(19) Europäisches Patentamt
European Patent Office
Office européen des brevets

(11) EP 4 180 427 A1

(12) **EUROPEAN PATENT APPLICATION**
published in accordance with Art. 153(4) EPC

(43) Date of publication:
**17.05.2023 Bulletin 2023/20**

(21) Application number: **21837764.6**

(22) Date of filing: **08.07.2021**

(51) International Patent Classification (IPC):
***C07D 401/14** (2006.01)* ***C07D 417/14** (2006.01)*
***C07D 471/04** (2006.01)* ***C07D 471/08** (2006.01)*
***C07D 471/10** (2006.01)* ***A61K 31/444** (2006.01)*
***A61K 31/496** (2006.01)* ***A61K 31/517** (2006.01)*
***A61P 35/00** (2006.01)*

(52) Cooperative Patent Classification (CPC):
**A61K 31/444; A61K 31/496; A61K 31/517; A61P 35/00; C07D 401/14; C07D 417/14; C07D 471/04; C07D 471/08; C07D 471/10**

(86) International application number:
**PCT/CN2021/105275**

(87) International publication number:
**WO 2022/007903 (13.01.2022 Gazette 2022/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.07.2020 CN 202010655931**
**07.08.2020 CN 202010776540**

(71) Applicant: **Sichuan Haisco Pharmaceutical Co., Ltd.**
**Sichuan Province 611130 (CN)**

(72) Inventors:
- **ZHANG, Chen**
**Chengdu City, Sichuan 611130 (CN)**
- **LIAO, Yuting**
**Chengdu City, Sichuan 611130 (CN)**
- **ZHU, Guozhi**
**Chengdu City, Sichuan 611130 (CN)**
- **YE, Fei**
**Chengdu City, Sichuan 611130 (CN)**
- **CHENG, Xinfan**
**Chengdu City, Sichuan 611130 (CN)**
- **CHEN, Xiaogang**
**Chengdu City, Sichuan 611130 (CN)**
- **TANG, Pingming**
**Chengdu City, Sichuan 611130 (CN)**
- **LI, Yao**
**Chengdu City, Sichuan 611130 (CN)**
- **NI, Jia**
**Chengdu City, Sichuan 611130 (CN)**
- **YAN, Pangke**
**Chengdu City, Sichuan 611130 (CN)**

(74) Representative: **Hoffmann Eitle**
**Patent- und Rechtsanwälte PartmbB**
**Arabellastraße 30**
**81925 München (DE)**

# (54) COMPOUND CAPABLE OF INHIBITING AND DEGRADING ANDROGEN RECEPTORS, AND PHARMACEUTICAL COMPOSITIONS AND PHARMACEUTICAL USES THEREOF

(57) A compound as shown in general formula B-L-K (I) or stereoisomers, deuterated compounds, solvates, prodrugs, metabolites, pharmaceutically acceptable salts or co-crystals thereof, and intermediates thereof and uses thereof for AR-related diseases such as prostate cancer.

EP 4 180 427 A1

## Description

### Technical Field

The present invention relates to a compound as shown in general formula (I) or a stereoisomer, a deuterated compound, a solvate, a prodrug, a metabolite, a pharmaceutically acceptable salt or a co-crystal thereof, and intermediates thereof and preparation methods therefor, as well as the use thereof in AR-related diseases such as prostate cancer.

### Background Art

Prostate cancer is mostly found in early stages, and its pathogenesis is often related to genetic factors, high-fat diet and endocrine. Generally, prostate cancer is more prevalent in developed countries than in developing countries. In 2016, there were 120,000 new prostate cancer patients in China, and by 2030, it is expected that the number of new patients will reach 237,000, and the market share will reach 4.8 billion US dollars. Patients with early-stage prostate cancer may be treated with radical therapy and have a longer survival time, while advanced patients with metastasis may be treated by a castration procedure combined with anti-androgen therapy and may develop castration-resistant prostate cancer. Clinical studies have shown that in most patients with resistant prostate cancer, androgen receptors (ARs) are overexpressed, and inhibition of androgen receptor (AR) signaling has significant efficacy in patients with hormonerefractory prostate cancer, so that inhibition of androgen receptors (ARs) is an effective means to directly block this pathway.

The androgen receptor (AR), a nuclear hormone receptor that is structurally comprised of an N-terminal domain (NTD), a DNA-binding domain (DBD) and a ligand-binding domain (LTD), can regulate the expression of genes that induce prostate cancer, and thus, inhibition of the androgen receptor is an effective method for treating prostate cancer. Currently marketed androgen receptor inhibitors such as enzalutamide and bicalutamide mainly exert an inhibitory effect by interacting with the ligand-binding domain (LTD) of the androgen receptor. However, some patients will develop drug resistance caused by androgen receptor splice variants (AR-Vs) with the deletion of LTD fragment during treatment. Preclinical studies have shown that androgen receptor splice variants can accelerate the progression of enzalutamide-resistant prostate cancer, and therefore, how to solve the problem of drug resistance has become the focus of clinical medicine.

PROTAC (proteolysis targeting chimera) molecules are a class of dual function compounds which are capable of binding to both targeted proteins and E3 ubiquitin ligases. Such compounds can be recognized by proteasomes in a cell to cause the degradation of the targeted proteins, which can effectively reduce the contents of the targeted proteins in the cell. By introducing a ligand capable of binding to various targeted proteins into the PROTAC molecules, it is possible to apply the PROTAC technology to the treatment of various diseases, and this technology has attracted extensive attention in recent years.

Therefore, it is necessary to develop a novel androgen receptor (AR) or/and AR splice variant inhibitor and a PROTAC drug of E3 ubiquitin ligase for the treatment of androgen receptor-related tumor diseases.

### Summary of the Invention

The present invention develops an AR or/and AR splice variant inhibitor with a novel structure, good efficacy, high bioavailability and higher safety, for use in the treatment of AR-related diseases such as prostate cancer.

The present invention provides a compound or a stereoisomer, a deuterated compound, a solvate, a prodrug, a metabolite, a pharmaceutically acceptable salt or a co-crystal thereof, wherein the compound is selected from a compound as shown in general formula (I),

B-L-K (I);

in certain embodiments, B is selected from

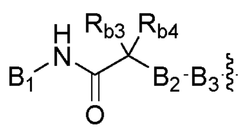

;

$B_1$ is selected from one of the following substituted or unsubstituted groups: 6-membered aryl or 6-membered heteroaryl, which, when substituted, is optionally further substituted with 0 to 4 $R^{b1}$, wherein the heteroaryl contains 1 to 4 heteroatoms selected from O, S and N;

$B_2$ is selected from one of the following substituted or unsubstituted groups: a 5- to 10-membered heterocyclic group or -NHC(=O)-, which, when substituted, is optionally further substituted with 0 to 4 selected from $R^{b2}$, wherein the heterocyclic group contains 1 to 4 heteroatoms selected from O, S and N;

$B_3$ is selected from substituted or unsubstituted 5- to 6-membered aryl or a bond, wherein the 5- to 6-membered aryl, when substituted, is optionally further substituted with 0 to 4 $R^{b2}$,

$R^{b1}$ and $R^{b2}$ are each independently selected from H, F, Cl, Br, I, OH, $NH_2$, CN, $CF_3$, $-C(=O)NH_2$, $-C(=O)NH-C_{1-4}$ alkyl, $-C(=O)N(C_{1-4}$ alkyl$)_2$, $C_{1-4}$ alkyl or $C_{1-4}$ alkoxy, wherein the alkyl or alkoxy is optionally further substituted with 0 to 4 substituents selected from H, F, Cl, Br, I or OH;

$R^{b3}$ and $R^{b4}$ are each independently selected from H or $C_{1-6}$ alkyl, wherein the alkyl is optionally further substituted with 0 to 4 substituents selected from H, F, Cl, Br, I, OH, $NH_2$, $C_{1-4}$ alkyl or $C_{1-4}$ alkoxy;

or $R^{b3}$ and $R^{b4}$ together with the carbon atoms to which they are attached form $C_{3-6}$ cycloalkyl or a $C_{3-6}$ mono-heterocyclic ring, wherein the cycloalkyl or mono-heterocyclic ring is optionally further substituted with 0 to 4 substituents selected from H, F, Cl, Br, I, OH, $NH_2$, $C_{1-4}$ alkyl or $C_{1-4}$ alkoxy, and the mono-heterocyclic ring contains 1 to 4 heteroatoms selected from O, S and N;

in certain embodiments, B is selected from

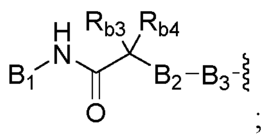

;

$B_1$ is selected from one of the following substituted or unsubstituted groups: 6-membered aryl or 6-membered heteroaryl, which, when substituted, is optionally further substituted with 0 to 4 $R^{b1}$, wherein the heteroaryl contains 1 to 4 heteroatoms selected from O, S and N;

$B_2$ is selected from one of the following substituted or unsubstituted groups: a 5- to 10-membered heterocyclic group or -NHC(=O)-, which, when substituted, is optionally further substituted with 0 to 4 selected from $R^{b2}$, wherein the heterocyclic group contains 1 to 4 heteroatoms selected from O, S and N;

$B_3$ is selected from substituted or unsubstituted phenyl or a bond, wherein the phenyl, when substituted, is optionally further substituted with 0 to 4 $R^{b2}$,

$R^{b1}$ and $R^{b2}$ are each independently selected from H, F, Cl, Br, I, OH, $NH_2$, CN, $CF_3$, $-C(=O)NH_2$, $-C(=O)NH-CH_3$, $-C(=O)N(CH_3)_2$, methyl, ethyl, propyl, isopropyl, methoxy or ethoxy, wherein the methyl, ethyl, propyl, isopropyl, methoxy or ethoxy is optionally further substituted with 0 to 4 substituents selected from H, F, Cl, Br, I or OH;

$R^{b3}$ and $R^{b4}$ are each independently selected from H or $C_{1-3}$ alkyl, wherein the alkyl is optionally further substituted with 0 to 4 substituents selected from H, F, Cl, Br, I, OH, $NH_2$, $C_{1-4}$ alkyl or $C_{1-4}$ alkoxy;

or $R^{b3}$ and $R^{b4}$ together with the carbon atoms to which they are attached form $C_{3-6}$ cycloalkyl or a $C_{3-6}$ mono-heterocyclic ring, wherein the cycloalkyl or mono-heterocyclic ring is optionally further substituted with 0 to 4 substituents selected from H, F, Cl, Br, I, OH, $NH_2$, $C_{1-4}$ alkyl or $C_{1-4}$ alkoxy, and the mono-heterocyclic ring contains 1 to 4 heteroatoms selected from O, S and N;

in certain embodiments, B is selected from

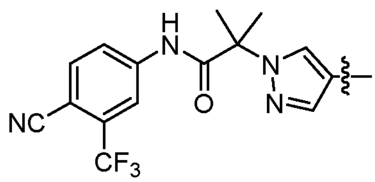

,

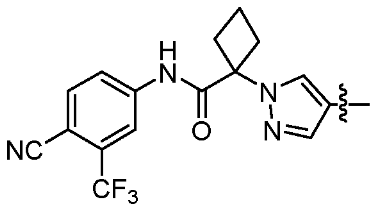,
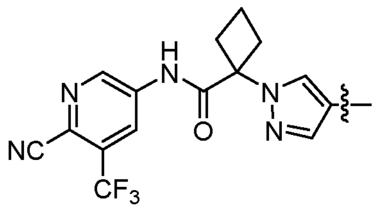,

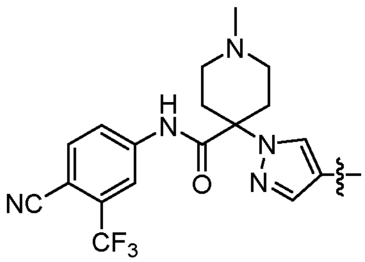,
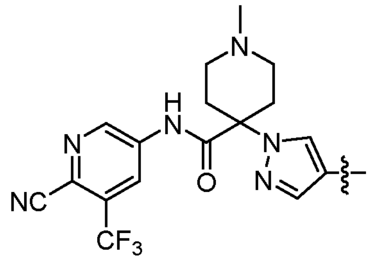,

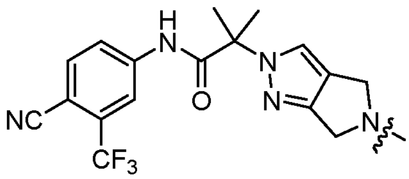, 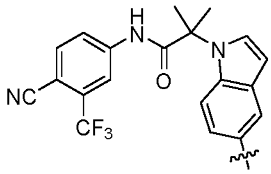, 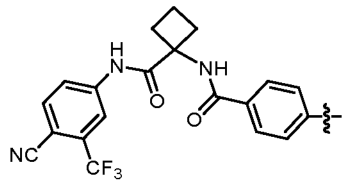,

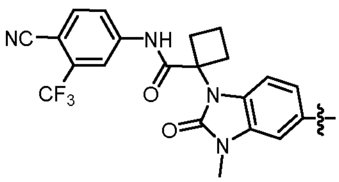,
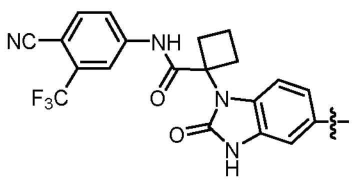,

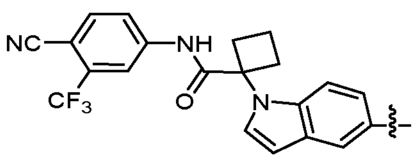 and 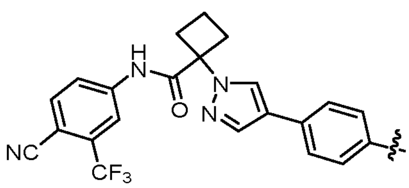;

in certain embodiments, L is selected from -Ak1-Cy1-Ak2-Cy2-Ak3-Cy3-Ak4-Cy4-Ak5-;

Ak1, Ak2, Ak3, Ak4 and Ak5 are each independently selected from $CH_2$, O, C≡C or a bond;

Cy1, Cy2, Cy3 and Cy4 are each independently selected from a bond, a 4- to 7-membered mono-heterocyclic ring, a 5- to 10-membered fused heterocyclic ring, a 6- to 12-membered spiro-heterocyclic ring, a 7- to 10-membered bridged-heterocyclic ring, 4-to 7-membered monocycloalkyl, 5- to 10-membered fused cycloalkyl, 6- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused heterocyclic ring, spiro-heterocyclic ring or bridged-heterocyclic ring is optionally further substituted with 0 to 4 substituents selected from H, F, Cl, Br, I, OH, COOH, CN, $NH_2$, oxo, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused heterocyclic ring, spiro-heterocyclic ring or bridged-heterocyclic ring contains 1 to 4 heteroatoms selected from O, S and N;

in certain embodiments, L is selected from -Ak1-Cy1-Ak2-Cy2-Ak3-Cy3-Ak4-Cy4-Ak5-;

Ak1, Ak2, Ak3, Ak4 and Ak5 are each independently selected from $CH_2$, O, C≡C or a bond;

Cy1, Cy2, Cy3 and Cy4 are each independently selected from a bond, a 4- to 7-membered nitrogen-containing mono-heterocyclic ring, a 5- to 10-membered nitrogen-containing fused heterocyclic ring, a 7- to 10-membered bridged-heterocyclic ring, or a 6- to 12-membered nitrogen-containing spiro-heterocyclic ring, wherein the mono-heterocyclic ring, fused heterocyclic ring, bridged-heterocyclic ring or spiro-heterocyclic ring is optionally further substituted with 0 to 4 substituents selected from H, F, Cl, Br, I, OH, COOH, CN, $NH_2$, oxo, $C_{1-4}$ alkyl, halogen-substituted $C_{1-4}$ alkyl, hydroxyl-substituted $C_{1-4}$ alkyl or $C_{1-4}$ alkoxy, and the mono-heterocyclic ring, fused heterocyclic ring, bridged-heterocyclic ring or spiro-heterocyclic ring contains 1 to 4 heteroatoms selected from O, S and N; and

in certain embodiments, L is selected from -Ak1-Cy1-Ak2-Cy2-Ak3-Cy3-Ak4-Cy4-Ak5-;

Ak1, Ak2, Ak3, Ak4 and Ak5 are each independently selected from $CH_2$, O, C≡C or a bond;

Cy1, Cy2, Cy3 and Cy4 are each independently selected from a bond or one of the following substituted or unsubstituted groups: azetidinyl, azacyclopentyl, azacyclohexyl, piperidine, morpholine, piperazine, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidine, cyclopentyl-spiro-piperidine, cyclohexyl-spiro-piperidine, azetidinyl-spiro-piperidine, azacyclopentyl-spiro-piperidine, azacyclohexyl-spiro-piperidine,

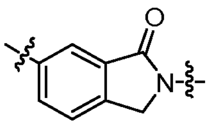,

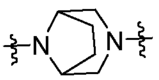, 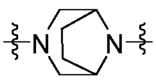, 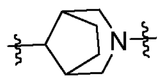, 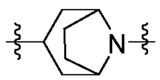, 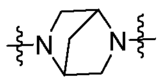, 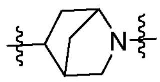,

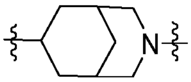, 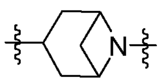, 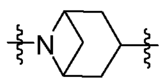, 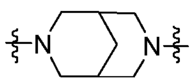, 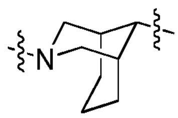,

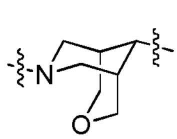 or 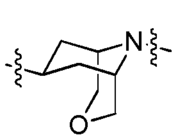,

which, when substituted, is optionally further substituted with 0 to 4 substituents selected from H, F, Cl, Br, I, OH, $NH_2$, COOH, CN, oxo, $C_{1-4}$ alkyl, halogen-substituted $C_{1-4}$ alkyl, hydroxyl-substituted $C_{1-4}$ alkyl or $C_{1-4}$ alkoxy;

in certain embodiments, L is selected from -Ak1-Cy1-Ak2-Cy2-Ak3-Cy3-Ak4-Cy4-Ak5-;

Ak1, Ak2, Ak3, Ak4 and Ak5 are each independently selected from $CH_2$, O, C≡C or a bond;

Cy1, Cy2, Cy3 and Cy4 are each independently selected from one of the following substituted or unsubstituted groups: a bond,

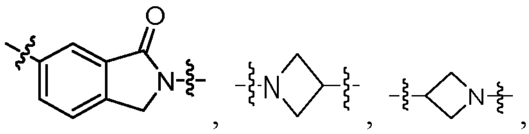

, , ,

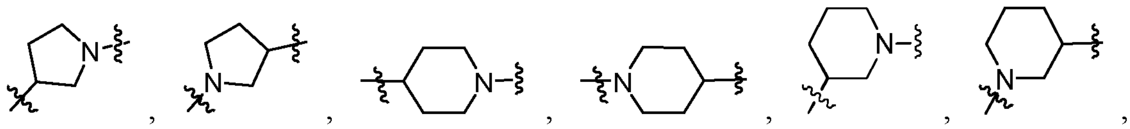

, , , , , ,

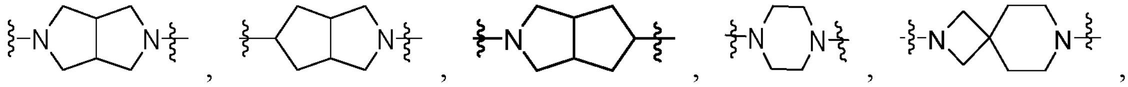

, , , , ,

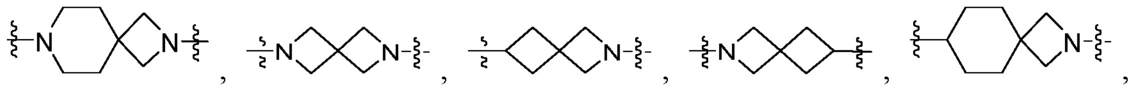

, , , , ,

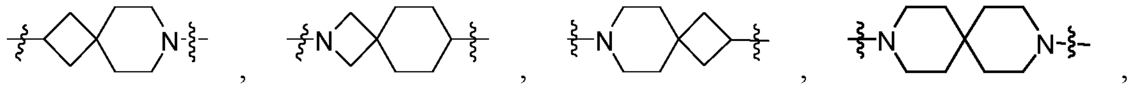

, , , ,

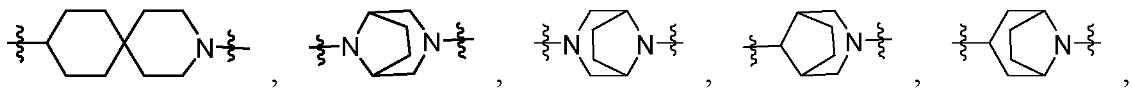

, , , , ,

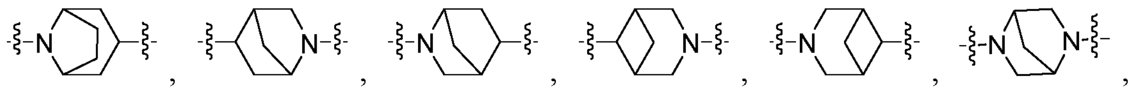

, , , , , ,

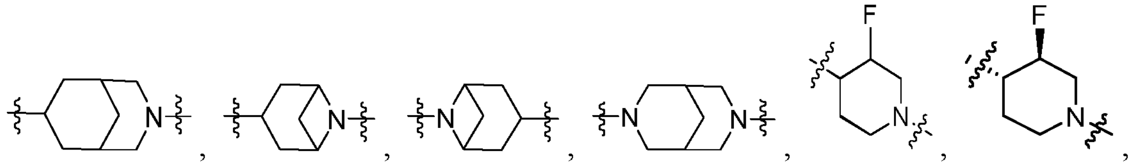

, , , , , ,

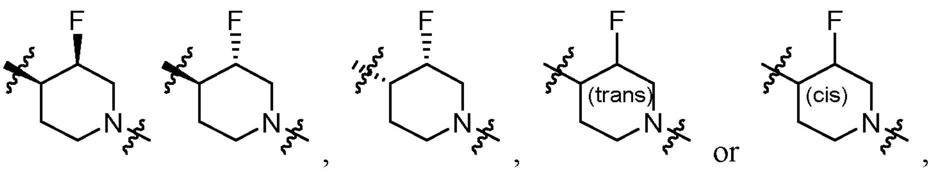

, , , or ,

which, when substituted, is optionally further substituted with 0 to 4 substituents selected from H, F, $CF_3$, methyl, oxo, hydroxymethyl, COOH, CN or $NH_2$;
in certain embodiments, L is selected from

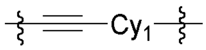

and

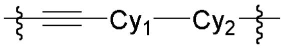

,

the left side of L is linked to B, and Cy1 and Cy2 are each independently selected from one of the following substituted or unsubstituted groups:

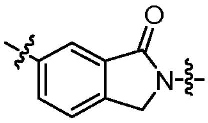, 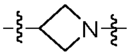, 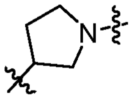, 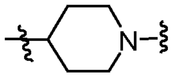, 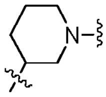, 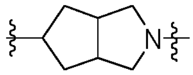,

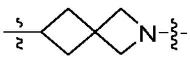, 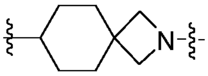, 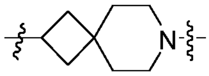, 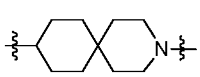, 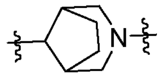,

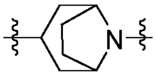, 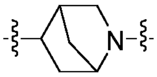, 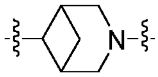, 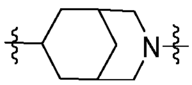, 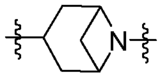, 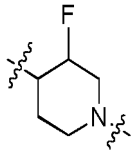,

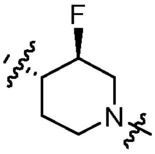, 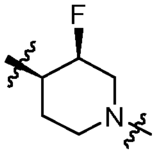 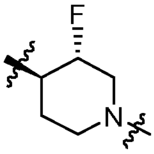, 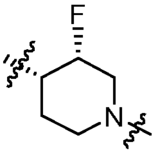, 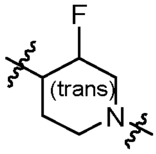

or 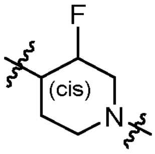

,

which, when substituted, is optionally further substituted with 0 to 4 substituents selected from H, F, $CF_3$, methyl, oxo, hydroxymethyl, COOH, CN or $NH_2$;
in certain embodiments, L is selected from

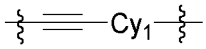

and

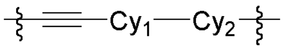,

the left side of L is linked to B, and Cy1 and Cy2 are each independently selected from one of the following substituted or unsubstituted groups:

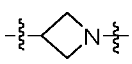, 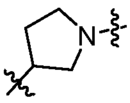, 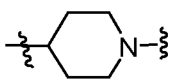, 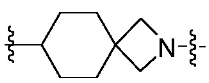 and 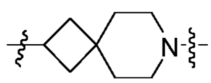,

which, when substituted, is optionally further substituted with 0 to 4 substituents selected from H, F, $CF_3$, methyl, oxo, hydroxymethyl, COOH, CN or $NH_2$;
in certain embodiments, L is selected from a bond,

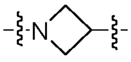, 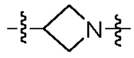,

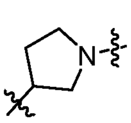, 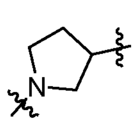, 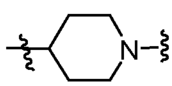, 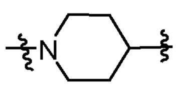, 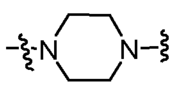, 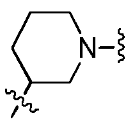,

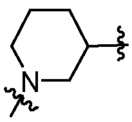, 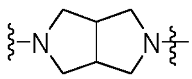, 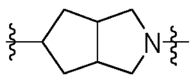, 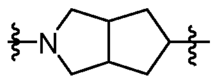, 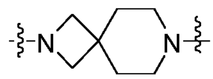,

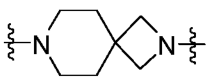, 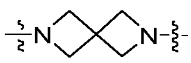, 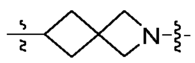, 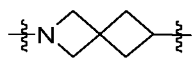, 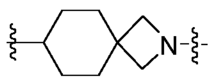,

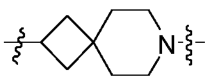, 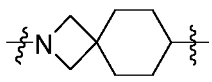, 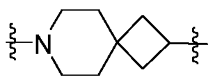, 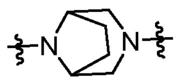, 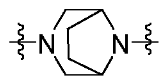,

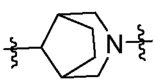, 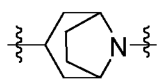, 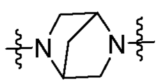, 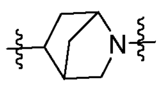, 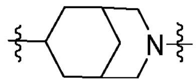, 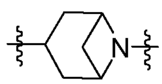,

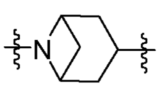, 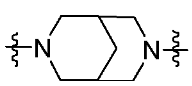, 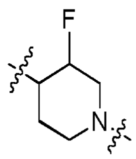, 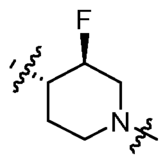, 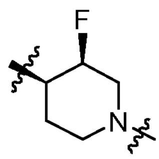, 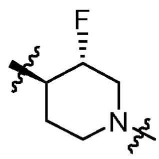,

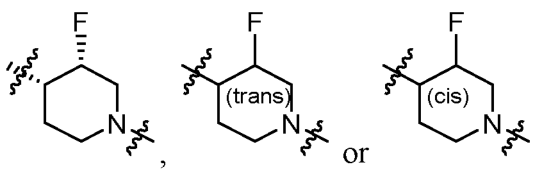
, or ,

wherein the left side of L is linked to B;
or L is selected from

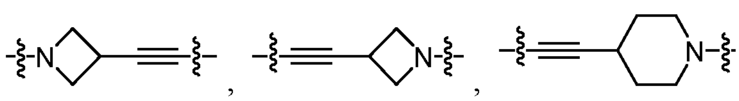
, , ,

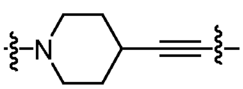, 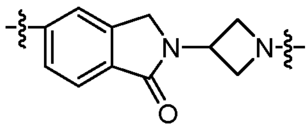, 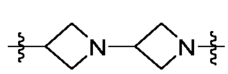, 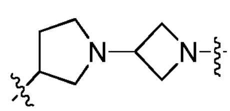,

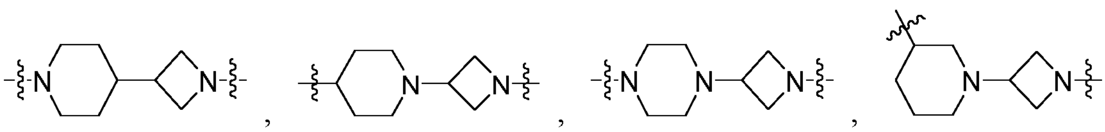
, , , ,

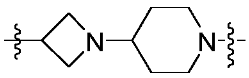, 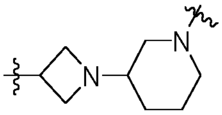, 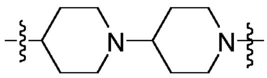, 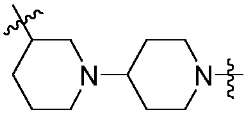,

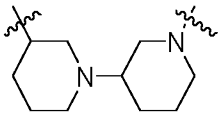, 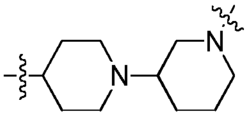, 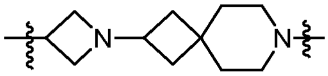,

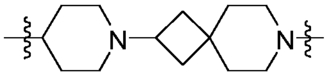, 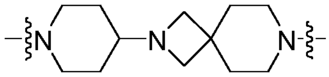, 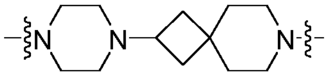,

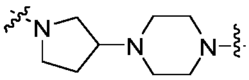, 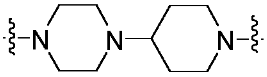, 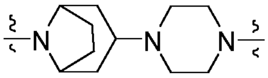, 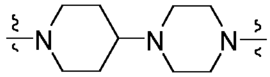,

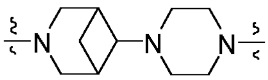, 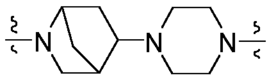, 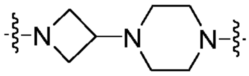,

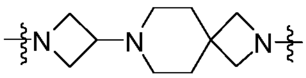, 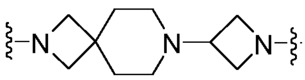, 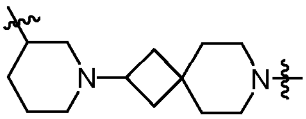,

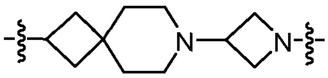, 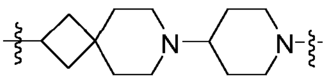, 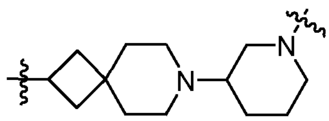,

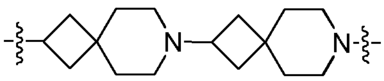, 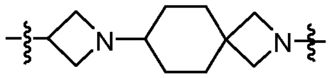, 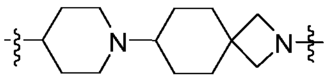,

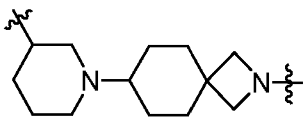, 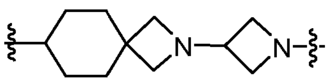, 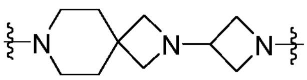,

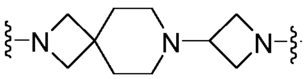, 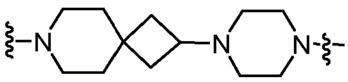, 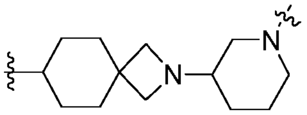,

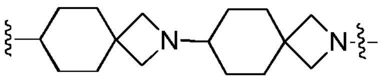, 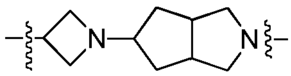, 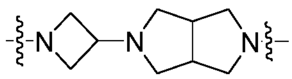,

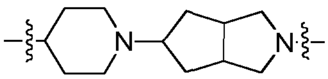 , 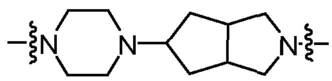 , 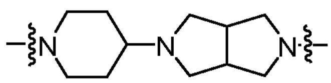 ,

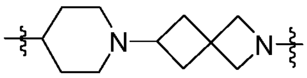 , 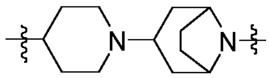 , 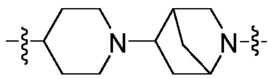 ,

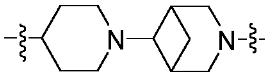 , 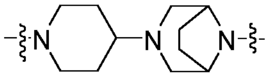 , 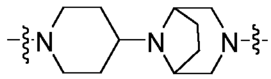 ,

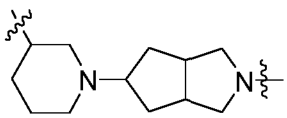 , 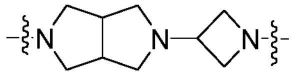 , 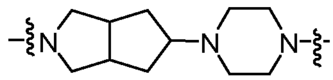 ,

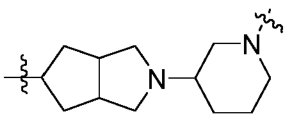 , 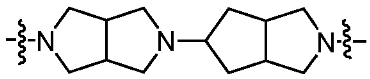 , 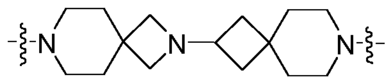 ,

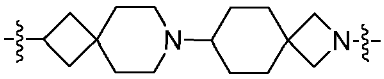 , 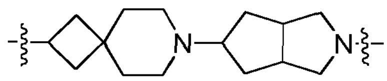 ,

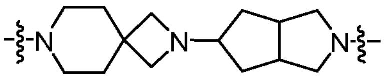 , 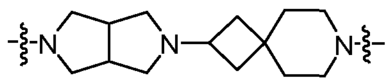 ,

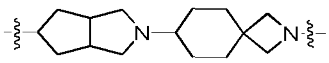 , 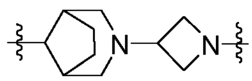 , 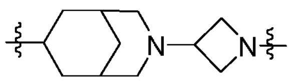 ,

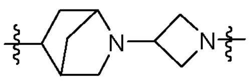 , 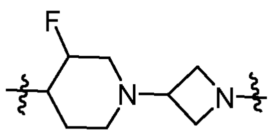 , 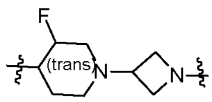 , 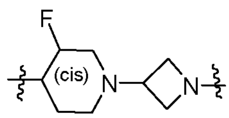 ,

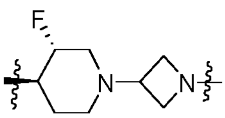 , 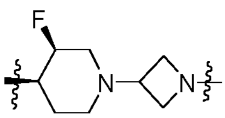 , 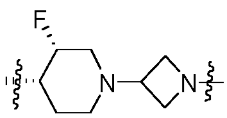 , 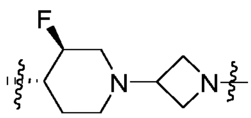 ,

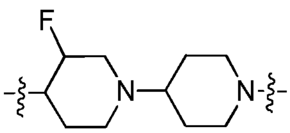 , 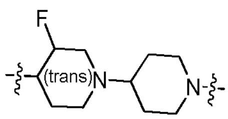 , 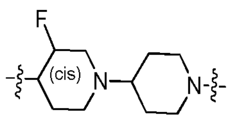 , 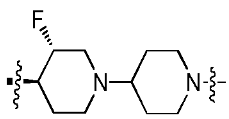 ,

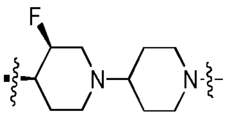 , 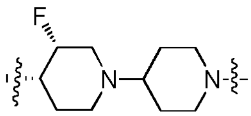 , 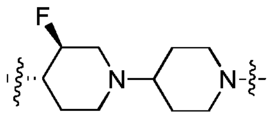 , 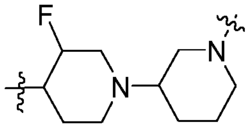 ,

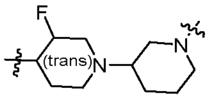 , 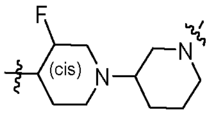 , 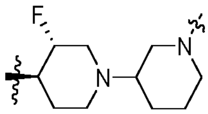 , 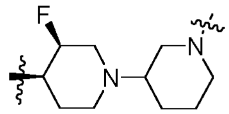 ,

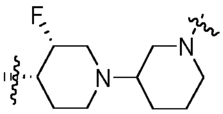 , 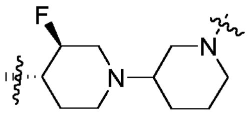 , 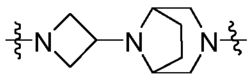 , 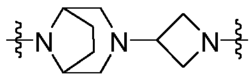 ,

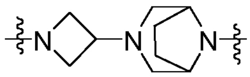 , 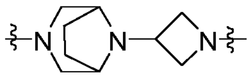 , 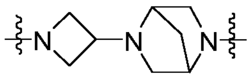 , 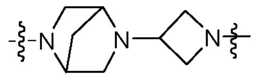

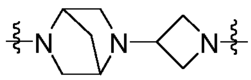 , 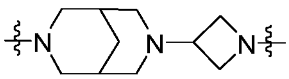 , 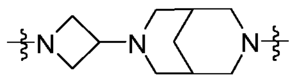

or

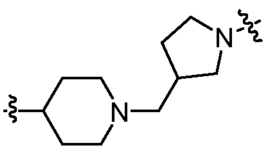 ,

wherein the left side of L is linked to B;
or L is selected from

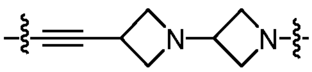 , 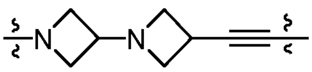 ,

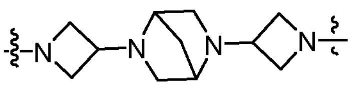 , 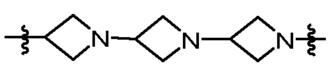 , 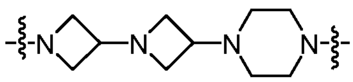 ,

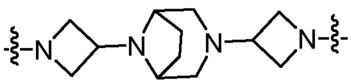 , 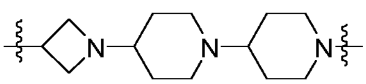 , 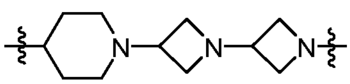 ,

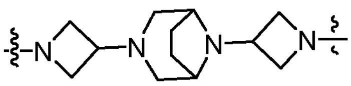 , 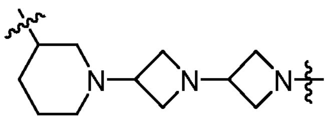 , 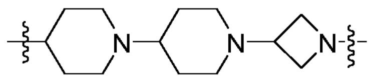 ,

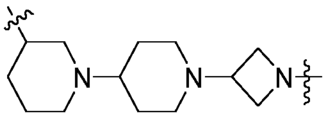, 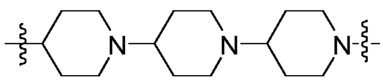, 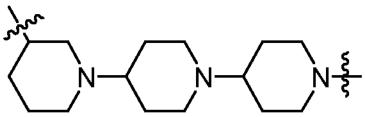,

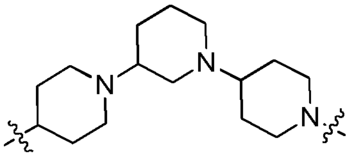, 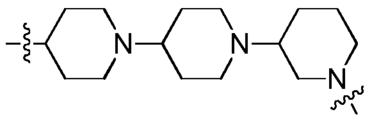, 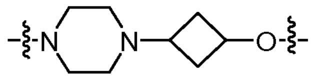,

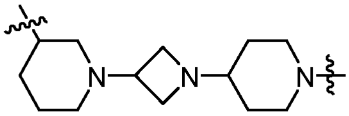, 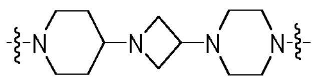, 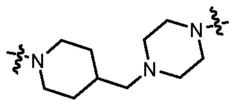

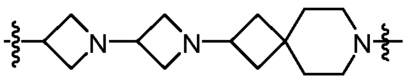, 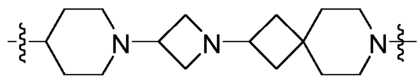,

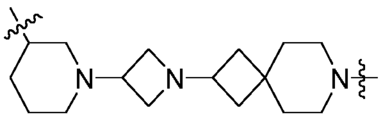, 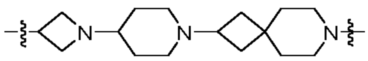,

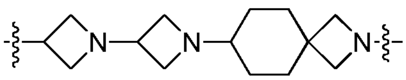, 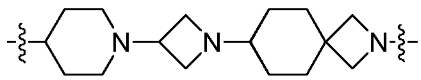,

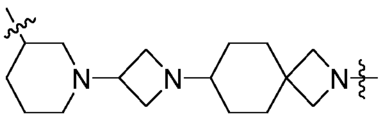, 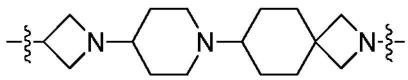,

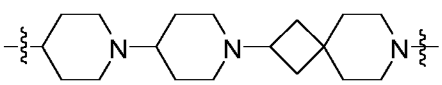, 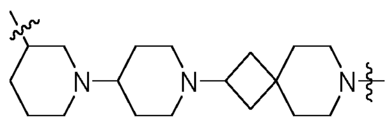,

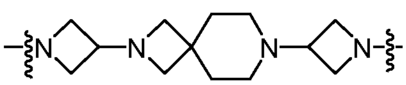, 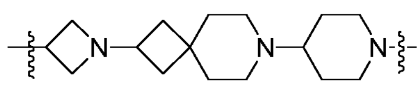,

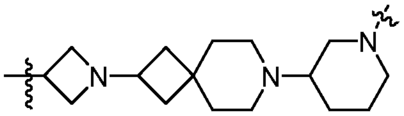, 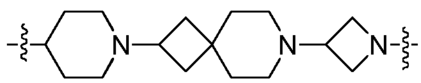,

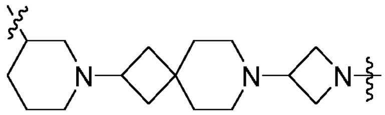,
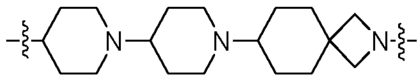,

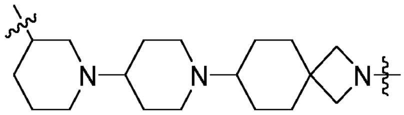,
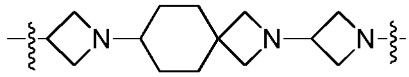,

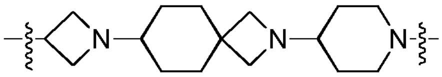,
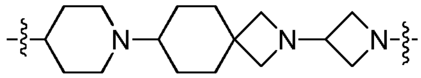,

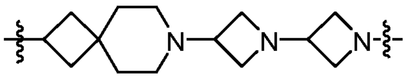,
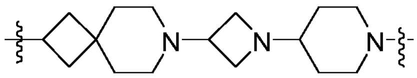,

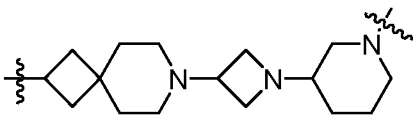,
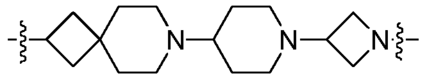,

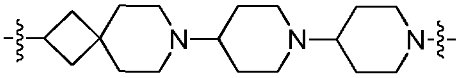,
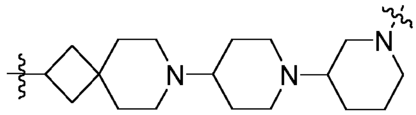.

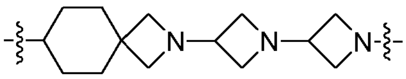,
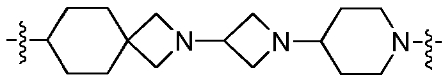,

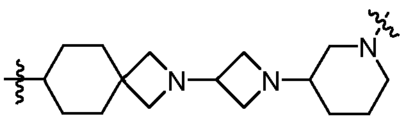,
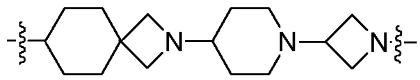,

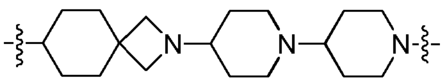,
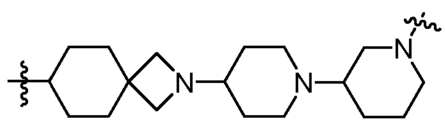,

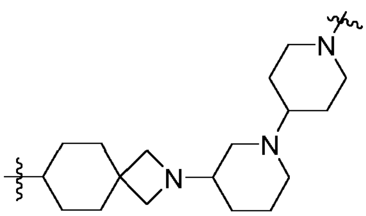,
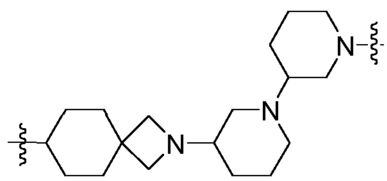,

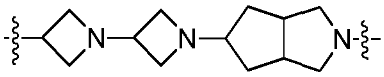 , 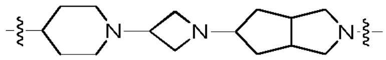 ,

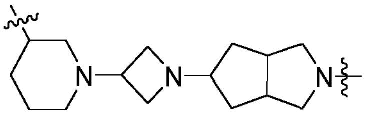 , 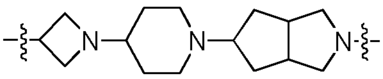 , 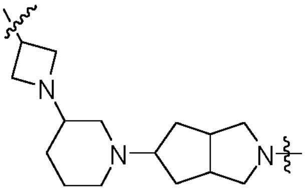 ,

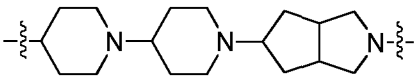 , 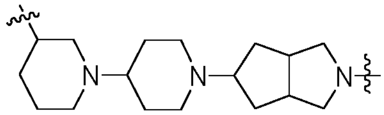 ,

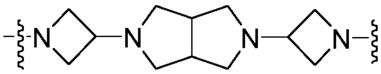 , 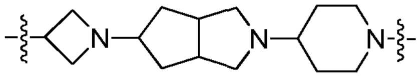 ,

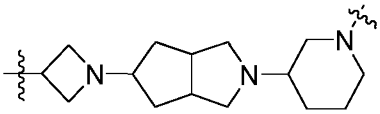 , 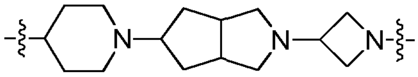 ,

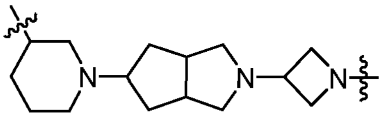 , 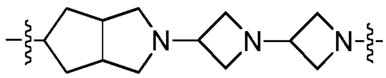 ,

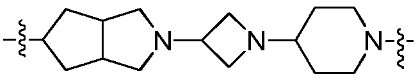 , 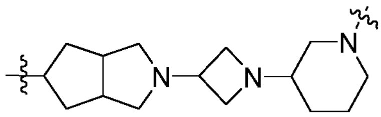 ,

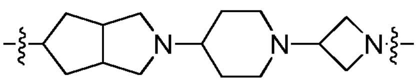 , 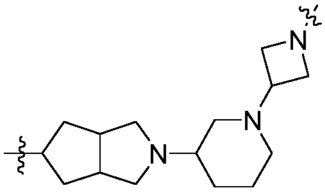 ,

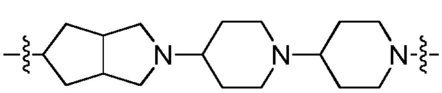 , 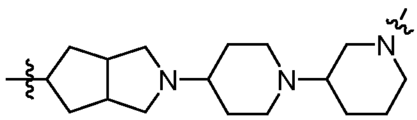 ,

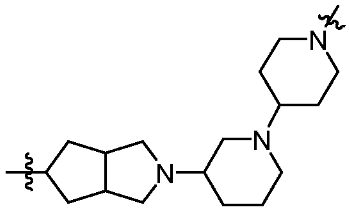
,
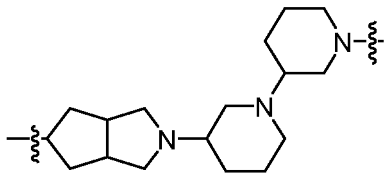
,
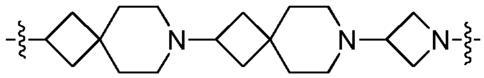
,
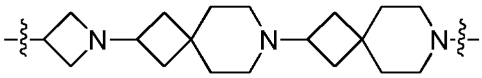
,
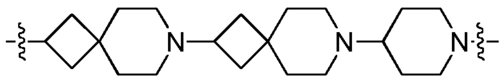
,
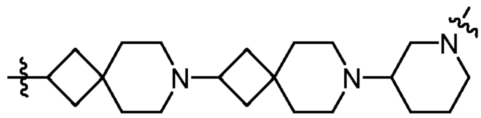
,
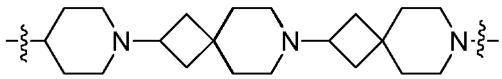
,
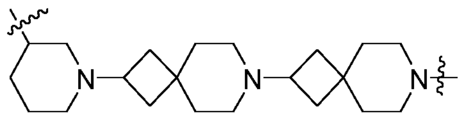
,
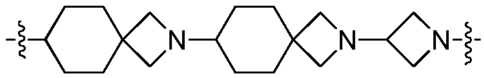
,
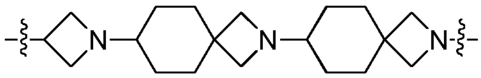
,
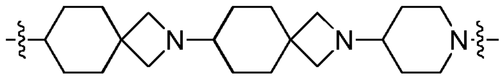
,
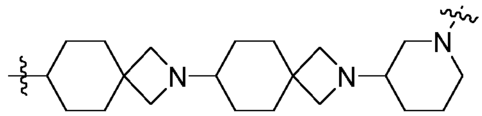
,
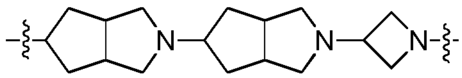
,
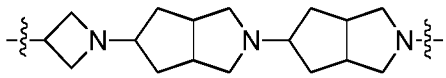
,
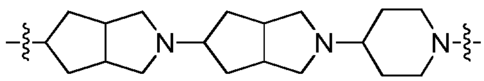
,
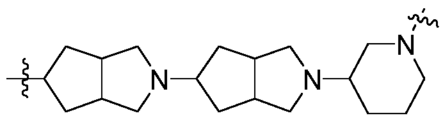
,
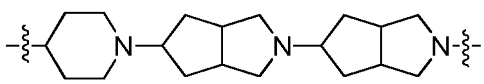
,
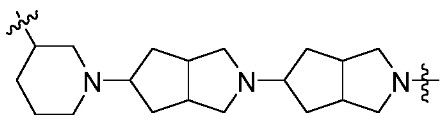
,
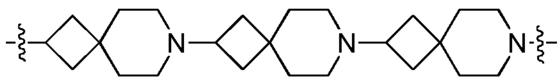
,
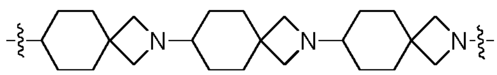
,
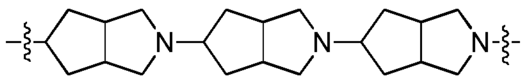
,
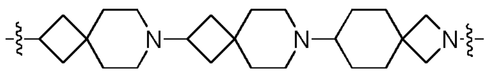
,

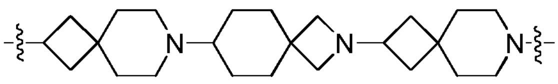 , 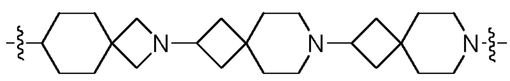 ,

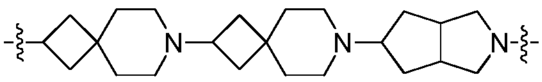 , 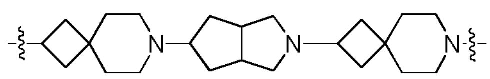 ,

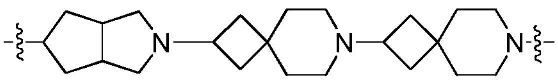 , 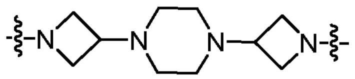 ,

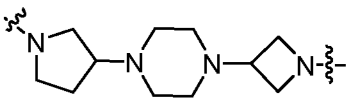 , 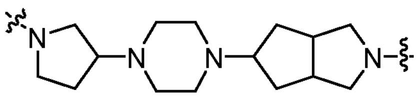 ,

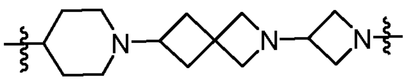 , 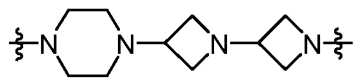 ,

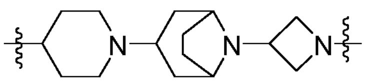 , 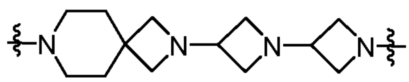 ,

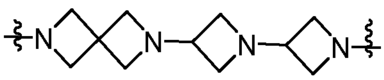 , 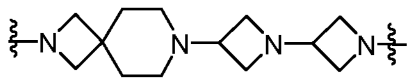 ,

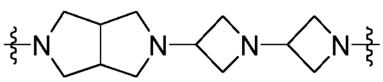 , 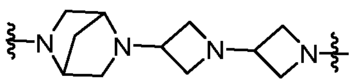 ,

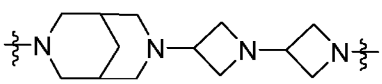 , 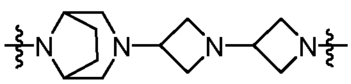 , 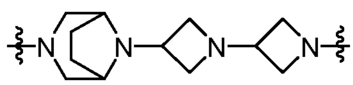 ,

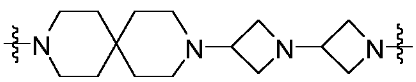 , 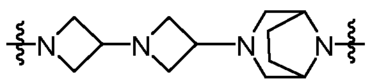 ,

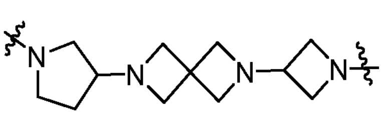 , 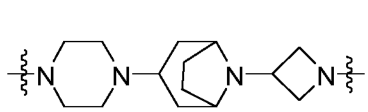 ,

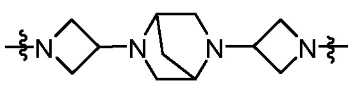 or 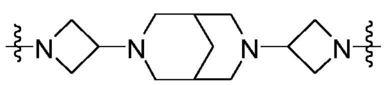 ,

wherein the left side of L is linked to B;
or L is selected from

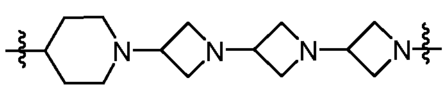 ,

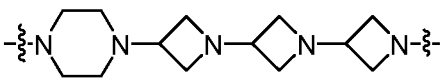,
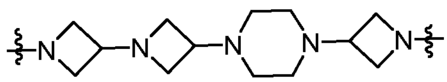,

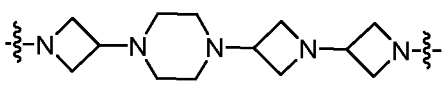 and 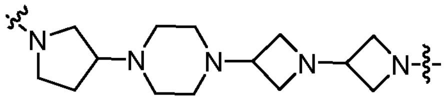,

wherein the left side of L is linked to B;
in certain embodiments, K is selected from

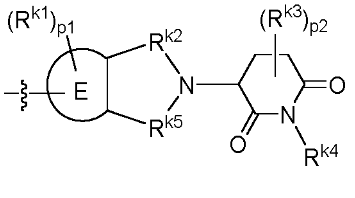,

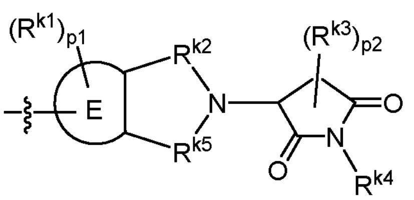, 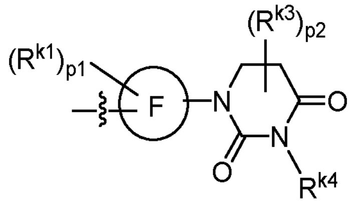, 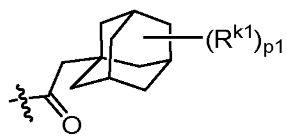,

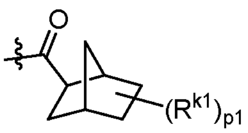, 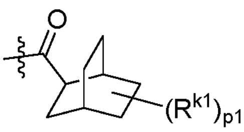, 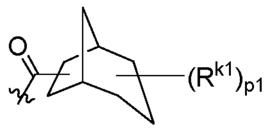, 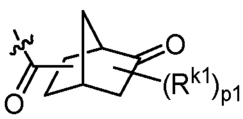,

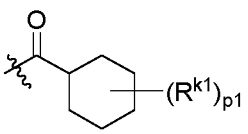, 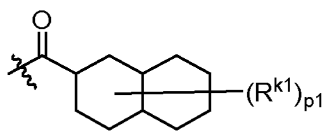, 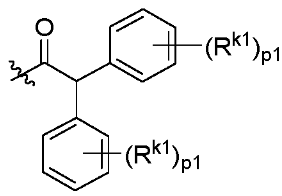,

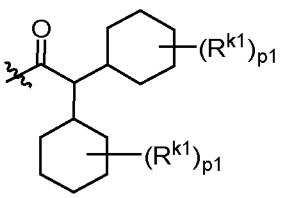, 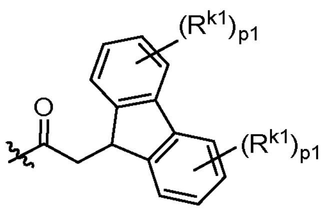, 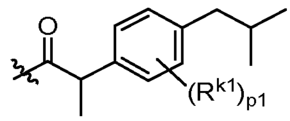,

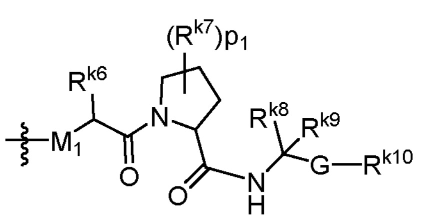, 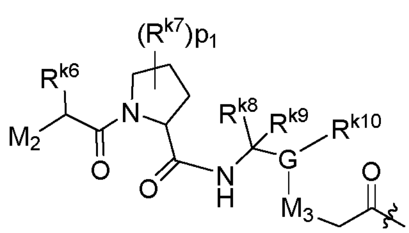

or

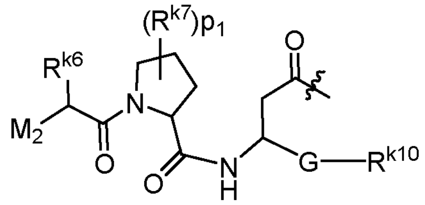

;

ring E or F is each independently selected from a phenyl ring or a 5- to 6-membered heteroaryl ring, wherein the heteroaryl ring contains 1 to 2 heteroatoms selected from O, S and N;
each $R^{k2}$ is independently selected from $CH_2$, C=O, S=O and $SO_2$;
$R^{k1}$, $R^{k3}$ or $R^{k4}$ is each independently selected from H, F, Cl, Br, I, OH, $NH_2$, $CF_3$, CN, COOH, $C_{1-4}$ alkyl or $C_{1-4}$ alkoxy;
$R^{k5}$ is selected from C=O or

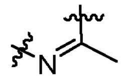

;

$M_1$ is selected from a bond, $-CH_2-C(=O)NH-$ or $-C(=O)CH_2NH-$;
$M_2$ is selected from $-NHC(=O)-C_{1-6}$ alkyl or $-NHC(=O)-C_{3-6}$ cycloalkyl, wherein the alkyl or cycloalkyl is optionally further substituted with 0 to 4 substituents selected from H, F, Cl, Br, I, OH, $NH_2$, $C_{1-4}$ alkyl or $C_{1-4}$ alkoxy;
$M_3$ is selected from -NH- or -O-;
$R^{k6}$ is selected from $C_{1-6}$ alkyl, wherein the alkyl is optionally further substituted with 0 to 4 substituents selected from H, F, Cl, Br, I, OH, $C_{1-6}$ alkyl or $C_{3-6}$ cycloalkyl;
each $R^{k7}$ is independently selected from H, F, Cl, Br, I, OH, SH, $C_{1-6}$ alkyl, $C_{1-6}$ alkoxy, $C_{1-6}$ alkylthio or $C_{1-6}$ alkylformyloxy, wherein the alkyl, alkoxy or alkylthio is optionally further substituted with 0 to 4 substituents selected from H, F, Cl, Br, I, OH, $C_{1-4}$ alkyl or $C_{1-4}$ alkoxy;
$R^{k8}$ and $R^{k9}$ are each independently selected from H, $C_{1-6}$ alkyl or $C_{3-6}$ cycloalkyl, wherein the alkyl or cycloalkyl is optionally further substituted with 0 to 4 substituents selected from H, F, Cl, Br, I, OH, $NH_2$, $C_{1-4}$ alkyl or $C_{1-4}$ alkoxy;
$R^{k10}$ is selected from 5- to 6-membered heteroaryl, wherein the heteroaryl is optionally further substituted with 0 to 4 substituents selected from H, F, Cl, Br, I, OH, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl;
G is selected from 6- to 10-membered aryl or 5- to 10-membered heteroaryl, wherein the aryl or heteroaryl is optionally further substituted with 0 to 4 substituents selected from H, F, Cl, Br, I, OH, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl; and
p1 or p2 is each independently selected from 0, 1, 2, 3 or 4;
in certain embodiments, K is selected from

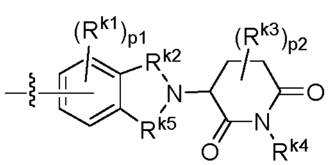

,

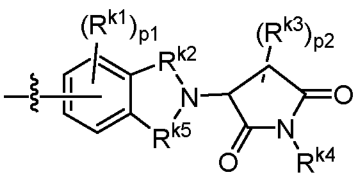

,

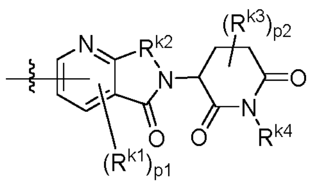

,

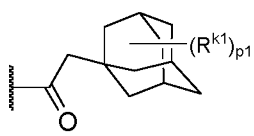

,

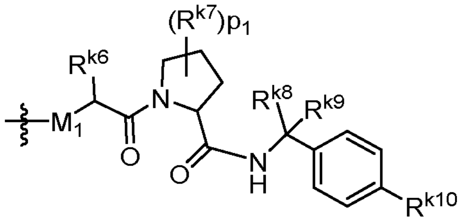
,
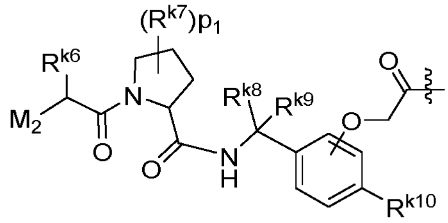

or

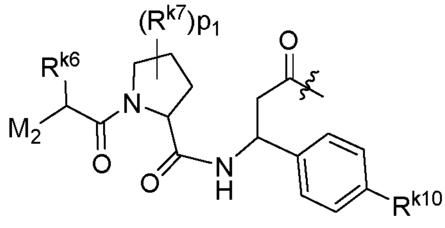
;

in certain embodiments, K is selected from

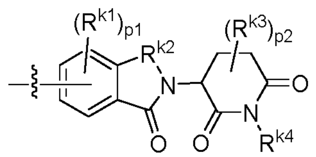
,

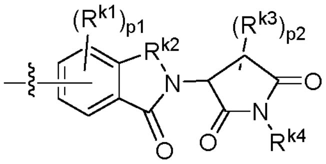
,
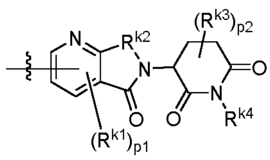
,
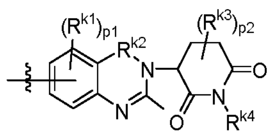
,
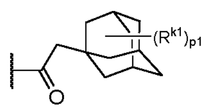
,

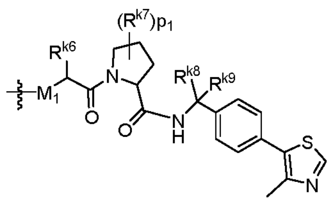
,
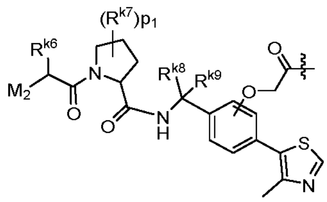
or
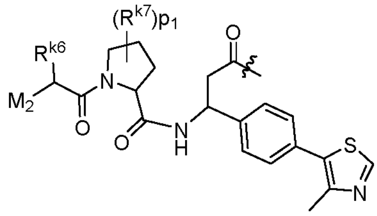
;

each $R^{k2}$ is independently selected from $CH_2$ or C=O;
$R^{k1}$, $R^{k3}$ or $R^{k4}$ is each independently selected from H, $CH_3$, F, Cl, Br, I, OH or $NH_2$;
$M_1$ is selected from a bond, $-CH_2-C(=O)NH-$ or $-C(=O)CH_2NH-$;
$M_2$ is selected from -NHC(=O)-methyl, -NHC(=O)-ethyl, -NHC(=O)-cyclopropyl, -NHC(=O)-cyclobutyl, -NHC(=O)-cyclopentyl or -NHC(=O)-cyclohexyl, wherein the methyl, ethyl, cyclopropyl, cyclobutyl, cyclopentyl or cyclohexyl is optionally further substituted with 0 to 4 substituents selected from H, F, Cl, Br, I, OH, $NH_2$, $C_{1-4}$ alkyl or $C_{1-4}$ alkoxy;
$R^{k6}$ is selected from methyl, ethyl, propyl, isopropyl, tert-butyl, isobutyl or sec-butyl;
each $R^{k7}$ is independently selected from H, F, OH, SH, methyl, methoxy or $-SCH_3$;
$R^{k8}$ and $R^{k9}$ are each independently selected from H, methyl, ethyl, cyclopropyl or cyclobutyl; and
p1 or p2 is each independently selected from 0, 1 or 2;
in certain embodiments, K is selected from

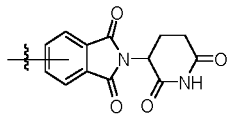,

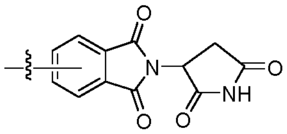, 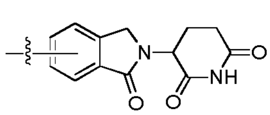, 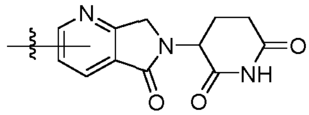,

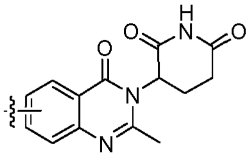, 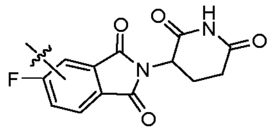, 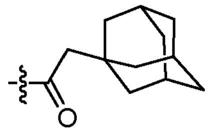,

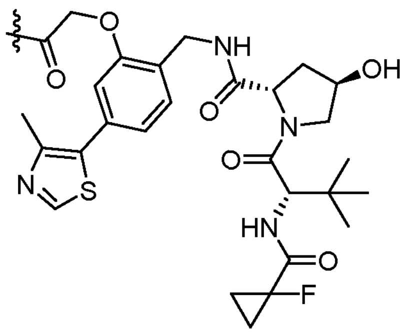, 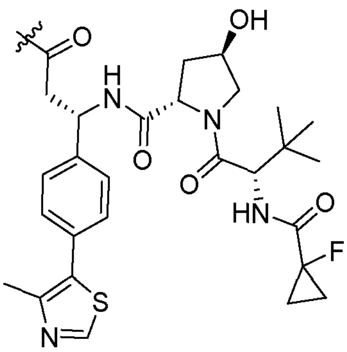,

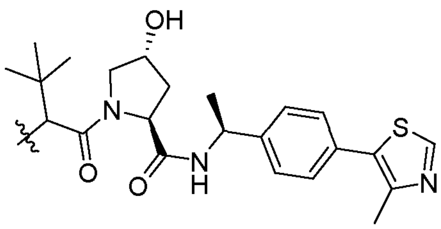, 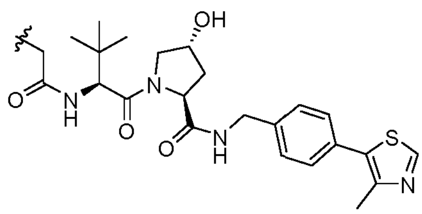,

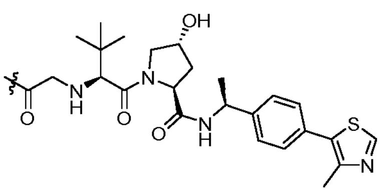, 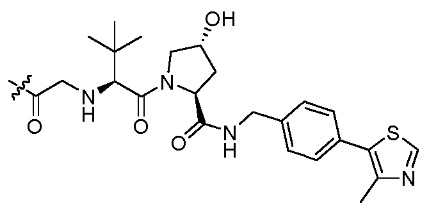,

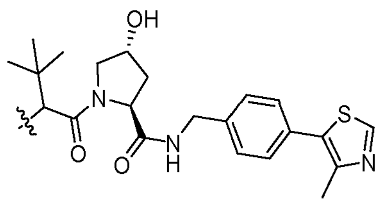 or 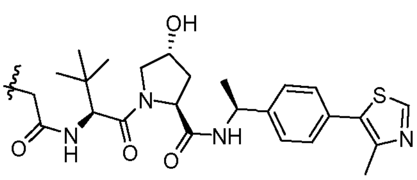;

in certain embodiments, K is selected from

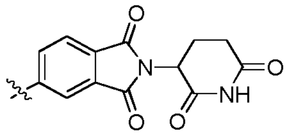,

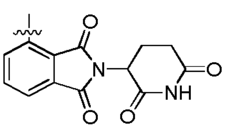, 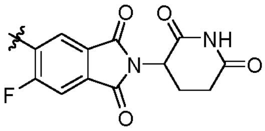, 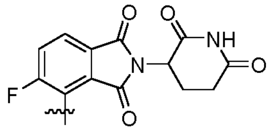, 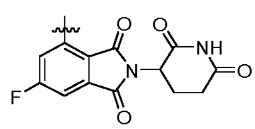,

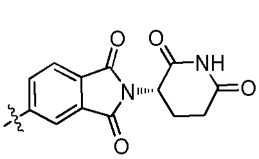, 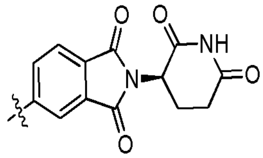 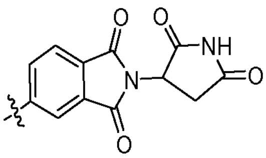, 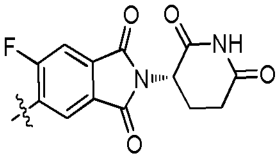,

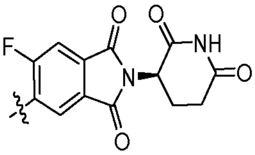, 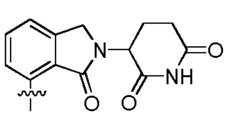, 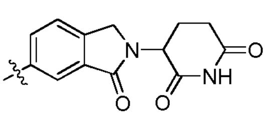, 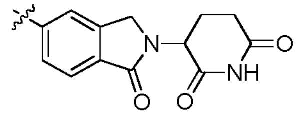,

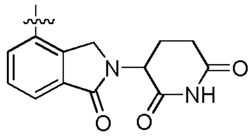, 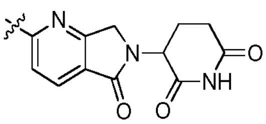, 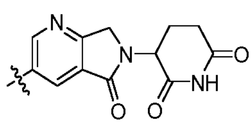, 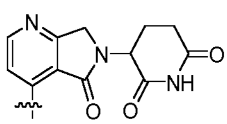,

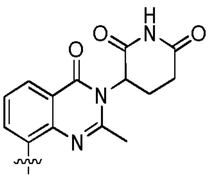, 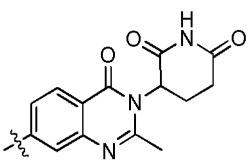, 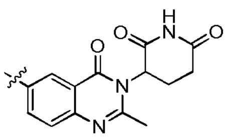, 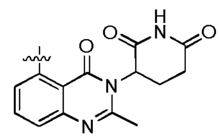,

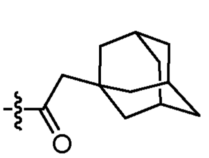, 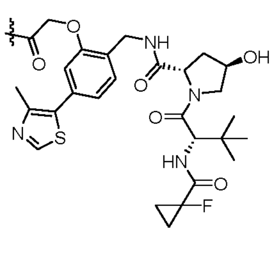, 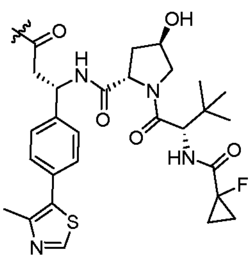,

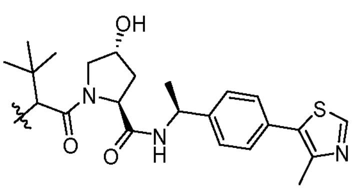, 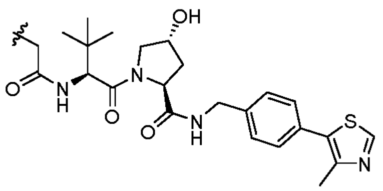,

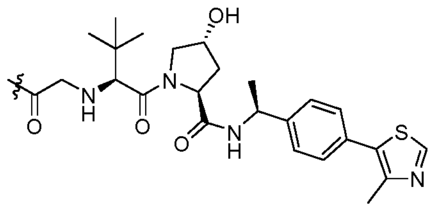 or 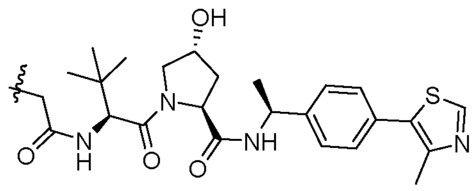 ; and

in certain embodiments, K is selected from

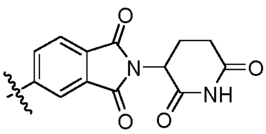 ,

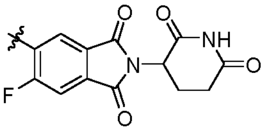 , 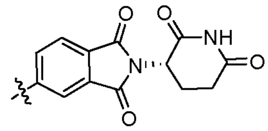 , 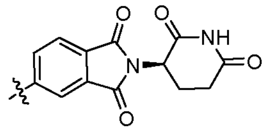 , 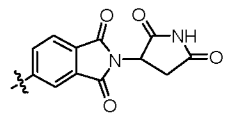 ,

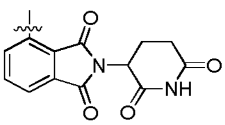 , 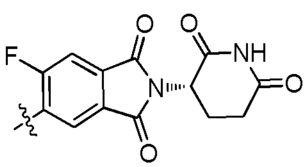 , 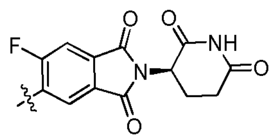 ,

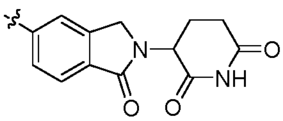 , 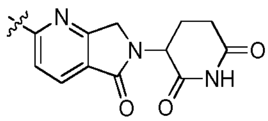 , 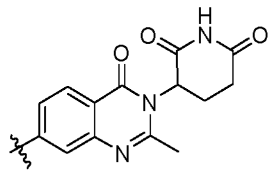 , 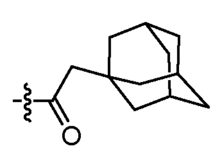 ,

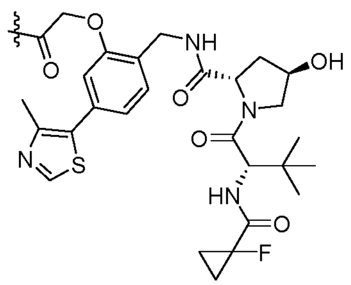 , 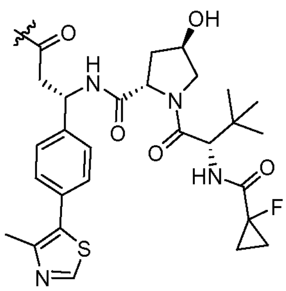 , 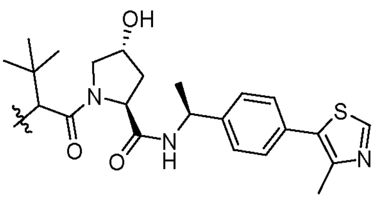 ,

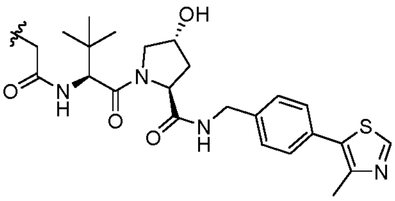 , 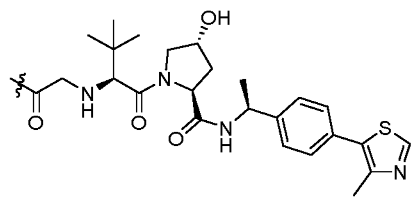 or

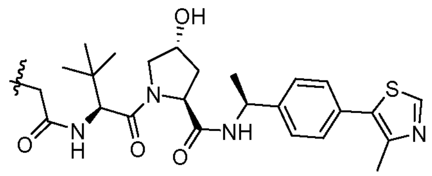.

As a first embodiment of the present invention, provided is a compound as shown in general formula (I) or a stereoisomer, a deuterated compound, a solvate, a prodrug, a metabolite, a pharmaceutically acceptable salt or a co-crystal thereof, wherein

B-L-K (I);

L is selected from -Ak1-Cy1-Ak2-Cy2-Ak3-Cy3-Ak4-Cy4-Ak5-;
Ak1, Ak2, Ak3, Ak4 and Ak5 are each independently selected from $CH_2$, O, C≡C or a bond;
Cy1, Cy2, Cy3 and Cy4 are each independently selected from a bond, a 4- to 7-membered mono-heterocyclic ring, a 5- to 10-membered fused heterocyclic ring, a 6- to 12-membered spiro-heterocyclic ring, a 7- to 10-membered bridged-heterocyclic ring, 4-to 7-membered monocycloalkyl, 5- to 10-membered fused cycloalkyl, 6- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused heterocyclic ring, spiro-heterocyclic ring or bridged-heterocyclic ring is optionally further substituted with 0 to 4 substituents selected from H, F, Cl, Br, I, OH, COOH, CN, $NH_2$, oxo, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused heterocyclic ring, spiro-heterocyclic ring or bridged-heterocyclic ring contains 1 to 4 heteroatoms selected from O, S and N;
B is selected from

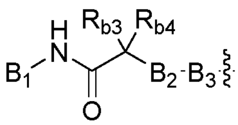;

$B_1$ is selected from one of the following substituted or unsubstituted groups: 6-membered aryl or 6-membered heteroaryl, which, when substituted, is optionally further substituted with 0 to 4 $R^{b1}$, wherein the heteroaryl contains 1 to 4 heteroatoms selected from O, S and N;
$B_2$ is selected from one of the following substituted or unsubstituted groups: a 5- to 10-membered heterocyclic group or -NHC(=O)-, which, when substituted, is optionally further substituted with 0 to 4 selected from $R^{b2}$, wherein the heterocyclic group contains 1 to 4 heteroatoms selected from O, S and N;
$B_3$ is selected from substituted or unsubstituted 5- to 6-membered aryl or a bond, wherein the 5- to 6-membered aryl, when substituted, is optionally further substituted with 0 to 4 $R^{b2}$,
$R^{b1}$ and $R^{b2}$ are each independently selected from H, F, Cl, Br, I, OH, $NH_2$, CN, $CF_3$, $-C(=O)NH_2$, $-C(=O)NH-C_{1-4}$ alkyl, $-C(=O)N(C_{1-4}$ alkyl$)_2$, $C_{1-4}$ alkyl or $C_{1-4}$ alkoxy, wherein the alkyl or alkoxy is optionally further substituted with 0 to 4 substituents selected from H, F, Cl, Br, I or OH;
$R^{b3}$ and $R^{b4}$ are each independently selected from H or $C_{1-6}$ alkyl, wherein the alkyl is optionally further substituted with 0 to 4 substituents selected from H, F, Cl, Br, I, OH, $NH_2$, $C_{1-4}$ alkyl or $C_{1-4}$ alkoxy;
or $R^{b3}$ and $R^{b4}$ together with the carbon atoms to which they are attached form $C_{3-6}$ cycloalkyl or a $C_{3-6}$ mono-heterocyclic ring, wherein the cycloalkyl or mono-heterocyclic ring is optionally further substituted with 0 to 4 substituents selected from H, F, Cl, Br, I, OH, $NH_2$, $C_{1-4}$ alkyl or $C_{1-4}$ alkoxy, and the mono-heterocyclic ring contains 1 to 4 heteroatoms selected from O, S and N;
K is selected from

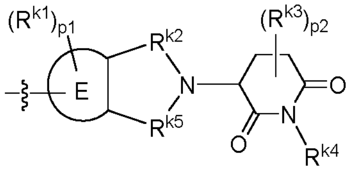, 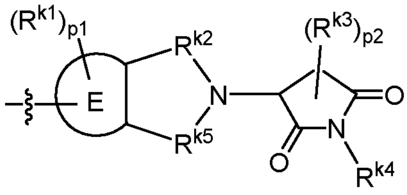,

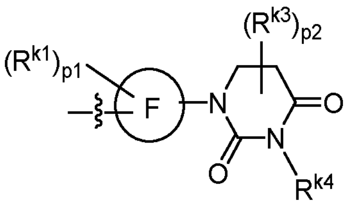, 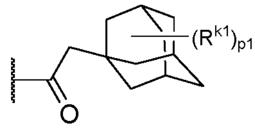, 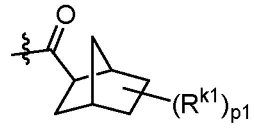,

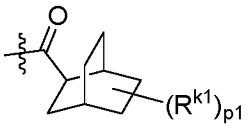, 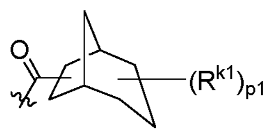, 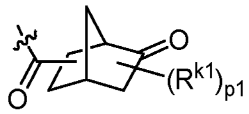, 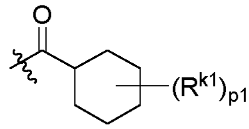,

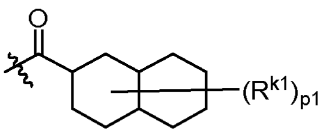, 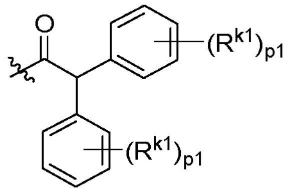, 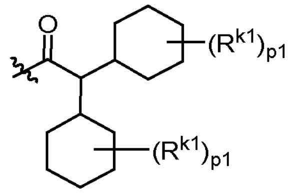,

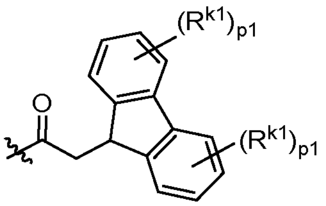, 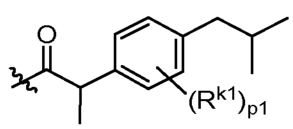, 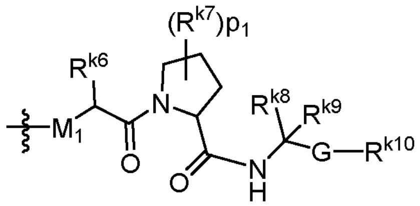,

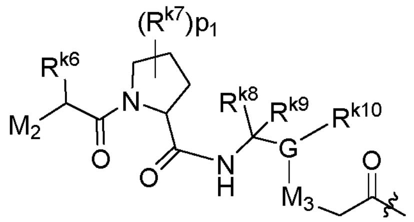 or 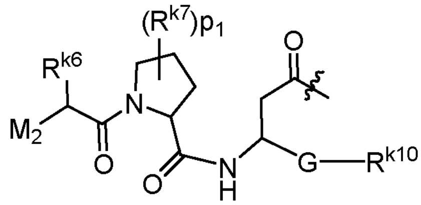;

ring E or F is each independently selected from a phenyl ring or a 5- to 6-membered heteroaryl ring, wherein the heteroaryl ring contains 1 to 2 heteroatoms selected from O, S and N;

each $R^{k2}$ is independently selected from $CH_2$, C=O, S=O and $SO_2$;

$R^{k1}$, $R^{k3}$ or $R^{k4}$ is each independently selected from H, F, Cl, Br, I, OH, $NH_2$, $CF_3$, CN, COOH, $C_{1-4}$ alkyl or $C_{1-4}$ alkoxy;

$R^{k5}$ is selected from C=O or

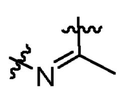;

$M_1$ is selected from a bond, $-CH_2-C(=O)NH-$ or $-C(=O)CH_2NH-$;

$M_2$ is selected from -NHC(=O)-$C_{1-6}$ alkyl or -NHC(=O)-$C_{3-6}$ cycloalkyl, wherein the alkyl or cycloalkyl is optionally further substituted with 0 to 4 substituents selected from H, F, Cl, Br, I, OH, $NH_2$, $C_{1-4}$ alkyl or $C_{1-4}$ alkoxy;
$M_3$ is selected from -NH- or -O-;
$R^{k6}$ is selected from $C_{1-6}$ alkyl, wherein the alkyl is optionally further substituted with 0 to 4 substituents selected from H, F, Cl, Br, I, OH, $C_{1-6}$ alkyl or $C_{3-6}$ cycloalkyl;
each $R^{k7}$ is independently selected from H, F, Cl, Br, I, OH, SH, $C_{1-6}$ alkyl, $C_{1-6}$ alkoxy, $C_{1-6}$ alkylthio or $C_{1-6}$ alkylformyloxy, wherein the alkyl, alkoxy or alkylthio is optionally further substituted with 0 to 4 substituents selected from H, F, Cl, Br, I, OH, $C_{1-4}$ alkyl or $C_{1-4}$ alkoxy;
$R^{k8}$ and $R^{k9}$ are each independently selected from H, $C_{1-6}$ alkyl or $C_{3-6}$ cycloalkyl, wherein the alkyl or cycloalkyl is optionally further substituted with 0 to 4 substituents selected from H, F, Cl, Br, I, OH, $NH_2$, $C_{1-4}$ alkyl or $C_{1-4}$ alkoxy;
$R^{k10}$ is selected from 5- to 6-membered heteroaryl, wherein the heteroaryl is optionally further substituted with 0 to 4 substituents selected from H, F, Cl, Br, I, OH, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl;
G is selected from 6- to 10-membered aryl or 5- to 10-membered heteroaryl, wherein the aryl or heteroaryl is optionally further substituted with 0 to 4 substituents selected from H, F, Cl, Br, I, OH, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl; and
p1 or p2 is each independently selected from 0, 1, 2, 3 or 4.

As a second embodiment of the present invention, provided is a compound as shown in general formula (I) or a stereoisomer, a deuterated compound, a solvate, a prodrug, a metabolite, a pharmaceutically acceptable salt or a co-crystal thereof, wherein

Cy1, Cy2, Cy3 and Cy4 are each independently selected from a bond, a 4- to 7-membered nitrogen-containing mono-heterocyclic ring, a 5- to 10-membered nitrogen-containing fused heterocyclic ring, a 7- to 10-membered bridged-heterocyclic ring, or a 6- to 12-membered nitrogen-containing spiro-heterocyclic ring, wherein the mono-heterocyclic ring, fused heterocyclic ring, bridged-heterocyclic ring or spiro-heterocyclic ring is optionally further substituted with 0 to 4 substituents selected from H, F, Cl, Br, I, OH, COOH, CN, $NH_2$, oxo, $C_{1-4}$ alkyl, halogen-substituted $C_{1-4}$ alkyl, hydroxyl-substituted $C_{1-4}$ alkyl or $C_{1-4}$ alkoxy, and the mono-heterocyclic ring, fused heterocyclic ring, bridged-heterocyclic ring or spiro-heterocyclic ring contains 1 to 4 heteroatoms selected from O, S and N; and

K is selected from

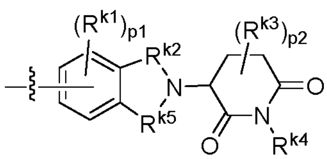 , 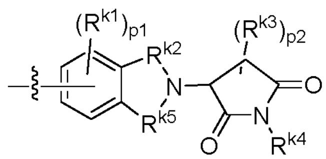 ,

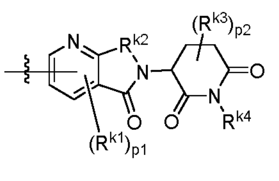 , 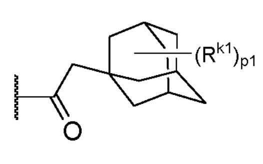 , 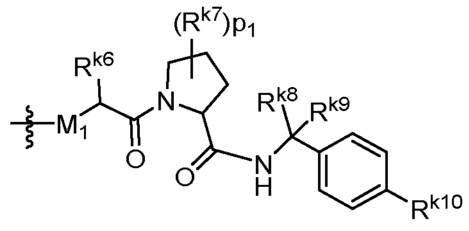 ,

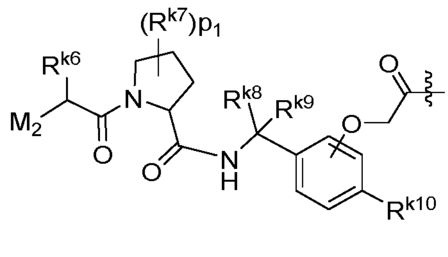 or 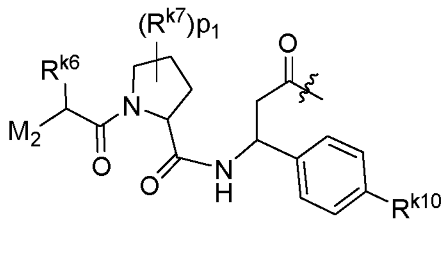 ;

other groups are as defined in the first embodiment.

As a third embodiment of the present invention, provided is a compound as shown in general formula (I) or a stereoisomer, a deuterated compound, a solvate, a prodrug, a metabolite, a pharmaceutically acceptable salt or a co-crystal thereof, wherein

Cy1, Cy2, Cy3 and Cy4 are each independently selected from a bond or one of the following substituted or unsubstituted groups: azetidinyl, azacyclopentyl, azacyclohexyl, piperidine, morpholine, piperazine, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidine, cyclopentyl-spiro-piperidine, cyclohexyl-spiro-piperidine, azetidinyl-spiro-piperidine, azacyclopentyl-spiro-piperidine, azacyclohexyl-spiro-piperidine,

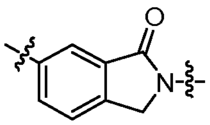,

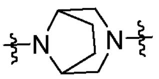, 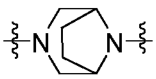, 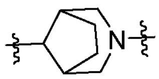, 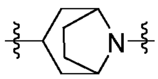, 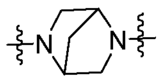, 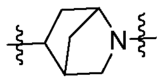,

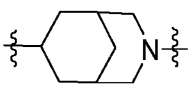, 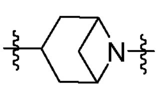, 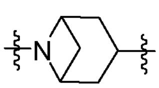, 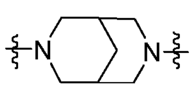, 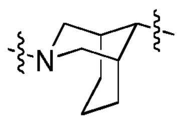,

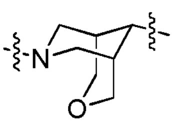 or 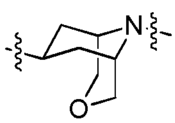,

which, when substituted, is optionally further substituted with 0 to 4 substituents selected from H, F, Cl, Br, I, OH, $NH_2$, COOH, CN, oxo, $C_{1-4}$ alkyl, halogen-substituted $C_{1-4}$ alkyl, hydroxyl-substituted $C_{1-4}$ alkyl or $C_{1-4}$ alkoxy;

$R^{b1}$ and $R^{b2}$ are each independently selected from H, F, Cl, Br, I, OH, $NH_2$, CN, $CF_3$, $-C(=O)NH_2$, $-C(=O)NH-CH_3$, $-C(=O)N(CH_3)_2$, methyl, ethyl, propyl, isopropyl, methoxy or ethoxy, wherein the methyl, ethyl, propyl, isopropyl, methoxy or ethoxy is optionally further substituted with 0 to 4 substituents selected from H, F, Cl, Br, I or OH;

K is selected from

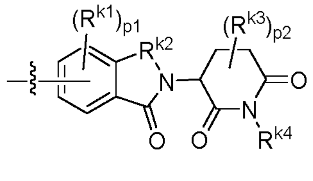 , 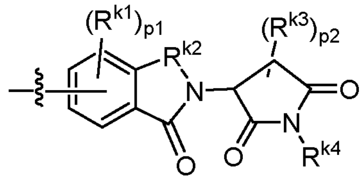 ,

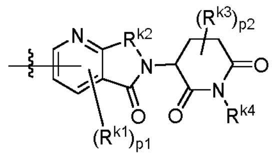 , 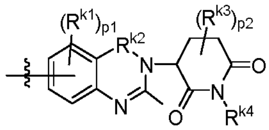 , 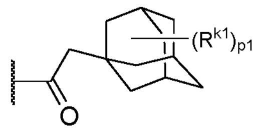 ,

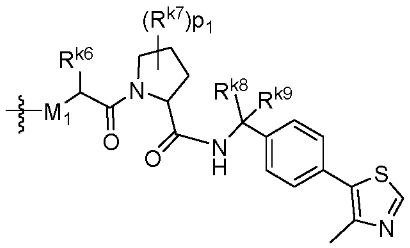 , 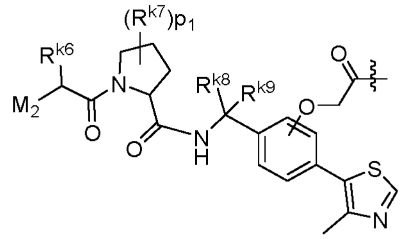

or

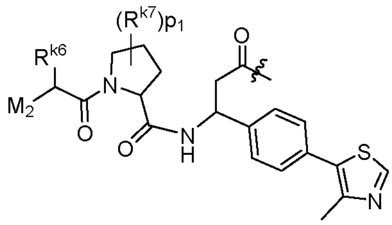 ;

each $R^{k2}$ is independently selected from $CH_2$ or C=O;
$R^{k1}$, $R^{k3}$ or $R^{k4}$ is each independently selected from H, $CH_3$, F, Cl, Br, I, OH or $NH_2$;
$M_1$ is selected from a bond, $-CH_2-C(=O)NH-$ or $-C(=O)CH_2NH-$;
$M_2$ is selected from -NHC(=O)-methyl, -NHC(=O)-ethyl, -NHC(=O)-cyclopropyl, -NHC(=O)-cyclobutyl, -NHC(=O)-cyclopentyl or -NHC(=O)-cyclohexyl, wherein the methyl, ethyl, cyclopropyl, cyclobutyl, cyclopentyl or cyclohexyl is optionally further substituted with 0 to 4 substituents selected from H, F, Cl, Br, I, OH, $NH_2$, $C_{1-4}$ alkyl or $C_{1-4}$ alkoxy;
$R^{k6}$ is selected from methyl, ethyl, propyl, isopropyl, tert-butyl, isobutyl or sec-butyl;
each $R^{k7}$ is independently selected from H, F, OH, SH, methyl, methoxy or $-SCH_3$;
$R^{k8}$ and $R^{k9}$ are each independently selected from H, methyl, ethyl, cyclopropyl or cyclobutyl; and
p1 or p2 is each independently selected from 0, 1 or 2;
other groups are as defined in the second embodiment.

As a fourth embodiment of the present invention, provided is a compound as shown in general formula (I) or a stereoisomer, a deuterated compound, a solvate, a prodrug, a metabolite, a pharmaceutically acceptable salt or a co-crystal thereof, wherein

Cy1, Cy2, Cy3 and Cy4 are each independently selected from one of the following substituted or unsubstituted groups: a bond,

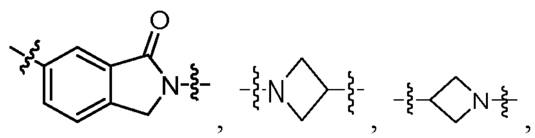

, , ,

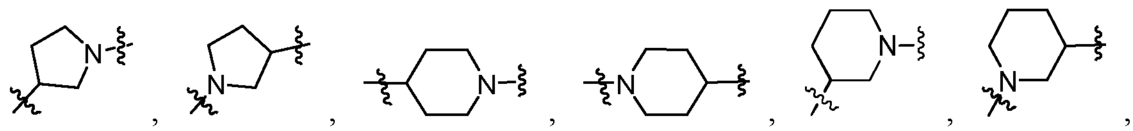

, , , , , ,

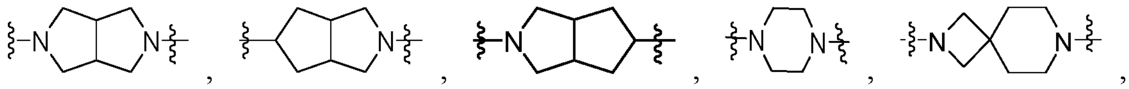

, , , , ,

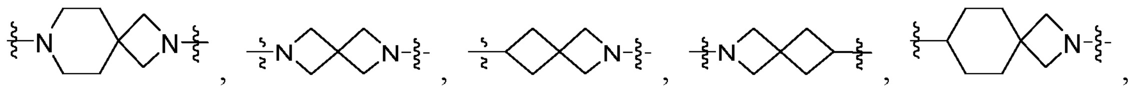

, , , , ,

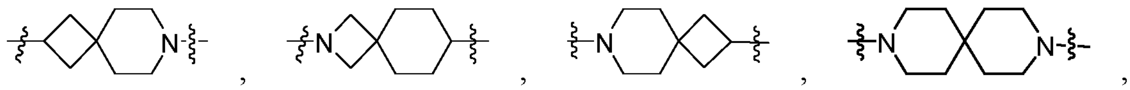

, , , ,

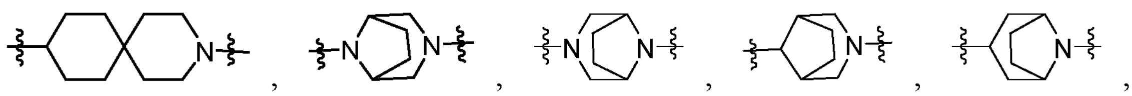

, , , , ,

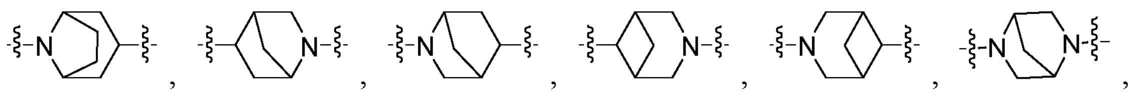

, , , , , ,

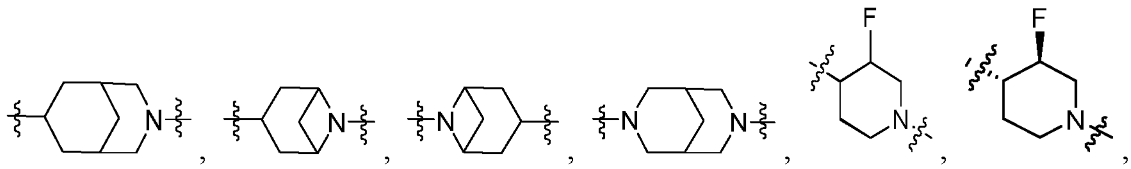

, , , , , ,

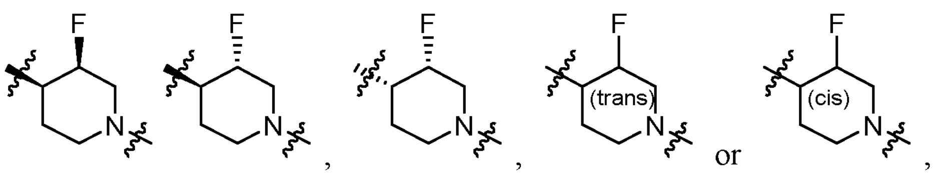

, , or ,

which, when substituted, is optionally further substituted with 0 to 4 substituents selected from H, F, $CF_3$, methyl, oxo, hydroxymethyl, COOH, CN or $NH_2$;

B is selected from

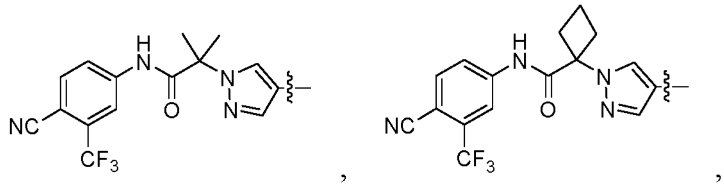

, ,

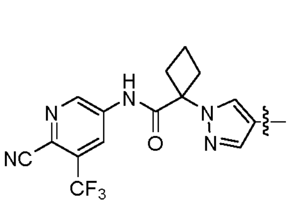,
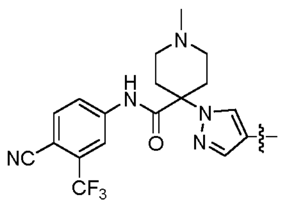,
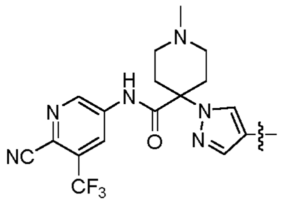,

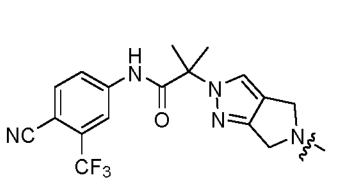,
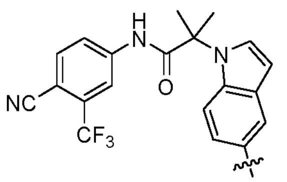,
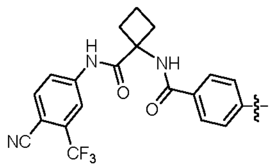,

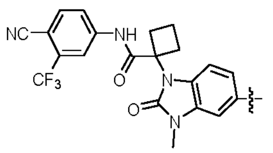,
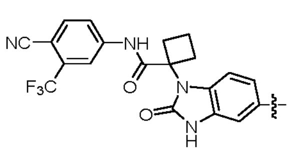,
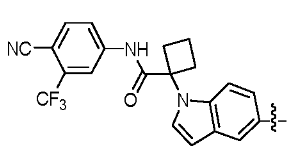

and

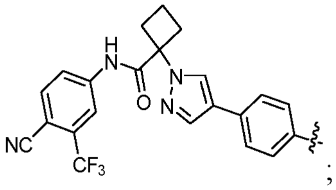;

K is selected from

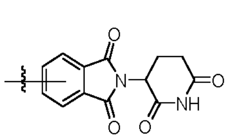,
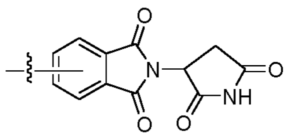
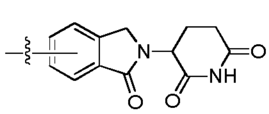,

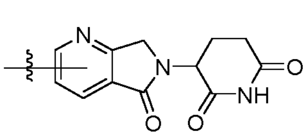,
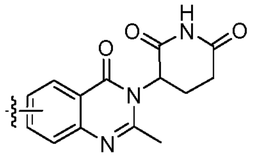,
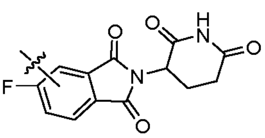,
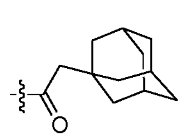,

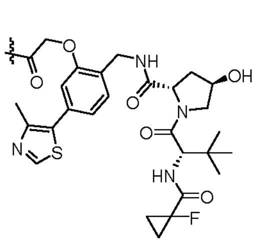,
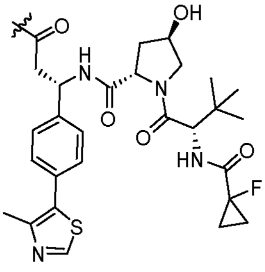,
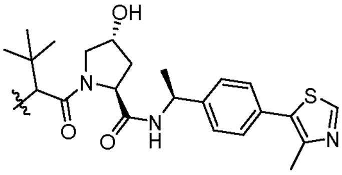,

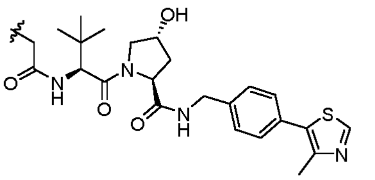, 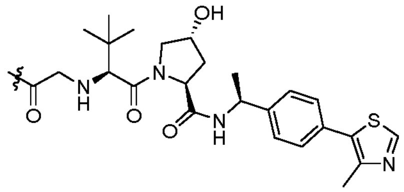,

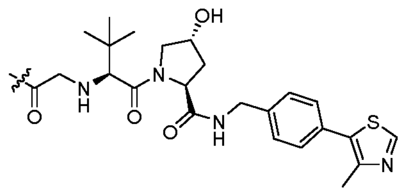, 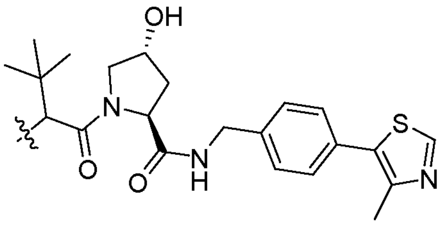

or

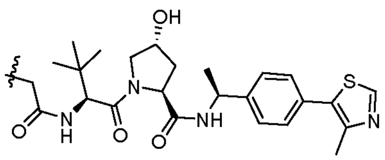;

other groups are as defined in the third embodiment.

As a fifth embodiment of the present invention, provided is a compound as shown in general formula (I) or a stereoisomer, a deuterated compound, a solvate, a prodrug, a metabolite, a pharmaceutically acceptable salt or a co-crystal thereof, wherein

L is selected from a bond,

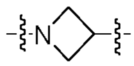, 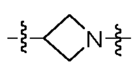, 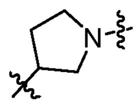, 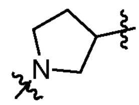,

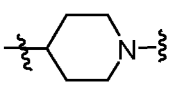, 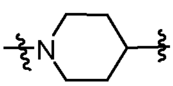, 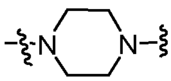, 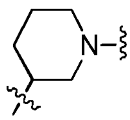, 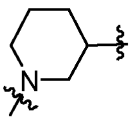, 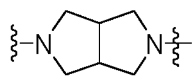,

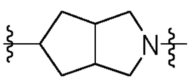, 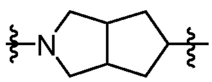, 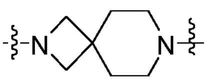, 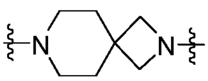, 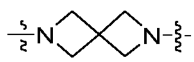,

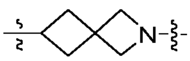, 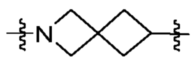, 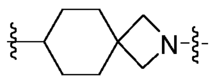, 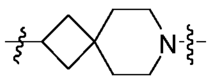, 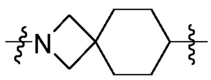,

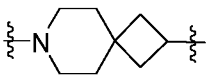, 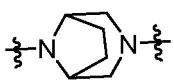, 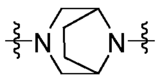, 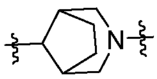, 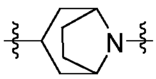, 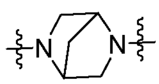,

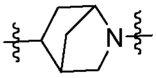, 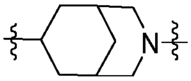, 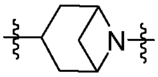, 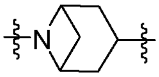, 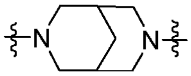, 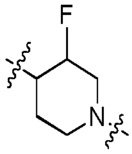,

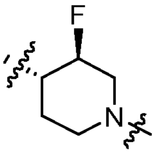, 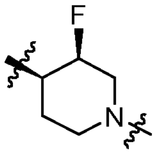 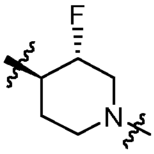, 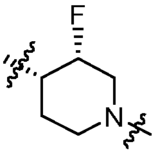, 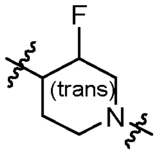 or 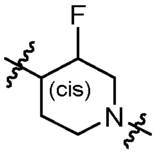,

wherein the left side of L is linked to B;
or L is selected from

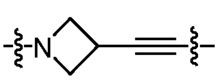, 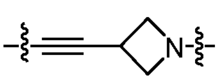, 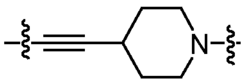,

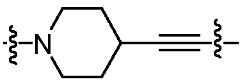, 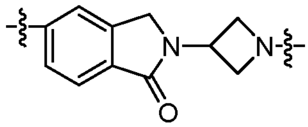, 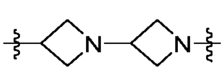, 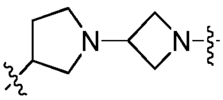,

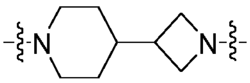, 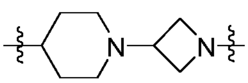, 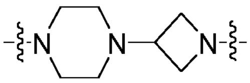, 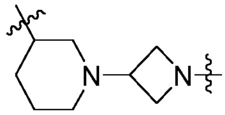,

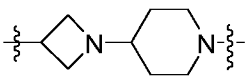, 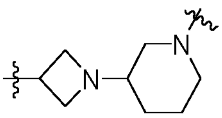, 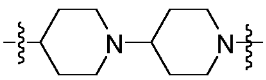, 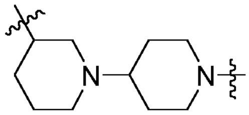,

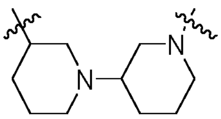, 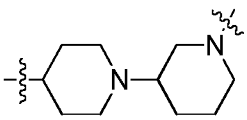, 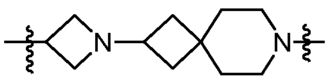,

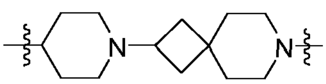, 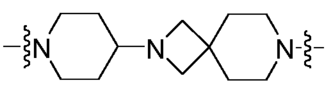, 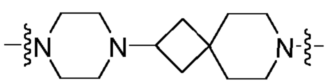,

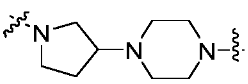, 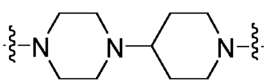, 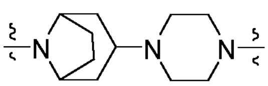, 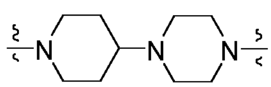,

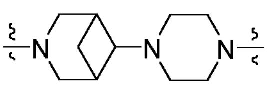, 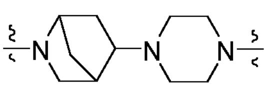, 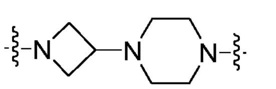,

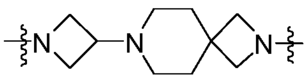 , 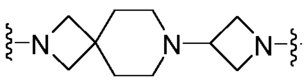 , 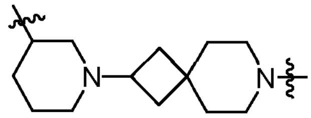 ,

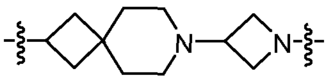 , 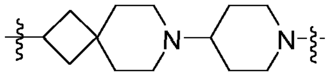 , 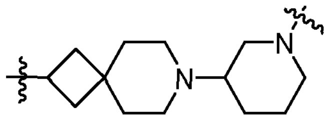 ,

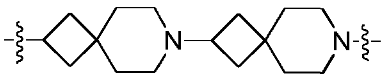 , 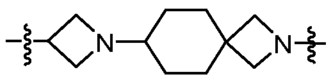 , 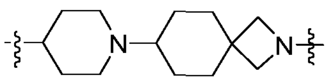 ,

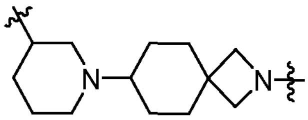 , 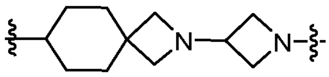 , 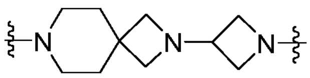 ,

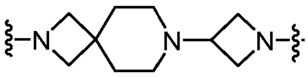 , 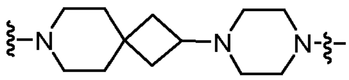 , 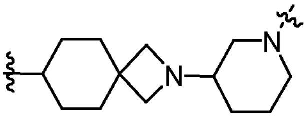 ,

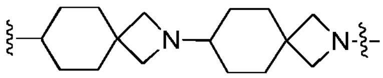 , 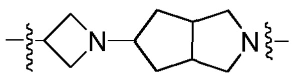 , 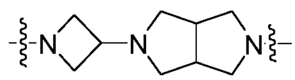 ,

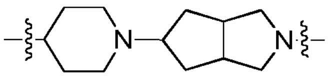 , 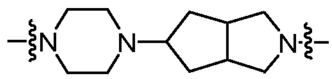 , 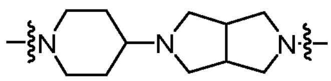 ,

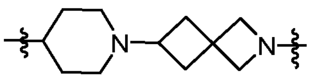 , 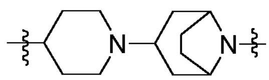 , 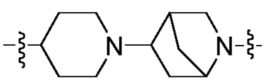 ,

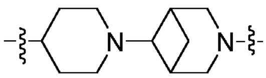 , 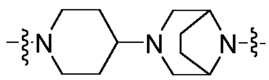 , 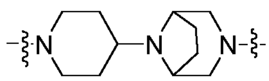 ,

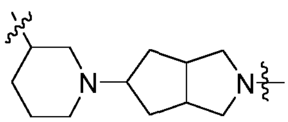 , 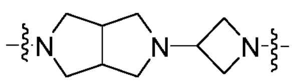 , 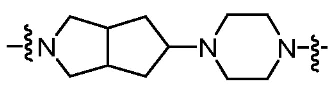 ,

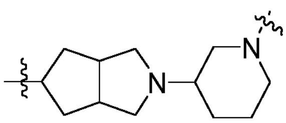 , 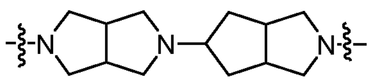 , 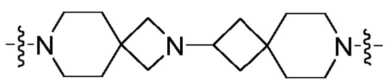 ,

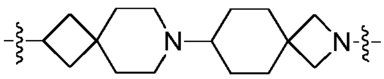 , 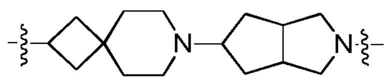 ,

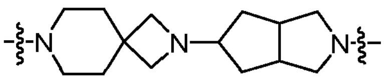 , 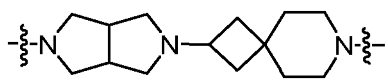 ,

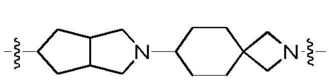 , 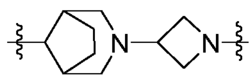 , 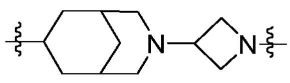 ,

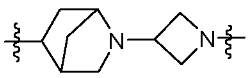 , 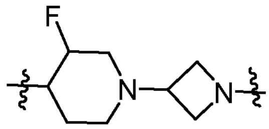 , 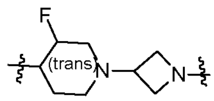 , 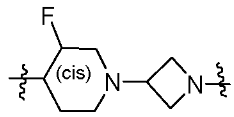 ,

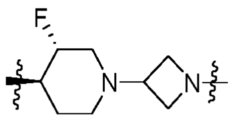 , 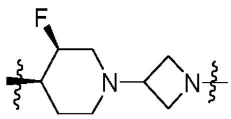 , 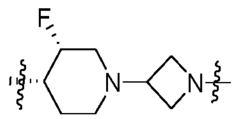 , 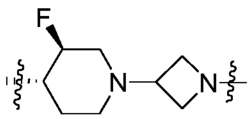 ,

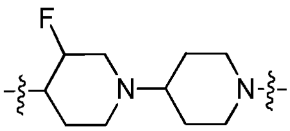 , 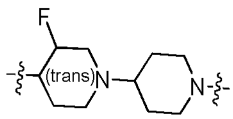 , 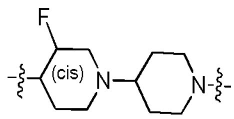 , 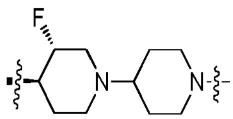 ,

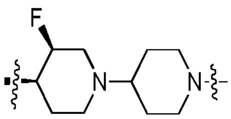 , 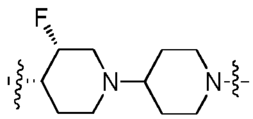 , 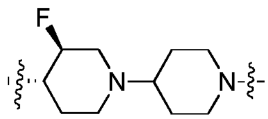 , 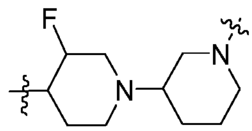 ,

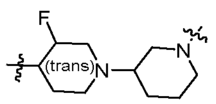 , 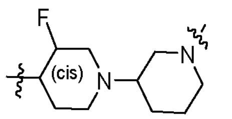 , 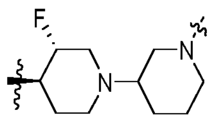 , 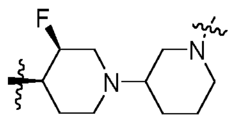 ,

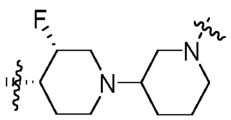 , 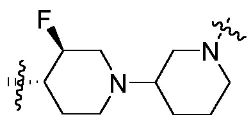 , 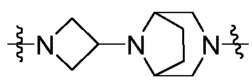 , 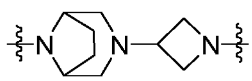 ,

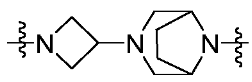 , 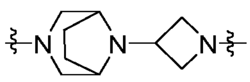 , 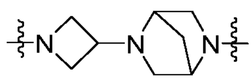 , 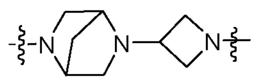

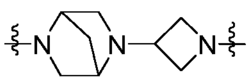 , 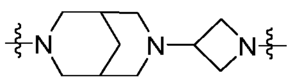 , 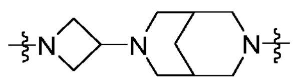 or

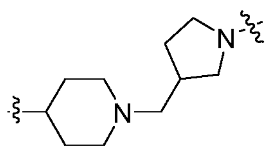,

wherein the left side of L is linked to B; or L is selected from

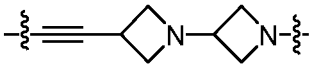, 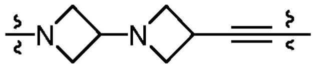,

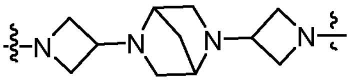, 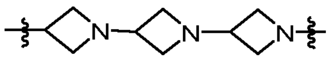, 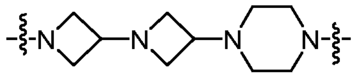,

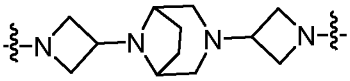, 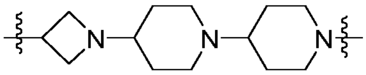, 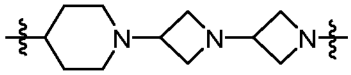,

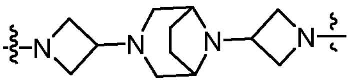, 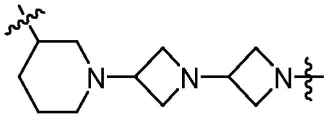, 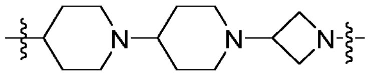,

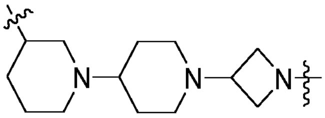, 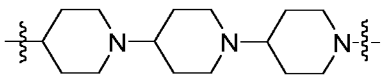, 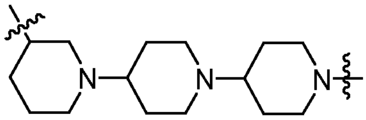,

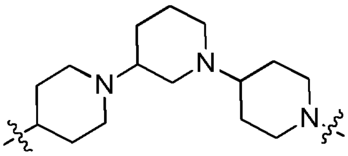, 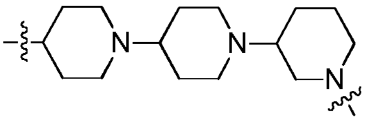, 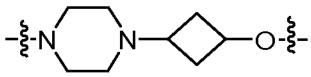,

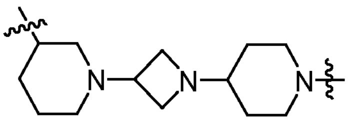, 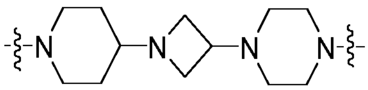, 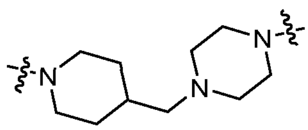

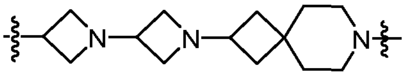, 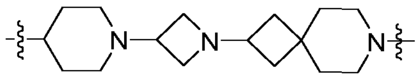,

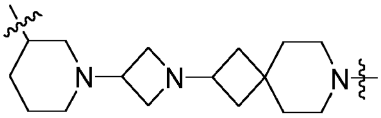, 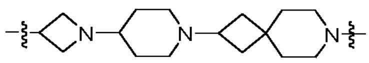,

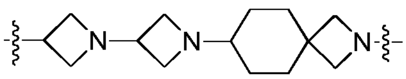,
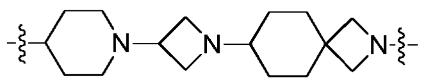,
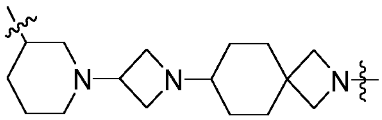,
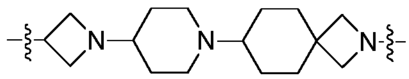,
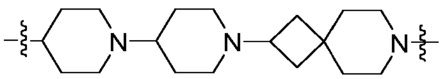,
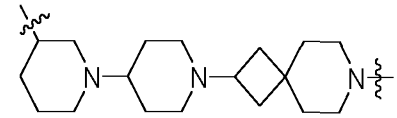,
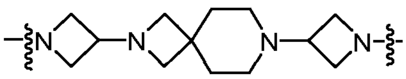,
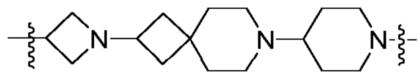,
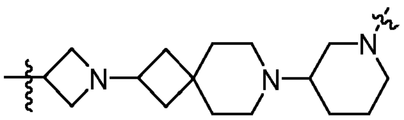,
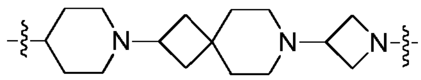,
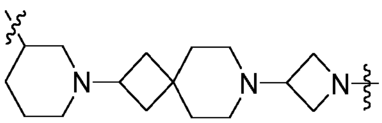,
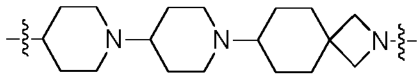,
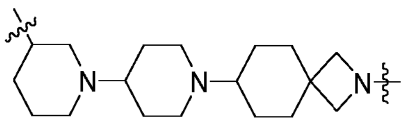,
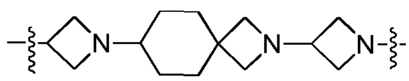,
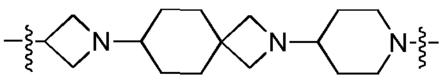,
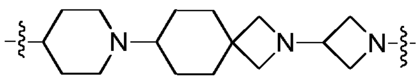,
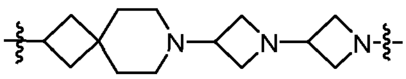,
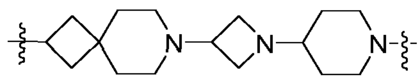,
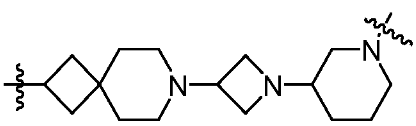,
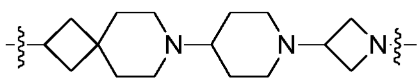,
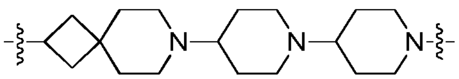,
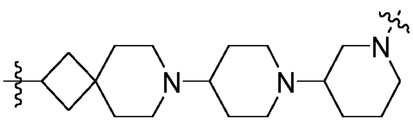,

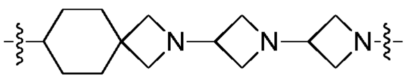,
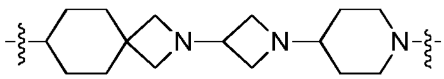,
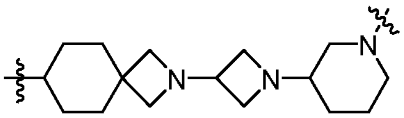,
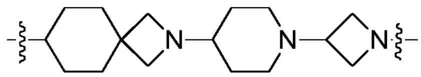,
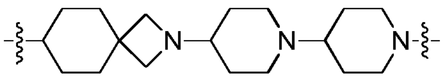,
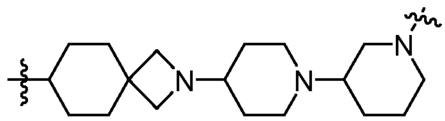,
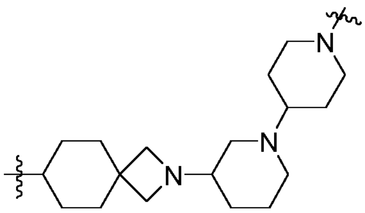,
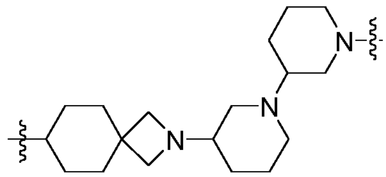,
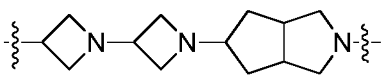,
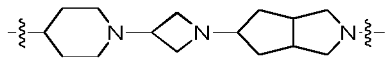,
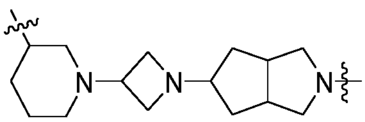,
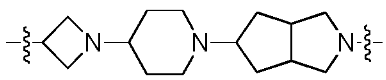,
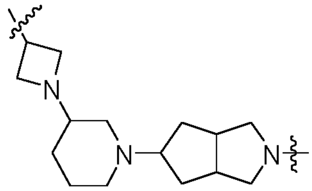,
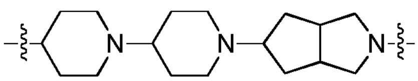,
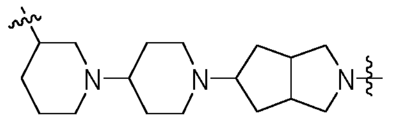,
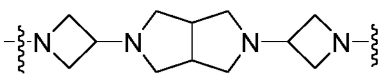,
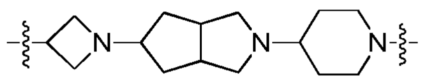,
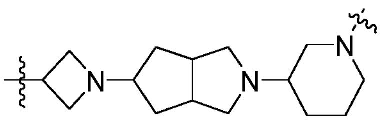,
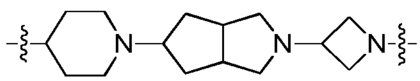,
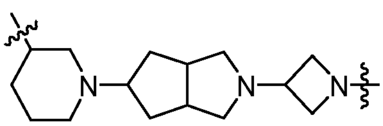,
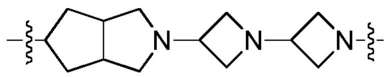,

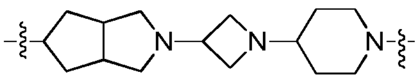 , 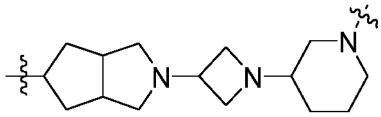 ,

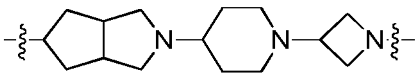 , 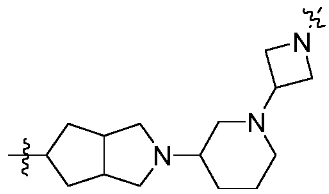 ,

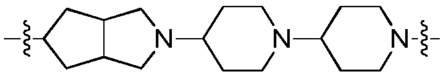 , 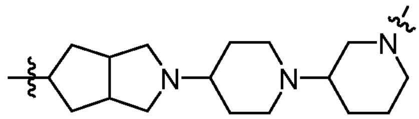 ,

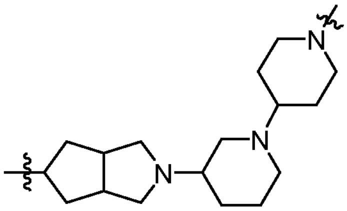 , 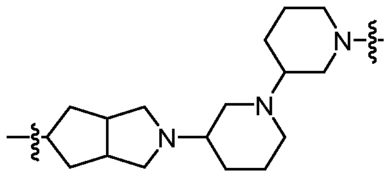 ,

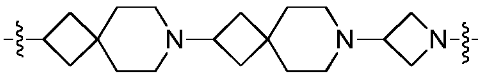 , 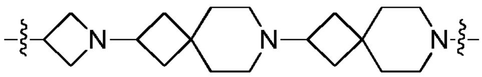 ,

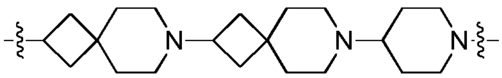 , 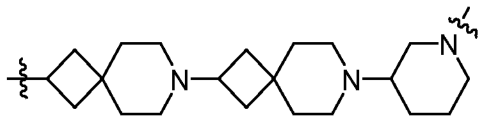 ,

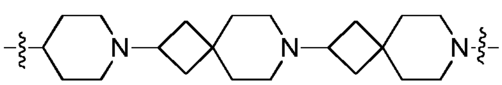 , 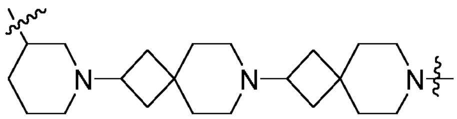 ,

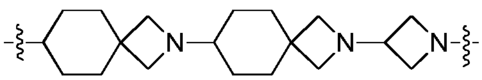 , 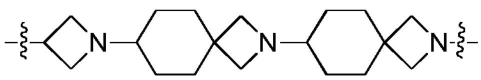 ,

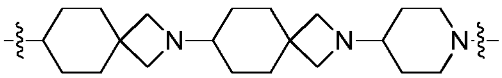 , 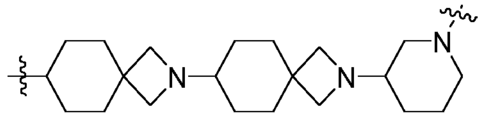 ,

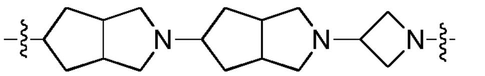
,
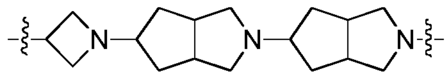
,

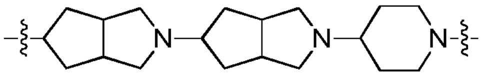
,
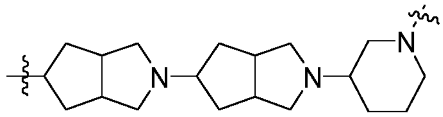
,

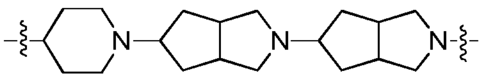
,
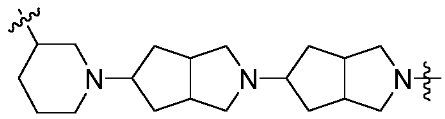
,

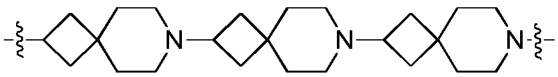
,
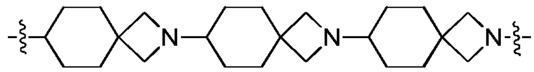
,

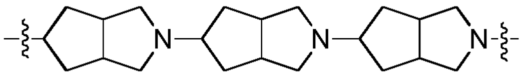
,
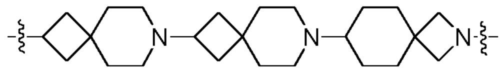
,

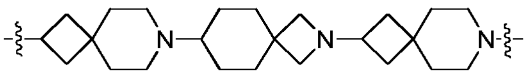
,
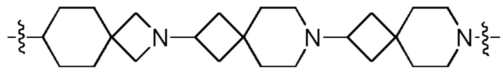
,

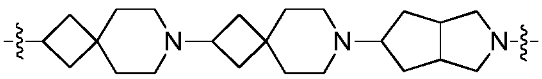
,
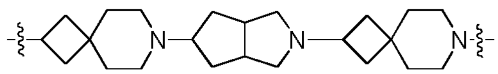
,

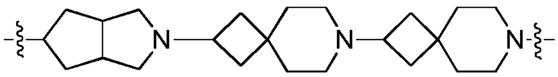
,
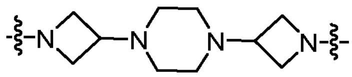
,

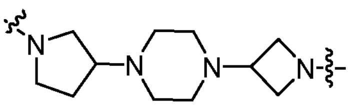
,
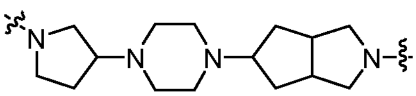
,

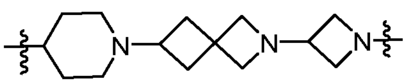
,
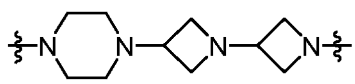
,

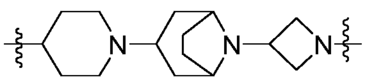
,
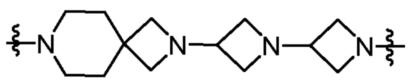
,

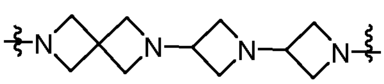
,
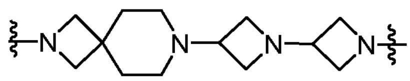
,

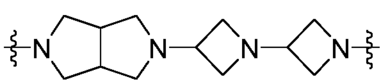
,
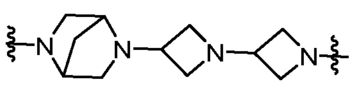
,

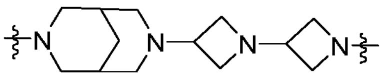, 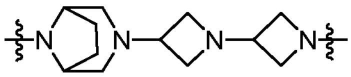, 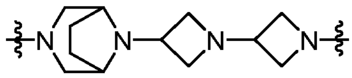,

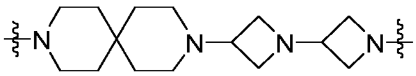, 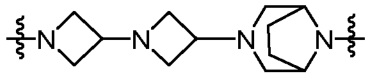,

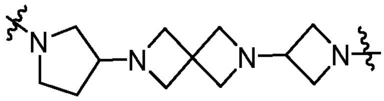, 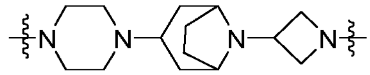,

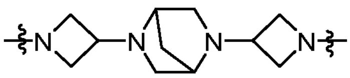 or 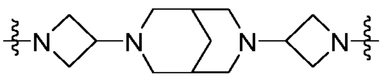,

wherein the left side of L is linked to B;
or L is selected from

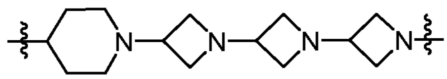,

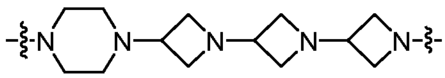, 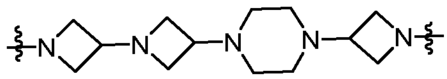,

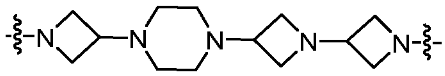 and 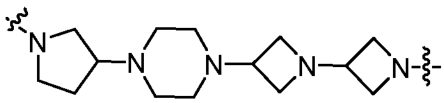,

wherein the left side of L is linked to B;
other groups are as defined in the fourth embodiment.

As a sixth embodiment of the present invention, provided is a compound as shown in general formula (I) or a stereoisomer, a deuterated compound, a solvate, a prodrug, a metabolite, a pharmaceutically acceptable salt or a co-crystal thereof, wherein

K is selected from

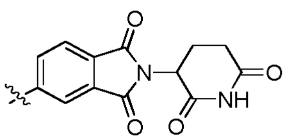, 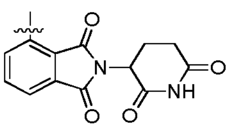, 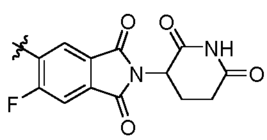,

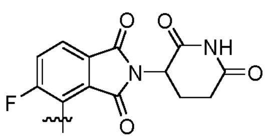, 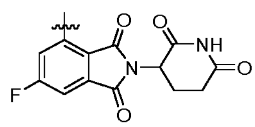, 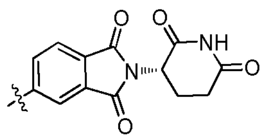, 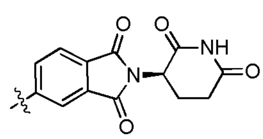,

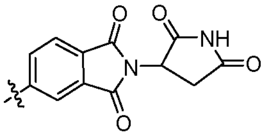, 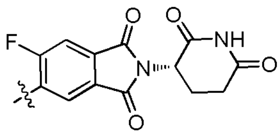, 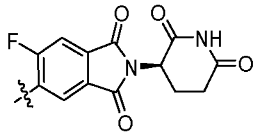, 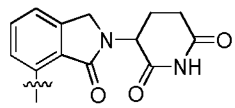,

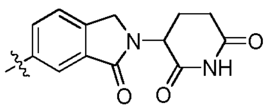, 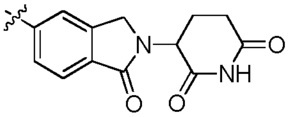, 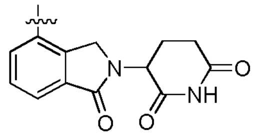, 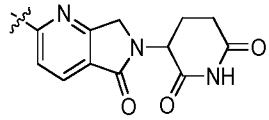

, 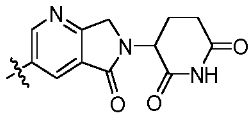, 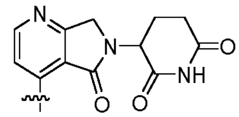, 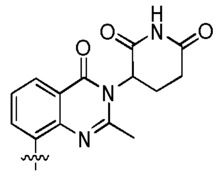, 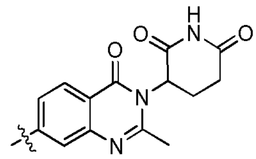,

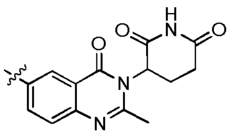, 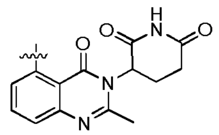, 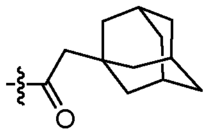, 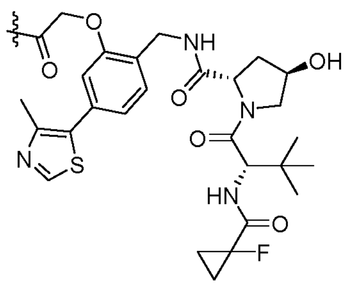,

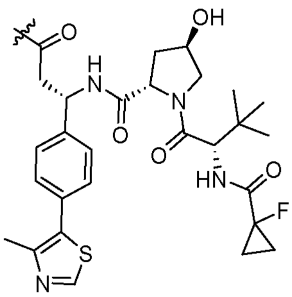, 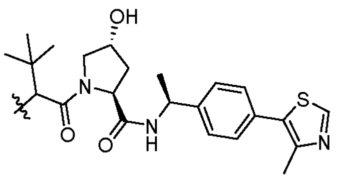, 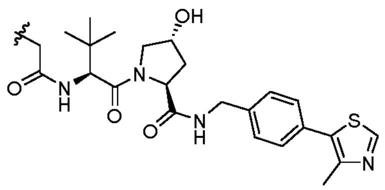,

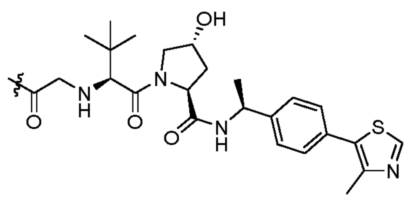 or 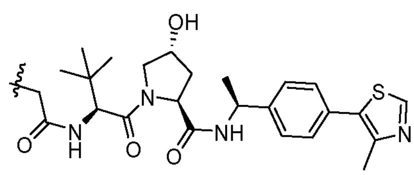;

other groups are as defined in the fifth embodiment.

As a seventh embodiment of the present invention, provided is a compound as shown in general formula (I) or a stereoisomer, a deuterated compound, a solvate, a prodrug, a metabolite, a pharmaceutically acceptable salt or a co-crystal thereof, wherein

K is selected from

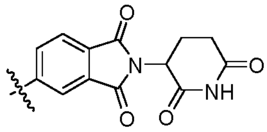, 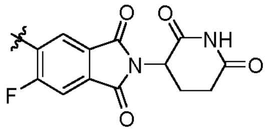,

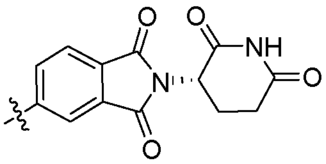, 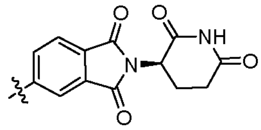, 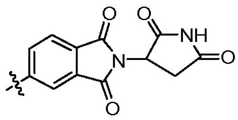, 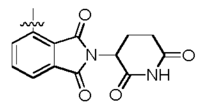,

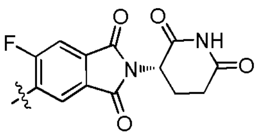, 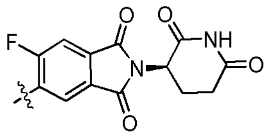, 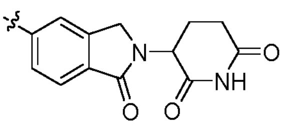, 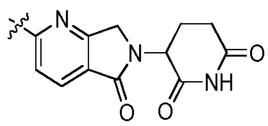,

, 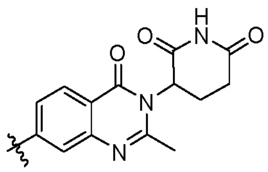, 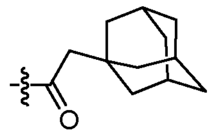, 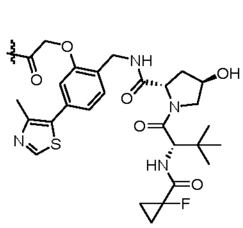, 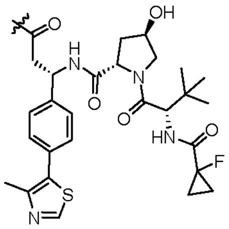,

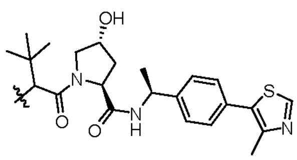, 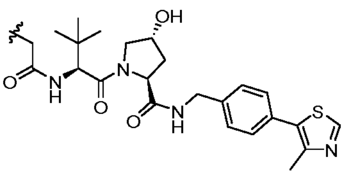, 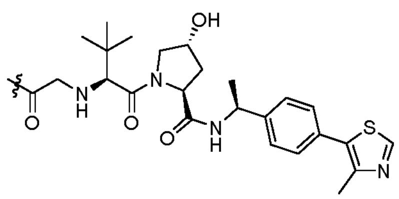 or

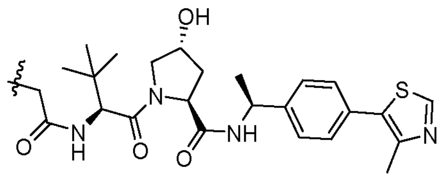;

other groups are as defined in the sixth embodiment.

As an embodiment of the present invention, provided is a compound as shown in general formula (I) or a stereoisomer, a deuterated compound, a solvate, a prodrug, a metabolite, a pharmaceutically acceptable salt or a co-crystal thereof, wherein the compound has a structure selected from one of the following structures:

| | |
|---|---|
| 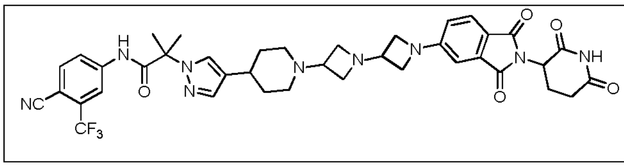 | 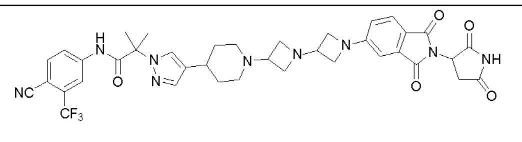 |

| | |
|---|---|
| 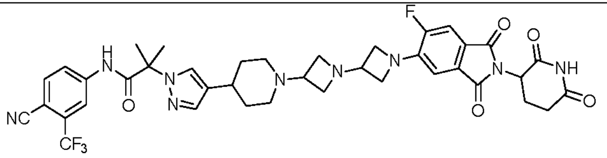 | 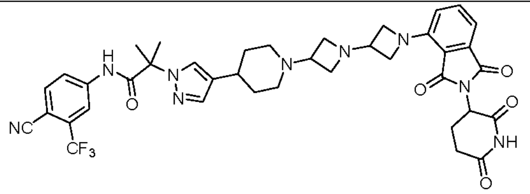 |
| 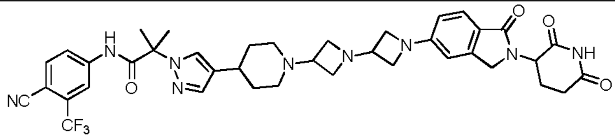 | 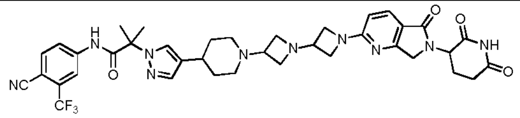 |
| 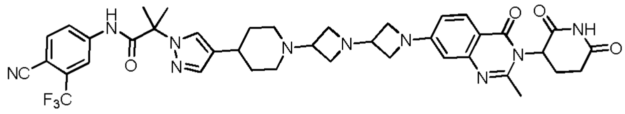 | 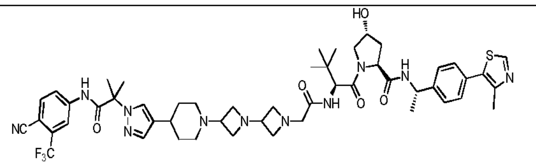 |
| 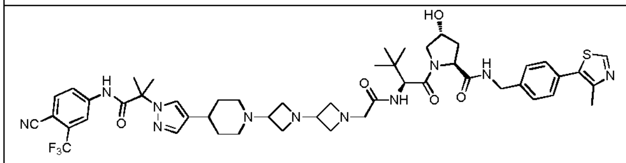 | 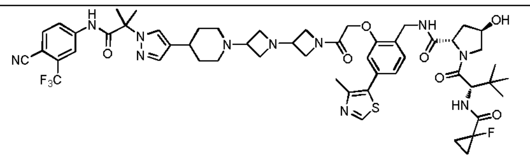 |
| 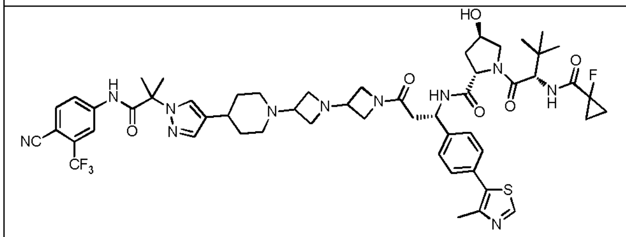 | 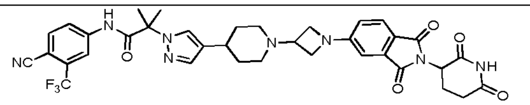 |
| 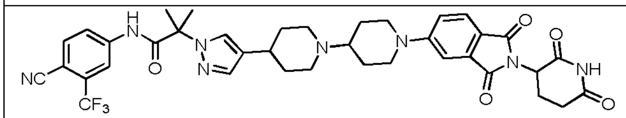 | 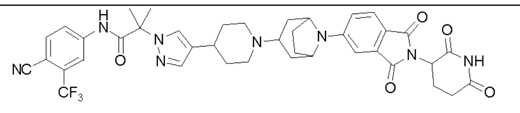 |
| 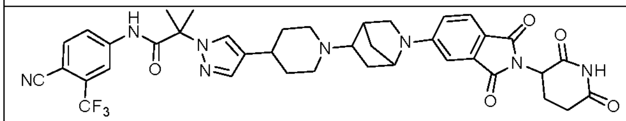 | 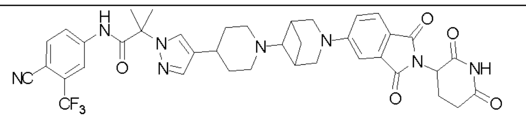 |
| 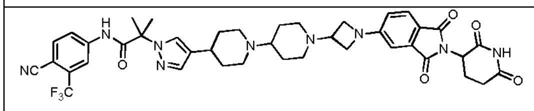 | 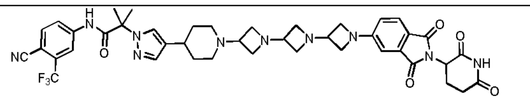 |
| 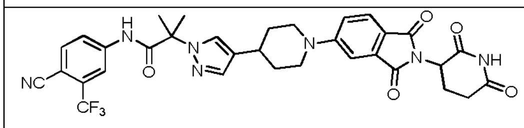 | 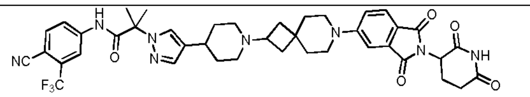 |
| 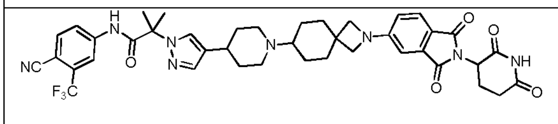 | 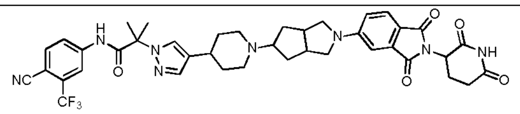 |
| 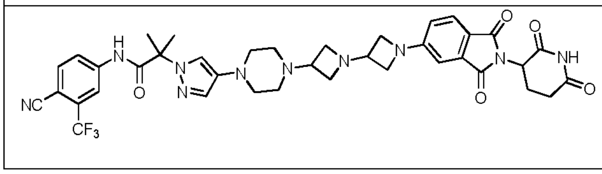 | 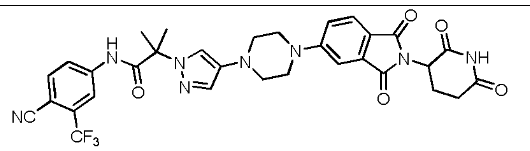 |

| | |
|---|---|
| 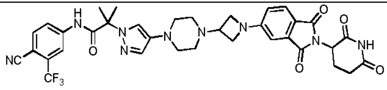 | 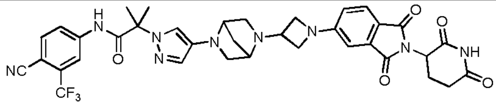 |
| 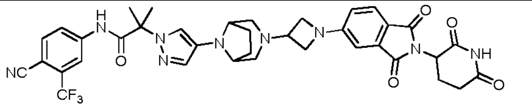 | 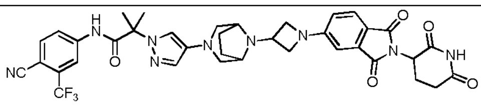 |
| 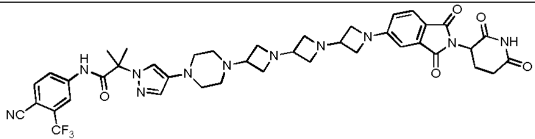 | 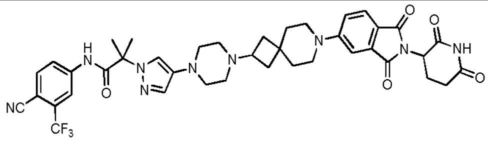 |
| 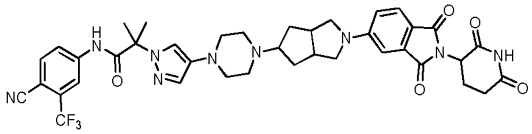 | 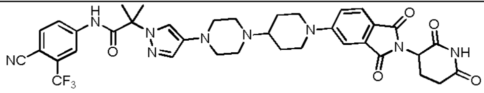 |
| 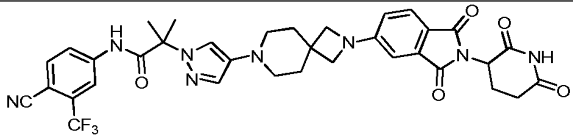 | 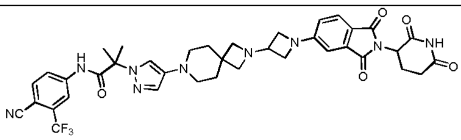 |
| 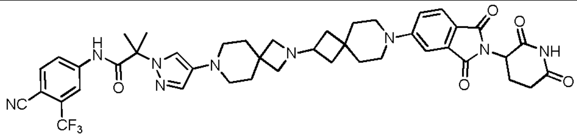 | 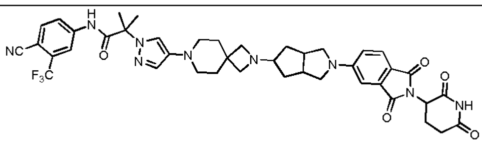 |
| 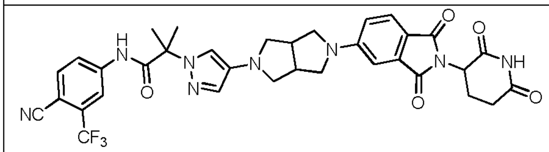 | 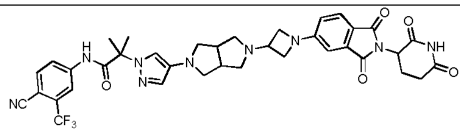 |
| 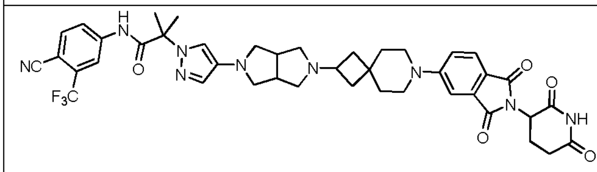 | 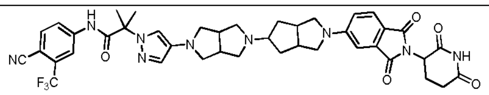 |
| 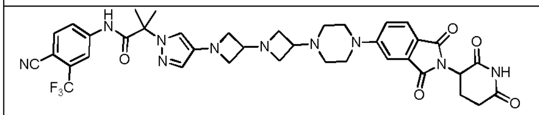 | 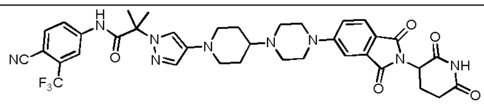 |
| 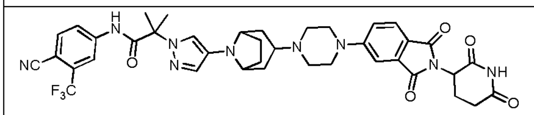 | 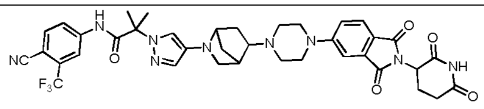 |
| 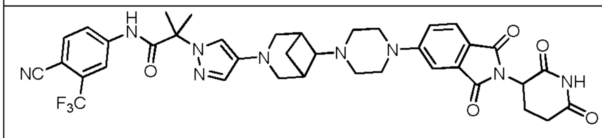 | 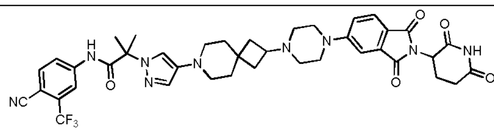 |
| 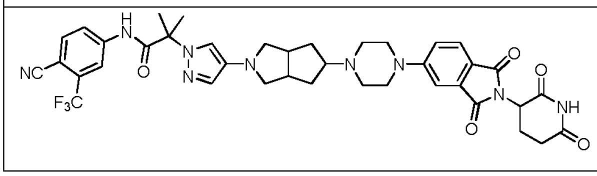 | 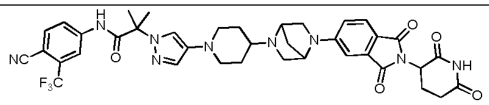 |

| | |
|---|---|
| 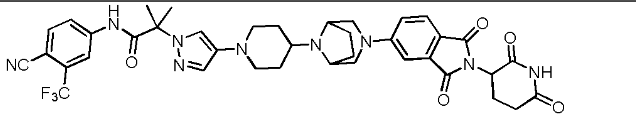 | 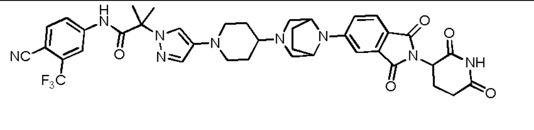 |
| 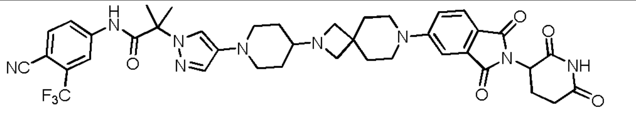 | 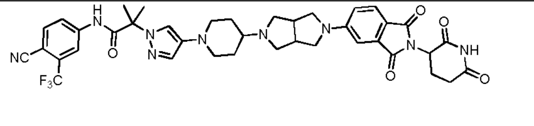 |
| 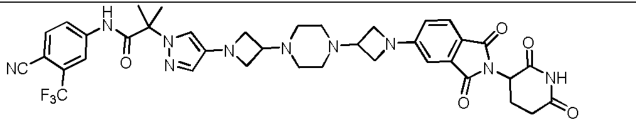 | 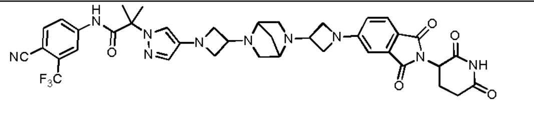 |
| 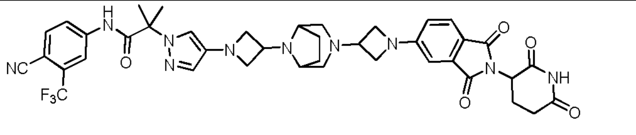 | 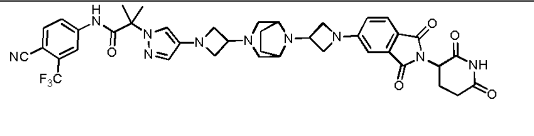 |
| 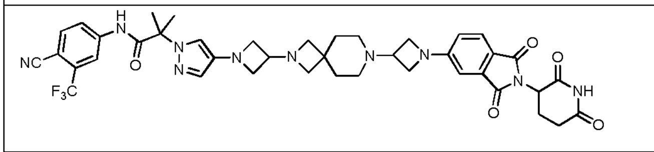 | 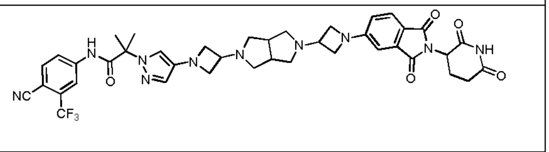 |
| 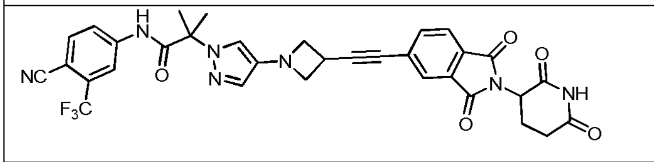 | 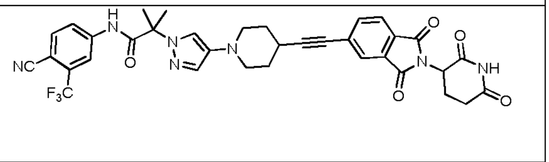 |
| 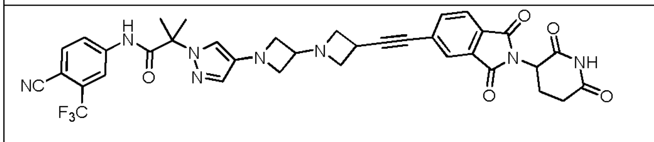 | 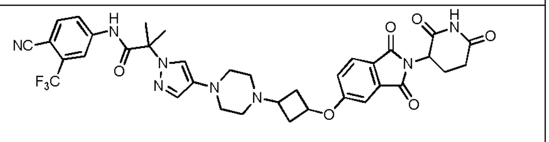 |
| 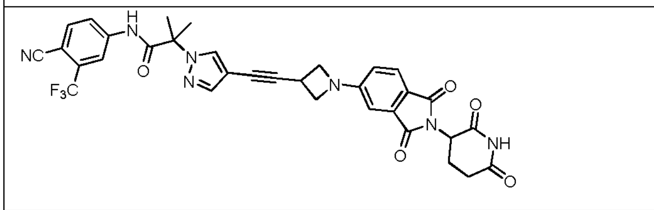 | 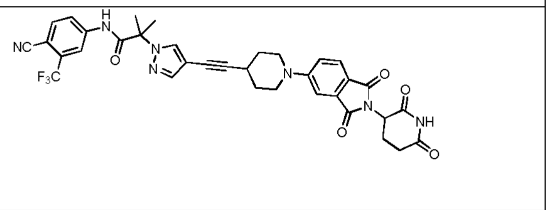 |
| 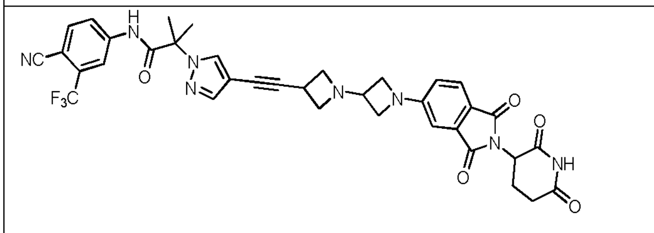 | 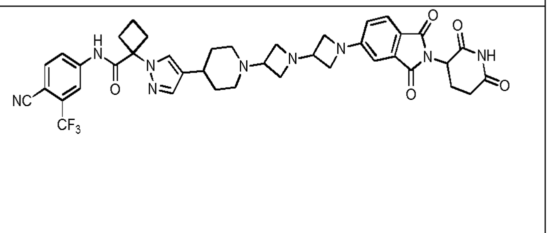 |
| 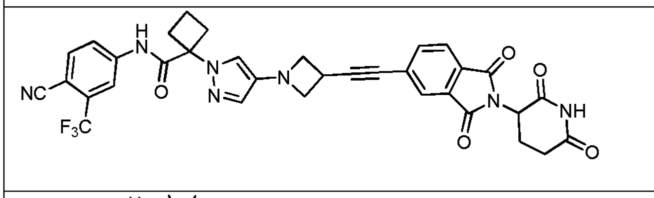 | 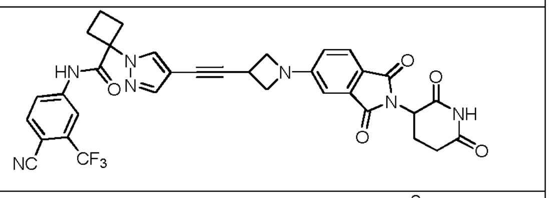 |
| 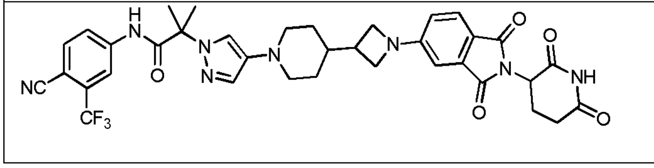 | 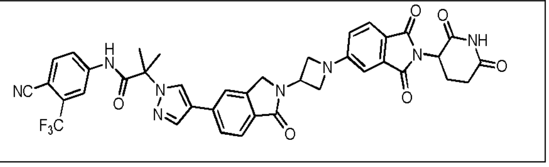 |

| | |
|---|---|
| 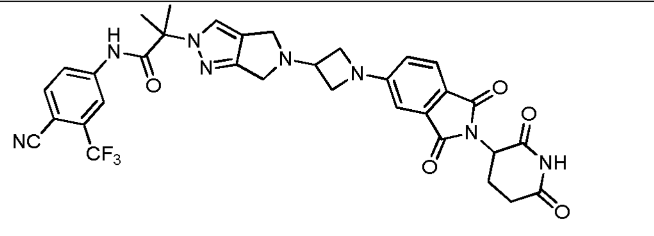 | 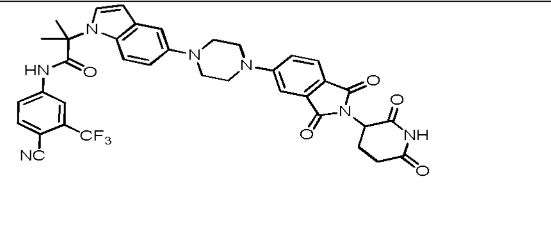 |
| 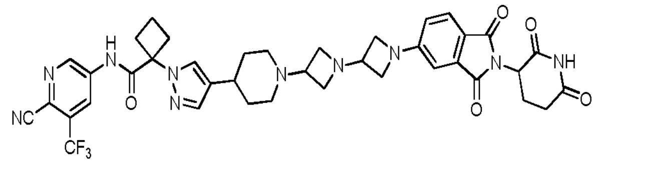 | 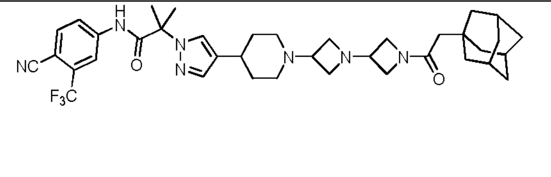 |
| 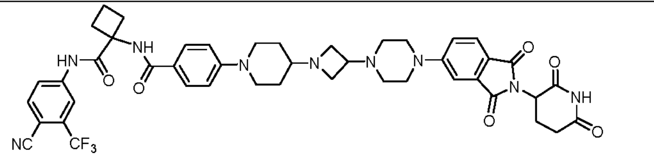 | 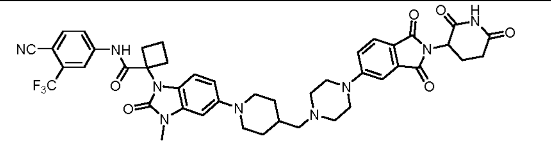 |
| 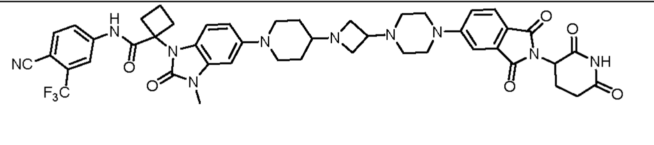 | 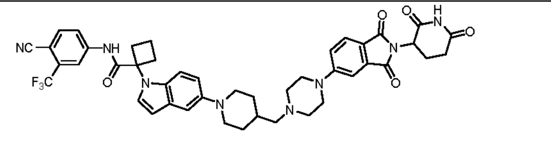 |
| 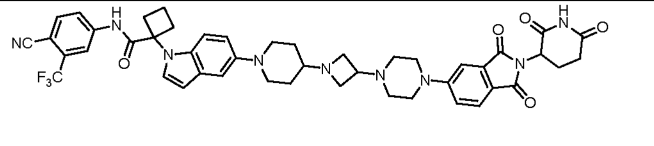 | 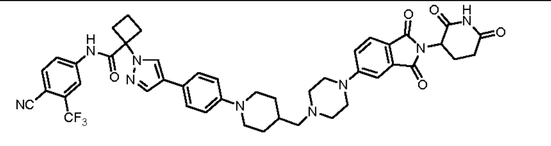 |
| 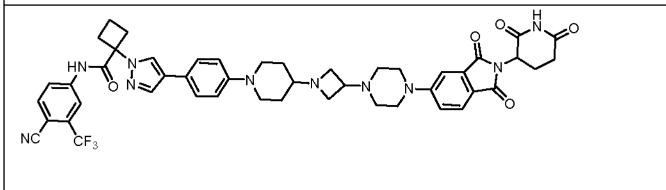 | 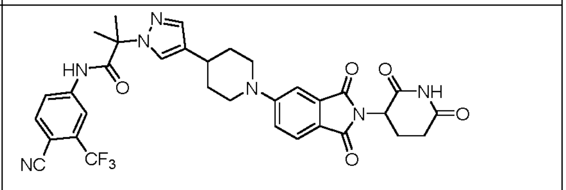 |
| 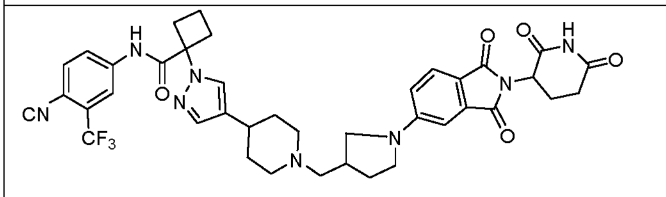 | 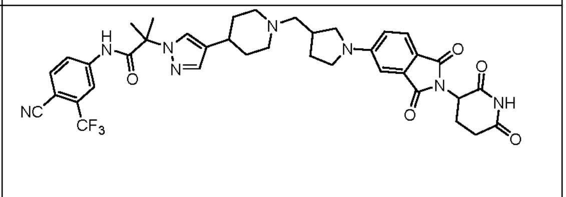 |
| 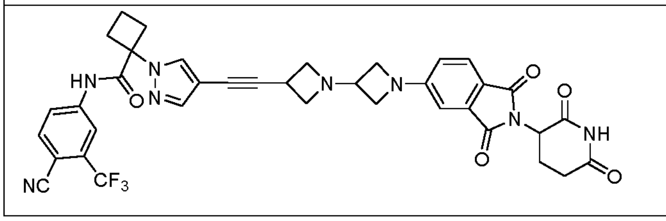 | 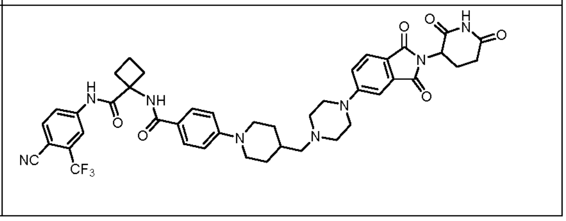 |

The present invention relates to a pharmaceutical composition, comprising the above-mentioned compound in the present invention or the stereoisomer, deuterated compound, solvate, prodrug, metabolite, pharmaceutically acceptable salt or co-crystal thereof, and a pharmaceutically acceptable carrier.

The present invention relates to the use of the above-mentioned compound in the present invention or the stereoisomer, deuterated compound, solvate, prodrug, metabolite, pharmaceutically acceptable salt or co-crystal thereof in the preparation of a medicament for treating a disease related to AR activity or expression level.

The present invention relates to the use of the above-mentioned compound in the present invention or the stereoisomer, deuterated compound, solvate, prodrug, metabolite, pharmaceutically acceptable salt or co-crystal thereof in the preparation of a medicament for treating a disease related to the inhibition or degradation of AR.

The present invention relates to the use of the above-mentioned compound in the present invention or the stereoisomer, deuterated compound, solvate, prodrug, metabolite, pharmaceutically acceptable salt or co-crystal thereof in the preparation of a medicament for treating a disease related to the activity or expression level of an AR or AR splice variant.

The present invention relates to the use of the above-mentioned compound in the present invention or the stereoisomer, deuterated compound, solvate, prodrug, metabolite, pharmaceutically acceptable salt or co-crystal thereof in the preparation of a medicament for treating a disease related to the inhibition or degradation of an AR or AR splice variant.

The present invention relates to the use of the above-mentioned compound in the present invention or the stereoisomer, deuterated compound, solvate, prodrug, metabolite, pharmaceutically acceptable salt or co-crystal thereof, wherein the disease is selected from prostate cancer.

Synthetic method:

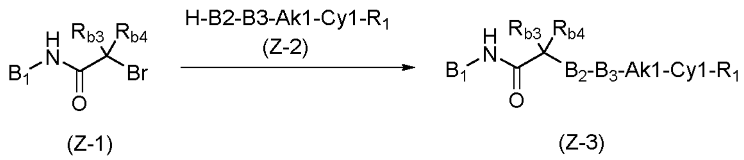

(Z-1) (Z-3)

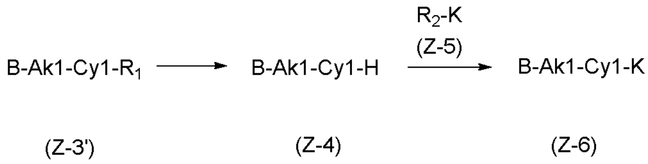

(Z-3') (Z-4) (Z-6)

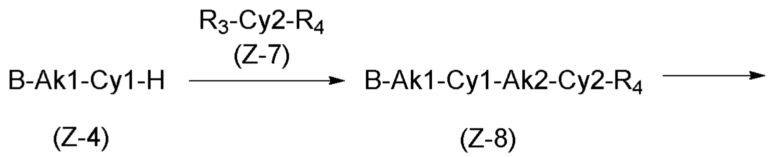

(Z-4) (Z-8)

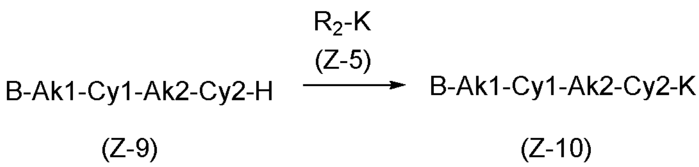

(Z-9) (Z-10)

$$\text{B-Ak1-Cy1-Ak2-Cy2-H} \xrightarrow[\text{(Z-11)}]{R_5\text{-Cy3-}R_6} \text{B-Ak1-Cy1-Ak2-Cy2-Ak3-Cy3-}R_6$$

(Z-9) (Z-12)

$$\longrightarrow \text{B-Ak1-Cy1-Ak2-Cy2-Ak3-Cy3-H}$$

(Z-13)

$$\xrightarrow[\text{(Z-5)}]{R_2\text{-K}} \text{B-Ak1-Cy1-Ak2-Cy2-Ak3-Cy3-K}$$

(Z-14)

$$\text{B-Ak1-Cy1-Ak2-Cy2-Ak3-Cy3-H} \xrightarrow[\text{(Z-15)}]{R_7\text{-Cy4-}R_8} \text{B-Ak1-Cy1-Ak2-Cy2-Ak3-Cy3-Ak4-Cy4-}R_8$$

(Z-13) (Z-16)

$$\longrightarrow \text{B-Ak1-Cy1-Ak2-Cy2-Ak3-Cy3-Ak4-Cy4-H}$$

(Z-17)

$$\xrightarrow[\text{(Z-5)}]{R_2\text{-K}} \text{B-Ak1-Cy1-Ak2-Cy2-Ak3-Cy3-Ak4-Cy4-K}$$

(Z-18)

general formula (Z-3), i.e., general formula (Z-3'), is generated by a nucleophilic substitution reaction of general formula (Z-1) with general formula (Z-2), and when $R_1$ is an amino protecting group, general formula (Z-3') is deprotected to obtain corresponding general formula (Z-4), and then general formula (Z-6), i.e., general formula (I), is obtained by a nucleophilic substitution reaction of general formula (Z-4) with general formula (Z-5); general formula (Z-8) is generated by a nucleophilic substitution or reductive amination reaction of general formula (Z-4) with general formula (Z-7), and when $R_4$ is an amino protecting group, general formula (Z-8) is deprotected to obtain corresponding general formula (Z-9), and then general formula (Z-10), i.e., general formula (I), is obtained by a nucleophilic substitution reaction of general formula (Z-9) with general formula (Z-5);

general formula (Z-12) is generated by a nucleophilic substitution or reductive amination reaction of general formula (Z-9) with general formula (Z-11), and when $R_6$ is an amino protecting group, general formula (Z-12) is deprotected to obtain corresponding general formula (Z-13), and then general formula (Z-14), i.e., general formula (I), is obtained by a nucleophilic substitution reaction of general formula (Z-13) with general formula (Z-5); and

general formula (Z-16) is generated by a nucleophilic substitution or reductive amination reaction of general formula (Z-13) with general formula (Z-15), and when $R_8$ is an amino protecting group, general formula (Z-16) is deprotected to obtain corresponding general formula (Z-17), and then general formula (Z-18), i.e., general formula (I), is obtained by a nucleophilic substitution reaction of general formula (Z-17) with general formula (Z-5).

The synthetic method of general formula (Z-5) is described in WO 2017197056;

$R_1$ is selected from H, (=O), -CHO, F, Cl, Br, I or an amino protecting group (preferably Boc);
$R_2$ is selected from $NH_2$, F, Cl, Br, I, OTf and OH; and
$R_3$, $R_4$, $R_5$, $R_6$, $R_7$ and $R_8$ are each independently selected from H, (=O), -CHO, H, F, Cl, Br, I, OTf or an amino protecting group (preferably Boc).

Unless stated to the contrary, the terms used in the description and claims have the following meanings.

The carbon, hydrogen, oxygen, sulfur, nitrogen or F, Cl, Br, I involved in the groups and compounds of the present invention all comprise their isotopes, and the carbon, hydrogen, oxygen, sulfur or nitrogen involved in the groups and compounds of the present invention is optionally further substituted with one or more of their corresponding isotopes, wherein the isotopes of carbon comprise $^{12}C$, $^{13}C$ and $^{14}C$, the isotopes of hydrogen comprise protium (H), deuterium (D, also known as heavy hydrogen), tritium (T, also known as superheavy hydrogen), the isotopes of oxygen comprise $^{16}O$, $^{17}O$ and $^{18}O$, the isotopes of sulfur comprise $^{32}S$, $^{33}S$, $^{34}S$ and $^{36}S$, the isotopes of nitrogen comprise $^{14}N$ and $^{15}N$, the isotopes of fluorine comprise $^{17}F$ and $^{19}F$, the isotopes of chlorine comprise $^{35}Cl$ and $^{37}Cl$, and the isotopes of bromine comprise $^{79}Br$ and $^{81}Br$.

"Alkyl" refers to a straight or branched saturated aliphatic hydrocarbon group containing 1 to 20 carbon atoms, preferably alkyl containing 1 to 8 carbon atoms, more preferably alkyl containing 1 to 6 carbon atoms, further preferably alkyl containing 1 to 4 carbon atoms. Non-limiting examples include methyl, ethyl, n-propyl, isopropyl, n-butyl, sec-butyl, neobutyl, tert-butyl, n-pentyl, isoamyl, neopentyl, n-hexyl and various branched isomers thereof; the alkyl can be optionally further substituted with 0 to 6 substituents selected from F, Cl, Br, I, hydroxyl, sulfhydryl, nitro, cyano, amino, alkylamino, amido, alkenyl, alkynyl, $C_{1-6}$ alkyl, $C_{1-6}$ hydroxyalkyl, $C_{1-6}$ alkoxy, 3- to 8-membered carbocyclyl, a 3- to 8-membered heterocyclic group, 3- to 8-membered carbocyclyloxy, 3- to 8-membered heterocyclyloxy, carboxyl or a carboxylate group, and the definition of the alkyl described herein is consistent with this definition.

"Alkoxy" refers to -O-alkyl. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy. The alkoxy can be optionally further substituted with 0 to 5 substituents selected from F, Cl, Br, I, hydroxyl, sulfhydryl, nitro, cyano, amino, alkylamino, alkenyl, alkynyl, alkyl, hydroxyalkyl, alkoxy, carbocyclyl, a heterocyclic group, carbocyclyloxy, heterocyclyloxy, carboxyl or a carboxylate group. The definition of the alkoxy described herein is consistent with this definition.

"Cycloalkyl" refers to a straight or branched saturated cyclic aliphatic hydrocarbon group containing 3 to 20 carbon atoms, preferably cycloalkyl containing 3 to 10 carbon atoms. Non-limiting examples include cyclopropyl, cyclobutyl, cyclopentyl, cyclohexyl and cycloheptyl. The cycloalkyl can be optionally further substituted with 0 to 5 substituents selected from F, Cl, Br, I, hydroxyl, sulfhydryl, nitro, cyano, amino, alkylamino, alkenyl, alkynyl, alkyl, hydroxyalkyl, alkoxy, carbocyclyl, a heterocyclic group, carbocyclyloxy, heterocyclyloxy, carboxyl or a carboxylate group. The definition of the cycloalkyl described herein is consistent with this definition.

"Heterocyclic group" or "heterocyclic ring" refers to a substituted or unsubstituted saturated or unsaturated aromatic ring or non-aromatic ring, and the aromatic ring or non-aromatic ring can be a 3- to 8-membered monocyclic ring, a 4- to 12-membered bicyclic ring or a 10- to 15-membered tricyclic ring and contains 1 to 3 heteroatoms selected from N, O or S, preferably a 3- to 8-membered heterocyclic group, and the optionally substituted N and S in the ring of the heterocyclic group can be oxidized into various oxidation states. Heterocyclic group can be connected to a heteroatom or carbon atom, and heterocyclic group can be connected to a bridged ring or spiro ring. Non-limiting examples include oxiranyl, azacyclopropyl, oxetanyl, azetidinyl, 1,3-dioxolane, 1,4-dioxolane, 1,3-dioxane, azacycloheptyl, pyridyl, furanyl, thienyl, pyranyl, N-alkylpyrrolyl, pyrimidinyl, pyrazinyl, pyridazinyl, imidazolyl, piperidyl, morpholinyl, thiomorpholinyl, 1,3-dithianyl, dihydrofuranyl, dihydropyranyl, dithiolanyl, tetrahydrofuranyl, tetrahydropyrrolyl, tetrahydroimidazolyl, tetrahydrothiazolyl, tetrahydropyranyl, benzimidazolyl, benzopyridyl, pyrrolopyridyl, benzodihydrofuranyl, azabicyclo[3.2.1]octyl, azabicyclo[5.2.0]nonyl, oxatricyclo[5.3.1.1]dodecyl, azaadamantyl and oxaspiro[3.3]heptyl. The heterocyclic group can be optionally further substituted with 0 to 5 substituents selected from F, Cl, Br, I, =O, hydroxyl, sulfhydryl, nitro, cyano, amino, alkylamino, amido, alkenyl, alkynyl, alkyl, hydroxyalkyl, alkoxy, carbocyclyl, a heterocyclic group, carbocyclyloxy, heterocyclyloxy, carboxyl or a carboxylate group. The definition of the heterocyclic group described herein is consistent with this definition.

"Spiro ring" refers to a 5- to 20-membered polycyclic group sharing one carbon atom (referred to as a spiro atom) between substituted or unsubstituted monocyclic rings, which may contain 0 to 5 double bonds, and may contain 0 to 5 heteroatoms selected from N, O or $S(=O)_n$. The spiro ring is preferably 6- to 14-membered, further preferably 6- to 12-membered, and more preferably 6- to 10-membered. Its non-limiting examples include:

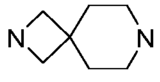, 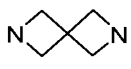, 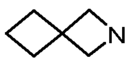,

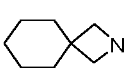, 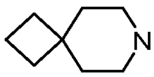, 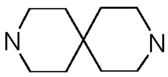, 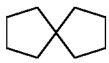, 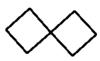, 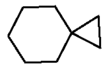, 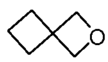

and

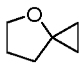, 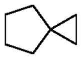 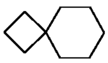, 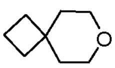, 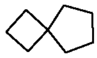. 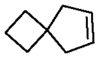, 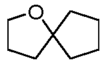,

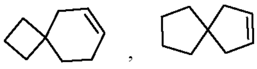, 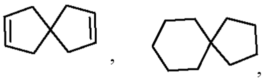, 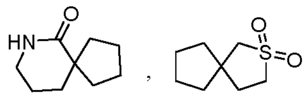.

When a substitution occurs, substituents can be 1 to 5 groups selected from F, Cl, Br, I, alkyl, cycloalkyl, alkoxy, haloalkyl, mercaptan, hydroxyl, nitro, sulfhydryl, amino, cyano, isocyano, aryl, heteroaryl, a heterocyclic group, a bridged ring group, a spiro ring group, a fused ring group, hydroxyalkyl, =O, carbonyl, aldehyde, carboxylic acid, formate, -$(CH_2)_m$-C(=O)-$R^a$, -O-$(CH_2)_m$-C(=O)-$R^a$, -$(CH_2)_m$-C(=O)-$NR^bR^c$, -$(CH_2)_m$S$(=O)_n$$R^a$, - $(CH_2)_m$-alkenyl-$R^a$, $OR^d$ or -$(CH_2)_m$-alkynyl-$R^a$ (wherein m and n are 0, 1 or 2), arylthio, thiocarbonyl, silyl or -$NR^bR^c$ and the like, wherein $R^b$ and $R^c$ are independently selected from H, hydroxyl, amino, carbonyl, alkyl, alkoxy, cycloalkyl, a heterocyclic group, aryl, heteroaryl, sulfonyl, trifluoromethylsulfonyl. Alternatively, $R^b$ and $R^c$ may form five- or six-membered cycloalkyl or a heterocyclic group. $R^a$ and $R^d$ are each independently selected from aryl, heteroaryl, alkyl, alkoxy, cycloalkyl, a heterocyclic group, carbonyl, an ester group, a bridged ring group, a spiro ring group or a fused ring group. The definition of the spiro ring described herein is consistent with this definition.

"Fused ring" refers to a polycyclic group in which each ring in the system shares an adjacent pair of carbon atoms with other rings in the system, wherein one or more of the rings may contain 0 or more double bonds, which may be substituted or unsubstituted, and each ring in the fused ring system may contain 0 to 5 heteroatoms selected from N, S$(=O)_n$ or O (n is selected from 0, 1 or 2). The fused ring is preferably 5- to 20-membered, further preferably 5- to 14-membered, more preferably 5- to 12-membered, and still further preferably 5- to 10-membered. Non-limiting examples include:

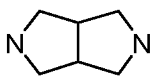, 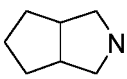,

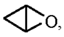, 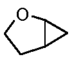, 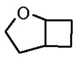, 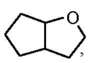, 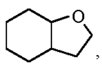, 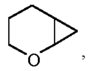, 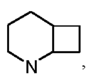, 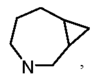,

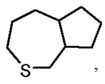, 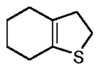, 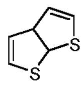, 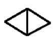, 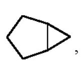, 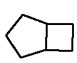, 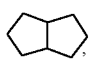, 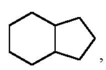,

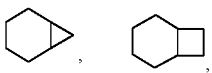, 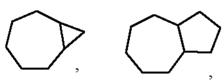, 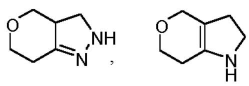, 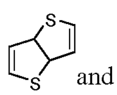 and 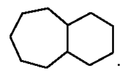.

When a substitution occurs, substituents can be 1 to 5 groups selected from F, Cl, Br, I, alkyl, cycloalkyl, alkoxy, haloalkyl, mercaptan, hydroxyl, nitro, sulfhydryl, amino, cyano, isocyano, aryl, heteroaryl, a heterocyclic group, a bridged ring group, a spiro ring group, a fused ring group, hydroxyalkyl, =O, carbonyl, aldehyde, carboxylic acid, formate, -$(CH_2)_m$-C(=O)-$R^a$, -O-$(CH_2)_m$-C(=O)-$R^a$, -$(CH_2)_m$-C(=O)-$NR^bR^c$, - $(CH_2)_m$S$(=O)_n$$R^a$, -$(CH_2)_m$-alkenyl-$R^a$, $OR^d$ or -$(CH_2)_m$-alkynyl-$R^a$ (wherein m and n are 0, 1 or 2), arylthio, thiocarbonyl, silyl or -$NR^bR^c$ and the like, wherein $R^b$ and $R^c$ are independently selected from H, hydroxyl, amino, carbonyl, alkyl, alkoxy, cycloalkyl, a heterocyclic group, aryl, heteroaryl, sulfonyl, trifluoromethylsulfonyl. Alternatively, $R^b$ and $R^c$ may form five- or six-membered cycloalkyl or a heterocyclic group. $R^a$ and $R^d$ are each independently selected from aryl, heteroaryl, alkyl, alkoxy, cycloalkyl, a heterocyclic group, carbonyl, an ester group, a bridged ring group, a spiro ring group or a fused ring group. The definition of the fused ring described herein is consistent with this definition.

"Bridged ring" refers to a polycyclic group containing any two carbon atoms that are not directly connected, which group may contain 0 or more double bonds and can be substituted or unsubstituted, and any ring in the fused ring system may contain 0 to 5 heteroatoms or groups selected from N, $S(=O)_n$ or O (wherein n is 1, 1, or 2). The ring atoms contain 5 to 20 atoms, preferably 5 to 14 atoms, further preferably 5 to 12 atoms, and still further preferably 5 to 10 atoms. Non-limiting examples include

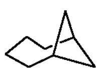, 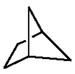, 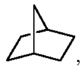, 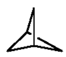, 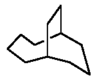, 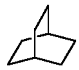, 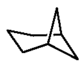, 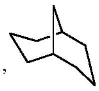, 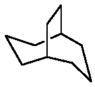, 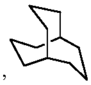,

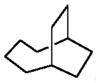, 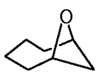, 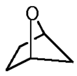, 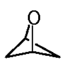, 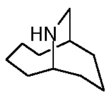, 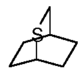, 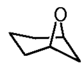, 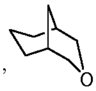, 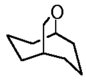, 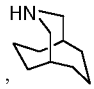,

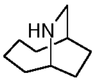, 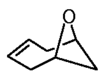, 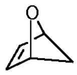, 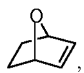, 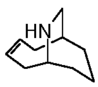, 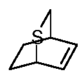, 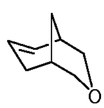, 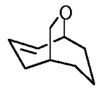, 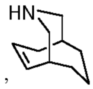,

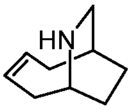, 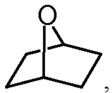, 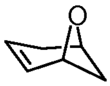, 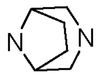, 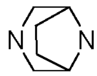, 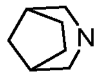, 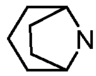, 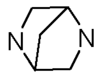, 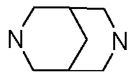,

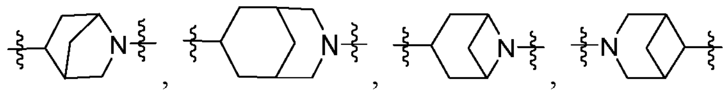, 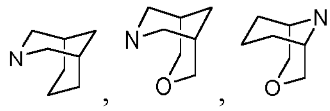

and adamantane. When a substitution occurs, substituents can be 1 to 5 groups selected from F, Cl, Br, I, alkyl, cycloalkyl, alkoxy, haloalkyl, mercaptan, hydroxyl, nitro, sulfhydryl, amino, cyano, isocyano, aryl, heteroaryl, a heterocyclic group, a bridged ring group, a spiro ring group, a fused ring group, hydroxyalkyl, =O, carbonyl, aldehyde, carboxylic acid, formate, $-(CH_2)_m-C(=O)-R^a$, $-O-(CH_2)_m-C(=O)-R^a$, $-(CH_2)_m-C(=O)-NR^bR^c$, $-(CH_2)_mS(=O)_mR^a$, $-(CH_2)_m$-alkenyl-$R^a$, $OR^d$ or $-(CH_2)_m$-alkynyl-$R^a$ (wherein m and n are 0, 1 or 2), arylthio, thiocarbonyl, silyl or $-NR^bR^c$ and the like, wherein $R^b$ and $R^c$ are independently selected from H, hydroxyl, amino, carbonyl, alkyl, alkoxy, cycloalkyl, a heterocyclic group, aryl, heteroaryl, sulfonyl, trifluoromethylsulfonyl. Alternatively, $R^b$ and $R^c$ may form five- or six-membered cycloalkyl or a heterocyclic group. $R^a$ and $R^d$ are each independently selected from aryl, heteroaryl, alkyl, alkoxy, cycloalkyl, a heterocyclic group, carbonyl, an ester group, a bridged ring group, a spiro ring group or a fused ring group. The definition of the bridged ring described herein is consistent with this definition.

"Mono-heterocyclic ring" refers to "heterocyclic group" or "heterocyclic ring" in a monocyclic ring system, and the definition of the mono-heterocyclic ring described herein is consistent with this definition.

"Fused heterocyclic ring" refers to a "fused ring" containing heteroatom(s). The definition of the fused heterocyclic ring described herein is consistent with this definition.

"Spiro-heterocyclic ring" refers to a "spiro ring" containing heteroatom(s). The definition of the spiro-heterocyclic ring described herein is consistent with this definition.

"Bridged-heterocyclic ring" refers to a "bridged ring" containing heteroatom(s). The definition of the bridged-heterocyclic ring described herein is consistent with this definition.

"Heteroaryl" or "heteroaryl ring" refers to a substituted or unsubstituted 5-to 14-membered aromatic ring, and contains 1 to 5 heteroatoms or groups selected from N, O or $S(=O)_n$, preferably 5- to 10-membered aromatic ring, further preferably 5- to 6-membered. Non-limiting examples of heteroaryl include, but are not limited to pyridyl, furanyl, thienyl, pyridinyl, pyranyl, N-alkylpyrrolyl, pyrimidinyl, pyrazinyl, pyridazinyl, imidazolyl, benzimidazole, benzimidazolyl, benzopyridine, pyrrolopyridine and the like. The heteroaryl ring can be fused to aryl, a heterocyclic group or a cycloalkyl ring, wherein the ring connected to the parent structure is a heteroaryl ring, and non-limiting examples include

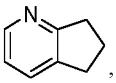, 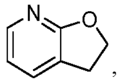, 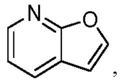, 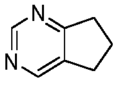, 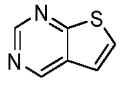, 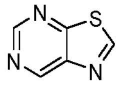, 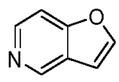

and

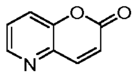.

When a substitution occurs, substituents can be 1 to 5 groups selected from F, Cl, Br, I, alkyl, cycloalkyl, alkoxy, haloalkyl, mercaptan, hydroxyl, nitro, sulfhydryl, amino, cyano, isocyano, aryl, heteroaryl, a heterocyclic group, a bridged ring group, a spiro ring group, a fused ring group, hydroxyalkyl, =O, carbonyl, aldehyde, carboxylic acid, formate, $-(CH_2)_m-C(=O)-R^a$, $-O-(CH_2)_m-C(=O)-R^a$, $-(CH_2)_m-C(=O)-NR^bR^c$, $-(CH_2)_mS(=O)_mR^a$, $-(CH_2)_m$-alkenyl-$R^a$, $OR^d$ or $-(CH_2)_m$-alkynyl-$R^a$ (wherein m and n are 0, 1 or 2), arylthio, thiocarbonyl, silyl or $-NR^bR^c$ and the like, wherein $R^b$ and $R^c$ are independently selected from H, hydroxyl, amino, carbonyl, alkyl, alkoxy, cycloalkyl, a heterocyclic group, aryl, heteroaryl, sulfonyl, trifluoromethylsulfonyl. Alternatively, $R^b$ and $R^c$ may form five- or six-membered cycloalkyl or a heterocyclic group. $R^a$ and $R^d$ are each independently selected from aryl, heteroaryl, alkyl, alkoxy, cycloalkyl, a heterocyclic group, carbonyl, an ester group, a bridged ring group, a spiro ring group or a fused ring group. The definition of the heteroaryl or heteroaryl ring described herein is consistent with this definition.

"Substituted with 0 to X substituents" refers to substituted with 0, 1, 2, 3 ... X substituents, wherein X is selected from any integer between 1 and 10. For example, "substituted with 0 to 4 substituents" refers to substituted with 0, 1, 2, 3 or 4 substituents. For example, "substituted with 0 to 5 substituents" refers to substituted with 0, 1, 2, 3, 4 or 5 substituents. For example, "bridged-heterocyclic ring is optionally further substituted with 0 to 4 substituents selected from H or F" means that the bridged-heterocyclic ring is optionally further substituted with 0, 1, 2, 3 or 4 substituents selected from H or F.

An X- to Y-membered ring (X is an integer, $3 \leq X < Y$, and Y is selected from any integer between 4 and 12) includes X+1-, X+2-, X+3-, X+4-,...to Y-membered rings. Rings include a heterocyclic ring, a carbocyclic ring, an aromatic ring, aryl, heteroaryl, cycloalkyl, a mono-heterocyclic ring, a fused heterocyclic ring, a spiro-heterocyclic ring or a bridged-heterocyclic ring. For example, a "4- to 7-membered mono-heterocyclic ring" refers to a 4-, 5-, 6- or 7-membered mono-heterocyclic ring, and a "5- to 10-membered fused heterocyclic ring" refers to a 5-, 6-, 7-, 8-, 9- or 10-membered fused heterocyclic ring.

The term "optional" or "optionally" refers to that the events or circumstances subsequently described may but not necessarily occur, and the description includes the occasions where the events or circumstances occur or do not occur. For example, "alkyl optionally substituted with F" means that an alkyl may but not necessarily be substituted with F, and the description includes the case where the alkyl is substituted with F and the case where the alkyl is not substituted with F.

"Pharmaceutically acceptable salt" or "pharmaceutically acceptable salt thereof' refers to a salt of the compound of the present invention maintaining the biological effectiveness and characteristics of the free acid or free base, and obtained by reacting the free acid with a non-toxic inorganic base or organic base, and reacting the free base with a non-toxic inorganic acid or organic acid.

"Pharmaceutical composition" refers to a mixture of one or more of the compounds of the present invention, a pharmaceutically acceptable salt or a prodrug thereof, and other chemical components, wherein "other chemical components" refer to pharmaceutically acceptable carriers, excipients and/or one or more other therapeutic agents.

"Carrier" refers to a material that does not cause significant irritation to the organism and does not eliminate the biological activity and characteristics of the administered compound.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

The term "stereoisomer" refers to an isomer produced as a result of different spatial arrangement of atoms in molecules, including cis-trans isomers, enantiomers and conformational isomers.

"$DC_{50}$" refers to the dose at which 50% of the protein is degraded.

"$IC_{50}$" refers to the concentration of a drug or inhibitor required to inhibit half of a given biological process (or a component of the process such as an enzyme, a receptor and a cell).

Detailed Description of Embodiments

The technical solutions of the present invention will be described in detail below in conjunction with examples, but the protection scope of the present invention includes but is not limited thereto.

To achieve the objects of the present invention, according to organic synthesis techniques known to those skilled in the art, and starting from commercially available chemicals and/or compounds described in chemical documents, the preparation of the compounds, "commercially available chemicals", for use in the reactions described herein is obtained from standard commercial sources, including Shanghai Aladdin Bio-Chem Technology Co., Ltd., Shanghai Macklin Biochemical Co., Ltd., Sigma-Aldrich, Alfa Aesar (China) Chemical Co., Ltd., Tokyo Chemical Industry (Shanghai) Co., Ltd., Energy Chemical Co., Ltd., Shanghai Titan Scientific Co., Ltd., Kelong Chemical Co., Ltd., J&K Scientific and the like.

References and monographs in the art introduce in detail the synthesis of reactants that can be used to prepare the compounds described herein, or provide articles describing the preparation method for reference. The references and monographs include: "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley-Interscience, New York, 1992; Fuhrhop, J. and Penzlin G. "Organic Synthesis: Concepts, Methods, Starting Materials", Second, Revised and Enlarged Edition (1994) John Wiley & Sons ISBN: 3 527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups", John Wiley & Sons, in 73 volumes.

Specific and similar reactants can be selectively identified by the indexes of known chemicals prepared by the Chemical Abstracts Service of the American Chemical Society, wherein the indexes are available in most public libraries or university libraries and online. Chemicals that are known but not commercially available in the catalog are optionally prepared by custom chemical synthesis plants, wherein many of standard chemical supply plants (for example, those listed above) provide custom synthesis services. Reference document for the preparation and selection of the pharmaceutically acceptable salts of the compounds described herein is P. H. Stahl & C. G. Wermuth "Handbook of Pharmaceutical Salts", Verlag Helvetica Chimica Acta, Zurich, 2002.

The technical solutions of the present invention will be described in detail by the following examples, but the scope of protection of the present invention includes but is not limited thereto.

The compounds used in the reactions described herein are prepared according to organic synthesis techniques known to those skilled in the art, and starting from commercially available chemicals and(or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific and the like.

The structures of the compounds are determined by nuclear magnetic resonance (NMR) or (and) mass spectrometry (MS). The NMR shift ($\delta$) is given in the unit of $10^{-6}$ (ppm). NMR is measured with (Bruker Avance III 400 and Bruker Avance 300) NMR instrument, and the solvent for determination is deuterated dimethyl sulfoxide (DMSO-$d_6$), deuterated chloroform ($CDCl_3$), deuterated methanol ($CD_3OD$), and the internal standard is tetramethylsilane (TMS);

MS is measured with (Agilent 6120B(ESI) and Agilent 6120B(APCI));
HPLC is determined with Agilent 1260DAD high pressure liquid chromatography (Zorbax SB-C18 100 $\times$ 4.6 mm, 3.5 $\mu$M);
Yantai Huanghai HSGF254 or Qingdao GF254 silica gel plate is used as a thin layer chromatography silica plate; the specification of the silica gel plate used for thin layer chromatography (TLC) is 0.15 mm-0.20 mm; and the specification when separating and purifying a product by thin layer chromatography is 0.4 mm - 0.5 mm.

For the column chromatography, Yantai Huanghai silica gel of 200-300 mesh silica gel is generally used as a carrier.

DMSO: dimethyl sulfoxide, DIPEA: N,N'-diisopropylethylamine, DCE: dichloroethane, DCM: dichloromethane, DIPEA: N,N-diisopropylethylamine.

**Synthesis of intermediate A:**

tert-butyl 4-(1H-pyrazol-4-yl)piperidine-1-carboxylate **(intermediate A)**

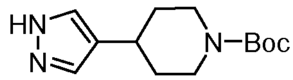

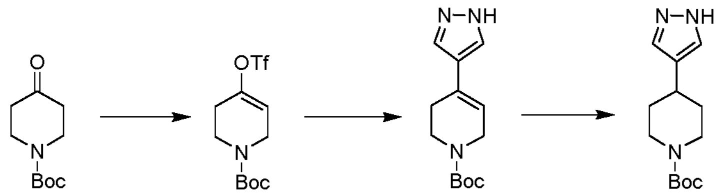

A1 A2 A3 Intermediate A

Step 1: tert-butyl 4-(trifluoromethylsulfonyloxy)-3,6-dihydro-2H-pyridine-1-carboxylate (A2)

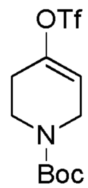

N-Boc-4-piperidone (A1) (5.21 g, 26.1 mmol) was dissolved in 26 mL of tetrahydrofuran, and then the mixture was cooled to -78°C under nitrogen atmosphere. A solution of lithium bis(trimethylsilyl)amide (1 mol/L) in tetrahydrofuran (28.5 mL) was slowly added dropwise, and after the addition was completed, the reaction was stirred at -78°C for 1 h. A solution of *N* phenylbis(trifluoromethanesulfonyl)imide (10.2 g, 28.6 mmol) in tetrahydrofuran (26 mL) was added dropwise, and after the addition was completed, the reaction solution was naturally warmed to room temperature and reacted for 3 h. The reaction was quenched by dropwise adding 20 mL of saturated sodium bicarbonate solution, and 50 mL of ethyl acetate was added. The liquid separation was conducted, and the organic layer was washed with 20 mL of saturated sodium chloride solution, dried over anhydrous sodium sulfate and concentrated under reduced pressure to obtain a crude product, which was separated and purified by silica gel column chromatography (petroleum ether/ethyl acetate (v/v) = 100/0-9/1) to obtain tert-butyl 4-(trifluoromethylsulfonyloxy)-3,6-dihydro-2H-pyridine-1-carboxylate (A2) (7.80 g, yield: 90%).

Step 2: tert-butyl 4-(1H-pyrazol-4-yl)-3,6-dihydro-2H-pyridine-1-carboxylate (A3)

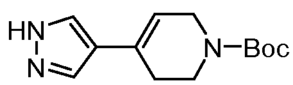

Tert-butyl 4-(trifluoromethylsulfonyloxy)-3,6-dihydro-2H-pyridine-1-carboxylate (A2) (1.00 g, 3.02 mmol) was dissolved in 5 mL of acetonitrile. 4-(4,4,5,5-tetramethyl-1,3,2-dioxaborolan-2-yl)-1H-pyrazole (0.761 g, 3.92 mmol), 5 mL of saturated sodium bicarbonate aqueous solution and $Pd(dppf)Cl_2 \cdot CH_2Cl_2$ (122 mg, 0.16 mmol) were successively added, and the mixture was reacted at 110°C for 30 min under a microwave condition. The reaction was cooled to room temperature, and 20 mL of water was added. The mixture was extracted with ethyl acetate (20 mL × 2). The organic

layers were combined, washed with 20 mL of saturated sodium chloride solution, dried over anhydrous sodium sulfate and concentrated under reduced pressure to obtain a crude product, which was separated and purified by silica gel column chromatography (petroleum ether/ethyl acetate (v/v) = 4/1-1/1) to obtain tert-butyl 4-(1H-pyrazol-4-yl)-3,6-dihydro-2H-pyridine-1-carboxylate (A3) (0.420 g, yield: 56%).
$^1$H NMR (400 MHz, $CDCl_3$) δ 7.61 (s, 2H), 5.92 (s, 1H), 4.07 - 4.00 (m, 2H), 3.62 (t, 2H), 2.46 - 2.37 (m, 2H), 1.48 (s, 9H).
LCMS m/z = 250.3 $[M+1]^+$

Step 3: tert-butyl 4-(1H-pyrazol-4-yl)piperidine-1-carboxylate (intermediate A)

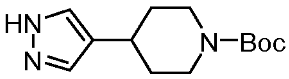

Tert-butyl 4-(1H-pyrazol-4-yl)-3,6-dihydro-2H-pyridine-1-carboxylate (A3) (0.400 g, 1.60 mmol) was dissolved in 10 mL of anhydrous ethanol. 200 mg of 10% palladium on carbon was added, and the mixture was subjected to hydrogen replacement three times and reacted at 40°C for 5 h under hydrogen atmosphere. The reaction solution was filtered with celite, and the filtrate was concentrated under reduced pressure to obtain tert-butyl 4-(1H-pyrazol-4-yl)piperidine-1-carboxylate (intermediate A) (0.4 g, yield: > 99%).
$^1$H NMR (400 MHz, $CDCl_3$) δ 8.94 (s, 1H), 7.43 (s, 2H), 4.30 - 4.00 (m, 2H), 2.83 (t, 2H), 2.74 - 2.63 (m, 1H), 1.96 - 1.84 (m, 2H), 1.60 - 1.39 (m, 11H).

**Synthesis of intermediate B:**

tert-butyl 3-ethynyl-[1,3'-biazetidine]-1'-carboxylate (intermediate B)

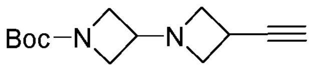

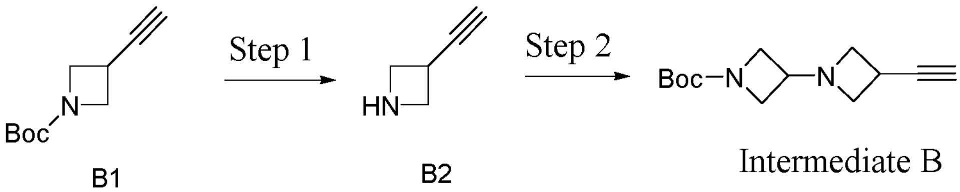


B1 B2 Intermediate B

Step 1: 3-ethynylazetidine (B2) hydrochloride

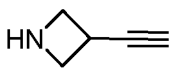

Tert-butyl 3-ethynylazetidine-carboxylate (B1) (2.77 g, 15.28 mmol) was added to a 250 mL single-necked round-bottom flask containing 40 mL of 3 mol/L ethyl acetate hydrochloride solution, and the mixture was stirred at room temperature for 4 h. The reaction solution was concentrated under reduced pressure to obtain crude 3-ethynylazetidine (B2) hydrochloride (1.74 g).
LCMS m/z = 82.2 $[M+1]^+$

Step 2: tert-butyl 3-ethynyl-[1,3'-biazetidine]-1'-carboxylate (intermediate B)

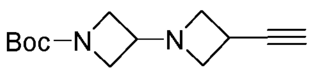

The crude 3-ethynylazetidine (B2) hydrochloride (1.74 g) was weighed into a 250 mL single-necked round-bottom flask and dissolved in 100 mL of 1,2-dichloroethane. 3 mL of triethylamine was added, and then tert-butyl 3-oxoazetidine-1-carboxylate (6.33 g, 37.00 mmol), acetic acid (3.11 g, 51.80 mmol) and 6 g of anhydrous sodium sulfate were successively added. The mixture was heated to 60°C and reacted for 2 h. The reaction solution was cooled to room temperature, sodium triacetoxyborohydride (18.82 g, 88.79 mmol) was added in portions, and the resulting solution was reacted at room temperature for 16 h. After the reaction was completed, 50 mL of water was added to the reaction solution, and the liquid separation was conducted. The organic phase was washed successively once with 50 mL of saturated sodium bicarbonate solution, 50 mL of water and 50 mL of saturated brine, dried over anhydrous sodium sulfate, filtered and concentrated under reduced pressure. The crude product was separated and purified by silica gel column chromatography (petroleum ether/ethyl acetate (v/v) = 4/1) to obtain tert-butyl 3-ethynyl-[1,3'-biazetidine]-1'-carboxylate (intermediate B) (3.33 g, two-step yield calculated from compound B 1: 92%). LCMS m/z = 237.2 $[M+1]^+$

**Synthesis of intermediate 1:**

N-(4-cyano-3-(trifluoromethyl)phenyl)-2-methyl-2-(4-(piperidin-4-yl)-1H-pyrazol-1-yl)propanamide **(intermediate 1)**

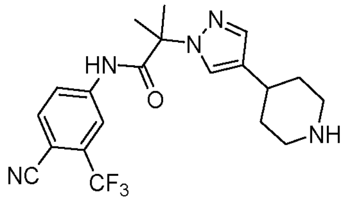

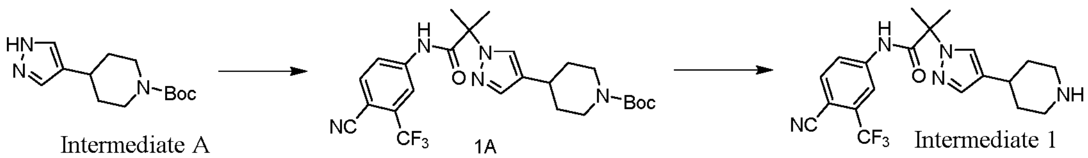

Intermediate A

1A

Intermediate 1

Step 1: tert-butyl 4-(1-(1-((4-cyano-3-(trifluoromethyl)phenyl)amino)-2-methyl-1-oxopropan-2-yl)-1H-pyrazol-4-yl)piperidine-1-carboxylate (1A)

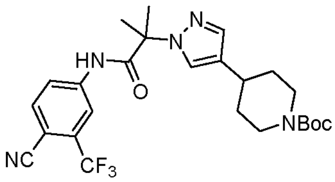

2-bromo-N-(4-cyano-3-(trifluoromethyl)phenyl)-2-methylpropanamide (see WO 2020063407 for the synthetic method) (4.0 g, 11.9 mmol) was dissolved in 50 mL of acetonitrile. Tert-butyl 4-(1H-pyrazol-4-yl)piperidine-1-carboxylate (intermediate A)(2.5 g, 9.9 mmol) and cesium carbonate (6.5 g, 19.9 mmol) were added, and the reaction was stirred at 50°C for 1 h. The reaction solution was concentrated under reduced pressure, diluted with 100 mL of ethyl acetate, washed with 100 mL of water, dried over anhydrous sodium sulfate and concentrated under reduced pressure. The crude product was separated and purified by silica gel column chromatography (petroleum ether/ethyl acetate (v/v) = 2 : 1) to obtain tert-butyl 4-(1-(1-((4-cyano-3-(trifluoromethyl)phenyl)amino)-2-methyl-1-oxopropan-2-yl)-1H-pyrazol-4-yl)piperidine-1-carboxylate (1A) (3.7 g, yield: 74%).

Step 2: N-(4-cyano-3-(trifluoromethyl)phenyl)-2-methyl-2-(4-(piperidin-4-yl)-1H-pyrazol-1-yl)propanamide (intermediate 1)

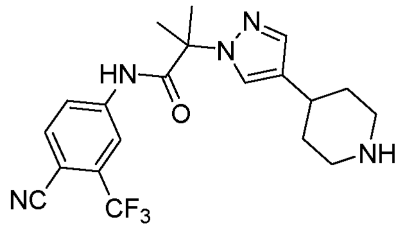

Tert-butyl 4-(1-(1-((4-cyano-3-(trifluoromethyl)phenyl)amino)-2-methyl-1-oxopropan-2-yl)-1H-pyrazol-4-yl)piperidine-1-carboxylate (1A) (3.7 g, 7.3 mmol) was dissolved in 50 mL of DCM. 15 mL of trifluoroacetic acid was added, and the mixture was stirred at room temperature for 3 h. The reaction solution was concentrated under reduced pressure and diluted with 50 mL of dichloromethane. The pH was adjusted to 9.0 with saturated sodium bicarbonate solution. The organic phase was separated, washed with 50 mL of water, dried over anhydrous sodium sulfate and concentrated under reduced pressure to obtain N-(4-cyano-3-(trifluoromethyl)phenyl)-2-methyl-2-(4-(piperidin-4-yl)-1H-pyrazol-1-yl)propanamide (intermediate 1) (2.9 g, yield: 98%). LCMS m/z = 406.3 $[M+1]^+$

**Synthesis of intermediate 2:**

N-(4-cyano-3-(trifluoromethyl)phenyl)-1-(4-(piperidin-4-yl)-1H-pyrazol-1-yl)cyclobutane-1-carboxamide **(intermediate 2)**

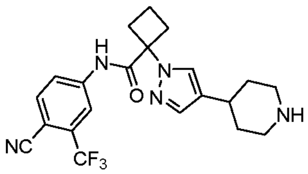

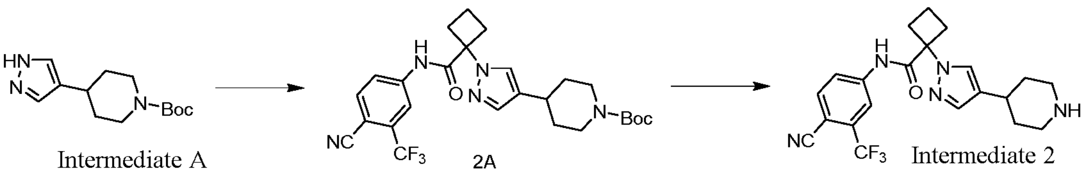


Step 1: tert-butyl 4-[1-[1-[[4-cyano-3-(trifluoromethyl)phenyl]carbamoyl]cyclobutyl]pyrazol-4-yl] piperidine-1-carboxylate (2A)

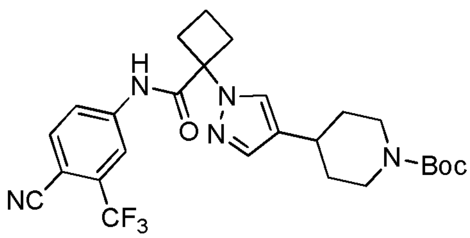

Tert-butyl 4-(1H-pyrazol-4-yl)piperidine-1-carboxylate (intermediate A) (0.145g, 0.576 mmol) was dissolved in 5 mL of acetonitrile. 1-bromo-N-(4-cyano-3-(trifluoromethyl)phenyl)cyclobutane-1-carboxamide (see WO 2020063407 for the synthetic method) (0.200 g, 0.576 mmol) and cesium carbonate (0.207 g, 0.635 mmol) were added successively, and the reaction was stirred at 80°C for 3 h. The reaction solution was cooled to room temperature, and 20 mL of ethyl acetate and 10 mL of water were added. The liquid separation was conducted. The organic layer was dried over anhydrous sodium sulfate and concentrated under reduced pressure to obtain a crude product, which was separated and purified

by silica gel column chromatography (petroleum ether/ethyl acetate (v/v) = 4 : 1) to obtain tert-butyl 4-[1-[1-[[4-cyano-3-(trifluoromethyl)phenyl]carbamoyl]cyclobutyl]pyrazol-4-yl] piperidine-1-carboxylate (2A) (0.060 g, yield: 20%).
$^1$H NMR (400 MHz, $CDCl_3$) δ 9.14 (s, 1H), 8.00 - 7.96 (m, 1H), 7.81 - 7.70 (m, 2H), 7.60 (s, 1H), 7.39 (s, 1H), 4.23 - 4.12 (m, 2H), 3.10 - 2.99 (m, 2H), 2.87 - 2.61 (m, 5H), 2.27 - 2.14 (m, 1H), 2.14 - 2.04 (m, 1H), 1.94 - 1.85 (m, 2H), 1.58 - 1.40 (m, 11H).

Step 2: N-(4-cyano-3-(trifluoromethyl)phenyl)-1-(4-(piperidin-4-yl)-1H-pyrazol-1-yl)cyclobutane-1-carboxamide (intermediate 2)

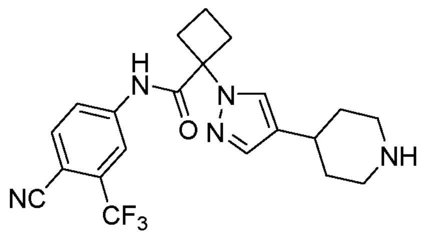

Tert-butyl 4-[1-[1-[[4-cyano-3-(trifluoromethyl)phenyl]carbamoyl]cyclobutyl]pyrazol-4-yl] piperidine-1-carboxylate (2A) (1.50 g, 2.90 mmol) was dissolved in 10 mL of dichloromethane. 4 mL of trifluoroacetic acid was added, and the mixture was reacted at room temperature for 20 min. The reaction solution was concentrated under reduced pressure, 50 mL of dichloromethane was added to the residue, the pH was adjusted to 9 with saturated sodium bicarbonate solution, and the liquid separation was conducted. The organic layer was dried over anhydrous sodium sulfate and concentrated under reduced pressure to obtain N-(4-cyano-3-(trifluoromethyl)phenyl)-1-(4-(piperidin-4-yl)-1H-pyrazol-1-yl)cyclobutane-1-carboxamide (intermediate 2) (1.20 g, yield: > 99%).
LCMS m/z = 418.2 $[M+1]^+$

**Example 1:**

N-(4-cyano-3-(trifluoromethyl)phenyl)-2-(4-(1-((3aR,6aS)-2-(2-(2,6-dioxopiperidin-3-yl)-1,3-dioxoisoindolin-5-yl)octahydrocyclopenta[c]pyrrol-5-yl)piperidin-4-yl)-1H-pyrazol-1-yl)-2-methylpropanamide (compound 1)

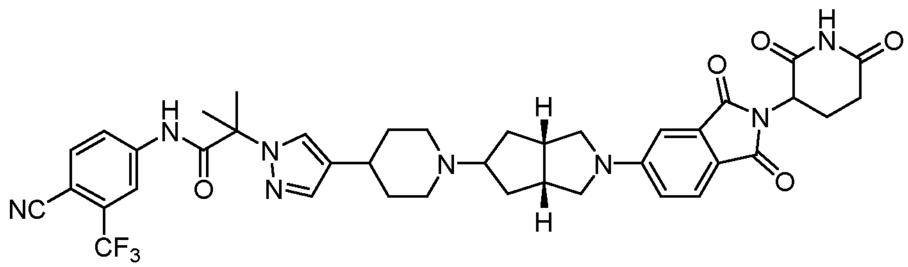

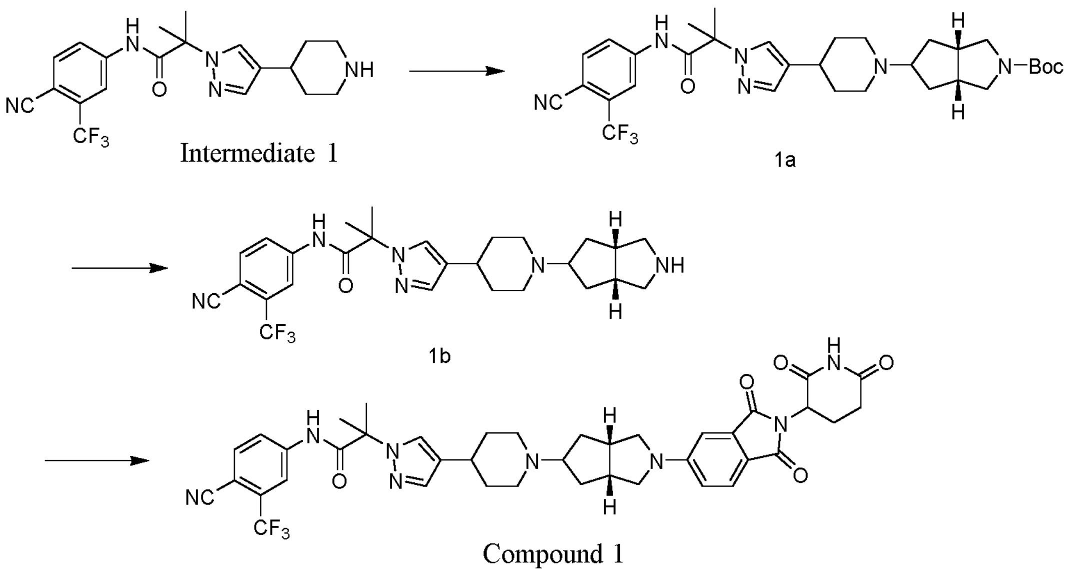

Intermediate 1

1a

1b

Compound 1

Step 1: tert-butyl(3aR,6aS)-5-(4-(1-(1-((4-cyano-3-(trifluoromethyl)phenyl)amino)-2-methyl-1-oxopropan-2-yl)-1H-pyrazol-4-yl)piperidin-1-yl)hexahydrocyclopenta[c]pyrrole-2(1H)-carboxylate (1a)

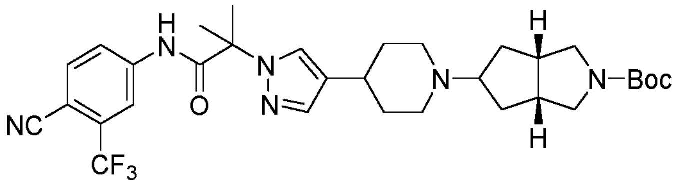

N-(4-cyano-3-(trifluoromethyl)phenyl)-2-methyl-2-(4-(piperidin-4-yl)-1H-pyrazol-1-yl)propanamide (intermediate 1) (202 mg, 0.50 mmol) was dissolved in 15 mL of DCE. (3aR,6aS)-5-oxo hexahydrocyclopenta[c]pyrrole-2(1H)-carboxylate (225 mg, 1.00 mmol) was added, and the mixture was stirred at room temperature for 0.5 h. After that, sodium triacetoxyborohydride (318 mg, 1.50 mmol) was added, and the resulting mixture was stirred at room temperature for 16 h. To the reaction solution, 20 mL of saturated sodium bicarbonate aqueous solution was slowly added, and the mixture was extracted with DCM (40 mL × 3). The organic phase was washed with 50 mL of water, dried over anhydrous sodium sulfate and concentrated under reduced pressure. The crude product was separated and purified by silica gel column chromatography (dichloromethane/methanol (v/v) = 20 : 1) to obtain tert-butyl(3aR,6aS)-5-(4-(1-(1-((4-cyano-3-(trifluoromethyl)phenyl)amino)-2-methyl-1-oxopropan-2-yl)-1H-pyrazol-4-yl)piperidin-1-yl)hexahydrocyclopenta[c]pyrrole-2(1H)-carboxylate (1a) (0.25 g, yield: 81%).
LCMS m/z = 615.4 $[M+1]^+$

Step 2: N-(4-cyano-3-(trifluoromethyl)phenyl)-2-methyl-2-(4-(1-((3aR,6aS)-octahydrocyclopenta[c]pyrrol-5-yl)piperidin-4-yl)-1H-pyrazol-1-yl)propanamide (1b)

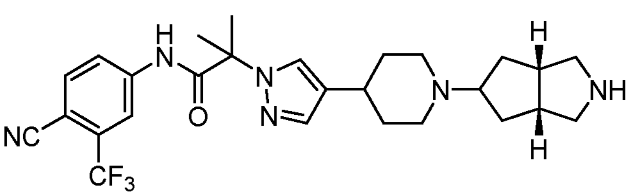

Tert-butyl(3aR,6aS)-5-(4-(1-(1-((4-cyano-3-(trifluoromethyl)phenyl)amino)-2-methyl-1-oxopropan-2-yl)-1H-pyrazol-4-yl)piperidin-1-yl)hexahydrocyclopenta[c]pyrrole-2(1H)-carboxylate (1a) (0.25 g, 0.41 mmol) was dissolved in

10 mL of DCM. 6 mL of trifluoroacetic acid was added, and the mixture was stirred at room temperature for 3 h. After the reaction was completed, the reaction system was concentrated under reduced pressure, and the residue was dissolved in 30 mL of 4 mol/L aqueous NaOH. The reaction solution was extracted with DCM (40 mL × 3). The organic phase was washed with 50 mL of water, dried over anhydrous sodium sulfate and concentrated under reduced pressure to obtain crude N-(4-cyano-3-(trifluoromethyl)phenyl)-2-methyl-2-(4-(1-((3aR,6aS)-octahydrocyclopenta[c]pyrrol-5-yl)piperidin-4-yl)-1H-pyrazol-1-yl)propanamide (1b) (0.18 g).
LCMS m/z = 515.3 $[M+1]^+$

Step 3: N-(4-cyano-3-(trifluoromethyl)phenyl)-2-(4-(1-((3aR,6aS)-2-(2-(2,6-dioxopiperidin-3-yl)-1,3-dioxoisoindolin-5-yl)octahydrocyclopenta[c]pyrrol-5-yl)piperidin-4-yl)-1H-pyrazol-1-yl)-2-methylpropanamide (compound 1)

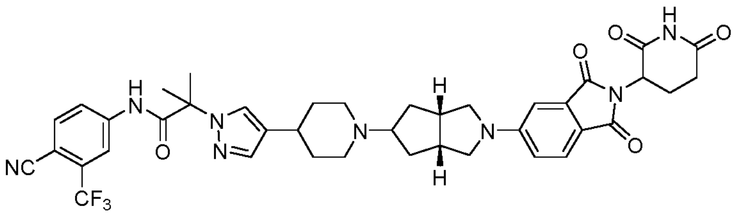

The crude N-(4-cyano-3-(trifluoromethyl)phenyl)-2-methyl-2-(4-(1-((3 aR, 6aS)-octahydrocyclopenta[c]pyrrol-5-yl)piperidin-4-yl)-1H-pyrazol-1-yl)propanamide (1b) (0.18 g) was dissolved in 10 mL of DMSO. 0.5 mL of DIPEA and 2-(2,6-dioxopiperidin-3-yl)-5-fluoroisoindoline-1,3-dione (see WO 2017197056 for the synthetic method) (145 mg, 0.53 mmol) were added, and the mixture was stirred at 80°C for 5 h. The reaction solution was cooled to room temperature, and 20 mL of water was added. The mixture was filtered to collect a solid, which was washed with 10 mL of water, dissolved with 50 mL of dichloromethane, dried over anhydrous sodium sulfate and concentrated under reduced pressure, and then the crude product was separated and purified by silica gel column chromatography (dichloromethane/methanol (v/v) = 15 : 1) to obtain N-(4-cyano-3-(trifluoromethyl)phenyl)-2-(4-(1-((3aR,6aS)-2-(2-(2,6-dioxopiperidin-3-yl)-1,3-dioxoisoindolin-5-yl)octahydrocyclopenta[c]pyrrol-5-yl)piperidin-4-yl)-1H-pyrazol-1-yl)-2-methylpropanamide (compound 1) (140 mg, two-step yield calculated from compound 1a: 44%).
$^1$H NMR (400 MHz, $CDCl_3$) δ 9.78 (s, 1H), 8.57 (br.s, 1H), 7.99 - 7.90 (m, 1H), 7.80 - 7.68 (m, 2H), 7.64 (d, 1H), 7.60 (s, 1H), 7.45 (s, 1H), 7.00 - 6.95 (m, 1H), 6.69 (dd, 1H), 4.92 (dd, 1H), 3.66 - 3.52 (m, 2H), 3.44 - 3.32 (m, 2H), 3.19 - 3.03 (m, 2H), 2.92 - 2.63 (m, 6H), 2.59 - 2.45 (m, 1H), 2.35 - 2.21 (m, 2H), 2.18 - 2.01 (m, 3H), 1.97 - 1.85 (m, 8H), 1.84 - 1.62 (m, 2H), 1.60 - 1.47 (m, 2H).
LCMS m/z = 771.3 $[M+1]^+$

**Example 2:**

N-(4-cyano-3-(trifluoromethyl)phenyl)-2-(4-(1-((1-(2-(2,6-dioxopiperidin-3-yl)-1,3-dioxoisoindolin-5-yl)pyrrolidin-3-yl)methyl)piperidin-4-yl)-1H-pyrazol-1-yl)-2-methylpropanamide **(compound 2)**

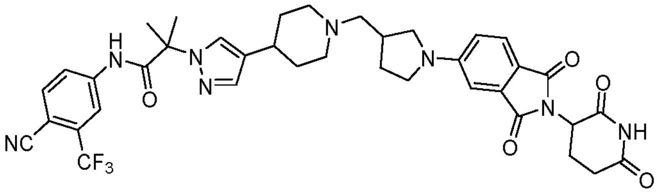

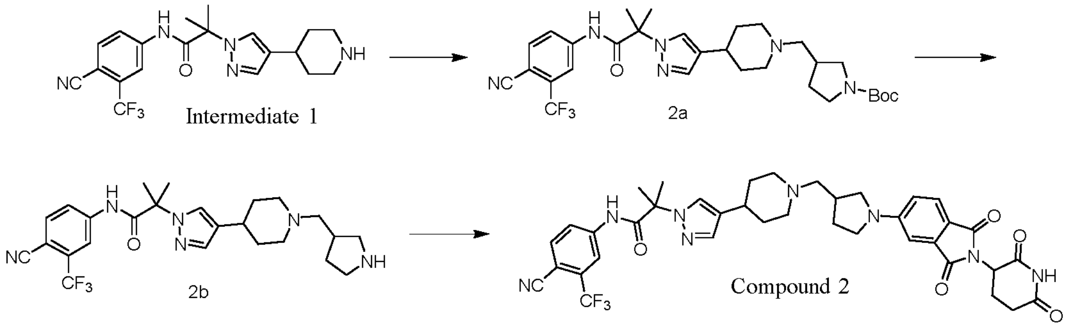

Intermediate 1 2a 2b Compound 2

Step 1: tert-butyl 3-((4-(1-(1-((4-cyano-3-(trifluoromethyl)phenyl)amino)-2-methyl-1-oxopropan-2-yl)-1H-pyrazol-4-yl)piperidin-1-yl)methyl)pyrrolidine-1-carboxylate (2a)

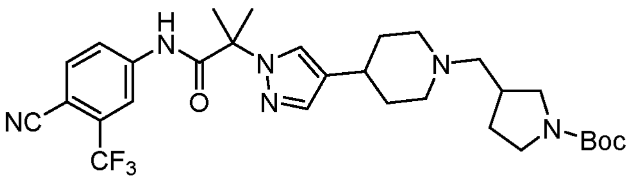

N-(4-cyano-3-(trifluoromethyl)phenyl)-2-methyl-2-(4-(piperidin-4-yl)-1H-pyrazol-1-yl)propanamide (intermediate 1) (202 mg, 0.50 mmol) was dissolved in 15 mL of DCE. Tert-butyl 3-formylpyrrole-1-carboxylate (200 mg, 1.00 mmol) was added, and the mixture was stirred at room temperature for 0.5 h. After that, sodium triacetoxyborohydride (318 mg, 1.5 mmol) was added, and the resulting mixture was stirred at room temperature for 16 h. To the reaction solution, 20 mL of saturated sodium bicarbonate aqueous solution was slowly added, and the mixture was extracted with DCM (40 mL × 3). The organic phase was washed with 50 mL of water, dried over anhydrous sodium sulfate and concentrated under reduced pressure. The crude product was separated and purified by silica gel column chromatography (dichloromethane/methanol (v/v) = 20 : 1) to obtain tert-butyl 3-((4-(1-(1-((4-cyano-3-(trifluoromethyl)phenyl)amino)-2-methyl-1-oxopropan-2-yl)-1H-pyrazol-4-yl)piperidin-1-yl)methyl)pyrrolidine-1-carboxylate (2a) (260 g, yield: 88%).
LCMS m/z = 589.2 $[M+1]^+$

Step 2: N-(4-cyano-3-(trifluoromethyl)phenyl)-2-methyl-2-(4-(1-(pyrrolidin-3-ylmethyl)piperidin-4-yl)-1H-pyrazol-1-yl)propanamide (2b)

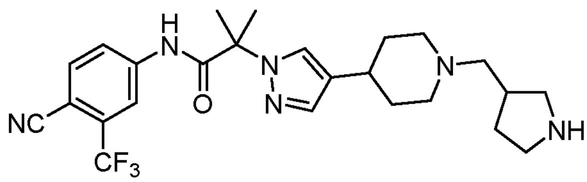

Tert-butyl 3-((4-(1-(1-((4-cyano-3-(trifluoromethyl)phenyl)amino)-2-methyl-1-oxopropan-2-yl)-1H-pyrazol-4-yl)piperidin-1-yl)methyl)pyrrolidine-1-carboxylate (2a) (0.26 g, 0.44 mmol) was dissolved in 10 mL of DCM. 6 mL of trifluoroacetic acid was added, and the mixture was stirred at room temperature for 3 h. After the reaction was completed, the reaction system was directly subjected to rotary evaporation, and the residue was dissolved in 30 mL of 4 mol/L aqueous NaOH. The reaction solution was extracted with DCM (40 mL × 3). The organic phase was washed with 50 mL of water, dried over anhydrous sodium sulfate and concentrated under reduced pressure to obtain crude N-(4-cyano-3-(trifluoromethyl)phenyl)-2-methyl-2-(4-(1-(pyrrolidin-3-ylmethyl)piperidin-4-yl)-1H-pyrazol-1-yl)propanamide (2b) (0.20 g).
LCMS m/z = 489.2 $[M+1]^+$

Step 3: N-(4-cyano-3-(trifluoromethyl)phenyl)-2-(4-(1-((1-(2-(2,6-dioxopiperidin-3-yl)-1,3-dioxoisoindolin-5-yl)pyrrolidin-3-yl)methyl)piperidin-4-yl)-1H-pyrazol-1-yl)-2-methylpropanamide (compound 2)

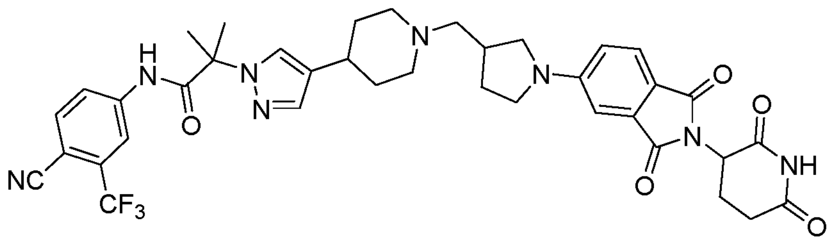

The crude N-(4-cyano-3-(trifluoromethyl)phenyl)-2-methyl-2-(4-(1-(pyrrolidin-3-ylmethyl)piperidin-4-yl)-1H-pyrazol-1-yl)propanamide (2b) (0.18 g) was dissolved in 10 mL of DMSO. 0.5 mL of DIPEA and 2-(2,6-dioxopiperidin-3-yl)-5-fluoroisoindoline-1,3-dione (see WO 2017197056 for the synthetic method) (153 mg, 0.55 mmol) were added, and the mixture was stirred at 80°C for 5 h. The reaction solution was cooled to room temperature, and 20 mL of water was added. The mixture was filtered to collect a solid, which was washed with 10 mL of water, dissolved with 50 mL of dichloromethane, dried over anhydrous sodium sulfate and concentrated under reduced pressure, and then the crude product was separated and purified by silica gel column chromatography (dichloromethane/methanol (v/v) = 15 : 1) to obtain N-(4-cyano-3-(trifluoromethyl)phenyl)-2-(4-(1-((1-(2-(2,6-dioxopiperidin-3-yl)-1,3-dioxoisoindolin-5-yl)pyrrolidin-3-yl)methyl)piperidin-4-yl)-1H-pyrazol-1-yl)-2-methylpropanamide (compound 2) (150 mg, two-step yield calculated from compound 2a: 51%).

$^1$H NMR (400 MHz, $CDCl_3$) $\delta$ 9.79 (s, 1H), 8.22 (s, 1H), 7.99 - 7.93 (m, 1H), 7.81 - 7.69 (m, 2H), 7.68 - 7.60 (m, 2H), 7.48 (s, 1H), 6.95 (d, 1H), 6.68 (dd, 1H), 4.93 (dd, 1H), 3.65 - 3.35 (m, 3H), 3.30 - 3.15 (m, 1H), 3.12 - 2.59 (m, 6H), 2.59 - 2.32 (m, 3H), 2.30 - 2.05 (m, 3H), 1.98 - 1.80 (m, 9H), 1.76 - 1.52 (m, 3H).

LCMS m/z = 745.3 $[M+1]^+$

**Example 3:**

N-(4-cyano-3-(trifluoromethyl)phenyl)-2-(4-(1-(2-(2-(2,6-dioxopiperidin-3-yl)-1,3-dioxoisoindolin-5-yl)-2-azaspiro[3.5]nonan-7-yl)piperidin-4-yl)-1H-pyrazol-1-yl)-2-methylpropanamide (**compound 3**)

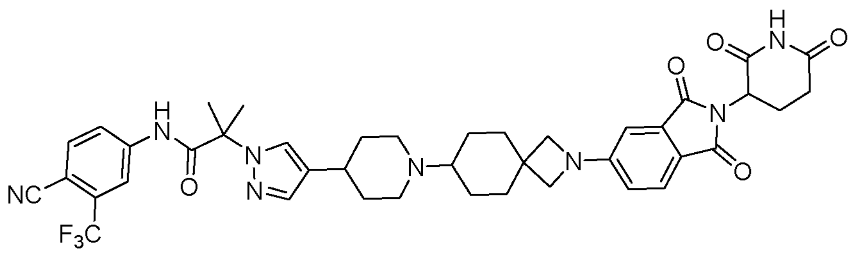

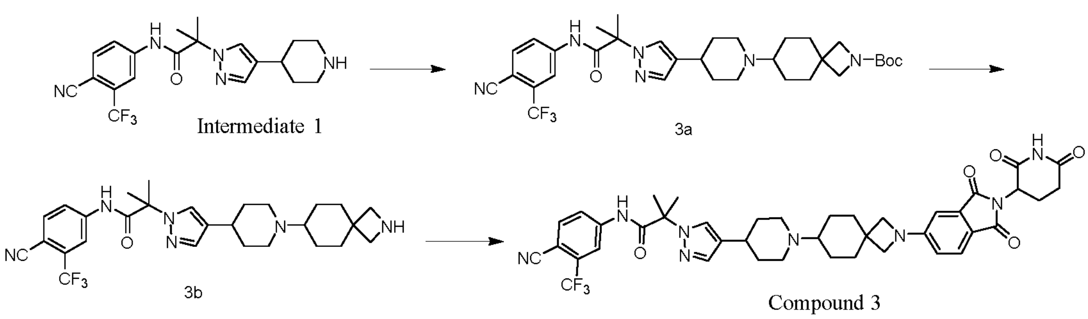
Intermediate 1
3a
3b
Compound 3

Step 1: tert-butyl 7-(4-(1-(1-((4-cyano-3-(trifluoromethyl)phenyl)amino)-2-methyl-1-oxopropan-2-yl)-1H-pyrazol-4-yl)piperidin-1-yl)-2-azaspiro[3.5]nonane-2-carboxylate (3a)

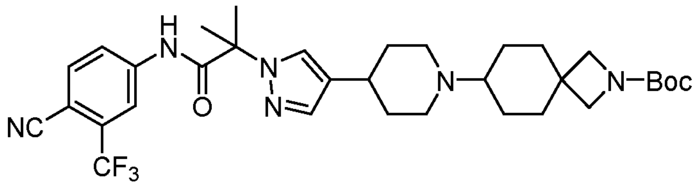

N-(4-cyano-3-(trifluoromethyl)phenyl)-2-methyl-2-(4-(piperidin-4-yl)-1H-pyrazol-1-yl)propanamide (intermediate 1) (202 mg, 0.50 mmol) was dissolved in 15 mL of DCE. 7-oxo-2-azaspiro[3.5]nonane-2-carboxylate (240 mg, 1.00 mmol) was added, and the mixture was stirred at room temperature for 0.5 h. After that, sodium triacetoxyborohydride (318 mg, 1.5 mmol) was added, and the resulting mixture was stirred at room temperature for 16 h. To the reaction solution, 20 mL of saturated sodium bicarbonate aqueous solution was slowly added, and the mixture was extracted with DCM (40 mL × 3). The organic phase was washed with 50 mL of water, dried over anhydrous sodium sulfate and concentrated under reduced pressure. The crude product was separated and purified by silica gel column chromatography (dichloromethane/methanol (v/v) = 20 : 1) to obtain tert-butyl 7-(4-(1-(1-((4-cyano-3-(trifluoromethyl)phenyl)amino)-2-methyl-1-oxopropan-2-yl)-1H-pyrazol-4-yl)piperidin-1-yl)-2-azaspiro[3.5]nonane-2-carboxylate (3a) (200 mg, yield: 64%).
LCMS m/z = 629.4 $[M+1]^+$

Step 2: 2-(4-(1-(2-azaspiro[3.5]nonan-7-yl)piperidin-4-yl)-1H-pyrazol-1-yl)-N-(4-cyano-3-(trifluoromethyl)phenyl)-2-methylpropanamide (3b)

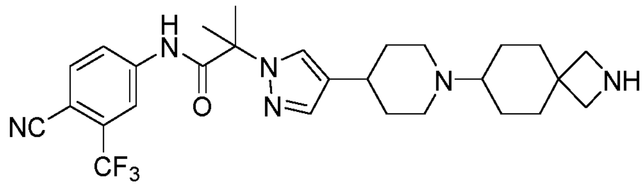

Tert-butyl 7-(4-(1-(1-((4-cyano-3-(trifluoromethyl)phenyl)amino)-2-methyl-1-oxopropan-2-yl)-1H-pyrazol-4-yl)piperidin-1-yl)-2-azaspiro[3.5]nonane-2-carboxylate (3a) (0.20 g, 0.32 mmol) was dissolved in 10 mL of DCM. 6 mL of trifluoroacetic acid was added, and the mixture was stirred at room temperature for 3 h. After the reaction was completed, the reaction system was directly concentrated under reduced pressure, and the residue was dissolved in 30 mL of 4 mol/L aqueous NaOH. The reaction solution was extracted with DCM (40 mL × 3). The organic phase was washed with 50 mL of water, dried over anhydrous sodium sulfate and concentrated under reduced pressure to obtain crude 2-(4-(1-(2-azaspiro[3.5]nonan-7-yl)piperidin-4-yl)-1H-pyrazol-1-yl)-N-(4-cyano-3-(trifluoromethyl)phenyl)-2-methylpropanamide (3b) (0.15 g).
LCMS m/z = 529.3 $[M+1]^+$

Step 3: N-(4-cyano-3-(trifluoromethyl)phenyl)-2-(4-(1-(2-(2-(2,6-dioxopiperidin-3-yl)-1,3-dioxoisoindolin-5-yl)-2-azaspiro[3.5]nonan-7-yl)piperidin-4-yl)-1H-pyrazol-1-yl)-2-methylpropanamide (compound 3)

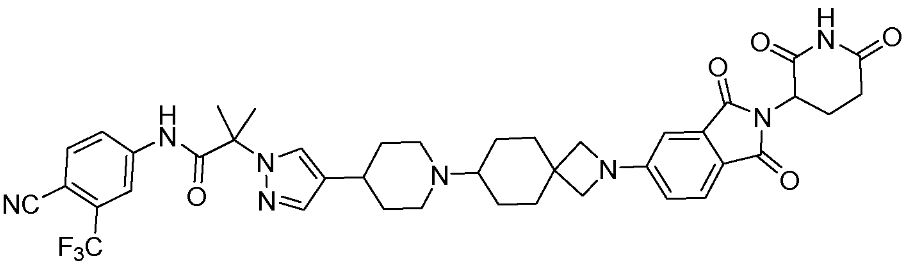

The crude 2-(4-(1-(2-azaspiro[3.5]nonan-7-yl)piperidin-4-yl)-1H-pyrazol-1-yl)-N-(4-cyano-3-(trifluoromethyl)phenyl)-2-methylpropanamide (3b) (0.15 g) was dissolved in 10 mL of DMSO. 0.5 mL of DIPEA and 2-(2,6-dioxopiperidin-3-yl)-5-fluoroisoindoline-1,3-dione (see WO 2017197056 for the synthetic method) (118 mg, 0.43 mmol) were added, and the mixture was stirred at 80°C for 5 h. The reaction solution was cooled to room temperature, and 20 mL of water was added. The mixture was filtered to collect a solid, which was washed with 10 mL of water, dissolved with 50 mL of dichloromethane, dried over anhydrous sodium sulfate and concentrated under reduced pressure, and then the crude product was separated and purified by silica gel column chromatography (dichloromethane/methanol (v/v) = 15 : 1) to obtain N-(4-cyano-3-(trifluoromethyl)phenyl)-2-(4-(1-(2-(2-(2,6-dioxopiperidin-3-yl)-1,3-dioxoisoindolin-5-yl)-2-azaspiro[3. 5]nonan-7-yl)piperidin-4-yl)-1H-pyrazol-1-yl)-2-methylpropanamide (compound 3) (120 mg, two-step yield calculated from compound 3a: 48%).

$^1$H NMR (400 MHz, $CDCl_3$) δ 9.78 (s, 1H), 8.22 (br.s, 1H), 7.97 - 7.93 (m, 1H), 7.81 - 7.69 (m, 2H), 7.66 - 7.59 (m, 2H), 7.46 (s, 1H), 6.76 (d, 1H), 6.49 (dd, 1H), 4.93 (dd, 1H), 3.73 (s, 2H), 3.68 (s, 2H), 3.16 - 2.95 (m, 2H), 2.93 - 2.67 (m, 3H), 2.59 - 2.46 (m, 1H), 2.44 - 2.24 (m, 2H), 2.16 - 2.02 (m, 3H), 2.00 - 1.84 (m, 10H), 1.80 - 1.52 (m, 5H), 1.45 - 1.31 (m, 2H).

LCMS m/z = 785.3 $[M+1]^+$

**Example 4:**

N-[4-cyano-3-(trifluoromethyl)phenyl]-1-[4-[1-[[1-[2-(2,6-dioxo-3-piperidyl)-1,3-dioxo-isoindolin-5-yl]pyrrolidin-3-yl]methyl]-4-piperidyl]pyrazol-1-yl]cyclobutanecarboxamide (**compound 4**)

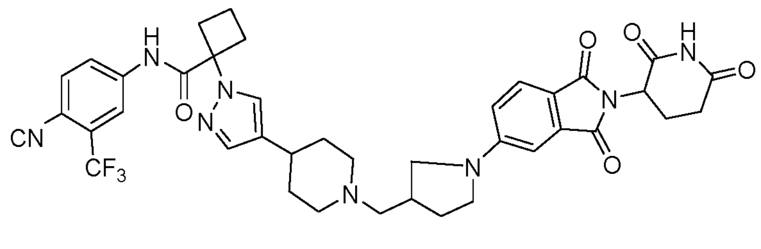

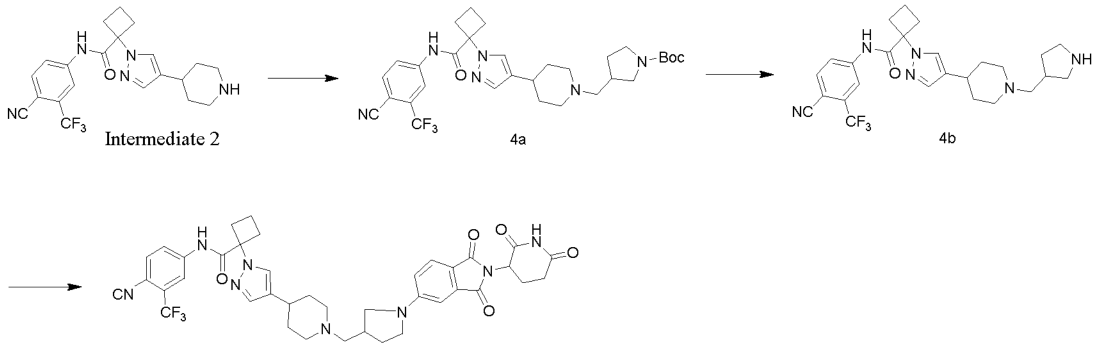

Intermediate 2

4a

4b

Compound 4

Step 1: tert-butyl 3-[[[4-[1-[1-[[4-cyano-3-(trifluoromethyl)phenyl]carbamoyl]cyclobutyl]pyrazol-4-yl]-1-piperidyl]methyl]pyrrolidine-1-carboxylate (4a)

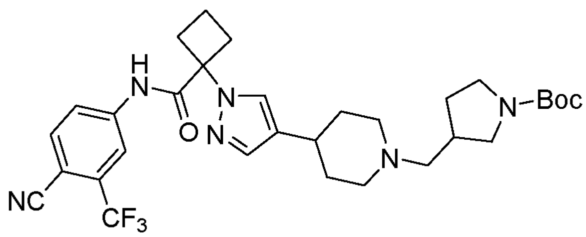

N-(4-cyano-3-(trifluoromethyl)phenyl)-1-(4-(piperidin-4-yl)-1H-pyrazol-1-yl)cyclobutane-1-carboxamide (intermediate 2) (0.100 g, 0.240 mmol) was dissolved in 3 mL of 1,2-dichloroethane. N-Boc-3-pyrrolecarboxaldehyde (0.0955 g, 0.479 mmol), glacial acetic acid (0.036 g, 0.599 mmol) and sodium triacetoxyborohydride (0.102 g, 0.481 mmol) were added successively. After the addition was completed, the mixture was reacted at room temperature for 16 h. The pH was adjusted to 9 by adding dropwise saturated sodium bicarbonate solution. The liquid separation was conducted, the aqueous layer was extracted with 20 mL of dichloromethane, and the organic layers were combined, dried over anhydrous sodium sulfate and concentrated under reduced pressure to obtain a crude product, which was separated and purified by silica gel column chromatography (DCM/MeOH (v/v) = 100/0-97/3) to obtain tert-butyl 3-[[[4-[1-[1-[[4-cyano-3-(trifluoromethyl)phenyl]carbamoyl]cyclobutyl]pyrazol-4-yl]-1-piperidyl]methyl]pyrrolidine-1-carboxylate (4a) (0.08 g, yield: 56%).
LCMS m/z = 601.3 $[M+1]^+$

Step 2: N-[4-cyano-3-(trifluoromethyl)phenyl]-1-[4-[1-(pyrrolidin-3-ylmethyl)-4-piperidyl]pyrazol-1-yl]cyclobutanecarboxamide (4b)

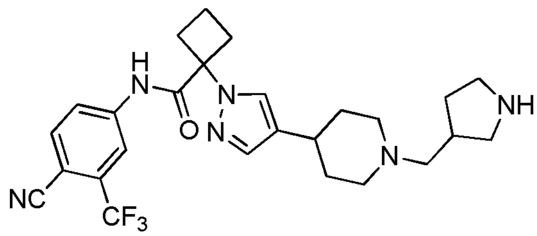

Tert-butyl 3-[[[4-[1-[1-[[4-cyano-3-(trifluoromethyl)phenyl]carbamoyl]cyclobutyl]pyrazol-4-yl]-1-piperidyl]methyl]pyrrolidine-1-carboxylate (4a) (0.08 g, 0.133 mmol) was dissolved in 2 mL of dichloromethane. 1 mL of trifluoroacetic acid was added, and the mixture was reacted at room temperature for 1 h. The reaction solution was concentrated under reduced pressure. 20 mL of dichloromethane was added to the residue, and the pH was adjusted to 9 with saturated sodium bicarbonate. The liquid separation was conducted, the aqueous layer was extracted with 10 mL of dichloromethane, and the organic layers were combined, dried over anhydrous sodium sulfate and concentrated under reduced pressure to obtain crude N-[4-cyano-3-(trifluoromethyl)phenyl]-1-[4-[1-(pyrrolidin-3-ylmethyl)-4-piperidyl]pyrazol-1-yl]cyclobutanecarboxamide (4b) (0.0667 g).
LCMS m/z = 501.3 $[M+1]^+$

Step 3: N-[4-cyano-3-(trifluoromethyl)phenyl]-1-[4-[1-[[1-[2-(2,6-dioxo-3-piperidyl)-1,3-dioxo-isoindolin-5-yl]pyrrolidin-3-yl]methyl]-4-piperidyl]pyrazol-1-yl]cyclobutanecarboxamide (compound 4)

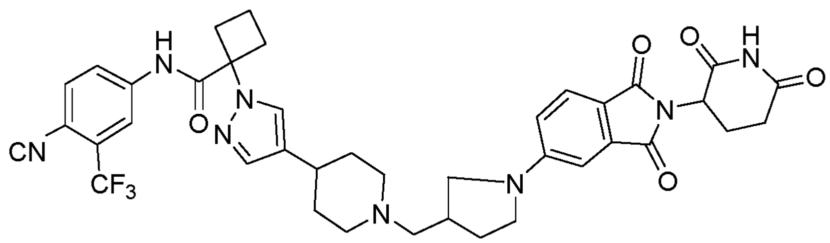

The crude N-[4-cyano-3-(trifluoromethyl)phenyl]-1-[4-[1-(pyrrolidin-3-ylmethyl)-4-piperidyl]pyrazol-1-yl]cyclobutanecarboxamide (4b) (0.060 g, 0.12 mmol) was dissolved in 3 mL of dimethylsulfoxide. 2-(2,6-dioxopiperidin-3-yl)-5-fluoroisoindoline-1,3-dione (see WO 2017197056 for the synthetic method) (0.036 g, 0.13 mmol) and diisopropylethylamine (0.031 g, 0.24 mmol) were added, and the mixture was stirred at 90°C for 2 h. The reaction solution was cooled to room temperature. 5 mL of water was added, and the mixture was stirred for 2 min and filtered. The filter cake was washed with 10 mL of water, then collected and dissolved with dichloromethane (30 mL). The resultant was washed with saturated aqueous sodium chloride solution (10 mL), dried over anhydrous sodium sulfate and filtered. The filtrate was concentrated under reduced pressure, and the crude product was separated and purified by silica gel column chromatography (methanol/dichloromethane (v/v) = 0 : 1-1 : 19) to obtain N-[4-cyano-3-(trifluoromethyl)phenyl]-1-[4-[1-[[1-[2-(2,6-dioxo-3-piperidyl)-1,3-dioxo-isoindolin-5-yl]pyrrolidin-3-yl]methyl]-4-piperidyl]pyrazol-1-yl]cyclobutanecarboxamide (compound 4) (0.040 g, yield: 44%).
$^1$H NMR (400 MHz, $CDCl_3$) δ 9.01 (s, 1H), 8.17 (s, 1H), 7.98 - 7.92 (m, 1H), 7.79 - 7.68 (m, 2H), 7.68 - 7.57 (m, 2H), 7.41 (s, 1H), 6.95 (d, 1H), 6.72 - 6.65 (m, 1H), 4.94 (dd, 1H), 3.66 - 3.46 (m, 2H), 3.45 - 3.37 (m, 1H), 3.28 - 3.15 (m, 1H), 3.09 - 2.97 (m, 3H), 2.95 - 2.65 (m, 6H), 2.60 - 2.32 (m, 3H), 2.30 - 1.76 (m, 9H), 1.75 - 1.60 (m, 1H), 1.37 - 1.20 (m, 2H).
LCMS m/z = 757.3 $[M+1]^+$

**Example 5:**

N-(4-cyano-3-(trifluoromethyl)phenyl)-2-(4-(1-(2-(2,6-dioxopiperidin-3-yl)-1,3-dioxoisoindolin-5-yl)piperidin-4-yl)-1H-pyrazol-1-yl)-2-methylpropanamide (**compound 5**)

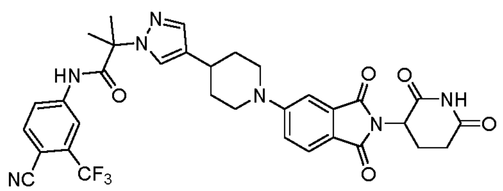

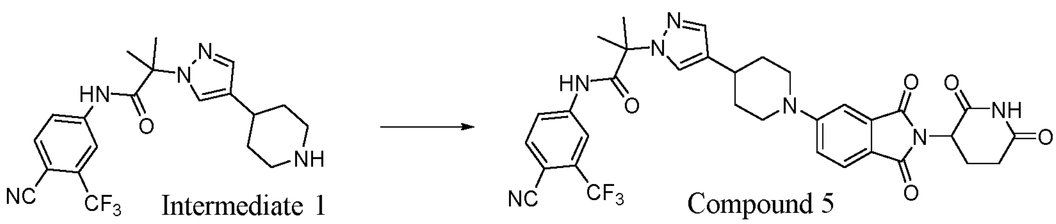

Intermediate 1

Compound 5

N-(4-cyano-3-(trifluoromethyl)phenyl)-2-methyl-2-(4-(piperidin-4-yl)-1H-pyrazol-1-yl)propanamide (intermediate 1) (0.1 g, 0.25 mmol) was dissolved in 5 mL of DMSO. 0.5 mL of DIPEA and 2-(2,6-dioxopiperidin-3-yl)-5-fluoroisoindoline-1,3-dione (see WO 2017197056 for the synthetic method) (90 mg, 0.33 mmol) was added, and the mixture was stirred at 85°C for 3 h. The reaction solution was cooled to room temperature, and 30 mL of water was added. The mixture was filtered to collect a solid, which was washed with 10 mL of water, dissolved with 50 mL of dichloromethane, dried over anhydrous sodium sulfate and concentrated under reduced pressure, and then the crude product was separated and purified by silica gel column chromatography (dichloromethane/methanol (v/v) = 15 : 1) to obtain N-(4-cyano-3-(trifluoromethyl)phenyl)-2-(4-(1-(2-(2,6-dioxopiperidin-3-yl)-1,3-dioxoisoindolin-5-yl)piperidin-4-yl)-1H-pyrazol-1-yl)-2-methylpropanamide (compound 5) (90 mg, yield: 54%).
$^1$H NMR (400 MHz, $CDCl_3$) δ 9.82 (s, 1H), 8.12 (br.s, 1H), 7.96 - 7.93 (m, 1H), 7.82 - 7.66 (m, 3H), 7.63 (s, 1H), 7.48 (s, 1H), 7.31 (d, 1H), 7.08 (dd, 1H), 4.94 (dd, 1H), 4.08 - 3.98 (m, 2H), 3.16 - 3.04 (m, 2H), 2.94 - 2.67 (m, 4H), 2.19 -

2.01 (m, 3H), 1.92 (s, 6H), 1.78 - 1.65 (m, 2H).
LCMS m/z = 662.2 $[M+1]^+$

**Example 6:**

N-[4-cyano-3-(trifluoromethyl)phenyl]-1-[4-[2-[1-[1-[2-(2,6-dioxo-3-piperidyl)-1,3-dioxo-isoindolin-5-yl]azetidin-3-yl]azetidin-3-yl]ethynyl]pyrazol-1-yl]cyclobutanecarboxamide (**compound 6**)

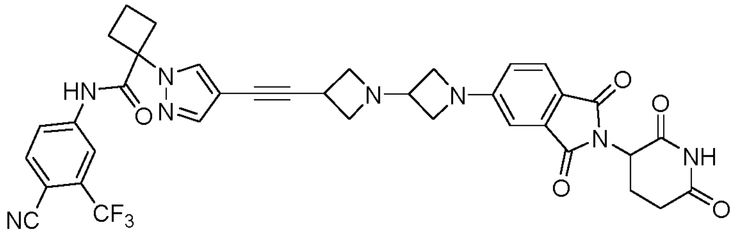

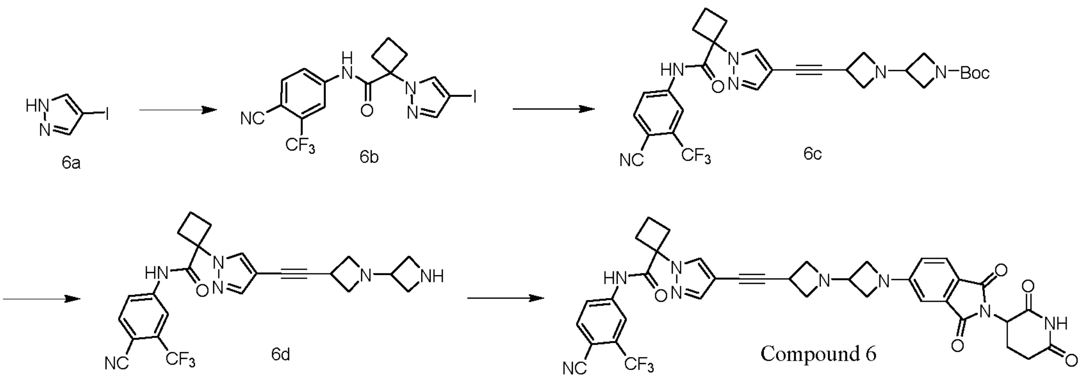

6a 6b 6c 6d Compound 6

Step 1: N-[4-cyano-3-(trifluoromethyl)phenyl]-1-(4-iodopyrazol-1-yl)cyclobutanecarboxamide (6b)

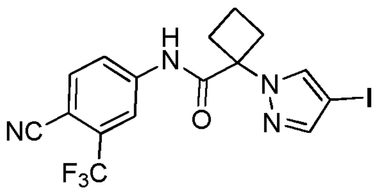

4-iodo-1H-pyrazole (6a) (0.559 g, 2.88 mmol) was dissolved in 10 mL of acetonitrile. 1-bromo-N-(4-cyano-3-(trifluoromethyl)phenyl)cyclobutane-1-carboxamide (see WO 2020063407 for the synthetic method) (1.00 g, 2.88 mmol) and cesium carbonate (1.03 g, 3.16 mmol) were added successively, and the mixture was reacted at 80°C for 1 h under a microwave condition. The reaction solution was cooled to room temperature, and 20 mL of ethyl acetate and 10 mL of water were added. The liquid separation was conducted. The organic layer was dried over anhydrous sodium sulfate and concentrated under reduced pressure to obtain a crude product, which was separated and purified by silica gel column chromatography (petroleum ether/ethyl acetate (v/v) = 9 : 1) to obtain N-[4-cyano-3-(trifluoromethyl)phenyl]-1-(4-iodopyrazol-1-yl)cyclobutanecarboxamide (6b) (0.130 g, yield: 10%).

Step 2: tert-butyl 3-[3-[2-[1-[1-[[4-cyano-3-(trifluoromethyl)phenyl]carbamoyl]cyclobutyl]pyrazol-4-yl]ethynyl]azetidin-1-yl]azetidine-1-carboxylate (6c)

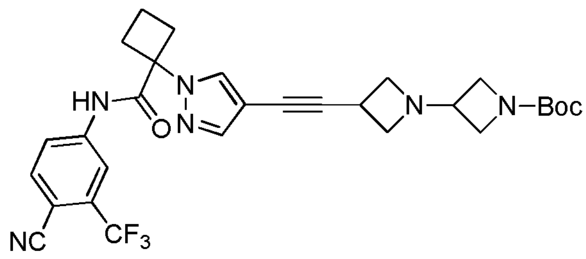

N-[4-cyano-3-(trifluoromethyl)phenyl]-1-(4-iodopyrazol-1-yl)cyclobutanecarboxamide (6b) (0.100 g, 0.217 mmol) was dissolved in 3 mL of dichloromethane. Triethylamine (0.0660 g, 0.652 mmol) was added, $PdCl_2(PPh_3)_2$ (0.0150 g, 0.0213 mmol) and cuprous iodide (0.0062 g, 0.0326 mmol) were added successively under nitrogen atmosphere, and then a solution of tert-butyl 3-ethynyl-[1,3'-biazetidine]-1'-carboxylate (intermediate B) (0.0770 g, 0.326 mmol) in dichloromethane (1 mL) was slowly added dropwise. After the addition was completed, the reaction solution was reacted at room temperature for 16 h. 5 mL of water and 10 mL of dichloromethane were added. The liquid separation was conducted, and the organic layer was washed with 5 mL of saturated sodium chloride solution, dried over anhydrous sodium sulfate and concentrated under reduced pressure to obtain a crude product, which was separated and purified by silica gel column chromatography (ethyl acetate) to obtain tert-butyl 3-[3-[2-[1-[1-[[4-cyano-3-(trifluoromethyl)phenyl]carbamoyl]cyclobutyl]pyrazol-4-yl]ethynyl]azetidin-1-yl]azetidine-1-carboxylate (6c) (0.0350 g, yield: 28%).

Step 3: 1-[4-[2-[1-(azetidin-3-yl)azetidin-3-yl]ethynyl]pyrazol-1-yl]-N-[4-cyano-3-(trifluoromethyl)phenyl]cyclobutanecarboxamide (6d)

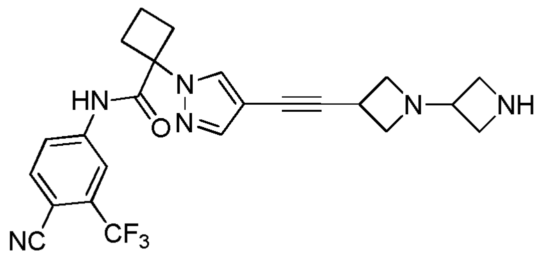

Tert-butyl 3-[3-[2-[1-[1-[[4-cyano-3-(trifluoromethyl)phenyl]carbamoyl]cyclobutyl]pyrazol-4-yl]ethynyl]azetidin-1-yl]azetidine-1-carboxylate (6c) (0.030 g, 0.053 mmol) was dissolved in 5 mL of dichloromethane. 2 mL of trifluoroacetic was added, and the mixture was reacted at room temperature for 1 h. The reaction solution was concentrated under reduced pressure. 20 mL of dichloromethane was added to the residue, and the pH was adjusted to 9 with saturated sodium bicarbonate. The liquid separation was conducted, the aqueous layer was extracted with 10 mL of dichloromethane, and the organic layers were combined, dried over anhydrous sodium sulfate and concentrated under reduced pressure to obtain crude 1-[4-[2-[1-(azetidin-3-yl)azetidin-3-yl]ethynyl]pyrazol-1-yl]-N-[4-cyano-3-(trifluoromethyl)phenyl]cyclobutanecarboxamide (6d) (0.020 g).
LCMS m/z = 469.2 $[M+1]^+$

Step 4: N-[4-cyano-3-(trifluoromethyl)phenyl]-1-[4-[2-[1-[1-[2-(2,6-dioxo-3-piperidyl)-1,3-dioxo-isoindolin-5-yl]azetidin-3-yl]azetidin-3-yl]ethynyl]pyrazol-1-yl]cyclobutanecarboxamide (compound 6)

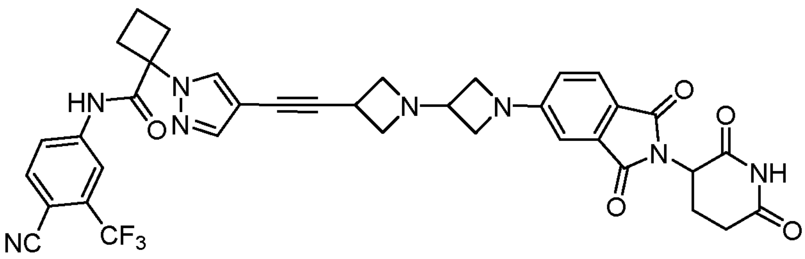

The crude 1-[4-[2-[1-(azetidin-3-yl)azetidin-3-yl]ethynyl]pyrazol-1-yl]-N-[4-cyano-3-(trifluoromethyl)phenyl]cyclobutanecarboxamide (6d) (0.020 g) was dissolved in dimethyl sulfoxide (3 mL). 2-(2,6-dioxopiperidin-3-yl)-5-fluoroisoindoline-1,3-dione (see WO 2017197056 for the synthetic method) (0.013 g, 0.047 mmol) and diisopropylethylamine (0.011 g, 0.085 mmol) were added, and the mixture was stirred at 90°C for 2 h. The reaction solution was cooled to room temperature. 5 mL of water was added, and the mixture was stirred for 2 min and filtered. The filter cake was washed with 10 mL of water, then collected and dissolved with dichloromethane (30 mL). The resultant was washed with 10 mL of saturated aqueous sodium chloride solution, dried over anhydrous sodium sulfate and filtered. The filtrate was concentrated under reduced pressure, and the crude product was separated and purified by silica gel column chromatography (methanol/dichloromethane (v/v) = 0 : 1-1 : 19) to obtain N-[4-cyano-3-(trifluoromethyl)phenyl]-1-[4-[2-[1-[1-[2-(2,6-dioxo-3-piperidyl)-1,3-dioxoisoindolin-5-yl]azetidin-3-yl]azetidin-3-yl]ethynyl]pyrazol-1-yl]cyclobutanecarboxamide (compound 6) (0.020 g, two-step yield calculated from compound 6c: 52%).
$^1$H NMR (400 MHz, $CDCl_3$) δ 8.11 - 7.97 (m, 2H), 7.95 - 7.87 (m, 1H), 7.80 - 7.74 (m, 1H), 7.72 - 7.64 (m, 2H), 7.58 - 7.50 (m, 1H), 6.83 - 6.77 (m, 1H), 6.58 - 6.51 (m, 1H), 4.93 (dd, 1H), 4.20 - 3.26 (m, 10H), 2.94 - 2.67 (m, 4H), 2.56 - 2.45 (m, 1H), 2.44 - 2.28 (m, 2H), 2.28 - 2.17 (m, 1H), 2.17 - 2.08 (m, 1H), 2.06 - 1.96 (m, 1H).
LCMS m/z = 725.2 $[M+1]^+$

**Example 7:**

N-[4-cyano-3-(trifluoromethyl)phenyl]-1-[4-[2-[1-[2-(2,6-dioxo-3-piperidyl)-1,3-dioxo-isoindolin-5-yl]azetidin-3-yl]ethynyl]pyrazol-1-yl]cyclobutanecarboxamide **(compound 7)**

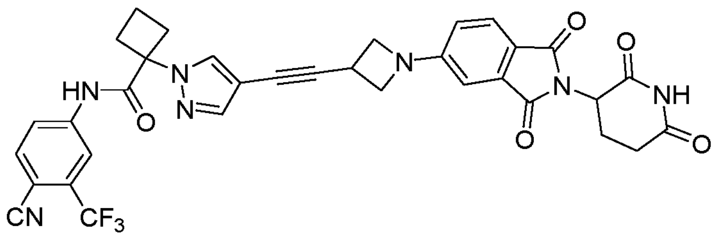

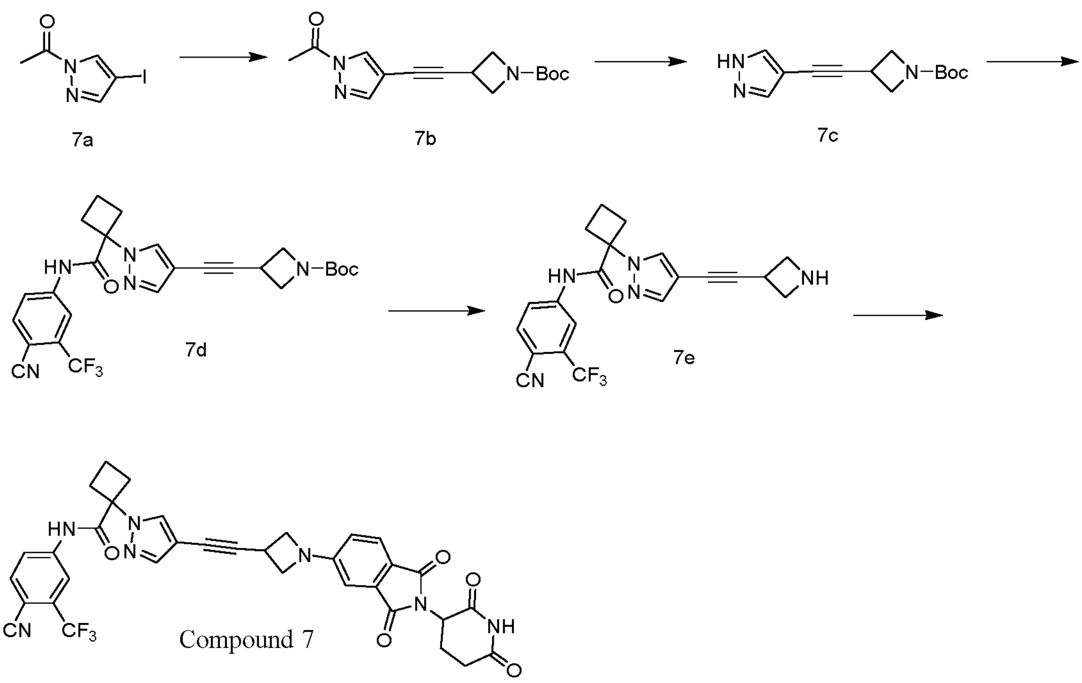

7a 7b 7c

7d 7e

Compound 7

Step 1: tert-butyl 3-[2-(1-(acetylpyrazol-4-yl)ethynyl]azetidine-1-carboxylate (7b)

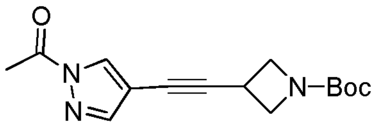

1-acetyl-4-iodo-1H-pyrazole (7a) (0.200 g, 0.847 mmol) was dissolved in 3 mL of dichloromethane. Triethylamine (0.257 g, 2.54 mmol) was added, $PdCl_2(PPh_3)_2$ (0.06 g, 0.0855 mmol) and cuprous iodide (0.0242 g, 0.127 mmol) were added successively under nitrogen atmosphere, and then a solution of tert-butyl 3-ethynyl-1-azetidinecarboxylate (0.200 g, 1.10 mmol) in dichloromethane (1 mL) was slowly added dropwise. After the addition was completed, the mixture was reacted at room temperature for 16 h. 5 mL of water and 10 mL of dichloromethane were added to the reaction system. The liquid separation was conducted, and the organic layer was washed with 5 mL of saturated sodium chloride solution, dried over anhydrous sodium sulfate and concentrated under reduced pressure to obtain a crude product, which was separated and purified by silica gel column chromatography (petroleum ether/ethyl acetate (v/v) = 5/1) to obtain tert-butyl 3-[2-(1-(acetylpyrazol-4-yl)ethynyl]azetidine-1-carboxylate (7b) (0.190 g, yield: 78%).
$^1$H NMR (400 MHz, $CDCl_3$) δ 8.27 (s, 1H), 7.69 (s, 1H), 4.23 - 4.16 (m, 2H), 4.04 - 3.96 (m, 2H), 3.56 - 3.45 (m, 1H), 2.69 (s, 3H), 1.45 (s, 9H).

Step 2: tert-butyl 3-[2-(1H-pyrazol-4-yl)ethynyl]azetidine-1-carboxylate (7c)

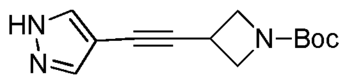

Tert-butyl 3-[2-(1-(acetylpyrazol-4-yl)ethynyl]azetidine-1-carboxylate (7b) (0.190 g, 0.657 mmol) was dissolved in 5 mL of anhydrous methanol. Potassium carbonate (0.182 g, 1.32 mmol) was added, and The mixture was stirred at room temperature for 5 min. 10 mL of water and 20 mL of ethyl acetate were added to the reaction solution. The liquid separation was conducted, and the organic layer was washed with 10 mL of saturated sodium chloride solution, dried over anhydrous sodium sulfate and concentrated under reduced pressure to obtain crude tert-butyl 3-[2-(1H-pyrazol-4-yl)ethynyl]azetidine-1-carboxylate (7c) (0.150 g).

Step 3: tert-butyl 3-[2-[1-[1-[[4-cyano-3-(trifluoromethyl)phenyl]carbamoyl]cyclobutyl]pyrazol-4-yl]ethynyl]azetidine-1-carboxylate (7d)

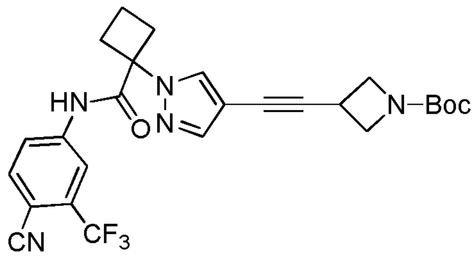

The crude tert-butyl 3-[2-(1H-pyrazol-4-yl)ethynyl]azetidine-1-carboxylate (7c) (0.142 g) was dissolved in 10 mL of acetonitrile. 1-bromo-N-(4-cyano-3-(trifluoromethyl)phenyl)cyclobutane-1-carboxamide (see WO 2020063407 for the synthetic method) (0.200 g, 0.576 mmol) and cesium carbonate (0.207 g, 0.635 mmol) were added, and the mixture was reacted at 80°C for 1 h under a microwave condition. The reaction solution was cooled to room temperature. 5 mL of water and 10 mL of dichloromethane were added. The liquid separation was conducted, and the organic layer was washed with 10 mL of saturated sodium chloride solution, dried over anhydrous sodium sulfate and concentrated under reduced pressure. The crude product was separated and purified by silica gel column chromatography (petroleum ether/ethyl acetate (v/v) = 9/1) to obtain tert-butyl 3-[2-[1-[1-[[4-cyano-3-(trifluoromethyl)phenyl]carbamoyl]cyclobutyl]pyrazol-4-yl]ethynyl]azetidine-1-carboxylate (7d) (0.130 g, two-step yield calculated from compound 7b: 41%).

Step 4: 1-[4-[2-(azetidin-3-yl)ethynyl]pyrazol-1-yl]-N-[4-cyano-3-(trifluoromethyl)phenyl]cyclobutanecarboxamide (7e)

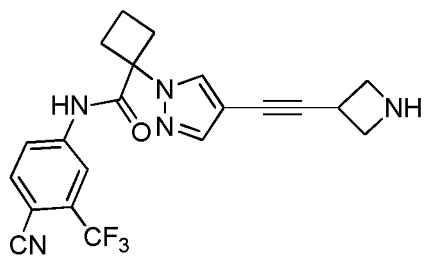

Tert-butyl 3-[2-[1-[1-[[4-cyano-3-(trifluoromethyl)phenyl]carbamoyl]cyclobutyl]pyrazol-4-yl]ethynyl]azetidine-1-carboxylate (7d) (0.050 g, 0.097 mmol) was dissolved in 5 mL of dichloromethane. 2 mL of trifluoroacetic was added, and the mixture was reacted at room temperature for 1 h. The reaction solution was concentrated under reduced pressure. 20 mL of dichloromethane was added to the residue, and the pH was adjusted to 10 with saturated sodium bicarbonate. The liquid separation was conducted, the aqueous layer was extracted with 10 mL of dichloromethane, and the organic layers were combined, dried over anhydrous sodium sulfate and concentrated under reduced pressure to obtain crude 1-[4-[2-(azetidin-3-yl)ethynyl]pyrazol-1-yl]-N-[4-cyano-3-(trifluoromethyl)phenyl]cyclobutanecarboxamide (7e) (0.040 g). LCMS m/z = 414.1 $[M+1]^+$

Step 5: N-[4-cyano-3-(trifluoromethyl)phenyl]-1-[4-[2-[1-[2-(2,6-dioxo-3-piperidyl)-1,3-dioxo-isoindolin-5-yl]azetidin-3-yl]ethynyl]pyrazol-1-yl]cyclobutanecarboxamide (compound 7)

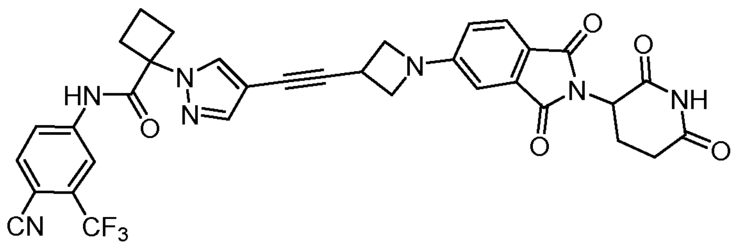

The crude 1-[4-[2-(azetidin-3-yl)ethynyl]pyrazol-1-yl]-N-[4-cyano-3-(trifluoromethyl)phenyl]cyclobutanecarboxamide (7e) (0.030 g) was dissolved in dimethyl sulfoxide (3 mL). 2-(2,6-dioxopiperidin-3-yl)-5-fluoroisoindoline-1,3-dione (see WO 2017197056 for the synthetic method) (0.022 g, 0.080 mmol) and diisopropylethylamine (0.019 g, 0.15 mmol) were added, and the mixture was stirred at 90°C for 2 h. The reaction solution was cooled to room temperature. 5 mL of water was added, and the mixture was stirred for 2 min and filtered. The filter cake was washed with 10 mL of water, then collected and dissolved with dichloromethane (30 mL). The organic phase was washed with 10 mL of saturated sodium chloride solution, dried over anhydrous sodium sulfate and filtered. The filtrate was concentrated under reduced pressure, and the crude product was separated and purified by silica gel column chromatography (petroleum ether/ethyl acetate (v/v) = 3/7) to obtain N-[4-cyano-3-(trifluoromethyl)phenyl]-1-[4-[2-[1-[2-(2,6-dioxo-3-piperidyl)-1,3-dioxo-isoindolin-5-yl]azetidin-3-yl]ethynyl]pyrazol-1-yl]cyclobutanecarboxamide (compound 7) (0.010 g, two-step yield calculated from compound 7d: 21%).

$^1$H NMR (400 MHz, $CDCl_3$) δ 8.83 (s, 1H), 8.23 (s, 1H), 7.97 - 7.91 (m, 1H), 7.83 - 7.61 (m, 5H), 6.80 (d, 1H), 6.55 (dd, 1H), 4.94 (dd, 1H), 4.40 - 4.31 (m, 2H), 4.11 - 4.00 (m, 2H), 3.87 - 3.74 (m, 1H), 3.11 - 2.99 (m, 2H), 2.96 - 2.67 (m, 5H), 2.26 - 1.96 (m, 3H).

LCMS m/z = 670.3 $[M+1]^+$

**Example 8:**

N-(4-cyano-3-(trifluoromethyl)phenyl)-2-(4-((1'-(2-(2,6-dioxopiperidin-3-yl)-1,3-dioxoisoindolin-5-yl)-[1,3'-biazetidin]-3-yl)ethynyl)-1H-pyrazol-1-yl)-2-methylpropanamide (**compound 8**)

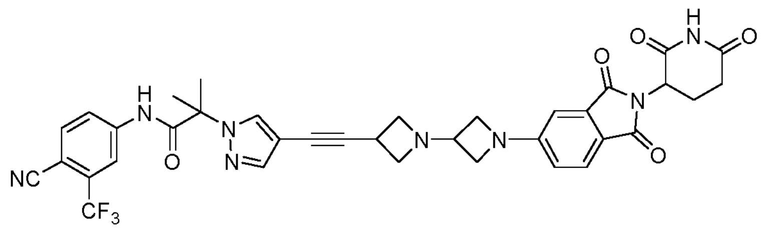

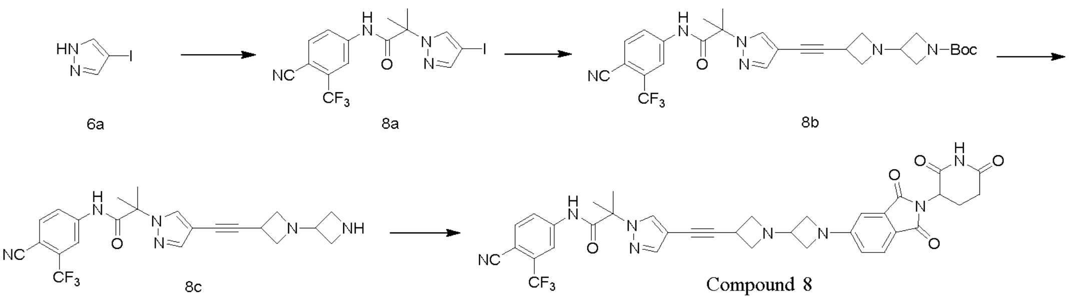

6a

8a

8b

8c

Compound 8

Step 1: N-(4-cyano-3-(trifluoromethyl)phenyl)-2-(4-iodo-1H-pyrazol-1-yl)-2-methylpropanamide (8a)

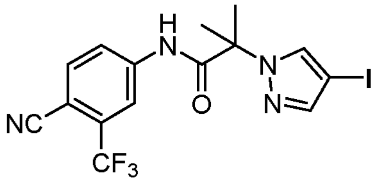

2-bromo-N-(4-cyano-3-(trifluoromethyl)phenyl)-2-methylpropanamide (see WO 2020063407 for the synthetic method) (1.72 g, 5.13 mmol) was dissolved in 35 mL of acetonitrile. 4-iodo-1H-pyrazole (6a) (1.0 g, 5.15 mmol) and cesium carbonate (3.36 g, 10.30 mmol) were added, and the mixture was stirred at 50°C for 3 h. The reaction solution was cooled to room temperature. 50 mL of water was added, and the mixture was extracted with DCM (40 mL × 3). The organic phase was washed with 50 mL of water, dried over anhydrous sodium sulfate and concentrated under reduced pressure. The crude product was separated and purified by silica gel column chromatography (petroleum ether/ethyl acetate (v/v) = 4 : 1) to obtain N-(4-cyano-3-(trifluoromethyl)phenyl)-2-(4-iodo-1H-pyrazol-1-yl)-2-methylpropanamide (8a) (1.5 g, yield: 65%).
$^1$H NMR (400 MHz, DMSO-$d_6$) δ 10.06 (s, 1H), 8.26 (d, 1H), 8.18 (s, 1H), 8.15 (dd, 1H), 8.08 (d, 1H), 7.62 (s, 1H), 1.81 (s, 6H).
LCMS m/z = 449.0 $[M+1]^+$

Step 2: tert-butyl 3-((1-(1-((4-cyano-3-(trifluoromethyl)phenyl)amino)-2-methyl-1-oxopropan-2-yl)-1H-pyrazol-4-yl)ethynyl)-[1,3'-biazetidine]-1'-carboxylate (8b)

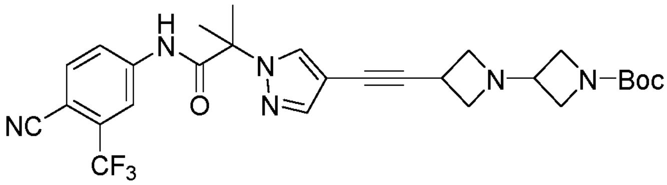

N-(4-cyano-3-(trifluoromethyl)phenyl)-2-(4-iodo-1H-pyrazol-1-yl)-2-methylpropanamide (8a) (224 mg, 0.50 mmol) was dissolved in 10 mL of dichloromethane. Triethylamine (150 mg, 1.48 mmol) was added, $PdCl_2(PPh_3)_2$ (35 mg, 0.05 mmol) and cuprous iodide (10 mg, 0.05 mmol) were added successively under nitrogen atmosphere, and then a solution of tert-butyl 3-ethynyl-[1,3'-biazetidine]-1'-carboxylate (intermediate B) (178 mg, 0.75 mmol) in dichloromethane (2 mL) was slowly added dropwise. After the addition was completed, the reaction solution was reacted at room temperature for 16 h. 15 mL of water and 10 mL of dichloromethane were added to the reaction solution. The liquid separation was conducted, and the organic layer was washed with 5 mL of saturated sodium chloride solution, dried over anhydrous sodium sulfate and concentrated under reduced pressure. The resulting crude product was separated and purified by silica gel column chromatography (petroleum ether/ethyl acetate (v/v) = 4 : 1) to obtain tert-butyl 3-((1-(1-((4-cyano-3-(trifluoromethyl)phenyl)amino)-2-methyl-1-oxopropan-2-yl)-1H-pyrazol-4-yl)ethynyl)-[1,3'-biazetidine]-1'-carboxylate (8b) (260 mg, yield: 93%).
LCMS m/z = 557.3 $[M+1]^+$

Step 3: 2-(4-([1,3'-biazetidin]-3-ylethynyl)-1H-pyrazol-1-yl)-N-(4-cyano-3-(trifluoromethyl)phenyl)-2-methylpropanamide (8c)

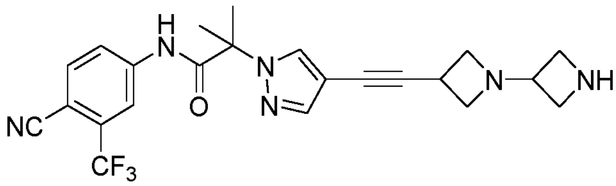

Tert-butyl 3 -((1 -(1 -((4-cyano-3 -(trifluoromethyl)phenyl)amino)-2-methyl-1-oxopropan-2-yl)-1H-pyrazol-4-yl)ethynyl)-[1,3'-biazetidine]-1'-carboxylate (8b) (0.24 g, 0.43 mmol) was dissolved in 10 mL of DCM. 6 mL of trifluoroacetic

acid was added, and the mixture was stirred at room temperature for 3 h. After the reaction was completed, the reaction system was directly concentrated under reduced pressure, and the residue was dissolved in 20 mL of 4 mol/L aqueous NaOH. The reaction solution was extracted with DCM (40 ml × 3). The organic phase was washed with 50 mL of water, dried over anhydrous sodium sulfate and concentrated under reduced pressure to obtain crude 2-(4-([1,3'-biazetidin]-3-ylethynyl)-1H-pyrazol-1-yl)-N-(4-cyano-3-(trifluoromethyl)phenyl)-2-methylpropanamide (8c) (0.18 g).
LCMS m/z = 457.1 $[M+1]^+$

Step 4: N-(4-cyano-3-(trifluoromethyl)phenyl)-2-(4-((1'-(2-(2,6-dioxopiperidin-3-yl)-1,3-dioxoisoindolin-5-yl)-[1,3'-biazetidin]-3-yl)ethynyl)-1H-pyrazol-1-yl)-2-methylpropanamide (compound 8)

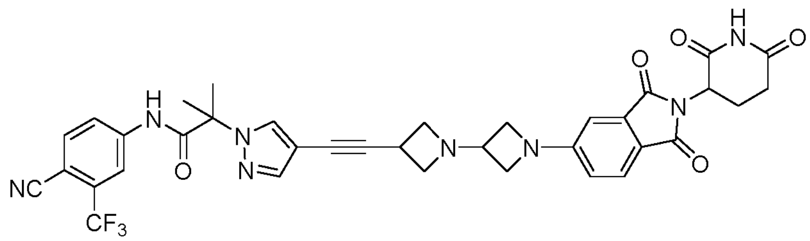

The crude 2-(4-([1,3'-biazetidin]-3-ylethynyl)-1H-pyrazol-1-yl)-N-(4-cyano-3-(trifluoromethyl)phenyl)-2-methylpropanamide (8c) (0.18 g) was dissolved in 10 mL of DMSO. 0.5 mL of DIPEA and 2-(2,6-dioxopiperidin-3-yl)-5-fluoroisoindoline-1,3-dione (see WO 2017197056 for the synthetic method) (181 mg, 0.66 mmol) were added, and the mixture was stirred at 80°C for 5 h. The reaction solution was cooled to room temperature, and 20 mL of water was added. The mixture was filtered to collect a solid, which was washed with 20 mL of water, dissolved with 50 mL of dichloromethane, dried over anhydrous sodium sulfate and concentrated under reduced pressure, and then the crude product was separated and purified by silica gel column chromatography (dichloromethane/methanol (v/v) = 15 : 1) to obtain N-(4-cyano-3-(trifluoromethyl)phenyl)-2-(4-((1'-(2-(2,6-dioxopiperidin-3-yl)-1,3-dioxoisoindolin-5-yl)-[1,3'-biazetidin]-3-yl)ethynyl)-1H-pyrazol-1-yl)-2-methylpropanamide (compound 8) (18 mg, two-step yield calculated from compound 8b: 6%).
$^1H$ NMR (400 MHz, $CDCl_3$) $\delta$ 9.47 (s, 1H), 8.31 (s, 1H), 7.96 - 7.90 (m, 1H), 7.84 - 7.70 (m, 4H), 7.64 (d, 1H), 6.78 (d, 1H), 6.52 (dd, 1H), 4.92 (dd, 1H), 4.11 - 4.01 (m, 2H), 3.96 - 3.84 (m, 2H), 3.83 - 3.63 (m, 3H), 3.58 - 3.47 (m, 1H), 3.39 - 3.25 (m, 2H), 2.94 - 2.64 (m, 3H), 2.17 - 2.07 (m, 1H), 1.92 (s, 6H).
LCMS m/z = 713.3 $[M+1]^+$

**Example 9:**

N-(4-cyano-3-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (**compound 9**)

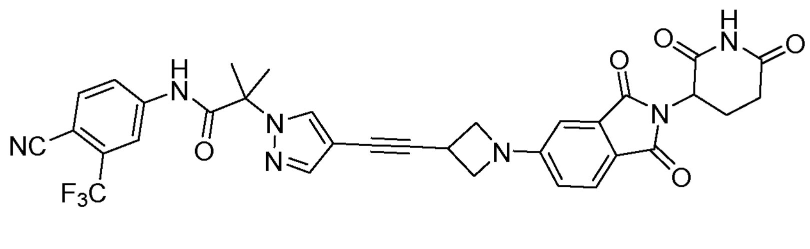

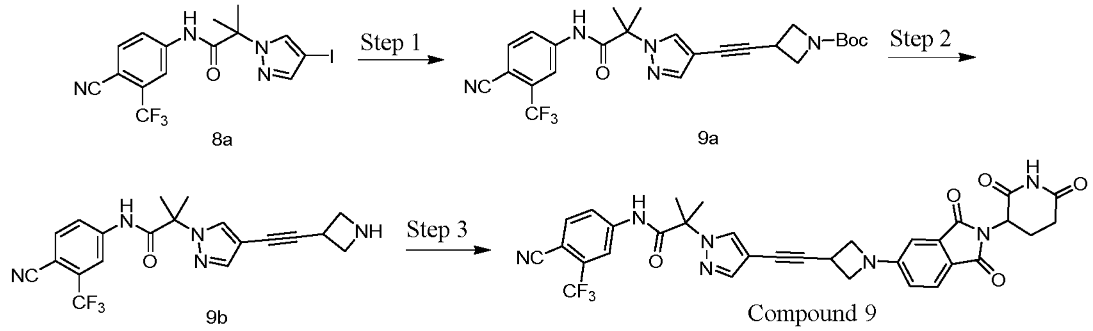


8a 9a 9b Compound 9

Step 1: tert-butyl 3-((1-(1-((4-cyano-3-(trifluoromethyl)phenyl)amino)-2-methyl-1-oxopropan-2-yl)-1H-pyrazol-4-yl)ethynyl)azetidine-1-carboxylate (9a)

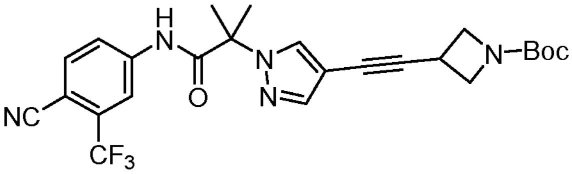

N-(4-cyano-3-(trifluoromethyl)phenyl)-2-(4-iodo-1H-pyrazol-1-yl)-2-methylpropanamide (8a) (224 mg, 0.50 mmol) was dissolved in 10 mL of dichloromethane. Triethylamine (150 mg, 1.48 mmol) was added, $PdCl_2(PPh_3)_2$ (35 mg, 0.05 mmol) and cuprous iodide (10 mg, 0.05 mmol) were added successively under nitrogen atmosphere, and then a solution of tert-butyl 3-ethynylazetidine-1-carboxylate (136 mg, 0.75 mmol) in dichloromethane (2 mL) was slowly added dropwise. After the addition was completed, the reaction solution was reacted at room temperature for 16 h. 15 mL of water and 10 mL of dichloromethane were added to the reaction solution. The liquid separation was conducted, and the organic layer was washed with 5 mL of saturated sodium chloride solution, dried over anhydrous sodium sulfate and concentrated under reduced pressure. The residue was separated and purified by silica gel column chromatography (petroleum ether/ethyl acetate (v/v) = 4 : 1) to obtain tert-butyl 3-((1-(1-((4-cyano-3-(trifluoromethyl)phenyl)amino)-2-methyl-1-oxopropan-2-yl)-1H-pyrazol-4-yl)ethynyl)azetidine-1-carboxylate (9a) (230 mg, yield: 92%).

Step 2: 2-(4-(azetidin-3-ylethynyl)-1H-pyrazol-1-yl)-N-(4-cyano-3-(trifluoromethyl)phenyl)-2-methylpropanamide (9b)

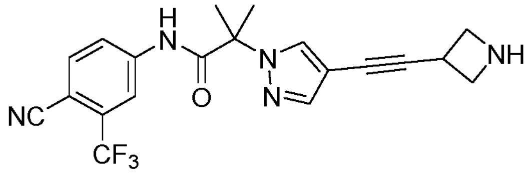

Tert-butyl 3 -((1 -(1 -((4-cyano-3 -(trifluoromethyl)phenyl)amino)-2-methyl-1-oxopropan-2-yl)-1H-pyrazol-4-yl)ethynyl)azetidine-1-carboxylate (9a) (0.23 g, 0.46 mmol) was dissolved in 10 mL of DCM. 6 mL of trifluoroacetic acid was added, and the mixture was stirred at room temperature for 3 h. After the reaction was completed, the system was directly concentrated under reduced pressure, and the residue was dissolved in 20 mL of 4 mol/L aqueous NaOH. The reaction solution was extracted with DCM (40 mL $\times$ 3). The organic phase was washed with 50 mL of water, dried over anhydrous sodium sulfate and concentrated under reduced pressure to obtain crude 2-(4-(azetidin-3-ylethynyl)-1H-pyrazol-1-yl)-N-(4-cyano-3-(trifluoromethyl)phenyl)-2-methylpropanamide (9b) (0.18 g).
LCMS m/z = 402.1 $[M+1]^+$

Step 3: N-(4-cyano-3-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (compound 9)

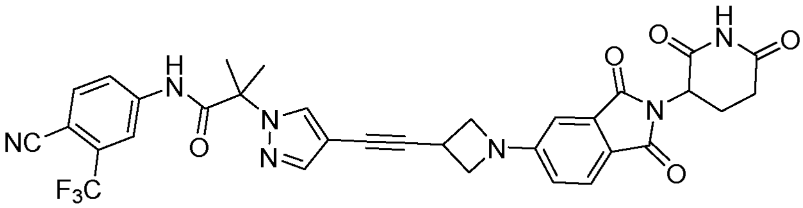

The crude 2-(4-(azetidin-3-ylethynyl)-1H-pyrazol-1-yl)-N-(4-cyano-3-(trifluoromethyl)phenyl)-2-methylpropanamide (9b) (0.17 g) was dissolved in 10 mL of DMSO. 0.5 mL of DIPEA and 2-(2,6-dioxopiperidin-3-yl)-5-fluoroisoindoline-1,3-dione (see WO 2017197056 for the synthetic method) (180 mg, 0.65 mmol) were added, and the mixture was stirred at 80°C for 5 h. The reaction solution was cooled to room temperature, and 20 mL of water was added. The mixture was filtered to collect a solid, which was washed with 20 mL of water, dissolved with 50 mL of dichloromethane, dried over anhydrous sodium sulfate and concentrated under reduced pressure, and then the crude product was separated and purified by silica gel column chromatography (dichloromethane/methanol (v/v) = 15 : 1) to obtain N-(4-cyano-3-(trifluoromethyl)phenyl)-2-(4-((1-(2-(2,6-dioxopiperidin-3-yl)-1,3-dioxoisoindolin-5-yl)azetidin-3-yl)ethynyl)-1H-pyrazol-1-yl)-2-methylpropanamide (compound 9) (80 mg, two-step yield calculated from compound 9a: 28%).
$^1$H NMR (400 MHz, $CDCl_3$) δ 9.49 (s, 1H), 8.04 (s, 1H), 7.95 - 7.90 (m, 1H), 7.84 - 7.70 (m, 4H), 7.67 (d, 1H), 6.80 (d, 1H), 6.56 (dd, 1H), 4.94 (dd, 1H), 4.40 - 4.32 (m, 2H), 4.10 - 4.02 (m, 2H), 3.86 - 3.75 (m, 1H), 2.94 - 2.65 (m, 3H), 2.18 - 2.07 (m, 1H), 1.92 (s, 6H).
LCMS m/z = 658.2 $[M+1]^+$

**Example 10:**

N-(4-cyano-3-(trifluoromethyl)phenyl)-2-(4-((1-(2-(2,6-dioxopiperidin-3-yl)-1,3-dioxoisoindolin-5-yl)piperidin-4-yl)ethynyl)-1H-pyrazol-1-yl)-2-methylpropanamide (**compound 10**)

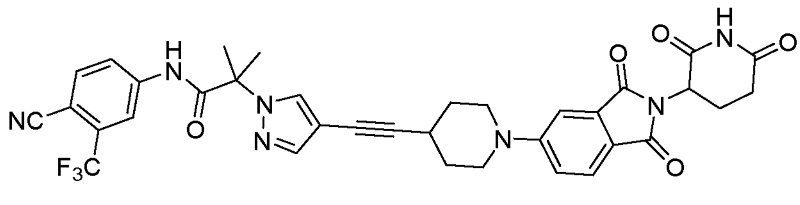

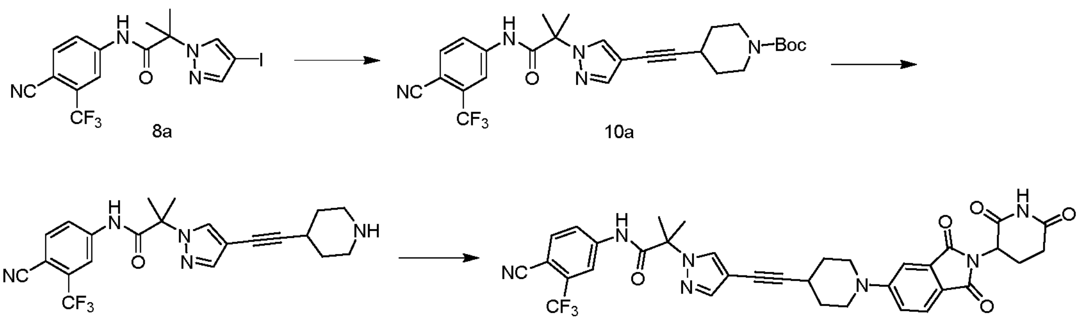

8a

10a

10b

Compound 10

Step 1: tert-butyl 4-((1-(1-((4-cyano-3-(trifluoromethyl)phenyl)amino)-2-methyl-1-oxopropan-2-yl)-1H-pyrazol-4-yl)ethynyl)piperidine-1-carboxylate (10a)

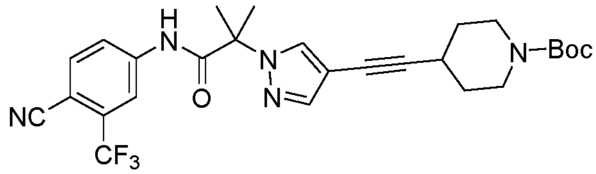

N-(4-cyano-3-(trifluoromethyl)phenyl)-2-(4-iodo-1H-pyrazol-1-yl)-2-methylpropanamide (8a) (224 mg, 0.50 mmol) was dissolved in 10 mL of dichloromethane. Triethylamine (150 mg, 1.48 mmol) was added, $PdCl_2(PPh_3)_2$ (35 mg, 0.05 mmol) and cuprous iodide (10 mg, 0.05 mmol) were added successively under nitrogen atmosphere, and then a solution of tert-butyl 4-ethynylpiperidine-1-carboxylate (170 mg, 0.81 mmol) in dichloromethane (2 mL) was slowly added dropwise. After the addition was completed, the reaction solution was reacted at room temperature for 16 h. 15 mL of water and 10 mL of dichloromethane were added to the reaction solution. The liquid separation was conducted, and the organic layer was washed with 5 mL of saturated sodium chloride solution, dried over anhydrous sodium sulfate and concentrated under reduced pressure. The residue was separated and purified by silica gel column chromatography (petroleum ether/ethyl acetate (v/v) = 4 : 1) to obtain tert-butyl 4-((1-(1-((4-cyano-3-(trifluoromethyl)phenyl)amino)-2-methyl-1-oxopropan-2-yl)-1H-pyrazol-4-yl)ethynyl)piperidine-1-carboxylate (10a) (200 mg, yield: 76%).

Step 2: N-(4-cyano-3-(trifluoromethyl)phenyl)-2-methyl-2-(4-(piperidin-4-ylethynyl)-1H-pyrazol-1-yl)propanamide (10b)

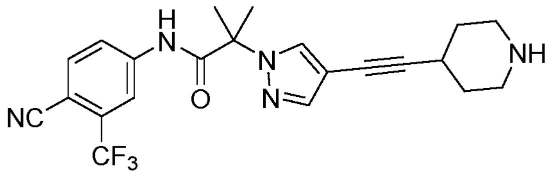

Tert-butyl 4-((1-(1-((4-cyano-3-(trifluoromethyl)phenyl)amino)-2-methyl-1-oxopropan-2-yl)-1H-pyrazol-4-yl)ethynyl)piperidine-1-carboxylate (10a) (0.20 g, 0.38 mmol) was dissolved in 10 mL of DCM. 6 mL of trifluoroacetic acid was added, and the mixture was stirred at room temperature for 3 h. After the reaction was completed, the reaction system was directly concentrated under reduced pressure, and the residue was dissolved in 20 mL of 4 mol/L aqueous NaOH. The reaction solution was extracted with DCM (40 mL × 3). The organic phase was washed with 50 mL of water, dried over anhydrous sodium sulfate and concentrated under reduced pressure to obtain crude N-(4-cyano-3-(trifluoromethyl)phenyl)-2-methyl-2-(4-(piperidin-4-ylethynyl)-1H-pyrazol-1-yl)propanamide (10b) (0.16 g).
LCMS m/z = 430.2 $[M+1]^+$

Step 3: N-(4-cyano-3-(trifluoromethyl)phenyl)-2-(4-((1-(2-(2,6-dioxopiperidin-3-yl)-1,3-dioxoisoindolin-5-yl)piperidin-4-yl)ethynyl)-1H-pyrazol-1-yl)-2-methylpropanamide (compound 10)

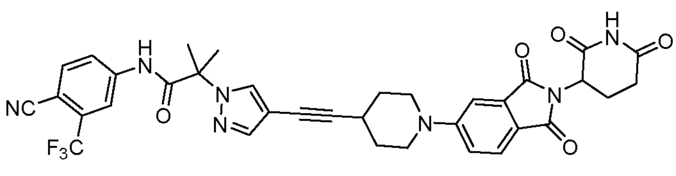

The crude N-(4-cyano-3-(trifluoromethyl)phenyl)-2-methyl-2-(4-(piperidin-4-ylethynyl)-1H-pyrazol-1-yl)propanamide (10b) (0.15 g) was dissolved in 10 mL of DMSO. 0.5 mL of DIPEA and 2-(2,6-dioxopiperidin-3-yl)-5-fluoroisoindoline-1,3-dione (see WO 2017197056 for the synthetic method) (145 mg, 0.53 mmol) was added, and the mixture was stirred at 80°C for 5 h. The reaction solution was cooled to room temperature, and 20 mL of water was added. The mixture was filtered to collect a solid, which was washed with 20 mL of water, dissolved with 50 mL of dichloromethane, dried over anhydrous sodium sulfate and concentrated under reduced pressure, and then the crude product was separated and purified by silica gel column chromatography (dichloromethane/methanol (v/v) = 15 : 1) to obtain N-(4-cyano-3-(trifluoromethyl)phenyl)-2-(4-((1-(2-(2,6-dioxopiperidin-3-yl)-1,3-dioxoisoindolin-5-yl)piperidin-4-yl)ethynyl)-1H-pyrazol-1-yl)-2-methylpropanamide (compound 10) (45 mg, two-step yield calculated from compound 10a: 18%).
$^1H$ NMR (400 MHz, $CDCl_3$) δ 9.49 (s, 1H), 8.13 (s, 1H), 7.96 - 7.90 (m, 1H), 7.82 - 7.65 (m, 5H), 7.30 (d, 1H), 7.10 (dd, 1H), 4.94 (dd, 1H), 3.78 - 3.68 (m, 2H), 3.39 - 3.28 (m, 2H), 2.96 - 2.66 (m, 4H), 2.18 - 1.99 (m, 3H), 1.92 (s, 6H), 1.88 - 1.78 (m, 2H).
LCMS m/z = 686.2 $[M+1]^+$

**Biological test examples**

**1. Experiment on inhibiting VCaP cell proliferation**

Prostate cancer cells VCaP were purchased from ATCC, the cell medium was 1640 + 10% FBS, and the cells were cultured in an incubator at 37°C under 5% $CO_2$. On day 1, the cells in an exponential phase were collected. With a 1% css-FBS phenol red-free medium, the cell suspension was adjusted to a corresponding concentration, plated into T0 wells at 7500 cells/well and incubated for 3 days. After the incubation was completed, R1881 at a final concentration of 0.1 nM and compounds at different concentrations were added, and the plate was placed in the incubator and incubated for another 7 days. On the day of the addition, $T_0$ plate was detected using a CellTiter-Glo kit and recorded as $RLU_0$. After the incubation was completed, 100 μL of mediums was pipetted from each well. According to operation instructions for a CellTiter-Glo kit (Promega, G7573), 50 μL of CellTiter-Glo reagents, which were already pre-melted and equilibrated to room temperature, were then added to each well, and the mixtures were uniformly mixed for 2 min using a microplate shaker. The plate was placed at room temperature for 10 min, and then fluorescence signal values were measured using a microplate reader (PHERAstar FSX). The results were processed according to formula (1), the inhibition rate of the compound at different concentrations was calculated, and the $GI_{50}$ value of the compound with an inhibition rate of 50% was calculated using origin9.2 software, wherein $RLU_{compound}$ was the readout of the treated group, and $RLU_{control}$ was the average value of the solvent control group.

$$\text{Inhibition \%} = [1 - (RLU_{compound} - RLU_0)/(RLU_{control} - RLU_0)] \times 100\% \quad \text{formula (1)}$$

The results for $GI_{50}$ values on proliferation inhibition of VCaP cells were shown in Table 1.

Table 1 $GI_{50}$ values for inhibition of VCaP cells by compounds of the present invention

| Serial No. | Compound No. | $GI_{50}$ (μM) |
|---|---|---|
| 1 | Compound 2 | 4.79 |
| 2 | Compound 3 | 3.09 |
| 3 | Compound 4 | 1.81 |
| 4 | Compound 5 | 1.91 |
| 5 | Compound 6 | 1.69 |
| 6 | Compound 7 | 0.47 |
| 7 | Compound 8 | 3.96 |
| 8 | Compound 9 | 0.18 |
| 9 | Compound 10 | 0.33 |
| Conclusion: the compounds of the present invention had an inhibition effect on prostate cancer cells VCaP. | | |

**2. Pharmacokinetic experiment in rats**

**Objective:** in this experiment, a single dose of each test compound was administered to SD rats intravenously and intragastrically, the concentrations of the test compound in plasma of rats were measured, and the pharmacokinetic characteristics and bioavailability of the test compound in rats were evaluated.
**Experimental animals:** male SD rats, 200-250 g, 6-8 weeks old, 6 rats/compound, purchased from CHENGDU DOSSY EXPERIMENTAL ANIMALS CO., LTD.
**Experimental method:** on the day of the experiment, six SD rats were randomly grouped according to their body weights. The animals were fasted but with water available for 12 to 14 hours one day before the administration,

and were fed 4 h after the administration.

Table 2

| Group | No. of rats | Administration information | | | | | | |
|---|---|---|---|---|---|---|---|---|
| | Male | Test compound | Administration dosage* (mg/kg) | Administration concentration (mg/mL) | Administration volume (mL/kg) | Collected samples | Mode of administration | Vehicle |
| G1 | 3 | Compound of the present invention | 5 | 1 | 5 | Plasma | Intravenously | 5% DMA + 5% Solutol + 90% Saline |
| G2 | 3 | Compound of the present invention | 20 | 2 | 10 | Plasma | Oral (intragastrically) | 5% DMSO+ 30%PEG-400 + 65% (20% SBE-β-CD) |
| *Dosage is calculated based on free base. | | | | | | | | |

**Sampling:** before and after the administration, 0.1 mL of blood was taken from the orbit of the rats under isoflurane anesthesia and placed in an EDTAK2 centrifuge tube. Centrifugation was carried out at 5000 rpm at 4°C for 10 min, and the plasma was collected.

Time points for plasma collection in G1 and G2 groups: 0, 5 min, 15 min, 30 min, 1 h, 2 h, 4 h, 6 h, 8 h, and 24 h.

Before analysis and detection, all samples were stored at -80°C. The samples were analyzed quantitatively by LC-MS/MS.

Table 3 Pharmacokinetic parameters of compounds of the present invention in plasma of rats

| Test compounds | Mode of administration* | $AUC_{0-t}$ (ng/mL·h) | $T_{1/2}$ (h) | Oral bioavailability |
|---|---|---|---|---|
| Compound 9 | i.g. (20 mg/kg) | 45604 ± 3725 | 24.0 ± 10 | Yes |
| *Note: i.g. (intragastrical) administration;<br>Conclusion: the compounds the present invention had a certain oral bioavailability in rats. | | | | |

**3. Experiment on inhibiting 22RV1 cell proliferation**

Prostate cancer cells 22RV1 were purchased from ATCC, the cell medium was RPMI 1640 + 10% FBS, and the cells were cultured in an incubator at 37°C under 5% $CO_2$. On day 1, the cells in an exponential phase were collected. With a 1% css-FBS phenol red-free medium, the cell suspension was adjusted to a corresponding concentration, plated at 2000 cells/well and incubated overnight. On day 2, compounds at different concentrations were added, and the plate was placed in the incubator and incubated for another 7 days. After the incubation was completed, according to operation instructions for a CellTiter-Glo kit (Promega, G7573), 50 μL of CellTiter-Glo reagents, which were already pre-melted and equilibrated to room temperature, were added to each well, and the mixtures were uniformly mixed for 2 min using a microplate shaker. The plate was placed at room temperature for 10 min, and then fluorescence signal values were measured using a microplate reader (PHERAstar FSX). The results were processed according to formula (2), the inhibition rate of the compound at different concentrations was calculated, and the $IC_{50}$ value of the compound with an inhibition rate of 50% was calculated using origin9.2 software with DoseResp function, wherein $RLU_{compound}$ was the readout of the treated group, and $RLU_{control}$ was the average value of the DMSO solvent control group.

$$\text{Inhibition } \% = [1 - RLU_{compound}/RLU_{control}] \times 100\% \quad \text{formula (2)}$$

The results for $IC_{50}$ values on proliferation inhibition of 22RV1 cells were shown in Table 4.

Table 4 IC50 values for inhibition of 22RV1 cells by compounds of the present invention

| Serial No. | Compound No. | $IC_{50}$ (μM) |
|---|---|---|
| 1 | Compound 9 | 0.078 |
| Conclusion: the compounds of the present invention had an inhibition effect on prostate cancer cells 22RV1. | | |

**4. Degradation experiment on full-length AR (AR-FL) and AR splice variants (AR-Vs) in 22RV1 cells**

Prostate cancer cells 22RV1 were purchased from ATCC, the cell medium was 1640 + 10% FBS, and the cells were cultured in an incubator at 37°C under 5% $CO_2$. On day 1, the cells in an exponential phase were collected. With a 1% css-FBS phenol red-free medium, the cell suspension was adjusted to a corresponding concentration and plated into a 6-well plate at 1 mL/well and 100000 cells/ well. The next day, a 1% css-FBS phenol-free medium containing the compound to be tested was added, a 1% css-FBS phenol-free medium containing 0.2% DMSO was added to one well as DMSO solvent control, and the 6-well plate was cultured in an incubator at 37°C under 5% $CO_2$. After 24 h, the cells were digested with trypsin and collected into a 1.5 mL centrifuge tube. 15 μL of RIPA lysate (containing 1X protease inhibitor cocktail) was added to each well, and the cells were lysed on ice for 15 min and then centrifuged at 12000 g at 4°C for 10 min. The supernatant protein samples were collected, and protein quantification was performed by BCA method. AR-FL and AR-Vs were detected using fully automatic protein expression quantitative analysis, which involved diluting the protein to be tested to 1 mg/mL. 4 μL of the diluted protein samples were added to 1 μL of 5× Master Mix (provided in the kit), and the prepared samples were denatured at 95°C for 5 min and placed on ice for use. The primary antibodies, AR(CST, 5153S) and β-actin (CST, 3700), were diluted with Antibody Diluent II (provided in the kit) at a dilution ratio of 1 : 20 and 1 : 200, respectively. The secondary antibody was a mixed goat anti-mouse and goat anti-rabbit secondary antibody in a ratio of 1 : 1, and the color development solution was a mixed solution of Lumino-S and Peroxide in a ratio of 1 : 1. The prepared reagents were successively added to an assay plate according to instructions of the kit and detected. Western blot band processing was performed using "Compass for SW", a fully automatic protein expression quantitative analysis software, in which western blot bands were automatically simulated based on signal values. The degradation rate of AR-FL (1) or AR-Vs (2) relative to solvent control at different compound concentrations was calculated according to formulas (1) and (2), wherein $\text{AR-FL}_{compound}$ was the relative peak area of AR-FL in the treated group, and $\text{AR-FL}_{solvent}$ was the relative peak area of AR-FL in the solvent control group. $\text{AR-Vs}_{compound}$ was the relative peak area of AR-Vs in the treated group, and $\text{AR-Vs}_{solvent}$ was the relative peak area of AR-Vs in the solvent control group.

$$\text{Degradation rate of AR-FL} = (1 - \text{AR-FL}_{compound}/\text{AR-FL}_{solvent}) \times 100\% \quad \text{formula (1)}$$

$$\text{Degradation rate of AR-Vs} = (1 - \text{AR-Vs}_{compound}/\text{AR-Vs}_{solvent}) \times 100\% \quad \text{formula (2)}$$

$DC_{50}$ calculation: the compound concentration $DC_{50}$ values at which the degradation rate of AR-FL or AR-Vs was 50% were calculated using OriginPro2015 software and analyzed using DoseResp function according to formula (1) or (2).

Conclusion: the compounds of the present invention had a certain degradation effect on AR-FL or AR-Vs in prostate cancer cells 22RV1.

## Claims

**1.** A compound or a stereoisomer, a deuterated compound, a solvate, a prodrug, a metabolite, a pharmaceutically acceptable salt or a co-crystal thereof, wherein the compound is selected from a compound as shown in general formula (I),

B-L-K (I);

L is selected from -Ak1-Cy1-Ak2-Cy2-Ak3-Cy3-Ak4-Cy4-Ak5-;
Ak1, Ak2, Ak3, Ak4 and Ak5 are each independently selected from $CH_2$, O, C≡C or a bond;
Cy1, Cy2, Cy3 and Cy4 are each independently selected from a bond, a 4- to 7-membered mono-heterocyclic

ring, a 5- to 10-membered fused heterocyclic ring, a 6- to 12-membered spiro-heterocyclic ring, a 7- to 10-membered bridged-heterocyclic ring, 4-to 7-membered monocycloalkyl, 5- to 10-membered fused cycloalkyl, 6- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused heterocyclic ring, spiro-heterocyclic ring or bridged-heterocyclic ring is optionally further substituted with 0 to 4 substituents selected from H, F, Cl, Br, I, OH, COOH, CN, $NH_2$, oxo, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused heterocyclic ring, spiro-heterocyclic ring or bridged-heterocyclic ring contains 1 to 4 heteroatoms selected from O, S and N;

B is selected from

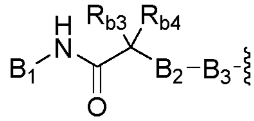
;

$B_1$ is selected from one of the following substituted or unsubstituted groups: 6-membered aryl or 6-membered heteroaryl, which, when substituted, is optionally further substituted with 0 to 4 $R^{b1}$, wherein the heteroaryl contains 1 to 4 heteroatoms selected from O, S and N;

$B_2$ is selected from one of the following substituted or unsubstituted groups: a 5- to 10-membered heterocyclic group or -NHC(=O)-, which, when substituted, is optionally further substituted with 0 to 4 selected from $R^{b2}$, wherein the heterocyclic group contains 1 to 4 heteroatoms selected from O, S and N;

$B_3$ is selected from substituted or unsubstituted 5- to 6-membered aryl or a bond, wherein the 5- to 6-membered aryl, when substituted, is optionally further substituted with 0 to 4 $R^{b2}$,

$R^{b1}$ and $R^{b2}$ are each independently selected from H, F, Cl, Br, I, OH, $NH_2$, CN, $CF_3$, -C(=O)$NH_2$, -C(=O)NH-$C_{1-4}$ alkyl, -C(=O)N($C_{1-4}$ alkyl)$_2$, $C_{1-4}$ alkyl or $C_{1-4}$ alkoxy, wherein the alkyl or alkoxy is optionally further substituted with 0 to 4 substituents selected from H, F, Cl, Br, I or OH;

$R^{b3}$ and $R^{b4}$ are each independently selected from H or $C_{1-6}$ alkyl, wherein the alkyl is optionally further substituted with 0 to 4 substituents selected from H, F, Cl, Br, I, OH, $NH_2$, $C_{1-4}$ alkyl or $C_{1-4}$ alkoxy;

or $R^{b3}$ and $R^{b4}$ together with the carbon atoms to which they are attached form $C_{3-6}$ cycloalkyl or a $C_{3-6}$ mono-heterocyclic ring, wherein the cycloalkyl or mono-heterocyclic ring is optionally further substituted with 0 to 4 substituents selected from H, F, Cl, Br, I, OH, $NH_2$, $C_{1-4}$ alkyl or $C_{1-4}$ alkoxy, and the mono-heterocyclic ring contains 1 to 4 heteroatoms selected from O, S and N;

K is selected from

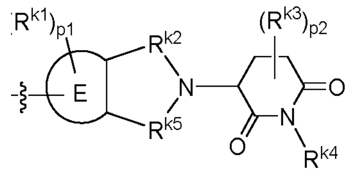
,
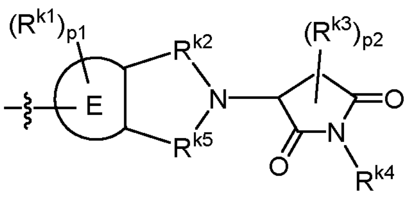
,

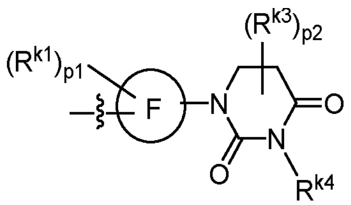
,
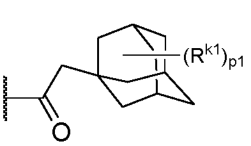
,
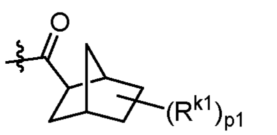
,

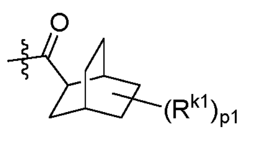
,
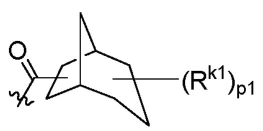
,
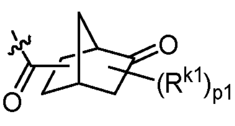
,
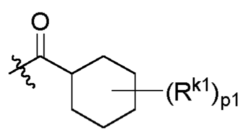
,

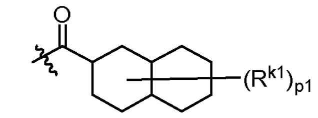 , 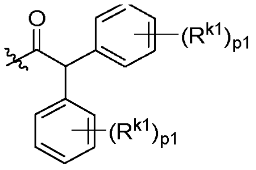 , 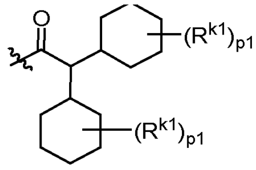 ,

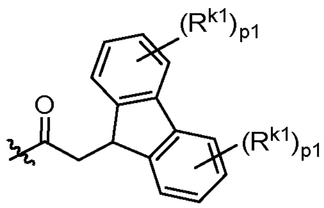 , 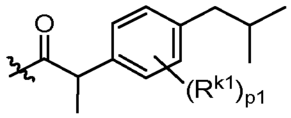 , 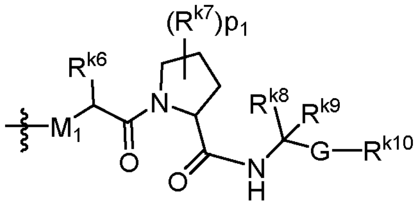 ,

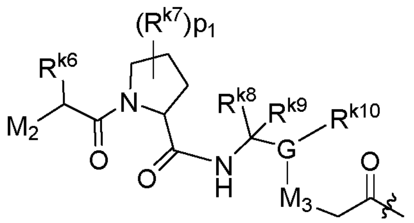 or 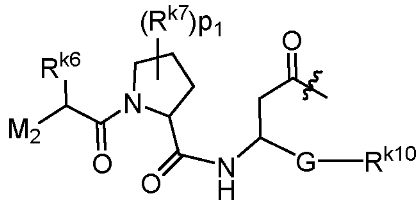 ;

ring E or F is each independently selected from a phenyl ring or a 5- to 6-membered heteroaryl ring, wherein the heteroaryl ring contains 1 to 2 heteroatoms selected from O, S and N;
each $R^{k2}$ is independently selected from $CH_2$, C=O, S=O and $SO_2$;
$R^{k1}$, $R^{k3}$ or $R^{k4}$ is each independently selected from H, F, Cl, Br, I, OH, $NH_2$, $CF_3$, CN, COOH, $C_{1-4}$ alkyl or $C_{1-4}$ alkoxy;
$R^{k5}$ is selected from C=O or

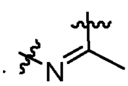 ;

;
$M_1$ is selected from a bond, $-CH_2-C(=O)NH-$ or $-C(=O)CH_2NH-$;
$M_2$ is selected from $-NHC(=O)-C_{1-6}$ alkyl or $-NHC(=O)-C_{3-6}$ cycloalkyl, wherein the alkyl or cycloalkyl is optionally further substituted with 0 to 4 substituents selected from H, F, Cl, Br, I, OH, $NH_2$, $C_{1-4}$ alkyl or $C_{1-4}$ alkoxy;
$M_3$ is selected from -NH- or -O-;
$R^{k6}$ is selected from $C_{1-6}$ alkyl, wherein the alkyl is optionally further substituted with 0 to 4 substituents selected from H, F, Cl, Br, I, OH, $C_{1-6}$ alkyl or $C_{3-6}$ cycloalkyl;
each $R^{k7}$ is independently selected from H, F, Cl, Br, I, OH, SH, $C_{1-6}$ alkyl, $C_{1-6}$ alkoxy, $C_{1-6}$ alkylthio or $C_{1-6}$ alkylformyloxy, wherein the alkyl, alkoxy or alkylthio is optionally further substituted with 0 to 4 substituents selected from H, F, Cl, Br, I, OH, $C_{1-4}$ alkyl or $C_{1-4}$ alkoxy;
$R^{k8}$ and $R^{k9}$ are each independently selected from H, $C_{1-6}$ alkyl or $C_{3-6}$ cycloalkyl, wherein the alkyl or cycloalkyl is optionally further substituted with 0 to 4 substituents selected from H, F, Cl, Br, I, OH, $NH_2$, $C_{1-4}$ alkyl or $C_{1-4}$ alkoxy;
$R^{k10}$ is selected from 5- to 6-membered heteroaryl, wherein the heteroaryl is optionally further substituted with 0 to 4 substituents selected from H, F, Cl, Br, I, OH, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl;

G is selected from 6- to 10-membered aryl or 5- to 10-membered heteroaryl, wherein the aryl or heteroaryl is optionally further substituted with 0 to 4 substituents selected from H, F, Cl, Br, I, OH, $CF_3$, CN, $C_{1-4}$ alkyl, halogen-substituted $C_{1-4}$ alkyl, hydroxyl-substituted $C_{1-4}$ alkyl, $C_{1-4}$ alkoxy or $C_{3-6}$ cycloalkyl; and
p1 or p2 is each independently selected from 0, 1, 2, 3 or 4.

**2.** The compound or the stereoisomer, deuterated compound, solvate, prodrug, metabolite, pharmaceutically acceptable salt or co-crystal thereof according to claim 1, wherein

Cy1, Cy2, Cy3 and Cy4 are each independently selected from a bond, a 4- to 7-membered nitrogen-containing mono-heterocyclic ring, a 5- to 10-membered nitrogen-containing fused heterocyclic ring, a 7- to 10-membered bridged-heterocyclic ring, or a 6- to 12-membered nitrogen-containing spiro-heterocyclic ring, wherein the mono-heterocyclic ring, fused heterocyclic ring, bridged-heterocyclic ring or spiro-heterocyclic ring is optionally further substituted with 0 to 4 substituents selected from H, F, Cl, Br, I, OH, COOH, CN, $NH_2$, oxo, $C_{1-4}$ alkyl, halogen-substituted $C_{1-4}$ alkyl, hydroxyl-substituted $C_{1-4}$ alkyl or $C_{1-4}$ alkoxy, and the mono-heterocyclic ring, fused heterocyclic ring, bridged-heterocyclic ring or spiro-heterocyclic ring contains 1 to 4 heteroatoms selected from O, S and N; and
K is selected from

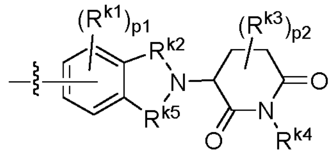 , 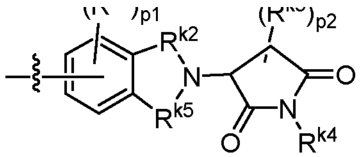 ,

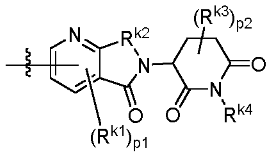 , 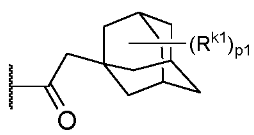 , 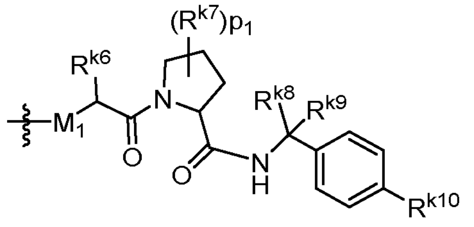 ,

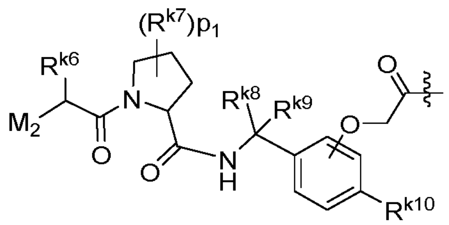 or 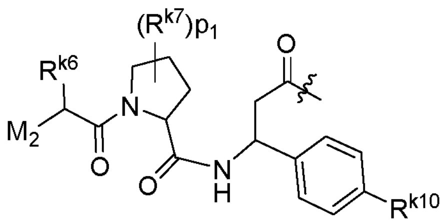 .

**3.** The compound or the stereoisomer, deuterated compound, solvate, prodrug, metabolite, pharmaceutically acceptable salt or co-crystal thereof according to claim 2, wherein

Cy1, Cy2, Cy3 and Cy4 are each independently selected from a bond or one of the following substituted or unsubstituted groups: azetidinyl, azacyclopentyl, azacyclohexyl, piperidine, morpholine, piperazine, cyclopropyl-fused-azetidinyl, cyclopropyl-fused-azacyclopentyl, cyclopropyl-fused-azacyclohexyl, cyclopropyl-fused-piperidine, cyclobutyl-fused-azetidinyl, cyclobutyl-fused-azacyclopentyl, cyclobutyl-fused-azacyclohexyl, cyclobutyl-fused-piperidine, cyclopentyl-fused-azetidinyl, cyclopentyl-fused-azacyclopentyl, cyclopentyl-fused-azacyclohexyl, cyclopentyl-fused-piperidine, cyclohexyl-fused-azetidinyl, cyclohexyl-fused-azacyclopentyl, cyclohexyl-fused-azacyclohexyl, cyclohexyl-fused-piperidine, azetidinyl-fused-azetidinyl, azetidinyl-fused-azacyclopentyl, azetidinyl-fused-azacyclohexyl, azetidinyl-fused-piperidine, azacyclopentyl-fused-azetidinyl, azacyclopentyl-fused-azacyclopentyl, azacyclopentyl-fused-azacyclohexyl, azacyclopentyl-fused-piperidine, azacyclohexyl-fused-azetidinyl, azacyclohexyl-fused-azacyclopentyl, azacyclohexyl-fused-azacyclohexyl, azacyclohexyl-fused-piperidine, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azetidinyl, azacyclopentyl-spiro-azacyclopentyl, azacyclopentyl-spiro-azacyclohexyl, azacyclohexyl-spiro-azetidinyl, azacyclohexyl-spiro-azacyclopentyl, azacyclohexyl-spiro-azacyclohexyl, cyclobutyl-spiro-piperidine, cyclopentyl-spiro-piperidine, cyclohexyl-spiro-piperidine, azetidinyl-spiro-piperidine, azacyclopentyl-spiro-piperidine, azacyclohexyl-spiro-piperidine,

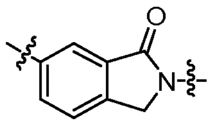
,

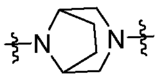
, 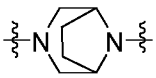
, 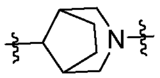
, 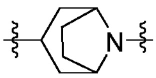
, 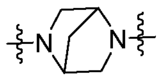
, 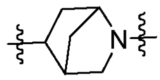
,

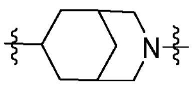
, 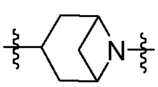
, 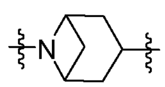
, 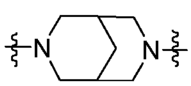
, 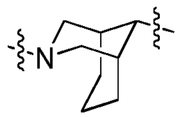
,

,

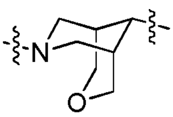
or
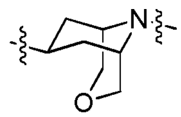
,

, which, when substituted, is optionally further substituted with 0 to 4 substituents selected from H, F, Cl, Br, I, OH, $NH_2$, COOH, CN, oxo, $C_{1-4}$ alkyl, halogen-substituted $C_{1-4}$ alkyl, hydroxyl-substituted $C_{1-4}$ alkyl or $C_{1-4}$ alkoxy;

$R^{b1}$ and $R^{b2}$ are each independently selected from H, F, Cl, Br, I, OH, $NH_2$, CN, $CF_3$, $-C(=O)NH_2$, $-C(=O)NH-CH_3$, $-C(=O)N(CH_3)_2$, methyl, ethyl, propyl, isopropyl, methoxy or ethoxy, wherein the methyl, ethyl, propyl, isopropyl, methoxy or ethoxy is optionally further substituted with 0 to 4 substituents selected from H, F, Cl, Br, I or OH;

K is selected from

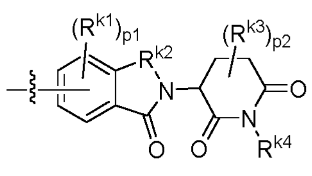
, 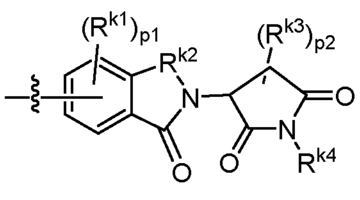
,

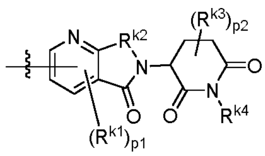, 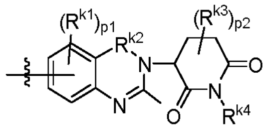, 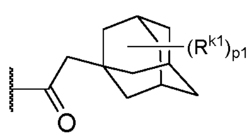,

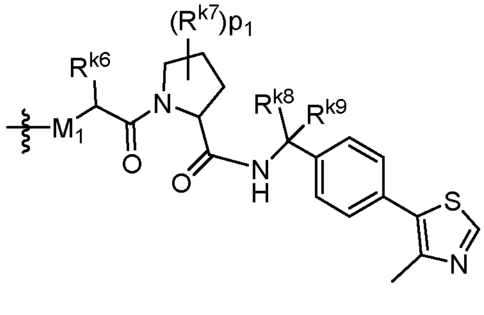, 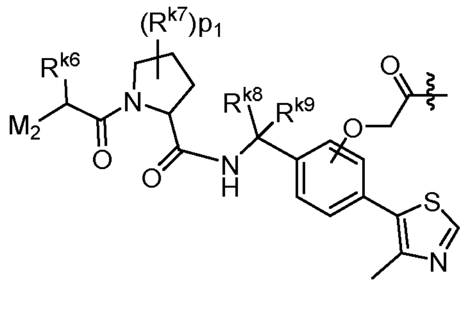

or

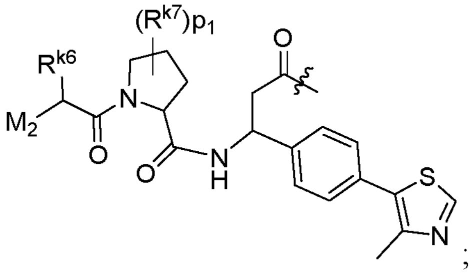;

each $R^{k2}$ is independently selected from $CH_2$ or C=O;
$R^{k1}$, $R^{k3}$ or $R^{k4}$ is each independently selected from H, $CH_3$, F, Cl, Br, I, OH or $NH_2$;
$M_1$ is selected from a bond, $-CH_2-C(=O)NH-$ or $-C(=O)CH_2NH-$;
$M_2$ is selected from -NHC(=O)-methyl, -NHC(=O)-ethyl, -NHC(=O)-cyclopropyl, -NHC(=O)-cyclobutyl, -NHC(=O)-cyclopentyl or -NHC(=O)-cyclohexyl, wherein the methyl, ethyl, cyclopropyl, cyclobutyl, cyclopentyl or cyclohexyl is optionally further substituted with 0 to 4 substituents selected from H, F, Cl, Br, I, OH, $NH_2$, $C_{1-4}$ alkyl or $C_{1-4}$ alkoxy;
$R^{k6}$ is selected from methyl, ethyl, propyl, isopropyl, tert-butyl, isobutyl or sec-butyl;
each $R^{k7}$ is independently selected from H, F, OH, SH, methyl, methoxy or $-SCH_3$;
$R^{k8}$ and $R^{k9}$ are each independently selected from H, methyl, ethyl, cyclopropyl or cyclobutyl; and
p1 or p2 is each independently selected from 0, 1 or 2.

**4.** The compound or the stereoisomer, deuterated compound, solvate, prodrug, metabolite, pharmaceutically acceptable salt or co-crystal thereof according to claim 3, wherein

Cy1, Cy2, Cy3 and Cy4 are each independently selected from one of the following substituted or unsubstituted groups: a bond,

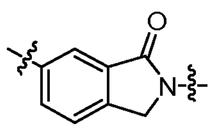, 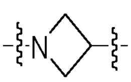, 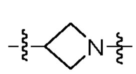,

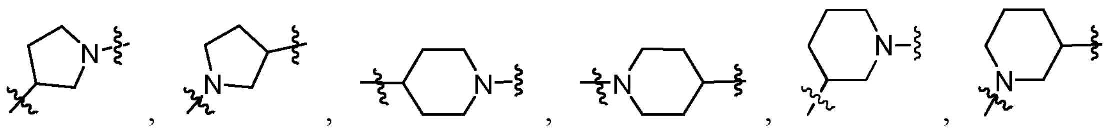

, , , , , ,

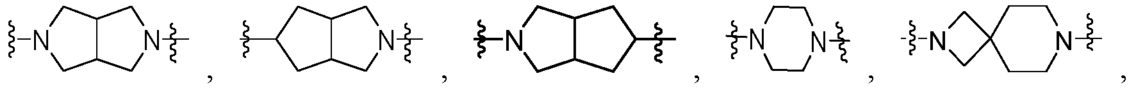

, , , , ,

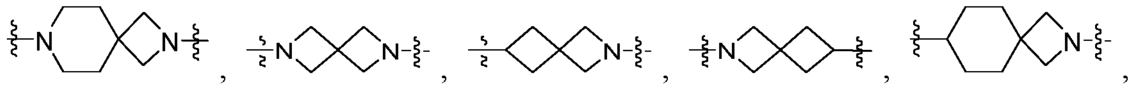

, , , , ,

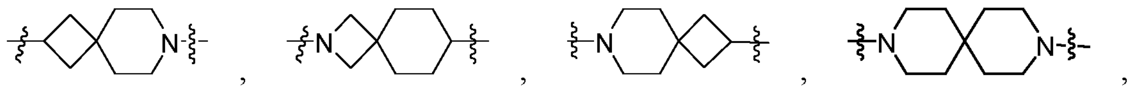

, , , ,

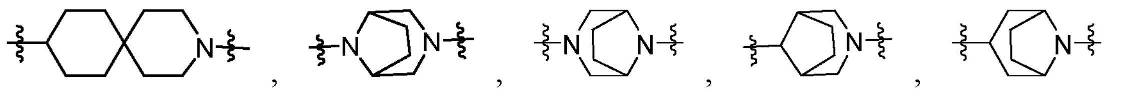

, , , , ,

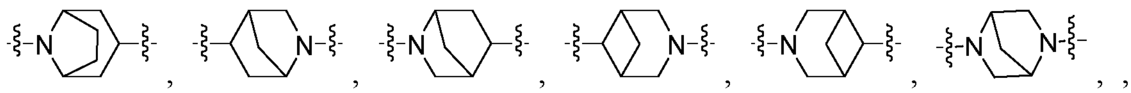

, , , , , , ,

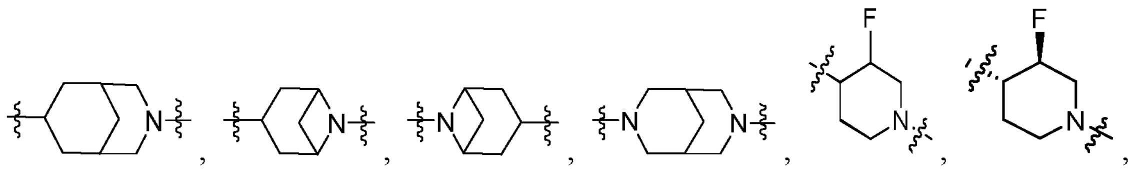

, , , , , ,

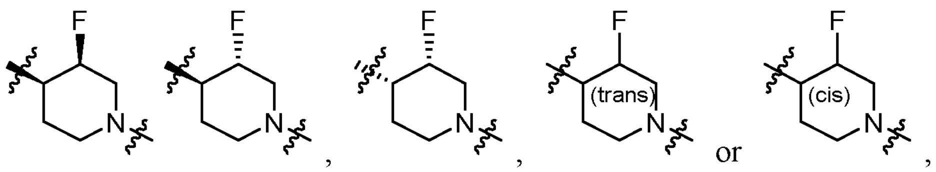

, , or ,

which, when substituted, is optionally further substituted with 0 to 4 substituents selected from H, F, $CF_3$, methyl, oxo, hydroxymethyl, COOH, CN or $NH_2$;

B is selected from

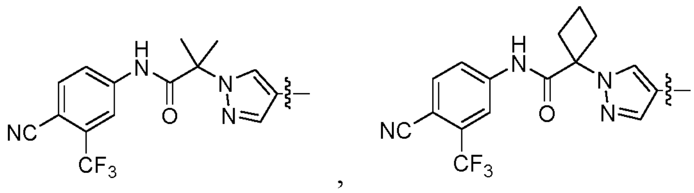

, ,

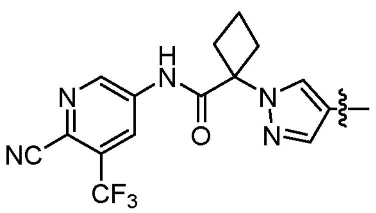,
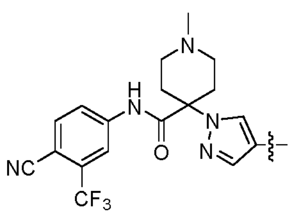,
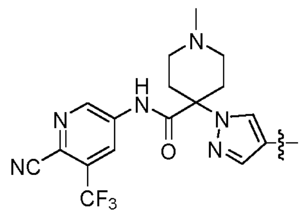,

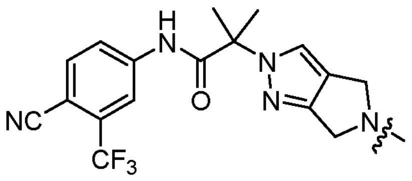,
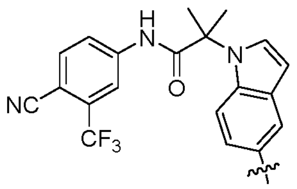,
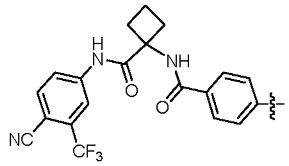,

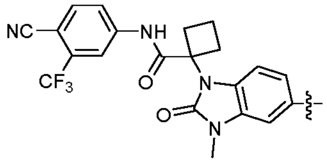,
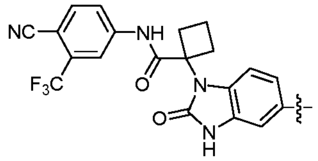,
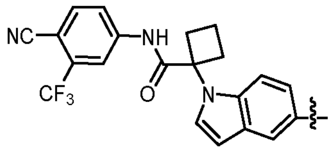

and

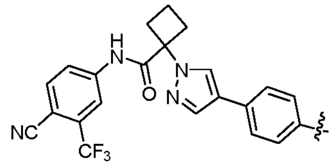;

and
K is selected from

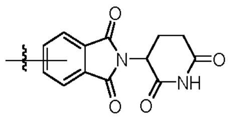,
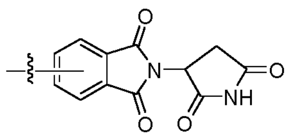,
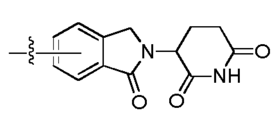,

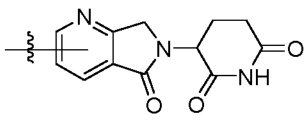,
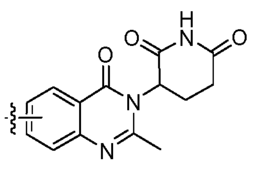,
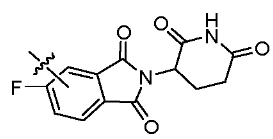,
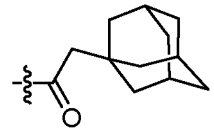,

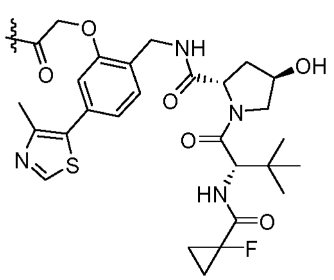,
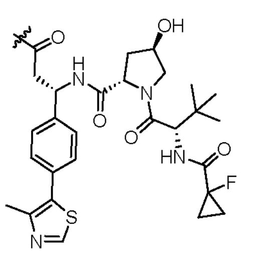,
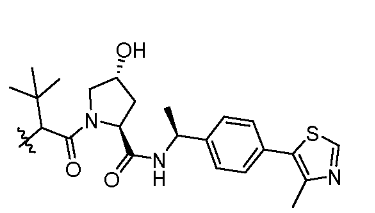,

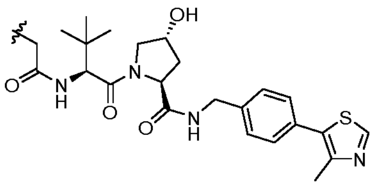,
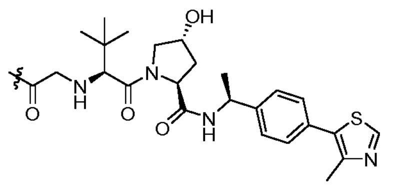,

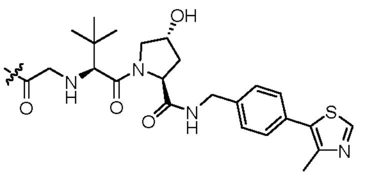,
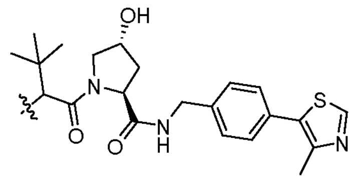

or

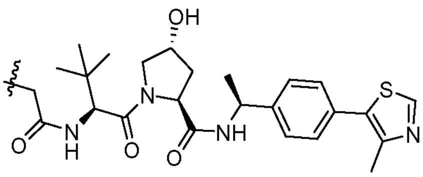.

5. The compound or the stereoisomer, deuterated compound, solvate, prodrug, metabolite, pharmaceutically acceptable salt or co-crystal thereof according to claim 4, wherein

L is selected from a bond,

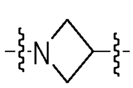, 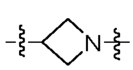, 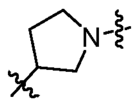, 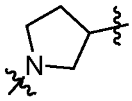,

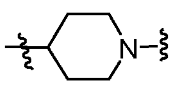, 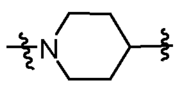, 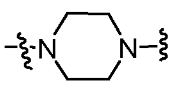, 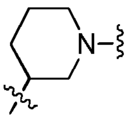, 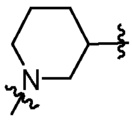, 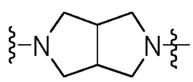,

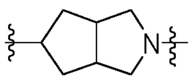, 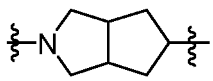, 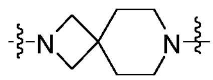, 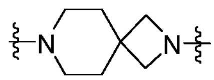, 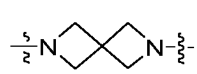,

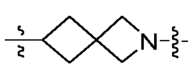, 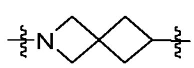, 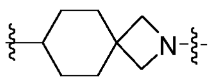, 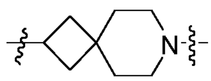, 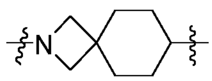,

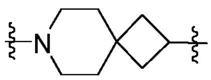, 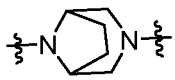, 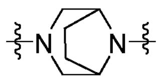, 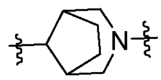, 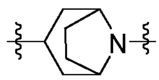, 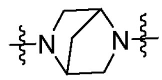,

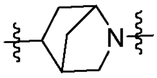, 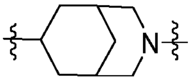, 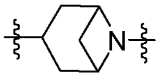, 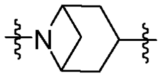, 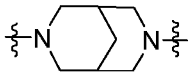, 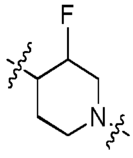, 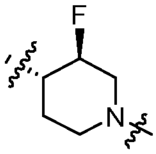, 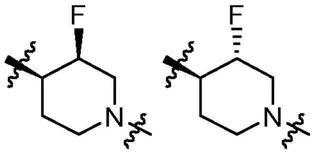, 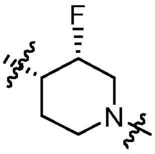, 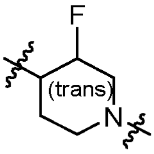 or 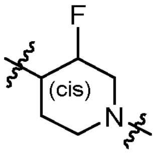,

wherein the left side of L is linked to B;
or L is selected from

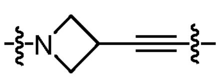, 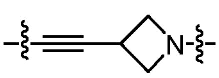, 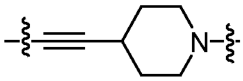,

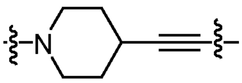, 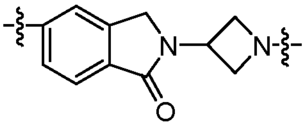, 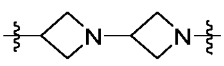, 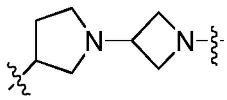,

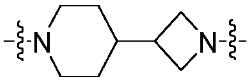, 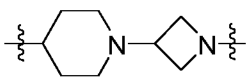, 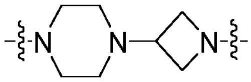, 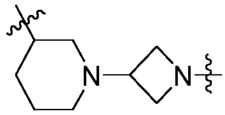,

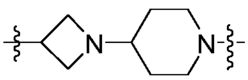, 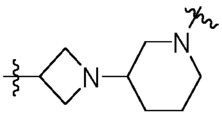, 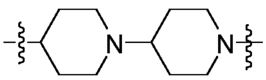, 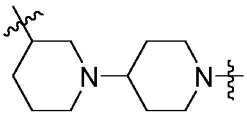,

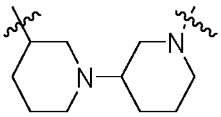, 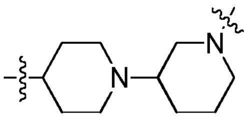, 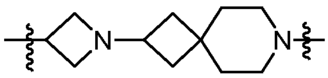,

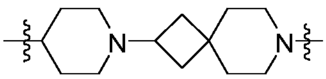, 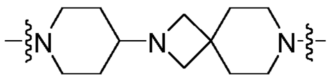, 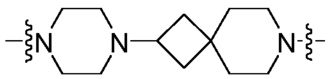,

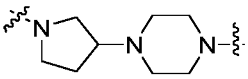, 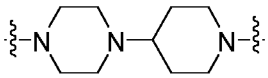, 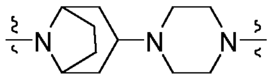, 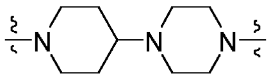,

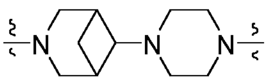, 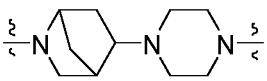, 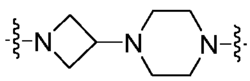,

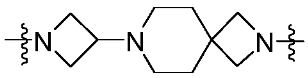 , 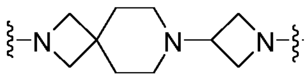 , 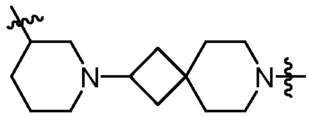 ,

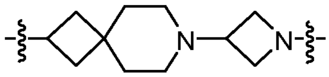 , 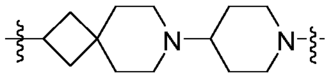 , 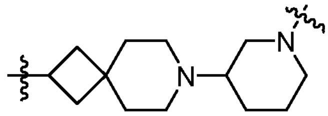 ,

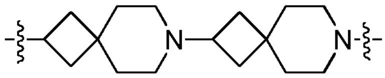 , 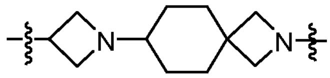 , 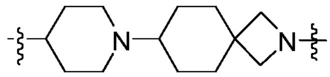 ,

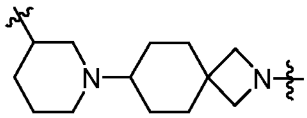 , 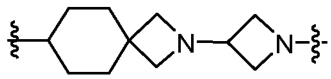 , 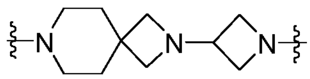 ,

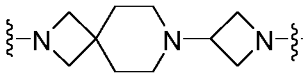 , 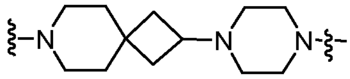 , 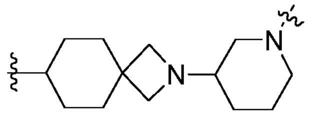 ,

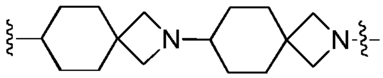 , 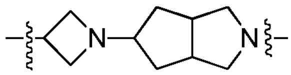 , 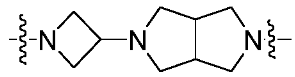 ,

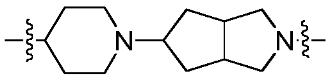 , 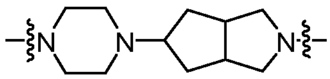 , 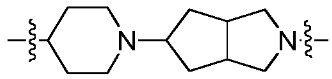 ,

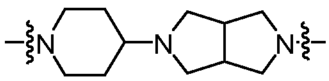 , 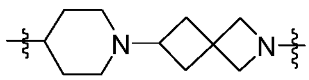 , 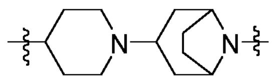 ,

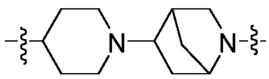 , 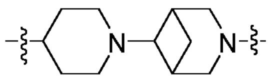 , 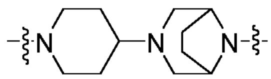 , 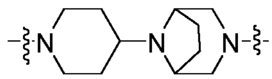

, 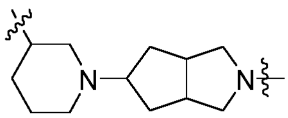 , 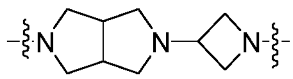 , 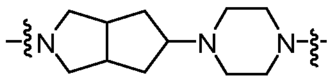 ,

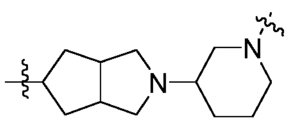 , 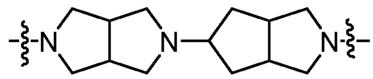 , 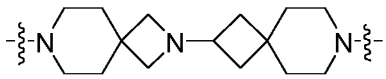 ,

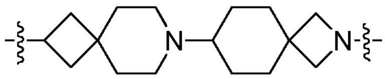 , 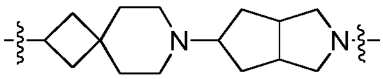 ,

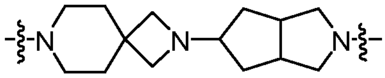 , 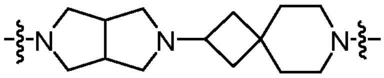 ,

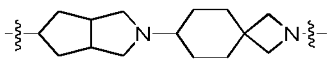 , 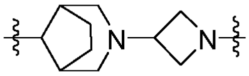 , 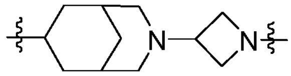 ,

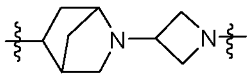 , 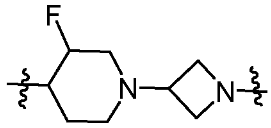 , 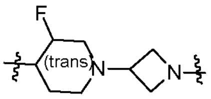 , 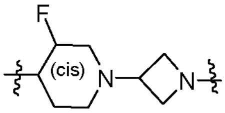 ,

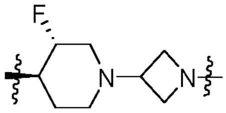 , 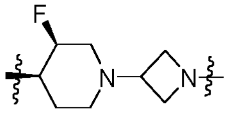 , 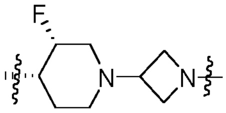 , 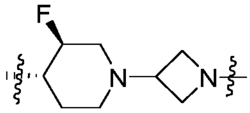 ,

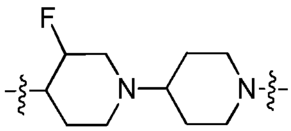 , 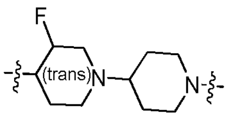 , 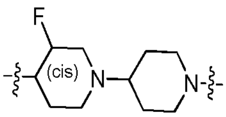 , 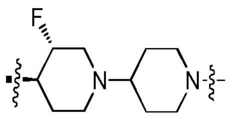 ,

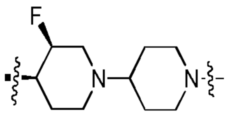 , 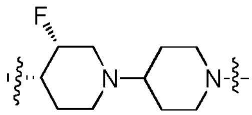 , 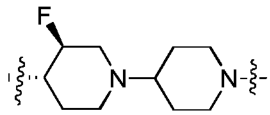 , 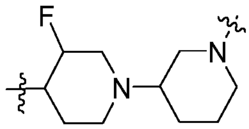 ,

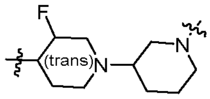 , 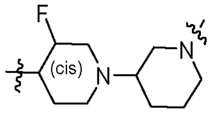 , 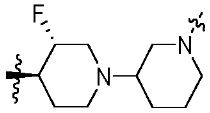 , 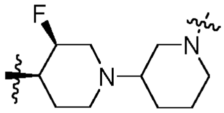 ,

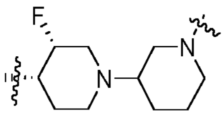 , 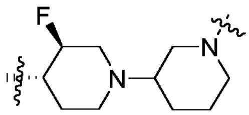 , 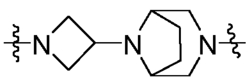 , 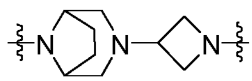 ,

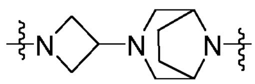 , 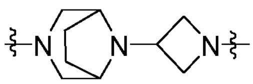 , 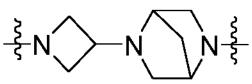 , 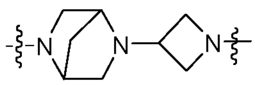 ,

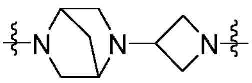 , 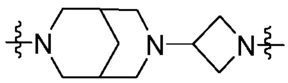 , 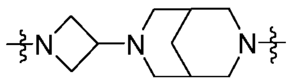 or

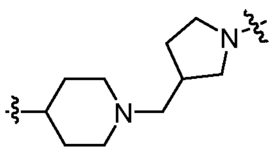,

wherein the left side of L is linked to B;
or L is selected from

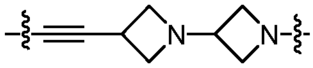, 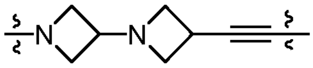,

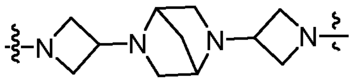, 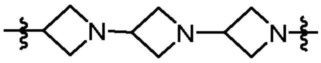, 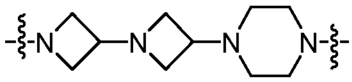,

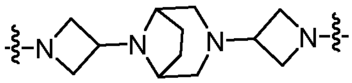, 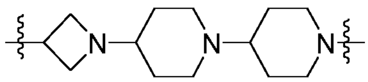, 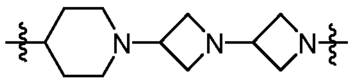,

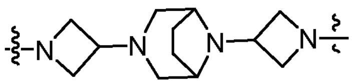, 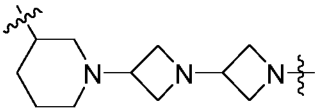, 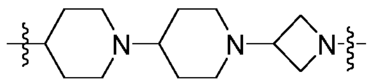,

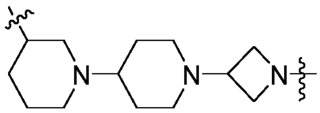, 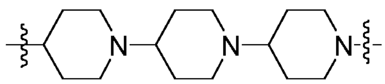, 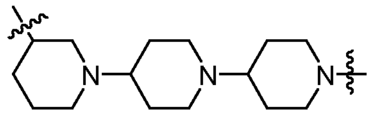,

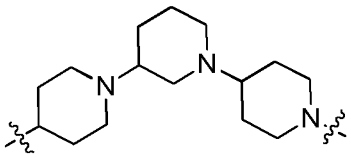, 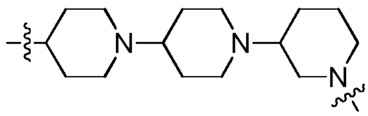, 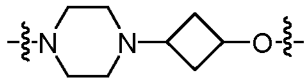,

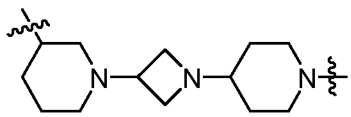, 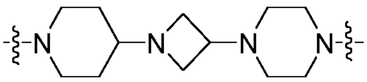, 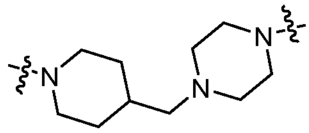

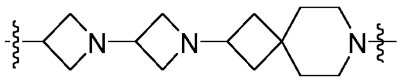, 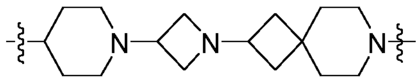,

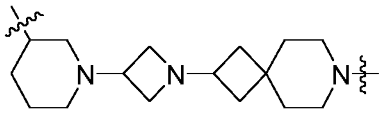, 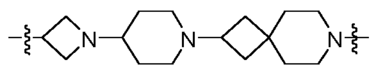,

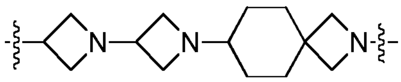, 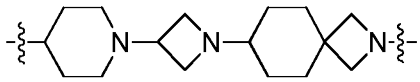,

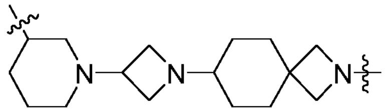 ,
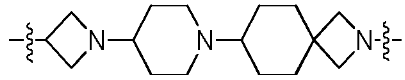 ,

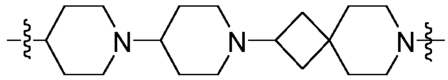 ,
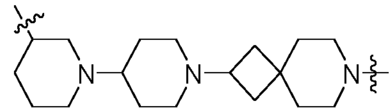 ,

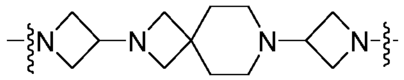 ,
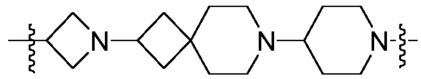 ,

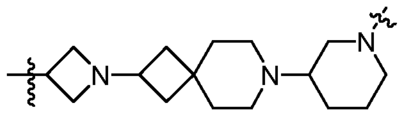 ,
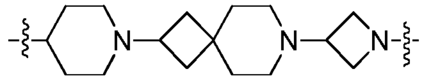 ,

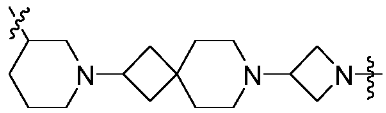 ,
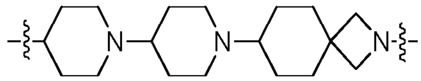 ,

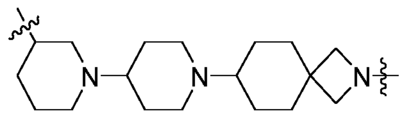 ,
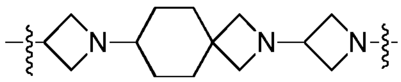 ,

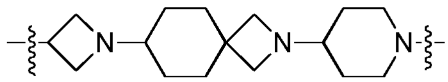 ,
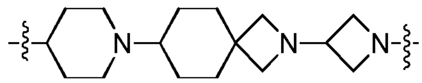 ,

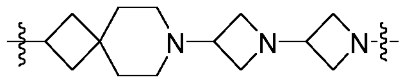 ,
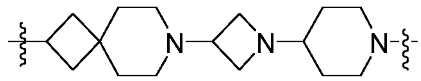 ,

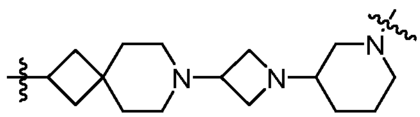 ,
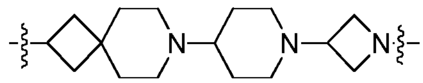 ,

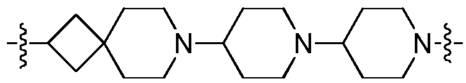 ,
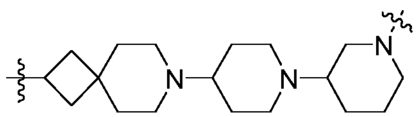 ,

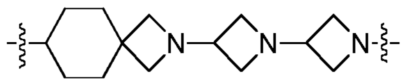 ,
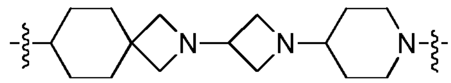 ,

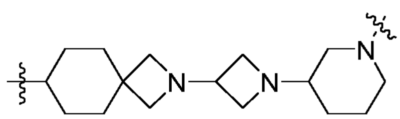 ,
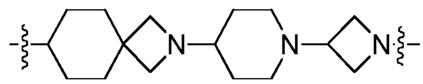 ,

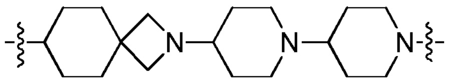,
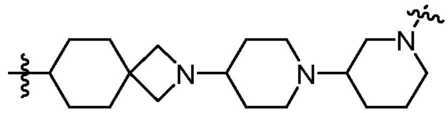,

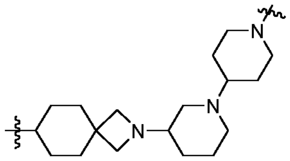,
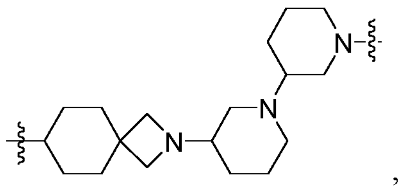,
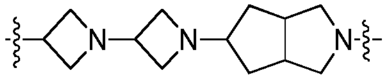,

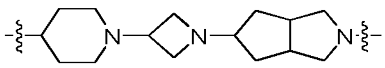,
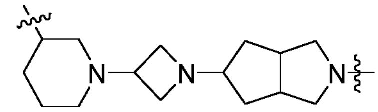,

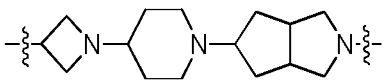,
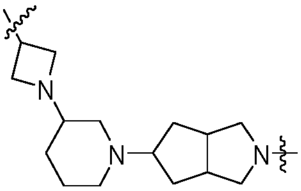,

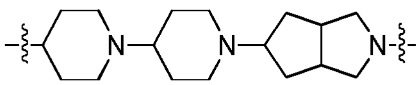,
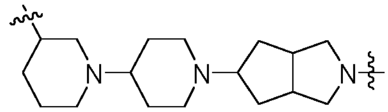,

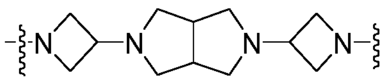,
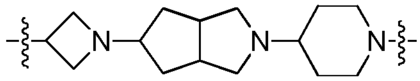,

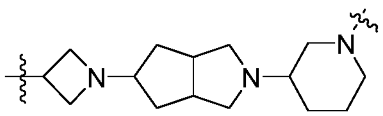,
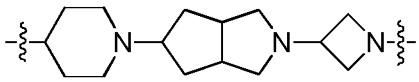,

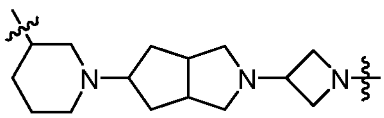,
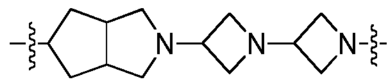,

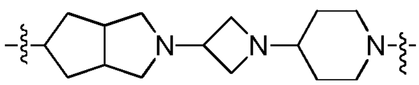,
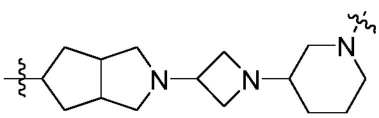,

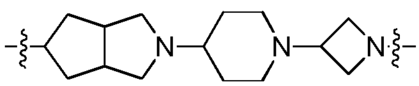,
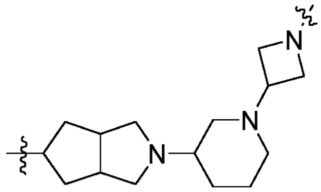,

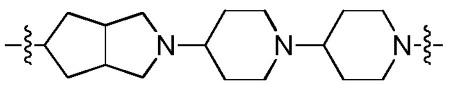 , 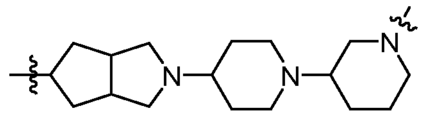 ,

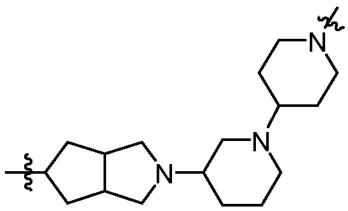 , 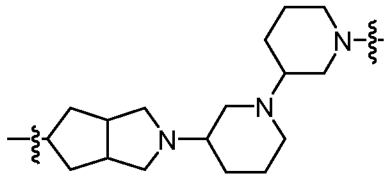 ,

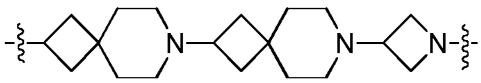 , 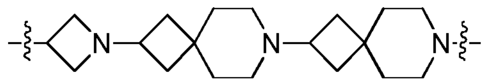 ,

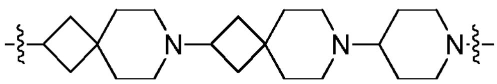 , 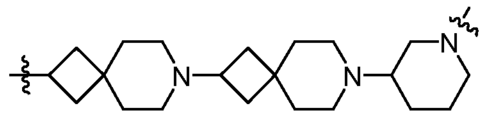 ,

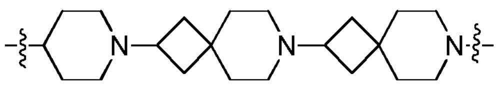 , 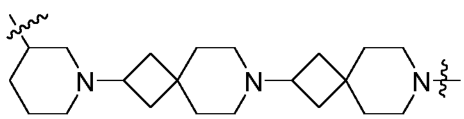 ,

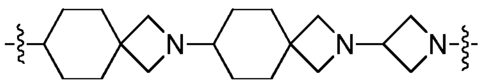 , 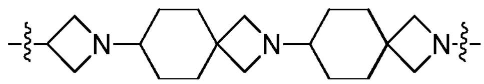 ,

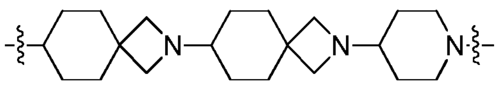 , 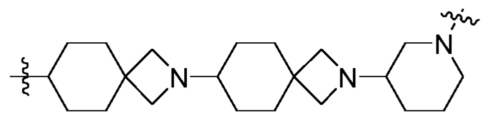 ,

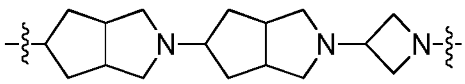 , 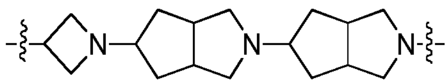 ,

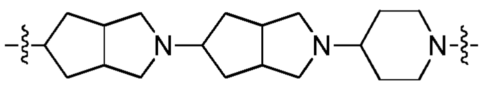 , 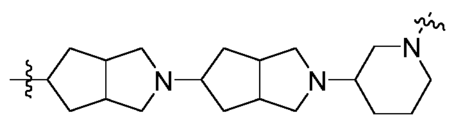 ,

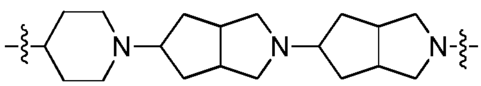 , 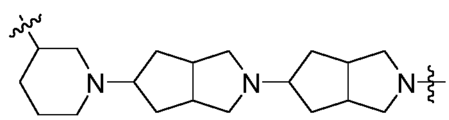 ,

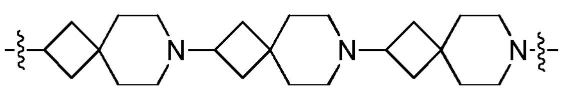 , 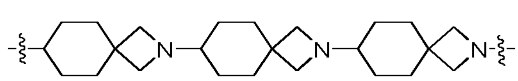 ,

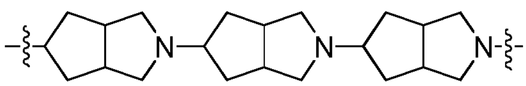 , 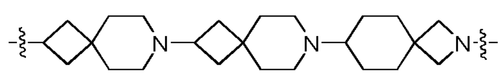 ,

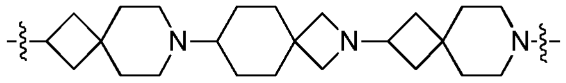, 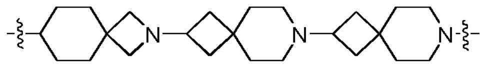,

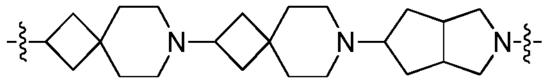, 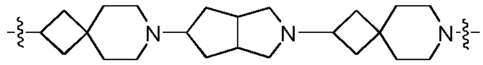,

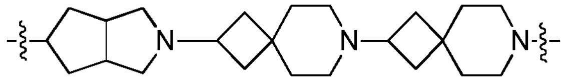, 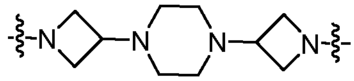,

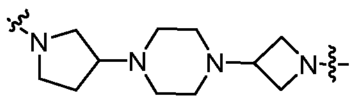, 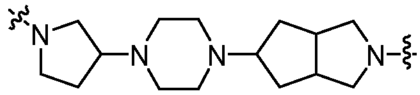,

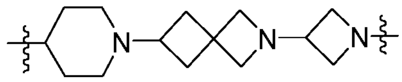, 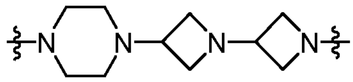,

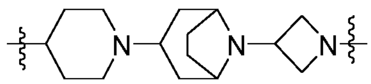, 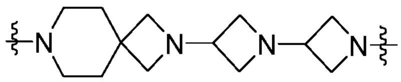,

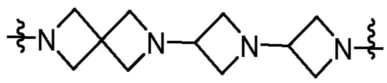, 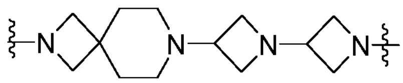,

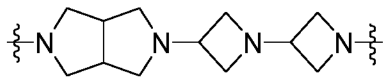, 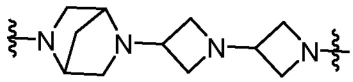,

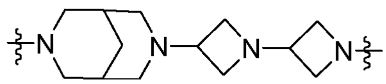, 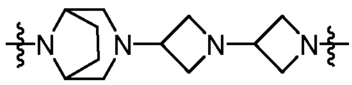, 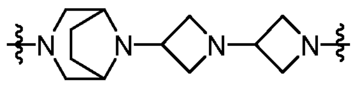,

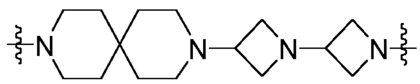, 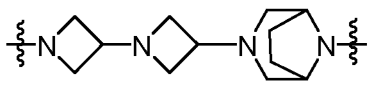,

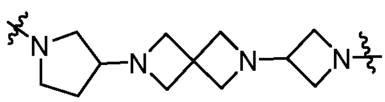, 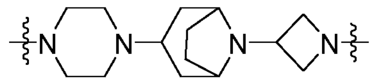,

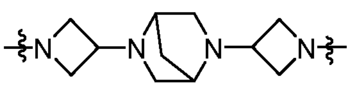 or 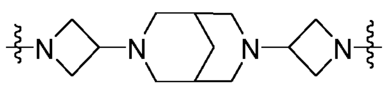,

wherein the left side of L is linked to B;
or L is selected from

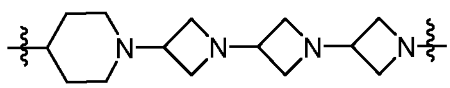,

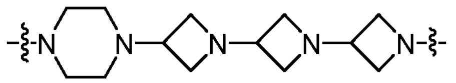, 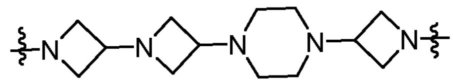,

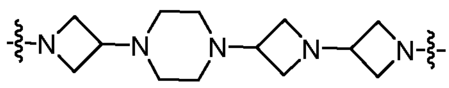 and 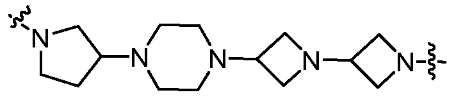,

wherein the left side of L is linked to B.

**6.** The compound or the stereoisomer, deuterated compound, solvate, prodrug, metabolite, pharmaceutically acceptable salt or co-crystal thereof according to claim 5, wherein
K is selected from

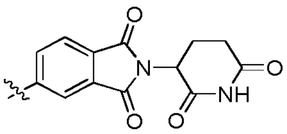, 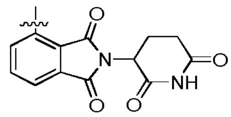, 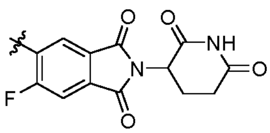

, 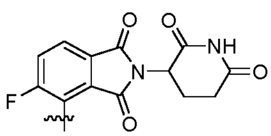, 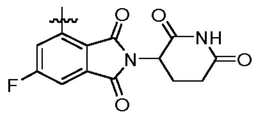, 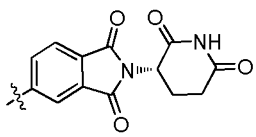, 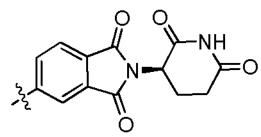

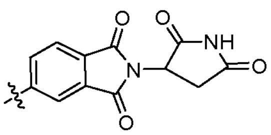, 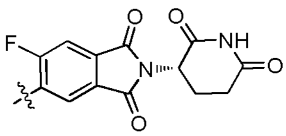, 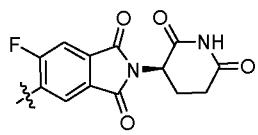, 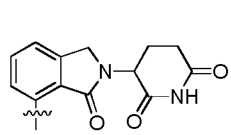,

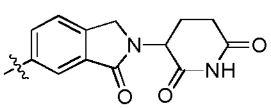, 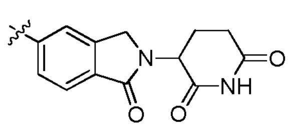, 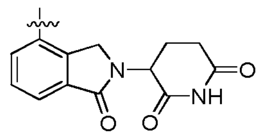,

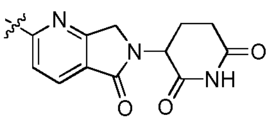, 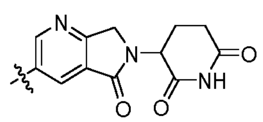, 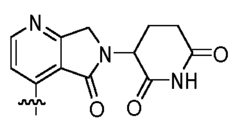, 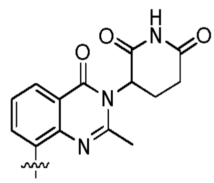,

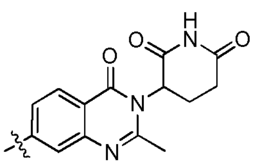, 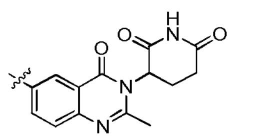, 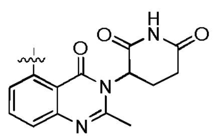, 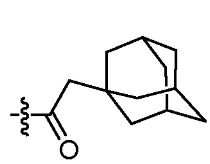,

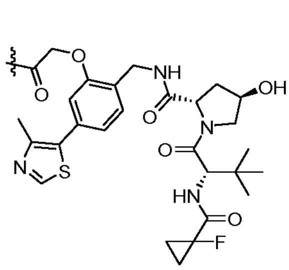, 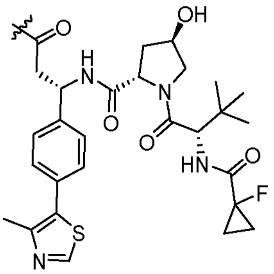, 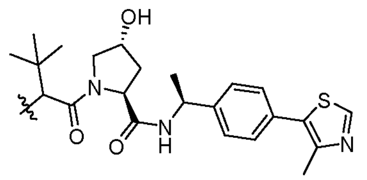,

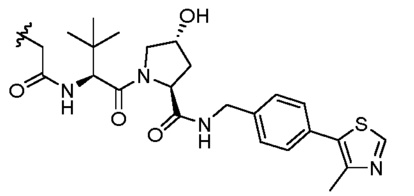, 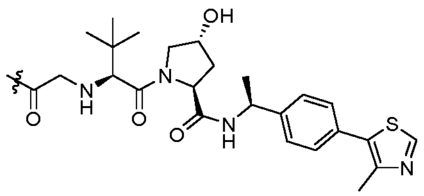

or

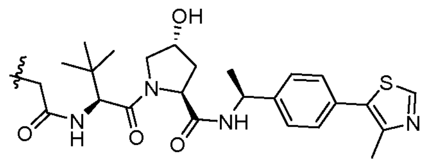.

7. The compound or the stereoisomer, deuterated compound, solvate, prodrug, metabolite, pharmaceutically acceptable salt or co-crystal thereof according to claim 5, wherein K is selected from

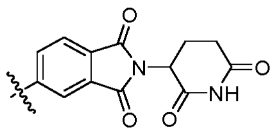, 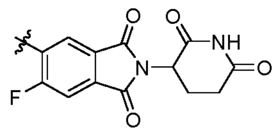,

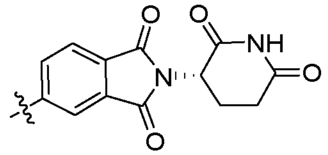, 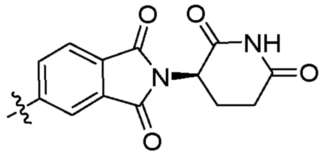, 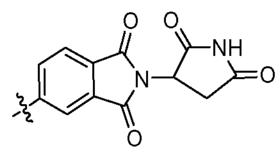,

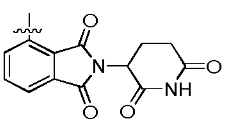, 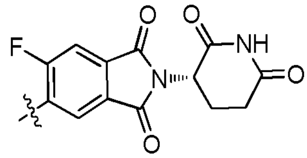, 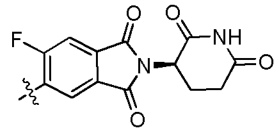,

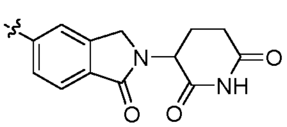, 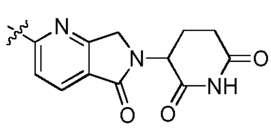, 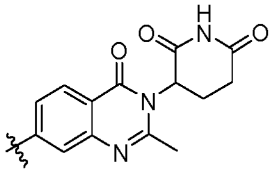, 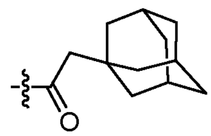,

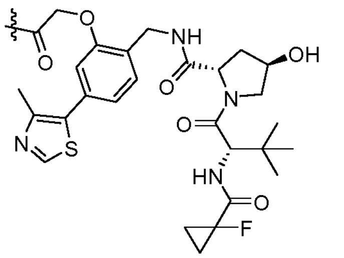,
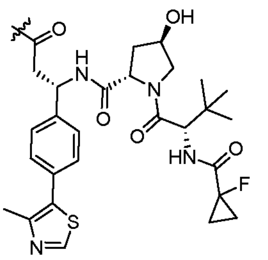,
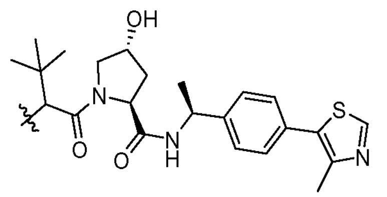,

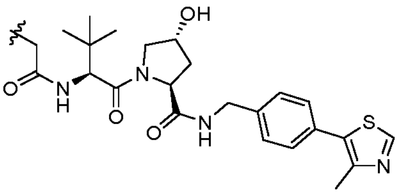,
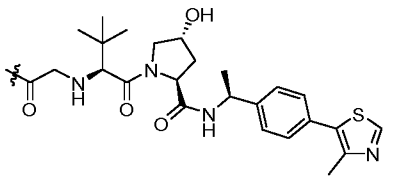

or

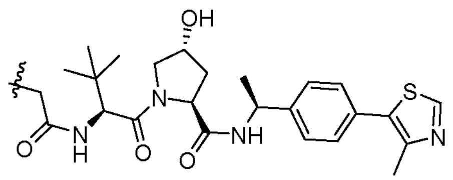.

8. The compound or the stereoisomer, deuterated compound, solvate, prodrug, metabolite, pharmaceutically acceptable salt or co-crystal thereof according to claim 7, wherein the compound has a structure selected from one of the following structures:

| | |
|---|---|
| 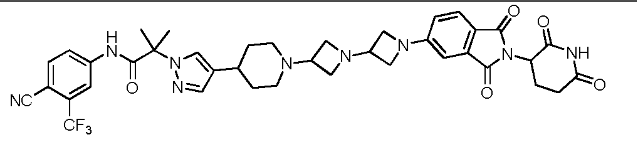 | 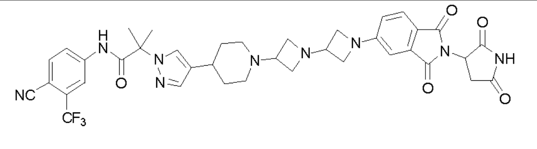 |
| 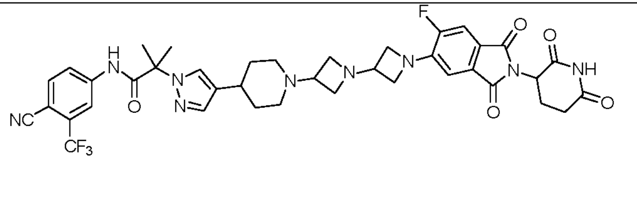 | 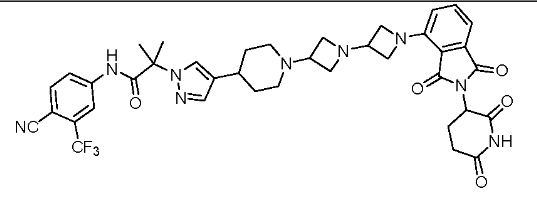 |
| 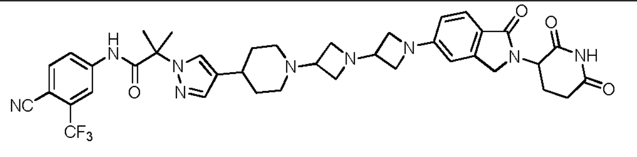 | 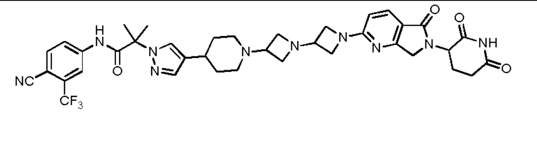 |
| 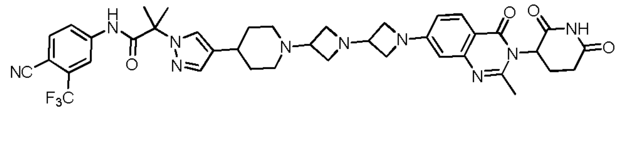 | 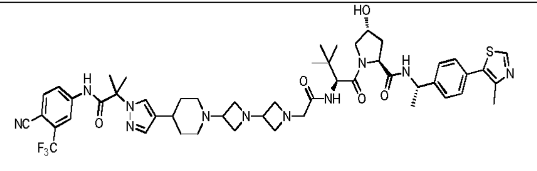 |
| 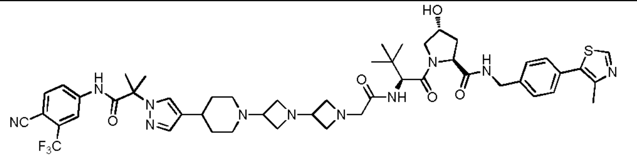 | 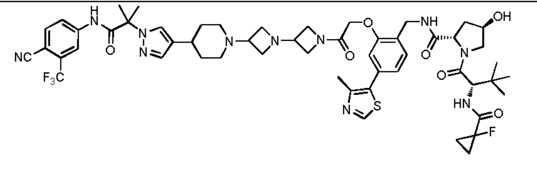 |
| 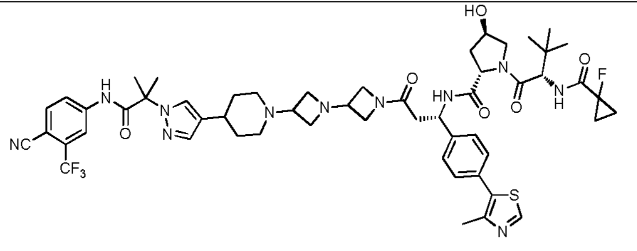 | 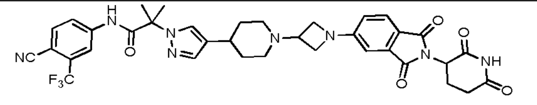 |
| 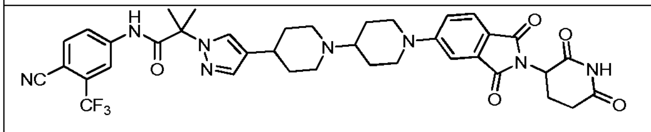 | 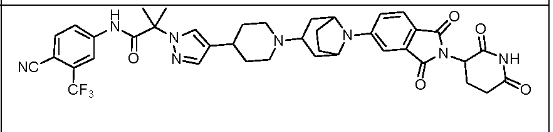 |
| 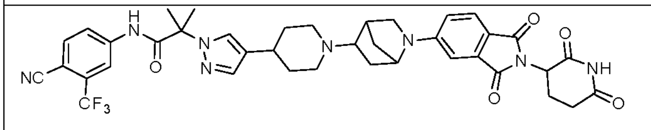 | 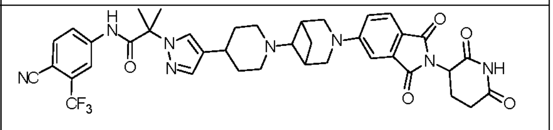 |
| 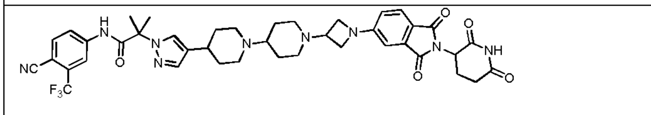 | 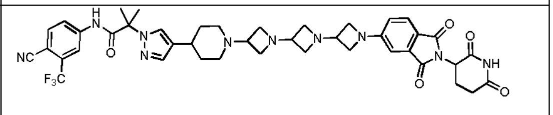 |
| 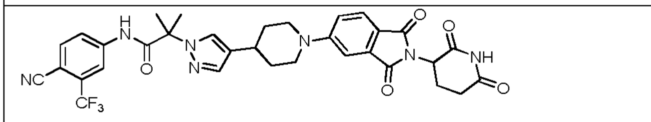 | 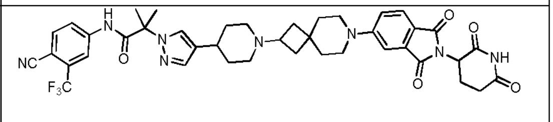 |
| 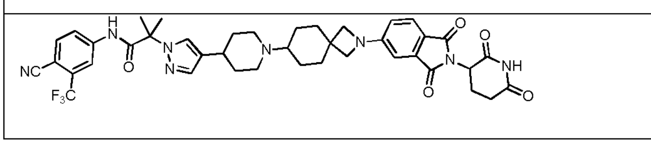 | 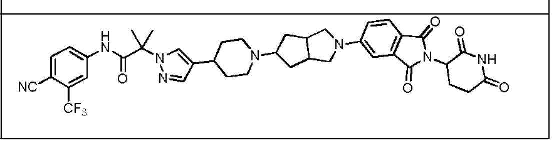 |

| | |
|---|---|
| 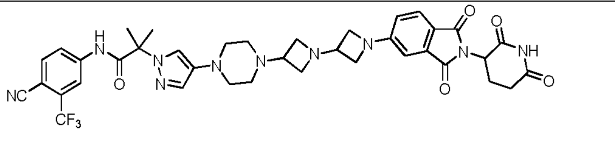 | 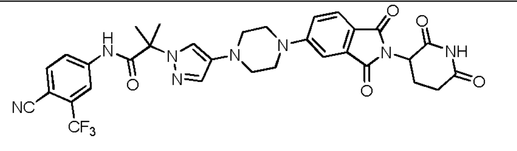 |
| 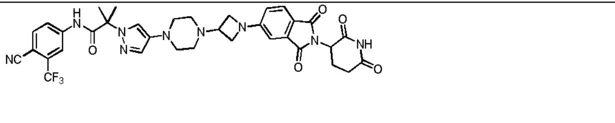 | 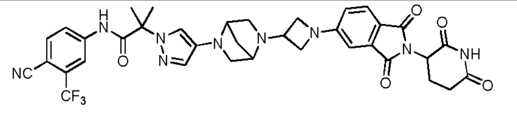 |
| 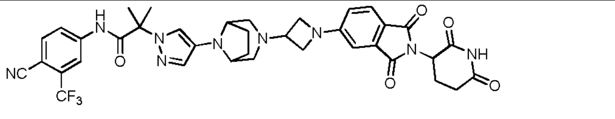 | 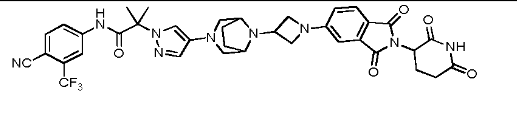 |
| 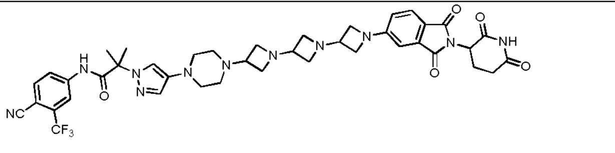 | 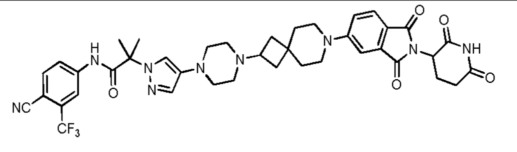 |
| 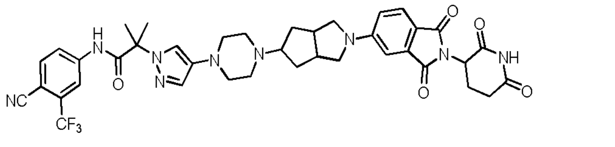 | 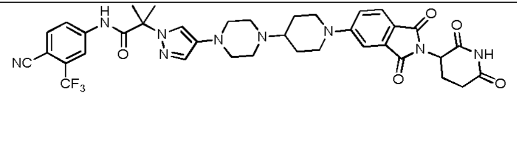 |
| 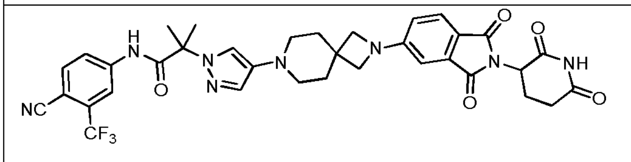 | 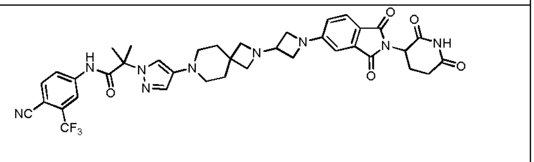 |
| 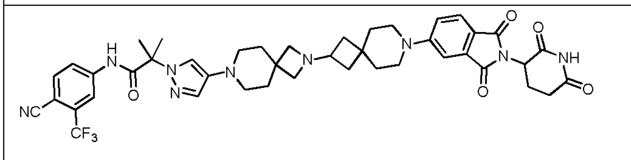 | 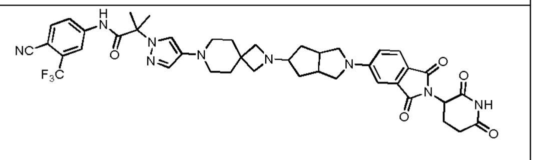 |
| 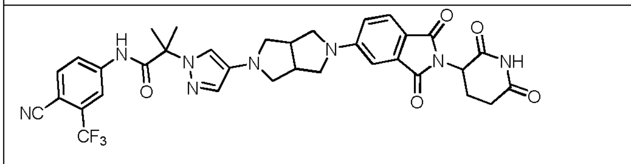 | 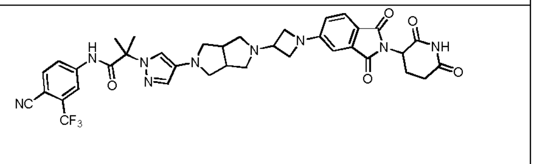 |
| 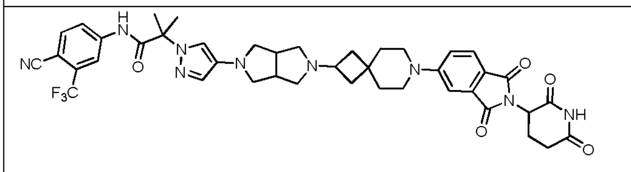 | 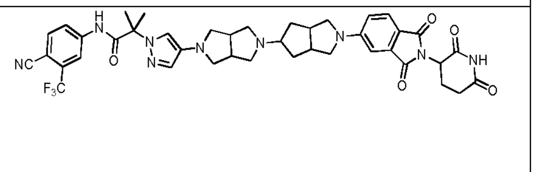 |
| 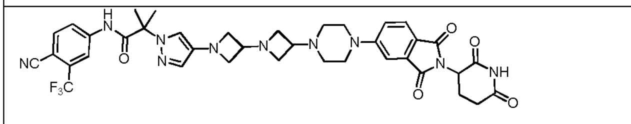 | 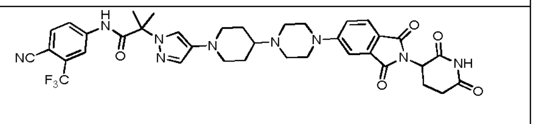 |
| 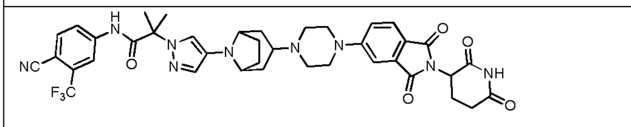 | 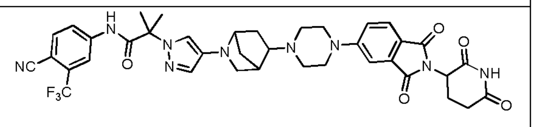 |
| 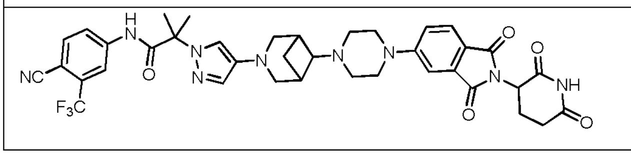 | 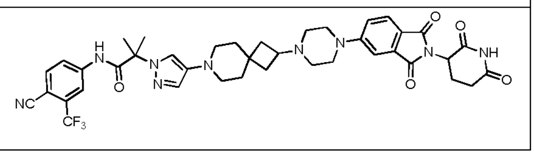 |

| | |
|---|---|
| 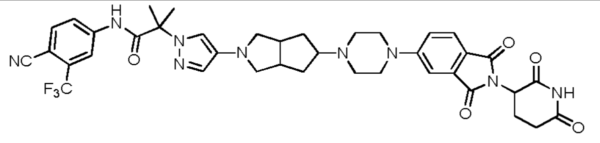 | 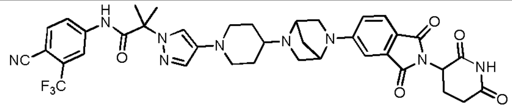 |
| 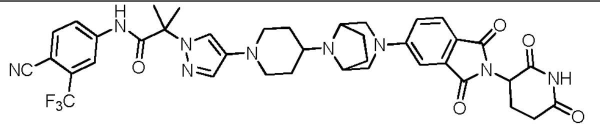 | 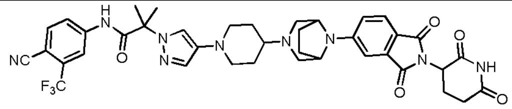 |
| 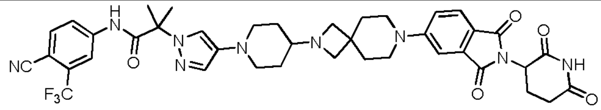 | 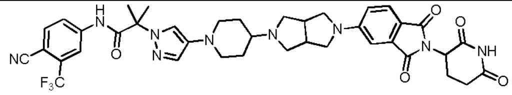 |
| 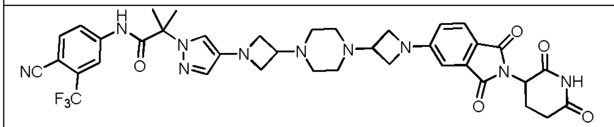 | 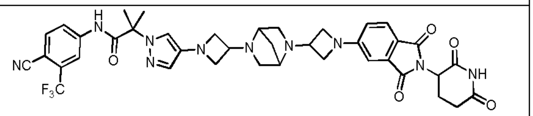 |
| 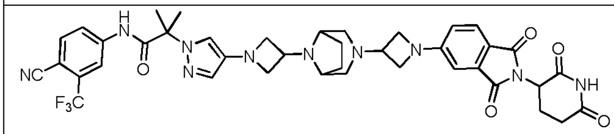 | 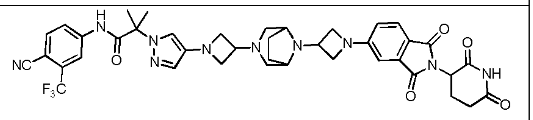 |
| 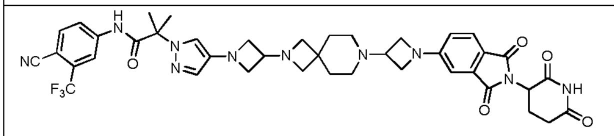 | 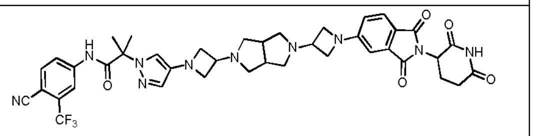 |
| 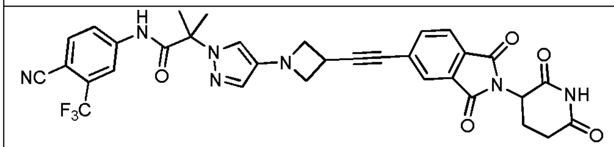 | 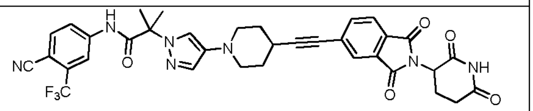 |
| 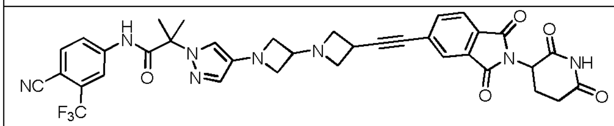 | 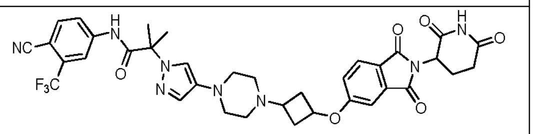 |
| 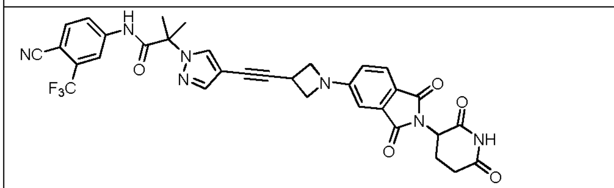 | 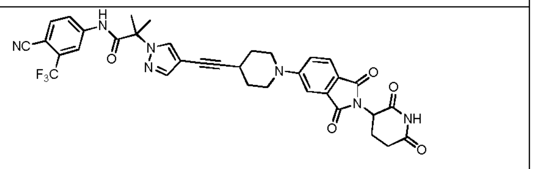 |
| 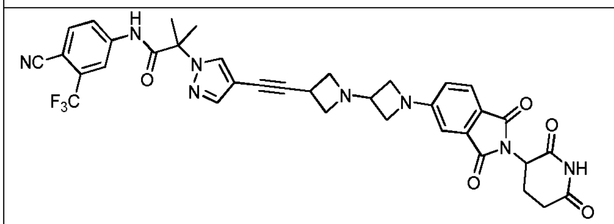 | 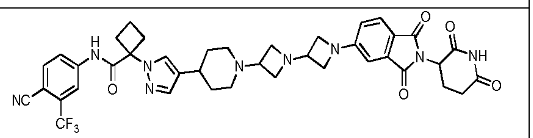 |
| 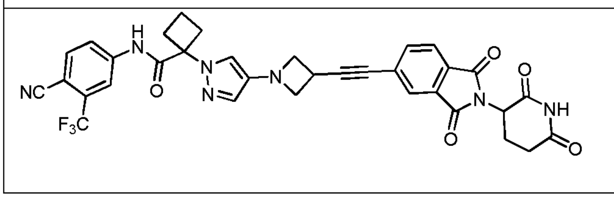 | 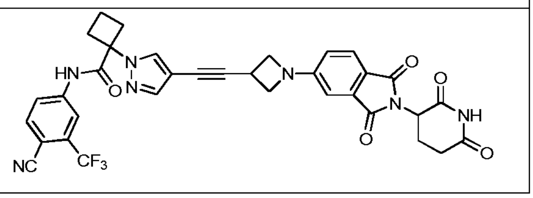 |

| | |
|---|---|
| 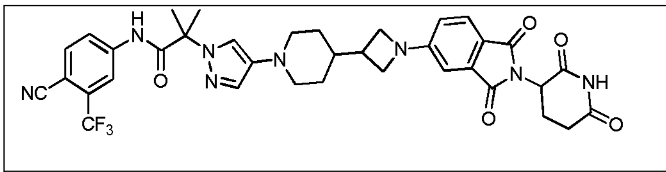 | 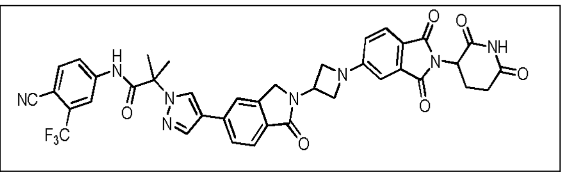 |
| 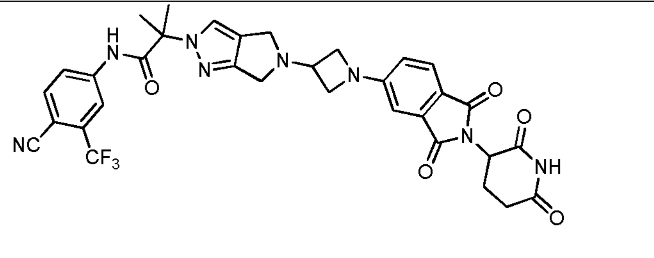 | 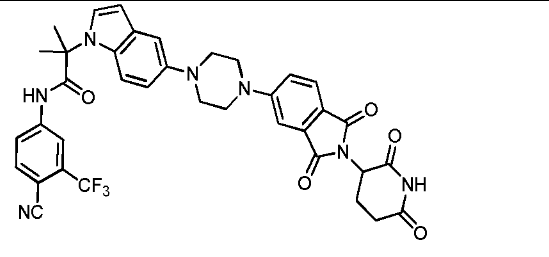 |
| 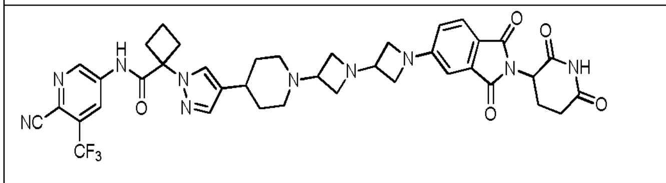 | 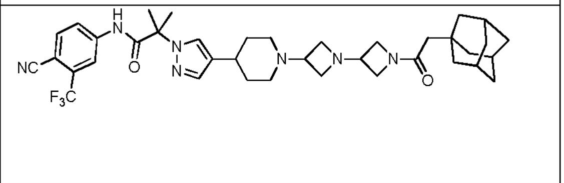 |
| 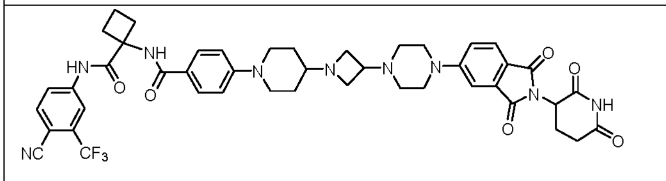 | 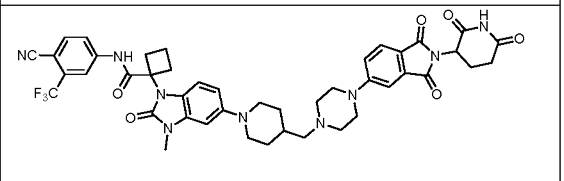 |
| 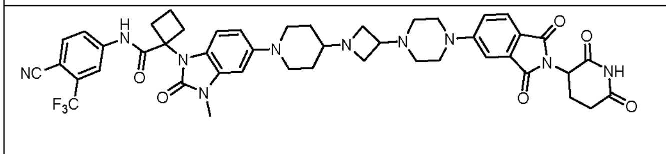 | 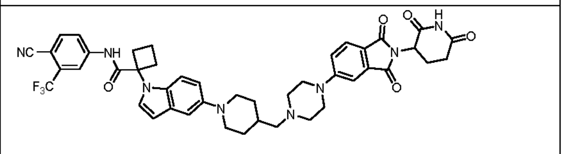 |
| 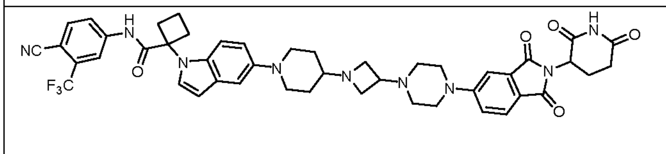 | 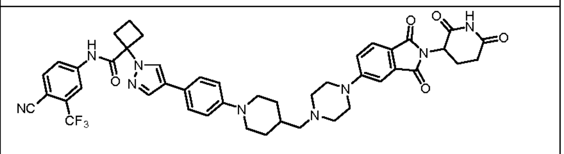 |
| 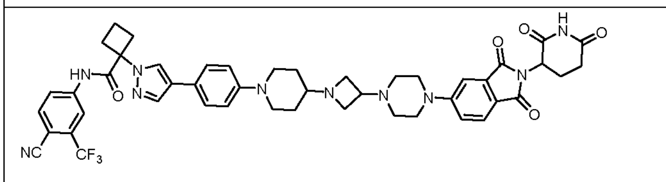 | 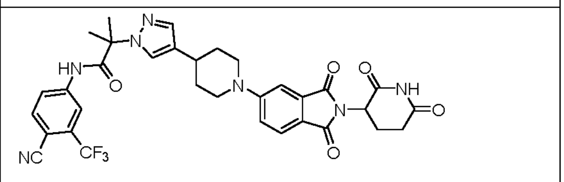 |
| 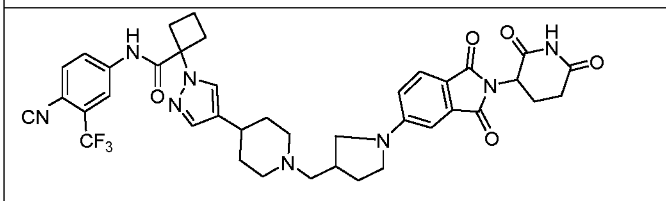 | 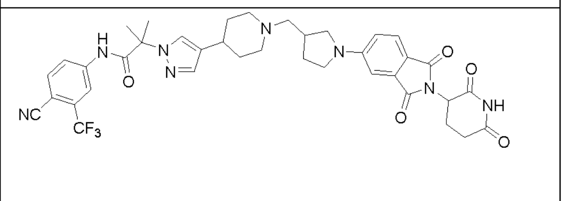 |
| 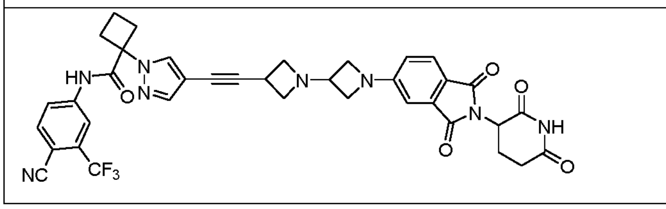 | 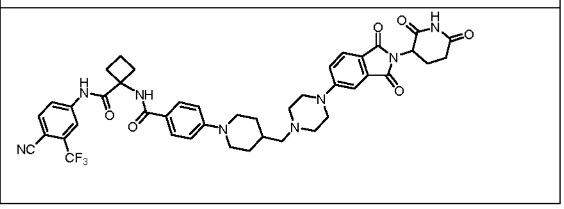 |

**9.** A pharmaceutical composition, comprising the compound or the stereoisomer, deuterated compound, solvate, prodrug, metabolite, pharmaceutically acceptable salt or co-crystal thereof according to any one of claims 1 to 8, and a pharmaceutically acceptable carrier.

**10.** Use of the compound or the stereoisomer, deuterated compound, solvate, prodrug, metabolite, pharmaceutically acceptable salt or co-crystal thereof according to any one of claims 1 to 8 in the preparation of a medicament for treating a disease related to AR activity or expression level.

**11.** Use of the compound or the stereoisomer, deuterated compound, solvate, prodrug, metabolite, pharmaceutically acceptable salt or co-crystal thereof according to any one of claims 1 to 8 in the preparation of a medicament for treating a disease related to the inhibition or degradation of AR.

**12.** The use according to claim 11, wherein the disease is selected from prostate cancer.

**INTERNATIONAL SEARCH REPORT**

International application No. **PCT/CN2021/105275**

**A. CLASSIFICATION OF SUBJECT MATTER**

C07D 401/14(2006.01)i; C07D 417/14(2006.01)i; C07D 471/04(2006.01)i; C07D 471/08(2006.01)i; C07D 471/10(2006.01)i; A61K 31/444(2006.01)i; A61K 31/496(2006.01)i; A61K 31/517(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, CNKI, EPODOC, WPI, STN: 四川海思科制药有限公司, 雄激素受体, E3泛素连接酶, androgen receptor, PROTAC, E3 ubiquitin ligase, structure search

**C. DOCUMENTS CONSIDERED TO BE RELEVANT**

| Category* | Citation of document, with indication, where appropriate, of the relevant passages | Relevant to claim No. |
|---|---|---|
| X | CN 111051298 A (NOVARTIS AG) 21 April 2020 (2020-04-21) see description paragraphs [0065],[0075],[0480] | 1-3,9-12 |
| X | ACS. "STN Registry" *RN 1280499-56-0,* 15 April 2011 (2011-04-15), see pp. 1-7 | 1-3 |
| X | ACS. "STN Registry" *RN 1297716-76-7,* 20 May 2011 (2011-05-20), see pp. 1-5 | 1-3 |
| Y | WO 9822432 A1 (YAMANOUCHI PHARMACEUTICAL CO., LTD. et al.) 28 May 1998 (1998-05-28) see abstract, claim 1, description tables 7-9,11,14 | 1-12 |
| Y | CN 110963957 A (SHENZHEN ENDUOKAI MEDICAL TECHNOLOGY CO., LTD.) 07 April 2020 (2020-04-07) see abstract, claims 1, 5 | 1-12 |
| Y | CN 108601764 A (ARVINAS, INC. et al.) 28 September 2018 (2018-09-28) see abstract, claims 1,16, table 3 | 1-12 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 September 2021** | **09 October 2021** |

Name and mailing address of the ISA/CN

**China National Intellectual Property Administration (ISA/CN)**
**No. 6, Xitucheng Road, Jimenqiao, Haidian District, Beijing 100088**
**China**

Facsimile No. **(86-10)62019451**

Authorized officer

Telephone No.

**INTERNATIONAL SEARCH REPORT**

International application No.
**PCT/CN2021/105275**

**C. DOCUMENTS CONSIDERED TO BE RELEVANT**

| Category* | Citation of document, with indication, where appropriate, of the relevant passages | Relevant to claim No. |
|---|---|---|
| Y | CN 110612294 A (ARVINAS OPERATIONS, INC.) 24 December 2019 (2019-12-24) see abstract, description paragraphs [0059]-[0060],[0298],[0704] | 1-12 |
| Y | WO 2019199816 A1 (ARVINAS OPERATIONS, INC.) 17 October 2019 (2019-10-17) see abstract, description paragraphs [0046],[0057]-[0132], table 2 | 1-12 |

Form PCT/ISA/210 (second sheet) (January 2015)

**INTERNATIONAL SEARCH REPORT**
**Information on patent family members**

International application No.
**PCT/CN2021/105275**

| Patent document cited in search report | Publication date (day/month/year) | Patent family member(s) | Publication date (day/month/year) |
|---|---|---|---|
| CN 111051298 A | 21 April 2020 | TW 201920143 A | 01 June 2019 |
| | | US 10640489 B2 | 05 May 2020 |
| | | US 10647701 B2 | 12 May 2020 |
| | | CA 3072694 A1 | 28 February 2019 |
| | | AR 112529 A1 | 06 November 2019 |
| | | US 2019367483 A1 | 05 December 2019 |
| | | JP 2020531498 A | 05 November 2020 |
| | | US 2020231569 A1 | 23 July 2020 |
| | | AU 2018319577 B2 | 15 October 2020 |
| | | IL 272748 D0 | 30 April 2020 |
| | | US 2019359594 A1 | 28 November 2019 |
| | | KR 20200044038 A | 28 April 2020 |
| | | EA 202090553 A1 | 08 June 2020 |
| | | SG 11202000490 P A | 30 March 2020 |
| | | PH 12020500125 A1 | 14 September 2020 |
| | | CL 2020000427 A1 | 28 August 2020 |
| | | UY 37854 A | 29 March 2019 |
| | | US 10414755 B2 | 17 September 2019 |
| | | EP 3672949 A1 | 01 July 2020 |
| | | BR 112020003373 A2 | 25 August 2020 |
| | | AU 2018319577 A1 | 06 February 2020 |
| | | US 2019062309 A1 | 28 February 2019 |
| | | EC SP20013248 A | 29 May 2020 |
| | | CR 20200081 A | 14 May 2020 |
| | | CU 20200014 A7 | 30 November 2020 |
| | | CO 2020001860 A2 | 29 May 2020 |
| | | WO 2019038717 A1 | 28 February 2019 |
| | | AU 2020277231 A1 | 24 December 2020 |
| | | JO P20200042 A1 | 23 February 2019 |
| WO 9822432 A1 | 28 May 1998 | AU 4966497 A | 10 June 1998 |
| CN 110963957 A | 07 April 2020 | WO 2020063407 A1 | 02 April 2020 |
| CN 108601764 A | 28 September 2018 | EP 3270917 A4 | 08 August 2018 |
| | | MX 2017011919 A | 22 May 2018 |
| | | AU 2016232705 A1 | 21 September 2017 |
| | | EP 3270917 A1 | 24 January 2018 |
| | | JP 2021073251 A | 13 May 2021 |
| | | BR 112017019751 A2 | 29 May 2018 |
| | | US 2016272639 A1 | 22 September 2016 |
| | | US 2020392131 A1 | 17 December 2020 |
| | | JP 2018512449 A | 17 May 2018 |
| | | KR 20180011759 A | 02 February 2018 |
| | | HK 1249058 A1 | 26 October 2018 |
| | | WO 2016149668 A1 | 22 September 2016 |
| | | RU 2017134730 A | 05 April 2019 |
| | | AU 2016232705 B2 | 22 October 2020 |
| | | CA 2979070 A1 | 22 September 2016 |
| | | US 10730870 B2 | 04 August 2020 |
| | | AU 2020273338 A1 | 17 December 2020 |
| | | RU 2017134730 A3 | 03 September 2019 |
| CN 110612294 A | 24 December 2019 | BR 112019015484 A2 | 28 April 2020 |

Form PCT/ISA/210 (patent family annex) (January 2015)

**INTERNATIONAL SEARCH REPORT**
**Information on patent family members**

International application No.
**PCT/CN2021/105275**

| Patent document cited in search report | Publication date (day/month/year) | Patent family member(s) | Publication date (day/month/year) |
|---|---|---|---|
| | | EP 3577109 A1 | 11 December 2019 |
| | | WO 2018144649 A8 | 22 August 2019 |
| | | IL 268069 D0 | 26 September 2019 |
| | | MX 2019009046 A | 30 October 2019 |
| | | KR 20190116315 A | 14 October 2019 |
| | | EP 3577109 A4 | 18 November 2020 |
| | | AU 2018215212 A1 | 11 July 2019 |
| | | RU 2019123462 A | 27 January 2021 |
| | | US 2018215731 A1 | 02 August 2018 |
| | | JP 2020506922 A | 05 March 2020 |
| | | CO 2019009424 A2 | 28 February 2020 |
| | | WO 2018144649 A1 | 09 August 2018 |
| | | RU 2019123462 A3 | 24 May 2021 |
| | | CA 3050309 A1 | 09 August 2018 |
| WO 2019199816 A1 | 17 October 2019 | IL 277934 D0 | 30 November 2020 |
| | | EP 3774772 A1 | 17 February 2021 |
| | | CA 3095912 A1 | 17 October 2019 |
| | | KR 20210003804 A | 12 January 2021 |
| | | CN 112262134 A | 22 January 2021 |
| | | AU 2019251223 A1 | 26 November 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017197056 A
- WO 2020063407 A


### Non-patent literature cited in the description


- Synthetic Organic Chemistry. John Wiley & Sons, Inc,
- **S. R. SANDLER et al.** Organic Functional Group Preparations. Academic Press, 1983
- **H. O. HOUSE.** Modern Synthetic Reactions. W. A. Benjamin, Inc, 1972
- **T. L. GILCHRIST.** Heterocyclic Chemistry. John Wiley & Sons, 1992
- **J. MARCH.** Advanced Organic Chemistry: Reactions, Mechanisms and Structure. Wiley-Interscience, 1992
- **FUHRHOP, J. ; PENZLIN G.** Organic Synthesis: Concepts, Methods, Starting Materials. John Wiley & Sons, 1994
- **HOFFMAN, R.V.** Organic Chemistry, An Intermediate Text. Oxford University Press, 1996
- **LAROCK, R. C.** Comprehensive Organic Transformations: A Guide to Functional Group Preparations. Wiley-VCH, 1999
- **MARCH, J.** Advanced Organic Chemistry: Reactions, Mechanisms, and Structure. John Wiley & Sons
- Modern Carbonyl Chemistry. Wiley-VCH, 2000
- **PATAI, S.** Patai's 1992 Guide to the Chemistry of Functional Groups. Interscience, 1992
- **SOLOMONS, T. W. G.** Organic Chemistry. John Wiley & Sons, 2000
- **STOWELL, J.C.** Intermediate Organic Chemistry. Wiley-Interscience, 1993
- Industrial Organic Chemicals: Starting Materials and Intermediates: An Ullmann's Encyclopedia. John Wiley & Sons, 1999
- Organic Reactions. John Wiley & Sons
- Chemistry of Functional Groups. John Wiley & Sons
- **P. H. STAHL ; C. G. WERMUTH.** Handbook of Pharmaceutical Salts. Verlag Helvetica Chimica Acta, 2002